# EUROPEAN PATENT SPECIFICATION

(11) **EP 3 268 014 B1**
(45) Date of publication and mention of the grant of the patent: **21.09.2022**
(21) Application number: 16721219.0
(22) Date of filing: 09.03.2016
(51) Int. Cl.: A61K 35/17, C07K 14/725, A61P 35/00, C12N 15/85, C07K 16/30

(54) **T-CELL RECEPTORS DIRECTED AGAINST THE PREFERENTIALLY EXPRESSED ANTIGEN OF MELANOMA AND USES THEREOF**
GEGEN DAS BEVORZUGT EXPRIMIERTE ANTIGEN VON MELANOMEN GERICHTETE T-ZELL-REZEPTOREN UND VERWENDUNGEN DAVON
RÉCEPTEURS DE LYMPHOCYTES T DIRIGÉS CONTRE L'ANTIGÈNE EXPRIMÉ DE PRÉFÉRENCE DANS LE MÉLANOME, ET LEURS UTILISATIONS

(30) Priority: 10.03.2015 US 201562130884 P
(43) Date of publication of application: 17.01.2018
(60) Divisional application: 22190314.9
(73) Proprietor: Academisch Ziekenhuis Leiden H.O.D.N. Leids Universitair Medisch Centrum, 2333 ZA Leiden (NL)
(72) Inventor: FALKENBURG, J.h., Frederik, 2300 RC Leiden (NL); HEEMSKERK, Mirjam, H.m., 2300 RC Leiden (NL)
(74) Representative: HGF
(86) International application number: PCT/IB2016/000399
(87) International publication number: WO 2016/142783

(56) References cited:
- WO-A2-2011/062634
- WO-A2-2013/040371
- WO-A2-2014/197638
- AMIR AVITAL L ET AL: "PRAME-specific Allo-HLA-restricted T cells with potent antitumor reactivity useful for therapeutic T-cell receptor gene transfer.", CLINICAL CANCER RESEARCH : AN OFFICIAL JOURNAL OF THE AMERICAN ASSOCIATION FOR CANCER RESEARCH 1 SEP 2011, vol. 17, no. 17, 1 September 2011 (2011-09-01), pages 5615-5625, XP002760143, ISSN: 1078-0432
- VAN LOENEN M M ET AL: "Multi-cistronic vector encoding optimized safety switch for adoptive therapy with T-cell receptor-modified T cells.", GENE THERAPY AUG 2013, vol. 20, no. 8, August 2013 (2013-08), pages 861-867, XP002760144, ISSN: 1476-5462
- DOSSETT MICHELLE L ET AL: "Adoptive immunotherapy of disseminated leukemia with TCR-transduced, CD8+ T cells expressing a known endogenous TCR.", MOLECULAR THERAPY : THE JOURNAL OF THE AMERICAN SOCIETY OF GENE THERAPY APR 2009, vol. 17, no. 4, April 2009 (2009-04), pages 742-749, XP002760145, ISSN: 1525-0024

## Description

### Field

The technology relates in part to compositions and methods for inducing an immune response against the Preferentially Expressed Antigen of Melanoma (PRAME). Provided are methods for treating hyperproliferative diseases by inducing an immune response against PRAME antigen; the immune response may be induced by specifically targeting PRAME-expressing cells using T cell receptors directed against PRAME.

### Background

T cell activation is an important step in the protective immunity against pathogenic microorganisms (e.g., viruses, bacteria, and parasites), foreign proteins, and harmful chemicals in the environment, and also as immunity against cancer and other hyperproliferative diseases. T cells express receptors on their surfaces (i.e., T cell receptors) that recognize antigens presented on the surface of cells. During a normal immune response, binding of these antigens to the T cell receptor, in the context of MHC antigen presentation, initiates intracellular changes leading to T cell activation.

Adoptive T cell therapy has been used to treat hyperproliferative diseases, including tumors, by providing an antigen-specific immune response. One method involves the use of genetically modified T cells that express an antigen-specific protein having an extracellular domain that binds to an antigen.

Amir et al discloses PRAME-specific Allo-HLA-restricted T cells with potent antitumor reactivity useful for therapeutic T-cell receptor gene transfer (Amir A.L et al, Clinical Cancer Research: an official journal of the American Association for Cancer Research, 1 Sep 2011, (2011-09-01), vol. 17, no. 17, pages 5615 - 5625).

Van Loenen et al discloses multi-cistronic vector encoding optimized safety switch for adoptive therapy with T-cell receptor-modified T cells (Van Loenen M.M et al, Gene Therapy, Aug 2013, (2013-08), vol. 20, no. 8, pages 861 - 867).

### Summary

The PRAME gene is expressed at a high level in a large proportion of tumors, including melanomas, non-small-cell lung carcinomas, renal cell carcinoma (RCC), breast carcinoma, cervix carcinoma, colon carcinoma, sarcoma, neuroblastoma, as well as several types of leukemia. PRAME-specific T cell clones were identified that recognize these different tumor types, including Ewing sarcoma, synovial sarcoma, and neuroblastoma cell lines. TCR gene transfer approaches using PRAME-specific TCRs can bring novel treatment modalities for patients with hyperproliferative diseases such as, for example, sarcomas, acute lymphoblastic leukemia, acute myeloid leukemia, uveal melanomas, and neuroblastomas.

Provided herein are compositions and methods comprising T cell receptors, nucleic acids coding for T cell receptors, and cells expressing T cell receptors that recognize PRAME. The cells may also express an inducible caspase-9 polypeptide.

In one aspect, the invention provides a nucleic acid molecule comprising a CDR3-encoding polynucleotide, wherein the CDR3-encoding polynucleotide encodes the CDR3 region of a T cell receptor that specifically binds to the preferentially expressed antigen in melanoma (PRAME), wherein: a) the CDR3-encoding polynucleotide comprises a first polynucleotide that encodes a first polypeptide comprising the VJ regions of a TCRα polypeptide, wherein the first polypeptide comprises the CDR3 region of the TCRα polypeptide, and wherein the CDR3 region comprises the amino acid sequence of SEQ ID NO: 25; and b) the CDR3-encoding polynucleotide comprises a second polynucleotide that encodes a second polypeptide comprising the VDJ regions of a TCRβ polypeptide, wherein the second polypeptide comprises the CDR3 region of the TCRβ polypeptide, and wherein the CDR3 region comprises the amino acid sequence of SEQ ID NO: 28; wherein the CDR3 region of the TCRα polypeptide and the CDR3 region of the TCRβ polypeptide together specifically bind to PRAME.

Suitably, the first polypeptide may comprise the amino acid sequence of SEQ ID NO: 31 and the second polypeptide may comprise the amino acid sequence of SEQ ID NO: 34.

Suitably, the nucleic acid molecule may encode a T cell receptor, wherein the CDR3 regions of the T cell receptor specifically binds to a PRAME polypeptide comprising the amino acid sequence SLLQHLIGL.

Suitably, the first polynucleotide may comprise the nucleotide sequence of SEQ ID NO:32 or SEQ ID NO:33; and the second polynucleotide may comprise the nucleotide sequence of SEQ ID NO:35 or SEQ ID NO: 36.

Suitably, the CDR3 region of the T cell receptor may specifically bind to a peptide-MHC complex, wherein the MHC molecule is a MHC Class I HLA molecule and the peptide is a PRAME epitope.

Suitably, the nucleic acid molecule may further comprise a promoter operatively linked to the CDR3-encoding polynucleotide.

Suitably, the nucleic acid molecule may further comprise a polynucleotide encoding a chimeric Caspase-9 polypeptide comprising a multimeric ligand binding region and a Caspase-9 polypeptide.

In another aspect, a plasmid vector or viral vector is provided comprising a nucleic acid molecule of the invention.

In another aspect, a modified cell transfected or transduced with a nucleic acid molecule of the invention is provided.

In another aspect, a modified cell transfected or transduced with a nucleic acid molecule encoding a T cell receptor that specifically binds to the preferentially expressed antigen in melanoma (PRAME), wherein the nucleic acid molecule comprises a first polynucleotide encoding a first polypeptide comprising the VJ regions of a TCRα polypeptide, wherein the first polypeptide comprises the amino acid sequence of SEQ ID NO: 25; and a second polynucleotide encodes a second polypeptide comprising the VDJ regions of a TCRβ polypeptide, wherein the second polypeptide comprises the amino acid sequence of SEQ ID NO: 28 is provided.

Suitably, the first polypeptide may comprise the amino acid sequence of SEQ ID NO: 31 and the second polypeptide may comprise the amino acid sequence of SEQ ID NO: 34.

In another aspect, a pharmaceutical composition comprising a nucleic acid molecule of the invention, a vector of the invention, or a modified cell of the invention and a pharmaceutically acceptable carrier is provided.

Suitably, the pharmaceutical composition of the invention may be for use in enhancing an immune response in a subject diagnosed with a hyperproliferative disease or condition, wherein a therapeutically effective amount of the pharmaceutical composition is to be administered to the subject.

Suitably, the pharmaceutical composition of the invention may be for use in stimulating a cell mediated immune response to a target cell population or tissue in a subject, wherein a therapeutically effective amount of the pharmaceutical composition is to be administered to the subject.

In another aspect, an in vitro method of expressing a T cell receptor that specifically binds to PRAME in a cell is provided, comprising contacting a nucleic acid molecule of the invention with a cell under conditions in which the nucleic acid is incorporated into the cell, whereby the cell expresses the T cell receptor from the incorporated nucleic acid.

Certain embodiments are described further in the following description, examples, claims and drawings.

The invention is defined by the appended claims. Any aspects or embodiments described herein that are not encompassed by the claims are included as aspects or embodiments of the disclosure only. Any aspects or embodiments described herein relating to methods of treatment are included as aspects or embodiments of the disclosure only.

### Brief Description of the Drawings

The drawings illustrate certain embodiments of the technology and are not limiting. For clarity and ease of illustration, the drawings are not made to scale and, in some instances, various aspects may be shown exaggerated or enlarged to facilitate an understanding of particular embodiments.
Figure 1A provides a schematic of components of adoptive T cell therapy; Figure 1B provides a bar graph of antigen expression in various tissues.

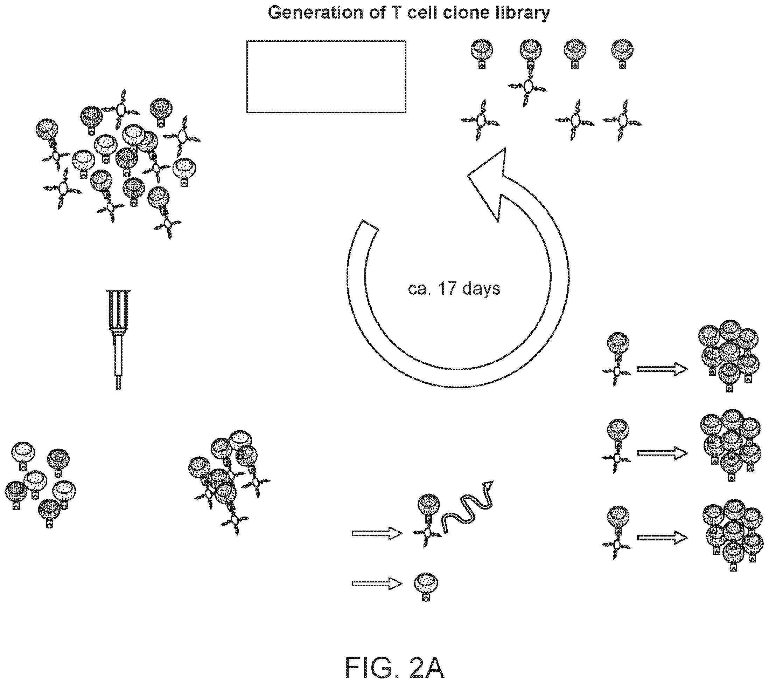

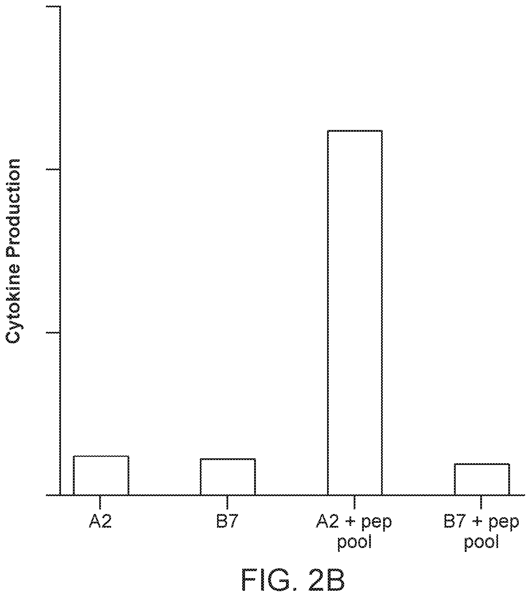

Figure 2A provides a schematic illustrating an example of a method used to generate a T cell clone library; Figure 2B provides a bar graph of cytokine production.
Figure 3 provides the results of a FACs analysis showing the isolation of activated CD8⁺ T cells after HLA mismatched stem cell therapy. The patient recipient was HLA-A2⁺ and the donor was HLA-A2-.
Figures 4A-4C provide bar graphs measuring tumor specificity of three different T cell clones. Figure 4A provides a bar graph for clone 12; Figure 4B provides a bar graph for clone 35; Figure 4C provides a bar graph for clone 54.
Figures 5A-5C provide graphs IFNγ showing that T cell clone is PRAME-specific. Figure 5A provides a graph of 1st dim: water/ACN/TFA HPLC fractions; Figure 5B provides a graph of 2nd dim: water/IPA/TFA HPLC fractions; Figure 5C provides a graph of 3rd dim: water/ANC/Formic acid HPLC fractions.
Figures 6A-6C provide results of experiments comparing the avidity of allo-restricted and non-allo-restricted PRAME-specific T cells. Figure 6A provides FACs results; Figure 6B provides a line graph; Figure 6C provides a bar graph.
Figures 7A and 7B are bar graphs showing the high specificity of PRAME-specific allo-HLA restricted T cells. Figure 7A provides a bar graph representing melanoma cell lines; Figure 7B provides a bar graph representing primary AML samples.
Figure 8 is a bar graph showing limited reactivity of the PRAME-specific T cell clone against matured dendritic cells (DCs) derived from healthy CD34⁺ cells.
Figure 9 is a scatter plot showing that recognition of cells by the PRAME-specific T cell clone correlates with the targeted cell's level of PRAME expression.
Figures 10A-10C are bar graphs showing that Silencing PRAME expression by shRNA correlated with reduced reactivity of a PRAME-specific T cell clone. Figure 10A provides a bar graph representing renal cell carcinoma RCC 1257; Figure 10B provides a bar graph representing mature dendritic cells derived from CD34⁺ cells (CD34 mDC); Figure 10C provides a bar graph representing proximal tubular epithelial cells (PTEC).
Figure 11 is a bar graph showing the high tumor reactivity of PRAME-TCR transduced T cells.
Figure 12 is a bar graph showing the reactivity of T cells transduced with PRAME TCR constructs, with or without a caspase-9 encoding polypeptide, against target cells with or without AP1903 treatment.
Figure 13 provides bar graphs showing the reactivity of PRAME specific T cell clones against Ewing sarcoma cells with or without treatment for 48 h with IFN-γ/IFN-α. HLA expression with or without IFN-γ/IFN-α of the Ewing sarcoma cell lines is shown on the right part of the Figure. Figure 13 is provided herein in 6 pages of drawings.
Figure 14.provides bar graphs showing the reactivity of PRAME specific T cell clones against neuroblastoma cells with or without treatment for 48 h with IFN-γ/IFN-α. HLA expression with or without IFN-γ/IFN-α of the neuroblastoma cell lines is shown on the right part of the Figure. Figure 14 is provided herein in 4 pages of drawings.
Figures 15A and 15B are bar graphs showing the reactivity of T cells transduced with PRAME TCR and PRAME TCR + icasp9 constructs against melanoma cells. 15A) without AP1903 treatment, 15B) with AP1903 treatment.
Figures 16A-16D are bar graphs showing the recognition of Ewing sarcoma cells by PRAME-specific T cell clones. Ewing sarcoma cell lines were treated with or without IFN-γ/IFN-α for 48h. Figure 16A provides a bar graph of EW3 cell lines; Figure 16B provides a bar graph of EW3 cell line HLA expression with or without IFN-γ/IFN-α; Figure 16C provides a bar graph of EW7 cell lines; Figure 16D provides a bar graph of EW3 cell line with or without IFN-γ/IFN-α.
Figure 17A provides the amino acid sequence of PRAME clone 54SLL. (TRAV8-4*04) in a PRAME/icasp9 construct; Figure 17B provides the amino acid sequence of PRAME clone 54SLL (TRBV9*01) in a PRAME/icasp9 construct.
Figure 18A provides the amino acid sequence of PRAME clone 46SLL (TRAV35*02), Figure 18B provides the amino acid sequence f PRAME clone 46SLL (TRBV28*01).
Figure 19A provides the amino acid sequence of PRAME clone DSK3 QLL (TRAV12-2*01), Figure 19B provides the amino acid sequence of PRAME clone DSK3 QII (TRBV9*01).
Figure 20A provides a bar chart of PRAME expression in samples of primary AML cells. Figure 20B provides a bar chart of reactivity of PRAME-specific T cells against samples of primary AML cells.

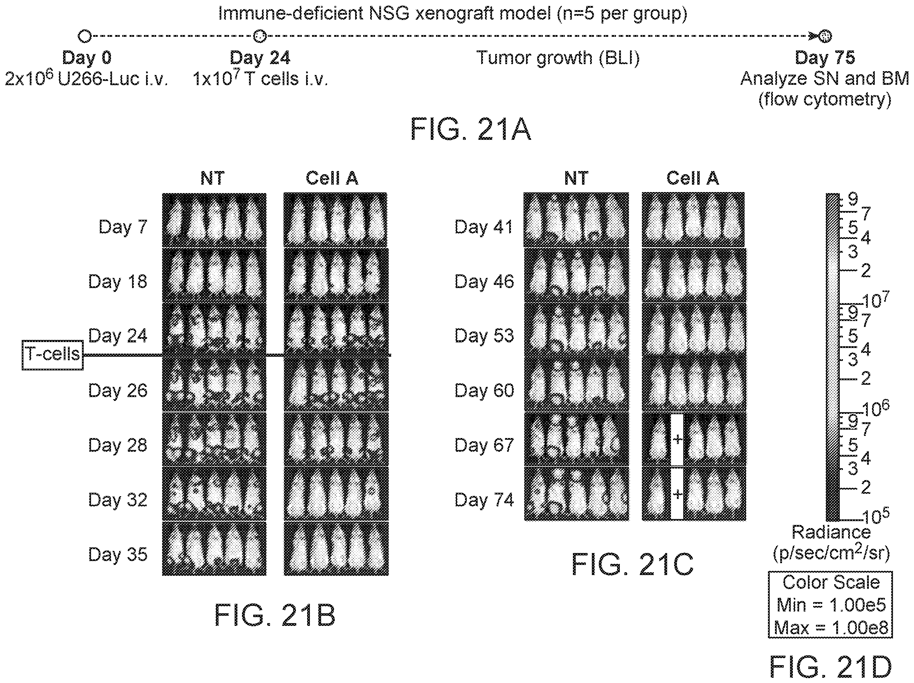

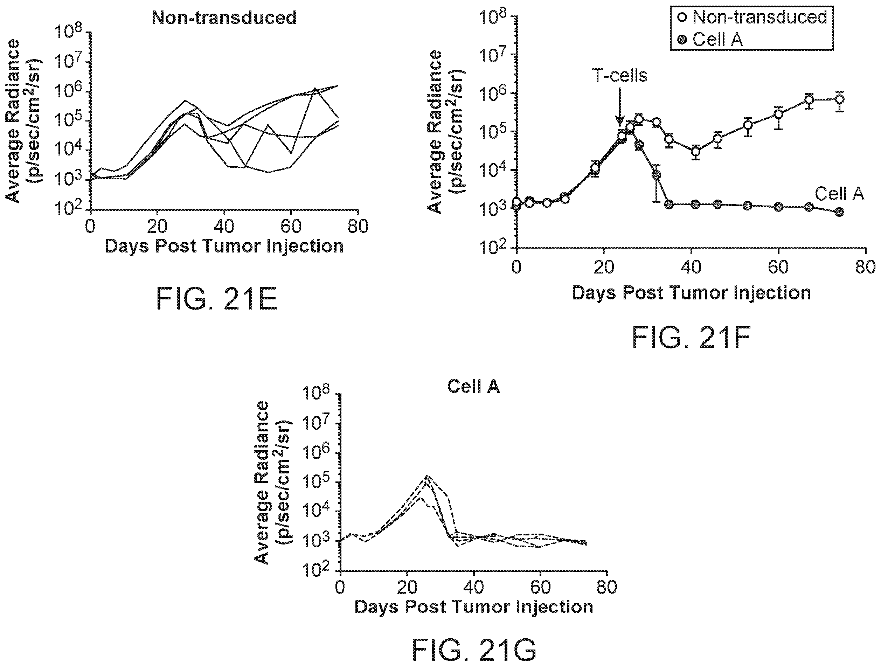

Figure 21A provides a timeline of an assay of tumor response in mice following treatment with PRAME TCR-expressing cells in an NSG immune deficient mouse xenograft model; Figure 21B provides photos of mice treated with non-transduced (NT) and PRAME TCR-expressing cells ((Cell A) at days 7-35; Figure 21C provides photos of mice treated with non-transduced (NT) and PRAME TCR-expressing cells at days 41-74; Figure 21D provides a fluorescence color scale; Figure 21E provides a line graph of average radiance of tumors in mice treated with the non-transduced cells; Figure 21F provides a line graph of average radiance of tumors in mice treated with the control (NT) cells, and mice treated with the PRAME TCR-expressing cells; Figure 21G provides a line graph of average radiance of tumors in mice treated with the PRAME TCR-expressing cells.
Figure 22A provides a FACs analysis of spleen from mice treated with the PRAME-TCR expressing T cells following administration of rimiducid; Figure 22B provdes a graph summarizing the FACs analysis of Figure 22A.

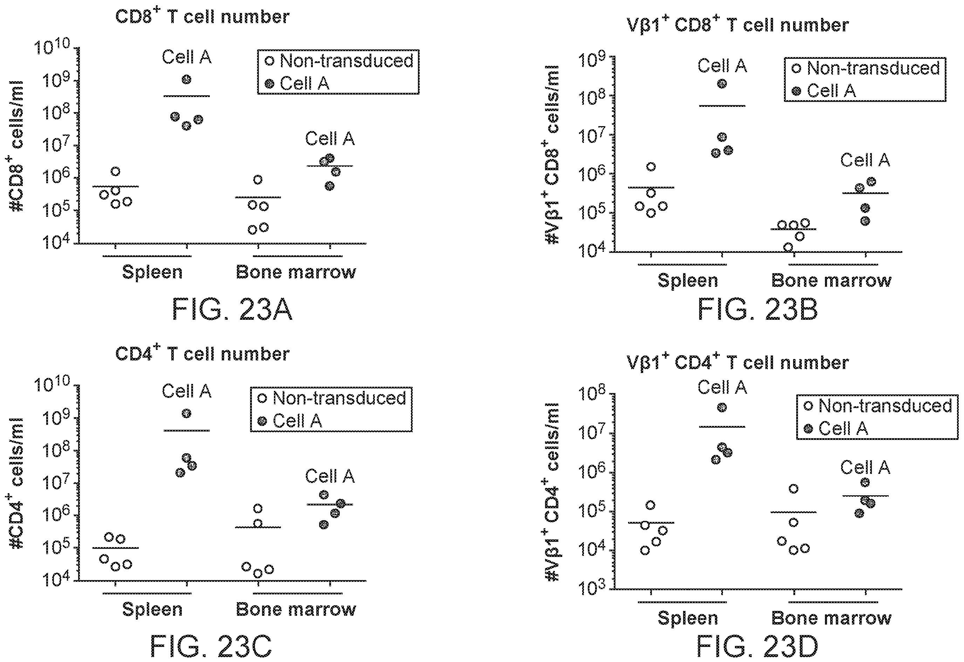

Figure 23A provides flow cytometry results of spleen and bone marrow cells were harvested from mice treated either with NT- or Cell A on day 51 after T cell injection (day 74 post-tumor implantation), counted, and analyzed for CD8+ expression; Figure 23B provides flow cytometry analysis of the cells counted and analyzed for CD4+ expression; Figure 23C provides flow cytometry analysis of the cells counted and analyzed for Vβ1 expression on CD8+ T cells; Figure 23D provides flow cytometry analysis of the cells counted and analyzed for Vβ1 expression on CD4+ T cells .
Figure 24 provides flow cytometry results of spleen and bone marrow cells isolated from Cell A-treated animals as analyzed above, and cultured overnight with or without 10 nM rimiducid.
Figure 25 provides a plasmid map of SFG.iC9-2A-SLL.TCR.

### Detailed Description

Recombinant T cell receptors, specific for a particular antigen, have been used to provide specificity to T cells, and to provide an antigen-specific immune response in patients. Certain methods involve the use of genetically modified T cells that express an antigen-specific protein having an extracellular domain that binds to an antigen.

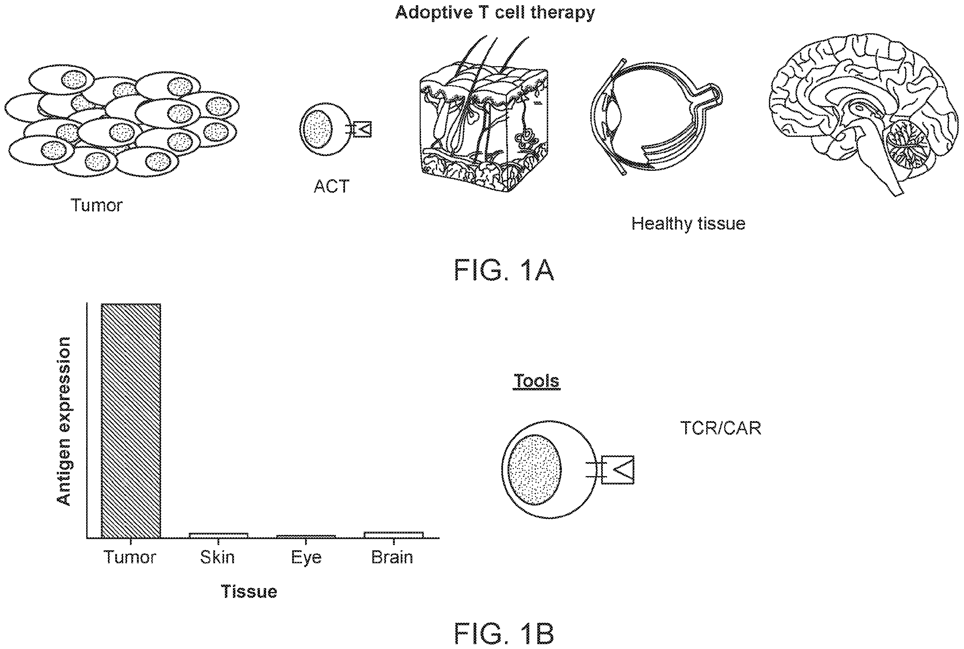

Adoptive T cell therapy, using genetically modified T cells that express a heterologous T cell receptor (TCR) can provide high avidity target cell-specific TCRs as part of the patient's T cell repertoire. Adoptive T cell therapy has been used to treat hyperproliferative diseases, including tumors, by providing an antigen-specific immune response. T cells are genetically modified to generate T cells with a defined specificity, such as, for example, specificity for tumor cells. Methods of adoptive T cell therapy are provided as a schematic in Figure 1. As discussed herein, T cells isolated from an allo-HLA repertoire may provide a higher avidity to the target, which is desirable to effectively eradicate tumors. Allo-HLA restricted T cells are compared to Self-restricted T cells in the following Table 1:

**Table 1**

| **Self-restricted T cells** | **Allo-HLA restricted T cells** |
|---|---|
| Tumor associated antigens / tissue specific antigens are self-peptides | No tolerance for self-peptides presented in the context of allo-HLA |
| Thymic selection: induction of tolerance for self-peptides in the context of self-HLA | In vivo derived allo-HLA reactive T cells are peptide specific Amir et al, Blood, 2011 |
| Only low avidity T cells left: no or low reactivity against tumors | Self-peptide specific allo-HLA reactive T cells exhibit high avidity |

Thus, provided in some examples are nucleic acid molecules comprising a CDR3-encoding polynucleotide, wherein: the CDR3-encoding polynucleotide encodes the CDR3 region of a T cell receptor that specifically binds to the preferentially expressed antigen in melanoma (PRAME); the CDR3-encoding polynucleotide comprises a first polynucleotide that encodes a first polypeptide comprising the CDR3 region of a TCRα polypeptide; the CDR3-encoding polynucleotide comprises a second polynucleotide that encodes a second polypeptide comprising the CDR3 region of a TCRβ polypeptide; and the CDR3 region of the TCRα polypeptide and TCR β polypeptide together specifically bind to PRAME. In some embodiments, the nucleic acid molecule encodes a T cell receptor. In some embodiments, the nucleotide sequences that encode the first or second, or first and second polypeptides are codon optimized; in some embodiments the first or second polypeptides. In some embodiments, the amino acid sequences of the first and second polypeptides, or first or second polypeptides are cysteine modified to increase expression of the recombinant TCR. By cysteine modified is meant that the nucleotide sequences encode polypeptides that comprise an additional cysteine residue. By codon optimized is meant that the nucleotide sequence includes codons that enhance expression of the encoded polypeptide. Examples of codon optimized nucleotide sequences are presented herein, however, it is understood that other nucleotide sequences may be used that also code for the CDR3 regions provided herein, and that the term codon optimized includes such nucleotide sequences that encode the CDR3 polypeptides, including the CDR3 regions of the TCR alpha and beta chains herein, or derivatives thereof that are 90% of more identical to the polypeptide sequences provided herein. In calculating the identity of an amino acid sequence and an amino acid sequence derivative, the cysteine used to enhance expression of the recombinant TCR alpha or beta polypeptide, or CDR3 region or other fragment thereof, is not considered as part of the percentage calculation where the sequence provided herein as a SEQ ID NO: does not have the cysteine modification.

In some embodiments, the CDR3 region of the T cell receptor specifically binds to a PRAME polypeptide comprising the amino acid sequence SLLQHLIGL.. In some examples, the first polypeptide comprises the amino acid sequence of SEQ ID NO: 1, and the second polypeptide comprises the amino acid sequence of SEQ ID NO: 4; or the first polypeptide comprises the amino acid sequence of SEQ ID NO: 23, and the second polypeptide comprises the amino acid sequence of SEQ ID NO: 26, or derivatives thereof having an amino acid sequence 90% or more identical to the sequence of SEQ ID NO: 1, SEQ ID NO: 4, SEQ ID NO: 23, or SEQ ID NO: 26. In some examples, the first polynucleotide comprises the nucleotide sequence of SEQ ID NO: 2 or SEQ ID NO: 3, or a derivative thereof, and the second polynucleotide comprises the nucleotide sequence of SEQ ID NO: 5 or SEQ ID NO: 6, or a derivative thereof; or the first polynucleotide comprises the nucleotide sequence of SEQ ID NO: 24 or SEQ ID NO: 25, or a derivative thereof having consecutive nucleotides 90% or more identical to the nucleotide sequence of SEQ ID NO: 24 or SEQ ID NO: 25, and the second polynucleotide comprises the nucleotide sequence of SEQ ID NO: 27 or SEQ ID NO: 28, or a derivative thereof having consecutive nucleotides 90% or more identical to the nucleotide sequence of SEQ ID NO: 27 or SEQ ID NO: 28.

In some examples, the CDR3 region of the T cell receptor specifically binds to a PRAME polypeptide comprising the amino acid sequence QLLALLPSL. In some embodiments, the first polypeptide comprises the amino acid sequence of SEQ ID NO: 45 and the second polypeptide comprises the amino acid sequence of SEQ ID NO: 48, having an amino acid sequence 90% or more identical to the sequence of SEQ ID NO: 45 or SEQ ID NO: 48. In some embodiments, the first polynucleotide comprises the nucleotide sequence of SEQ ID NO: 46 or SEQ ID NO: 47, or a derivative thereof having consecutive nucleotides 90% or more identical to the nucleotide sequence of SEQ ID NO: 46 or SEQ ID NO: 47, and the second polynucleotide comprises the nucleotide sequence of SEQ ID NO: 49 or SEQ ID NO: 50, or a derivative thereof having consecutive nucleotides 90% or more identical to the nucleotide sequence of SEQ ID NO: 49 or SEQ ID NO: 50.

In some embodiments, the CDR3 region of the T cell receptor binds to human PRAME. In some embodiments, the CDR3 region of the T cell receptor binds to PRAME in the context of MHC Class I HLA presentation. In some embodiments, the CDR3 region of the T cell receptor specifically binds to a peptide-MHC complex, wherein the MHC molecule is a MHC Class I HLA molecule and the peptide is a PRAME epitope. In some embodiments, the MHC molecule is a MHC Class 1 HLA A2.01 molecule. In some embodiments, the PRAME epitope is SLLQHLIGL.

In some embodiments, the nucleic acid further comprises a promoter operatively linked to the CDR3-encoding polynucleotide. In some embodiments, the nucleic acid molecule further comprises a polynucleotide encoding a chimeric Caspase-9 polypeptide comprising a multimeric ligand binding region and a Caspase-9 polypeptide. In some embodiments, modified cells that are transfected or transduced with the nucleic acids of the present application that encode the CDR3 region and the chimeric Caspase-9 polypeptide, are provided. In some embodiments, the modified cell is a T cell.

Also provided in some embodiments are plasmid or viral vectors that comprise nucleic acid molecules of the present application. In some embodiments, modified cells are provided that are transfected or transduced with a nucleic acid molecule of the present application. In some embodiments, the cell further comprises a nucleic acid molecule comprising a polynucleotide encoding a chimeric Caspase-9 polypeptide comprising a multimeric ligand binding region and a Caspase-9 polypeptide. In some embodiments, the modified cell is a T cell.

Also provided in some embodiments are pharmaceutical compositions that comprise a modified cell of the present application, a nucleic acid of the present application, or a plasmid or viral vector of the present application, and a pharmaceutically acceptable carrier.

Provided in some examples are methods of enhancing an immune response in a subject diagnosed with a hyperproliferative disease or condition, comprising administering a therapeutically effective amount of a modified cell of the present application to the subject. In some embodiments, the subject has at least one tumor and wherein the size of at least one tumor is reduced following administration of the modified cell. In some embodiments, the subject has been diagnosed with a disease selected from the group consisting of diagnosed with a condition or disease selected from the group consisting of sarcoma, acute lymphoblastic leukemia, acute myeloid leukemia, and neuroblastoma, melanoma, leukemia, lung cancer, colon cancer renal cell cancer, breast cancer, sarcoma, acute lymphoblastic leukemia, acute myeloid leukemia, and neuroblastoma.

Provided in some examples, are methods for stimulating a cell mediated immune response to a target cell population or tissue in a subject, comprising administering a modified cell of the present application to the subject. In some embodiments, the number or concentration of target cells in the subject is reduced following administration of the modified cell. Where, in some examples, the modified cell comprises a nucleic acid coding for a chimeric Caspase-9 polypeptide, the method further comprises administering a multimeric ligand that binds to the multimeric ligand binding region to the subject following administration of the modified cells to the subject. In some embodiments, administration of the multimeric ligand, the number or concentration of modified cells comprising the chimeric Caspase-9 polypeptide is reduced in a sample obtained from the subject after administering the multimeric ligand compared to the number or concentration of modified cells comprising the chimeric Caspase-9 polypeptide in a sample obtained from the subject before administering the multimeric ligand.

Also provided in some embodiments are in vitro methods for expressing a T cell receptor that specifically binds to PRAME in a cell, comprising contacting a nucleic acid molecule of the present application with a cell under conditions in which the nucleic acid is incorporated into the cell, whereby the cell expresses the T cell receptor from the incorporated nucleic acid.

As used herein, the use of the word "a" or "an" when used in conjunction with the term "comprising" in the claims and/or the specification may mean "one," but it is also consistent with the meaning of "one or more," "at least one," and "one or more than one." Still further, the terms "having", "including", "containing" and "comprising" are interchangeable and one of skill in the art is cognizant that these terms are open ended terms.

The term "allogeneic" as used herein, refers to HLA or MHC loci that are antigenically distinct between the host and donor cells.

Thus, cells or tissue transferred from the same species can be antigenically distinct. Syngeneic mice can differ at one or more loci (congenics) and allogeneic mice can have the same background.

The term "autologous" as used herein, refers to HLA or MHC loci that are not antigenically distinct between the host and donor cells, for example, where the donor cells are obtained from the host.

The term "antigen" as used herein is defined as a molecule that provokes an immune response. This immune response may involve either antibody production, or the activation of specific immunologically competent cells, or both. An antigen can be derived from organisms, subunits of proteins/antigens, killed or inactivated whole cells or lysates. Exemplary organisms include but are not limited to, Helicobacters, Campylobacters, Clostridia, Corynebacterium diphtheriae, Bordetella pertussis, influenza virus, parainfluenza viruses, respiratory syncytial virus, Borrelia burgdorfei, Plasmodium, herpes simplex viruses, human immunodeficiency virus, papillomavirus, Vibrio cholera, E. coli, measles virus, rotavirus, shigella, Salmonella typhi, Neisseria gonorrhea. Therefore, any macromolecules, including virtually all proteins or peptides, can serve as antigens. Furthermore, antigens can be derived from recombinant or genomic DNA. Any DNA that contains nucleotide sequences or partial nucleotide sequences of a pathogenic genome or a gene or a fragment of a gene for a protein that elicits an immune response results in synthesis of an antigen. Furthermore, the present methods are not limited to the use of the entire nucleic acid sequence of a gene or genome. The present compositions and methods include, but are not limited to, the use of partial nucleic acid sequences of more than one gene or genome and that these nucleic acid sequences are arranged in various combinations to elicit the desired immune response.

The term "antigen-presenting cell" is any of a variety of cells capable of displaying, acquiring, or presenting at least one antigen or antigenic fragment on (or at) its cell surface. In general, the term "cell" can be any cell that accomplishes the goal of aiding the enhancement of an immune response (i.e., from the T cell or -B-cell arms of the immune system) against an antigen or antigenic composition. As discussed in Kuby, 2000, Immunology, .supp. 4th edition, W.H. Freeman and company, for example, and used herein in certain embodiments, a cell that displays or presents an antigen normally or with a class II major histocompatibility molecule or complex to an immune cell is an "antigen-presenting cell." In certain aspects, a cell (e.g., an APC cell) may be fused with another cell, such as a recombinant cell or a tumor cell that expresses the desired antigen. Methods for preparing a fusion of two or more cells are discussed in, for example, Goding, J.W., Monoclonal Antibodies: Principles and Practice, pp. 65-66, 71-74 (Academic Press, 1986); Campbell, in: Monoclonal Antibody Technology, Laboratory Techniques in Biochemistry and Molecular Biology, Vol. 13, Burden & Von Knippenberg, Amsterdam, Elseview, pp. 75-83, 1984; Kohler & Milstein, Nature, 256:495-497, 1975; Kohler & Milstein, Eur. J. Immunol., 6:511-519, 1976, Gefter et al., Somatic Cell Genet., 3:231-236, 1977. In some cases, the immune cell to which a cell displays or presents an antigen to is a CD4⁺TH cell. Additional molecules expressed on the APC or other immune cells may aid or improve the enhancement of an immune response. Secreted or soluble molecules, such as for example, cytokines and adjuvants, may also aid or enhance the immune response against an antigen. Various examples are discussed herein.

An "antigen recognition moiety" may be any polypeptide or fragment thereof, such as, for example, an antibody fragment variable domain, either naturally derived, or synthetic, which binds to an antigen. Examples of antigen recognition moieties include, but are not limited to, polypeptides derived from antibodies, such as, for example, single chain variable fragments (scFv), Fab, Fab', F(ab')₂, and Fv fragments; polypeptides derived from T cell receptors, such as, for example, TCR variable domains; secreted factors (e.g., cytokines, growth factors) that can be artificially fused to signaling domains (e.g., "zytokines"), and any ligand or receptor fragment (e.g., CD27, NKG2D)that binds to the extracellular cognate protein. Combinatorial libraries could also be used to identify peptides binding with high affinity to tumor-associated targets.

The term "autologous" means a cell, nucleic acid, protein, polypeptide, or the like derived from the same individual to which it is later administered. The modified cells of the present methods may, for example, be autologous cells, such as, for example, autologous T cells.

The term "cancer" as used herein is defined as a hyperproliferation of cells whose unique trait-loss of normal controls-results in unregulated growth, lack of differentiation, local tissue invasion, and metastasis. Examples include but are not limited to, melanoma, non-small cell lung, small-cell lung, lung, hepatocarcinoma, leukemia, retinoblastoma, astrocytoma, glioblastoma, gum, tongue, neuroblastoma, head, neck, breast, pancreatic, prostate, eye, renal, bone, testicular, ovarian, mesothelioma, cervical, gastrointestinal, lymphoma, brain, colon, sarcoma or bladder.

The terms "cell," "cell line," and "cell culture" as used herein may be used interchangeably. All of these terms also include their progeny, which are any and all subsequent generations. It is understood that all progeny may not be identical due to deliberate or inadvertent mutations.

As used herein, the term "cDNA" is intended to refer to DNA prepared using messenger RNA (mRNA) as template. The advantage of using a cDNA, as opposed to genomic DNA or DNA polymerized from a genomic, non- or partially processed RNA template, is that the cDNA primarily contains coding sequences of the corresponding protein. There are times when the full or partial genomic sequence is used, such as where the non-coding regions are required for optimal expression or where non-coding regions such as introns are to be targeted in an antisense strategy.

As used herein, the term "expression construct" or "transgene" is defined as any type of genetic construct containing a nucleic acid coding for gene products in which part or all of the nucleic acid encoding sequence is capable of being transcribed can be inserted into the vector. The transcript is translated into a protein, but it need not be. In certain embodiments, expression includes both transcription of a gene and translation of mRNA into a gene product. In other embodiments, expression only includes transcription of the nucleic acid encoding genes of interest. The term "therapeutic construct" may also be used to refer to the expression construct or transgene. The expression construct or transgene may be used, for example, as a therapy to treat hyperproliferative diseases or disorders, such as cancer, thus the expression construct or transgene is a therapeutic construct or a prophylactic construct.

As used herein, the term "expression vector" refers to a vector containing a nucleic acid sequence coding for at least part of a gene product capable of being transcribed. In some cases, RNA molecules are then translated into a protein, polypeptide, or peptide. In other cases, these sequences are not translated, for example, in the production of antisense molecules or ribozymes. Expression vectors can contain a variety of control sequences, which refer to nucleic acid sequences necessary for the transcription and possibly translation of an operatively linked coding sequence in a particular host organism. In addition to control sequences that govern transcription and translation, vectors and expression vectors may contain nucleic acid sequences that serve other functions as well and are discussed infra.

As used herein, the term "ex vivo" refers to "outside" the body. The terms "ex vivo" and "in vitro" can be used interchangeably herein.

T cell receptors (TCRs) are immune proteins that specifically bind to antigenic molecules. TCRs are composed of two different polypeptides that are on the surface of T cells. They recognize, or specifically bind to, antigens bound to major histocompatibility complex molecules; upon binding to the antigen, the T cell is activated. By "recognize" is meant, for example, that the T cell receptor, or fragment or fragments thereof, such as TCRα polypeptide and TCRβ together, is capable of contacting the antigen and identifying it as a target. TCRs may comprise α and β polypeptides, or chains. The α and β polypeptides include two extracellular domains, the variable and the constant domains. The variable domain of the α and β polypeptides has three complementarity determining regions (CDRs); CDR3 is considered to be the main CDR responsible for recognizing the epitope. The α polypeptide includes the V and J regions, generated by VJ recombination, and the β polypeptide includes the V, D, and J regions, generated by VDJ recombination. The intersection of the VJ regions and VDJ regions corresponds to the CDR3 region. TCRs are often named using the International Immunogenetics (IMGT) TCR nomenclature (IMGT Database, www. IMGT.org; Giudicelli, V., et al.,IMGT/LIGM-DB, the IMGT® comprehensive database of immunoglobulin and T cell receptor nucleotide sequences, Nucl. Acids Res., 34, D781-D784 (2006). PMID: 16381979;T cell Receptor Factsbook, LeFranc and LeFranc, Academic Press ISBN 0-12-441352-8).

By "specifically bind(s) to" as it relates to a T cell receptor, or as it refers to a recombinant T cell receptor, polypeptide, polypeptide fragment, variant, or analog, or a modified cell, such as, for example, the PRAME T cell receptors, T cell receptor CDR3 regions, and PRAME TCR-expressing modified cells herein, is meant that the T cell receptor, or fragment thereof, recognizes, or binds selectively to the PRAME antigen. Under certain conditions, for example, in an immunoassay, for example an immunoassay discussed herein, the T cell receptor binds to PRAME and does not bind in a significant amount to other polypeptides. Thus the T cell receptor binds to PRAME with at least 5, 10, 20, 30, 40, 50, or 100 fold more affinity than to a control antigenic polypeptide. This binding may also be determined indirectly in the context of a modified T cell that expresses a PRAME TCR. In assays such as, for example, an assay discussed herein, the modified T cell is specifically reactive against a PRAME expressing cell line, cells, or tissue, such as, for example, myeloma, AML, or ALL cells. Thus, the modified PRAME TCR-expressing T cell binds to a PRAME-expressing cell line with at least 5, 10, 20, 30, 40, 50, or 100 fold more reactivity when compared to its reactivity against a control cell line that is not a PRAME-expressing cell line. The PRAME T cell receptors, T cell receptor CDR3 regions, and PRAME TCR-expressing cell lines may, for example bind to PRAME that is expressed on a cell surface, and typically bind to PRAME in the context of major histocompatibility marker (MHC) presentation, for example, MHC Class I HLA presentation, for example, in the context of MHC Class 1 HLA A2.01 presentation.

As used herein, the term "functionally equivalent," as it relates to a T cell receptor, for example, or as it refers to a T cell receptor nucleic acid fragment, variant, or analog, refers to a nucleic acid that codes for a T cell receptor or T cell receptor polypeptide, that stimulates an immune response against an antigen or cell. In the context of TCR recognition or binding to an antigen, the TCR may recognize the antigen as an epitope as part of a peptide-MHC complex. "Functionally equivalent" or "a functional fragment" of a T cell receptor polypeptide refers, for example, to a T cell receptor that is lacking a T cell receptor domain, such as a constant region, but is capable of stimulating an immune response typical for a T cell. A functionally equivalent T cell receptor fragment, may, for example, specifically bind to or recognize an antigen, alone, or in an MHC complex, and upon binding or recognition, activates the T lymphocyte. Derivatives of nucleic acid molecules or nucleotide sequences coding for, for example, CDR3 regions of a TCR, or of a TCR alpha polypeptide or TCR beta polypeptide are nucleic acid molecules or nucleotide sequences that code for functional CDR3 regions of TCR or of TCR alpha or beta polypeptides, and, for example, encode polypeptides that specifically bind to or recognize an antigen, alone, or in an MHC complex. These derivatives of nucleotide sequences that code for TCR alpha or TCR beta polypeptides may, for example, have consecutive nucleotides 80%, 85%, 90%, 95% or more identical to the corresponding nucleotide sequence coding for the TCR alpha or TCR beta polypeptides. "Functionally equivalent" or "a functional fragment" of a CDR3 region of a T cell receptor polypeptide refers, for example, to a T cell receptor that may have a modified amino acid sequence and may, for example, have an amino acid sequence of the alpha or beta polypeptides, or alpha and beta polypeptides that is 80%, 85%, 90%, 95% or more identical to the corresponding alpha and beta polypeptides, but is capable of stimulating an immune response typical for a T cell when included as part of a T cell receptor, such as a recombinant T cell receptor. In calculating the identity of an amino acid sequence and an amino acid sequence derivative, or a nucleotide sequence, the cysteine used to enhance expression of the recombinant TCR alpha or beta polypeptide, or CDR3 region or other fragment thereof, or the codon that encodes the cysteine residue, is not considered as part of the percentage calculation where the sequence provided herein as a SEQ ID NO: does not have the cysteine modification. When the term "functionally equivalent" or "functional fragment thereof" is applied to other nucleic acids or polypeptides, such as, for example, Caspase-9 or truncated Caspase-9, it refers to fragments, variants, and the like that have the same or similar activity as the reference polypeptides of the methods herein. For example, a functional fragment of a tumor antigen polypeptide, such as, for example, PSMA may be antigenic, allowing for antibodies to be produced that recognize the particular tumor antigen. A functional fragment of a ligand binding region, for example, Fvls, would include a sufficient portion of the ligand binding region polypeptide to bind the appropriate ligand. "Functionally equivalent" refers, for example, to a co-stimulatory polypeptide that is lacking the extracellular domain, but is capable of amplifying the T cell-mediated tumor killing response when expressed in T cells.

The term "hyperproliferative disease" is defined as a disease that results from a hyperproliferation of cells. Exemplary hyperproliferative diseases include, but are not limited to cancer or autoimmune diseases. Other hyperproliferative diseases may include vascular occlusion, restenosis, atherosclerosis, or inflammatory bowel disease.

As used herein, the term "gene" is defined as a functional protein, polypeptide, or peptide-encoding unit. As will be understood, this functional term includes genomic sequences, cDNA sequences, and smaller engineered gene segments that express, or are adapted to express, proteins, polypeptides, domains, peptides, fusion proteins, and mutants.

The term "immunogenic composition" or "immunogen" refers to a substance that is capable of provoking an immune response. Examples of immunogens include, e.g., antigens, autoantigens that play a role in induction of autoimmune diseases, and tumor-associated antigens expressed on cancer cells.

The term "immunocompromised" as used herein is defined as a subject that has reduced or weakened immune system. The immunocompromised condition may be due to a defect or dysfunction of the immune system or to other factors that heighten susceptibility to infection and/or disease. Although such a categorization allows a conceptual basis for evaluation, immunocompromised individuals often do not fit completely into one group or the other. More than one defect in the body's defense mechanisms may be affected. For example, individuals with a specific T-lymphocyte defect caused by HIV may also have neutropenia caused by drugs used for antiviral therapy or be immunocompromised because of a breach of the integrity of the skin and mucous membranes. An immunocompromised state can result from indwelling central lines or other types of impairment due to intravenous drug abuse; or be caused by secondary malignancy, malnutrition, or having been infected with other infectious agents such as tuberculosis or sexually transmitted diseases, e.g., syphilis or hepatitis.

As used herein, the term "pharmaceutically or pharmacologically acceptable" refers to molecular entities and compositions that do not produce adverse, allergic, or other untoward reactions when administered to an animal or a human.

As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the vectors or cells presented herein, its use in therapeutic compositions is contemplated. Supplementary active ingredients also can be incorporated into the compositions. In some embodiments, the subject is a mammal. In some embodiments, the subject is a human.

As used herein, the term "polynucleotide" is defined as a chain of nucleotides. Furthermore, nucleic acids are polymers of nucleotides. Thus, nucleic acids and polynucleotides as used herein are interchangeable. Nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR^{™}, and the like, and by synthetic means. Furthermore, polynucleotides include mutations of the polynucleotides, include but are not limited to, mutation of the nucleotides, or nucleosides by methods well known in the art. A nucleic acid may comprise one or more polynucleotides.

As used herein, the term "polypeptide" is defined as a chain of amino acid residues, usually having a defined sequence. As used herein the term polypeptide may be interchangeable with the term "proteins".

As used herein, the term "promoter" is defined as a DNA sequence recognized by the synthetic machinery of the cell, or introduced synthetic machinery, required to initiate the specific transcription of a gene.

As used herein, the terms "regulate an immune response," "modulate an immune response," or "control an immune response," refer to the ability to modify the immune response. For example, the composition is capable of enhancing and/or activating the immune response. Still further, the composition is also capable of inhibiting the immune response. The form of regulation is determined by the ligand that is used with the composition. For example, a dimeric analog of the chemical results in dimerization of the co-stimulating polypeptide leading to activation of the T cell, however, a monomeric analog of the chemical does not result in dimerization of the co-stimulating polypeptide, which would not activate the T cells.

The term "transfection" and "transduction" are interchangeable and refer to the process by which an exogenous DNA sequence is introduced into a eukaryotic host cell. Transfection (or transduction) can be achieved by any one of a number of means including electroporation, microinjection, gene gun delivery, retroviral infection, lipofection, superfection and the like.

As used herein, the term "syngeneic" refers to cells, tissues or animals that have genotypes that are identical or closely related enough to allow tissue transplant, or are immunologically compatible. For example, identical twins or animals of the same inbred strain. Syngeneic and isogeneic can be used interchangeably.

The term "patient" or "subject" are interchangeable, and, as used herein include, but are not limited to, an organism or animal; a mammal, including, e.g., a human, non-human primate (e.g., monkey), mouse, pig, cow, goat, rabbit, rat, guinea pig, hamster, horse, monkey, sheep, or other non-human mammal; a non-mammal, including, e.g., a non-mammalian vertebrate, such as a bird (e.g., a chicken or duck) or a fish, and a non-mammalian invertebrate.

As used herein, the term "vaccine" refers to a formulation that contains a composition presented herein which is in a form that is capable of being administered to an animal. Typically, the vaccine comprises a conventional saline or buffered aqueous solution medium in which the composition is suspended or dissolved. In this form, the composition can be used conveniently to prevent, ameliorate, or otherwise treat a condition. Upon introduction into a subject, the vaccine is able to provoke an immune response including, but not limited to, the production of antibodies, cytokines and/or other cellular responses.

As used herein, the term "under transcriptional control" or "operatively linked" is defined as the promoter is in the correct location and orientation in relation to the nucleic acid to control RNA polymerase initiation and expression of the gene.

As used herein, the terms "treatment", "treat", "treated", or "treating" refer to prophylaxis and/or therapy. When used with respect to a solid tumor, such as a cancerous solid tumor, for example, the term refers to prevention by prophylactic treatment, which increases the subject's resistance to solid tumors or cancer. In some examples, the subject may be treated to prevent cancer, where the cancer is familial, or is genetically associated. When used with respect to an infectious disease, for example, the term refers to a prophylactic treatment which increases the resistance of a subject to infection with a pathogen or, in other words, decreases the likelihood that the subject will become infected with the pathogen or will show signs of illness attributable to the infection, as well as a treatment after the subject has become infected in order to fight the infection, for example, reduce or eliminate the infection or prevent it from becoming worse.

The methods provided herein may be used, for example, to treat a disease, disorder, or condition wherein there is an elevated expression of a tumor antigen.

As used herein, the term "vaccine" refers to a formulation which contains a composition presented herein which is in a form that is capable of being administered to an animal. Typically, the vaccine comprises a conventional saline or buffered aqueous solution medium in which the composition is suspended or dissolved. In this form, the composition can be used conveniently to prevent, ameliorate, or otherwise treat a condition. Upon introduction into a subject, the vaccine is able to provoke an immune response including, but not limited to, the production of antibodies, cytokines and/or other cellular responses.

Blood disease: The terms "blood disease", "blood disease" and/or "diseases of the blood" as used herein, refers to conditions that affect the production of blood and its components, including but not limited to, blood cells, hemoglobin, blood proteins, the mechanism of coagulation, production of blood, production of blood proteins, the like and combinations thereof. Non-limiting examples of blood diseases include anemias, leukemias, lymphomas, hematological neoplasms, albuminemias, haemophilias and the like.

Bone marrow disease: The term "bone marrow disease" as used herein, refers to conditions leading to a decrease in the production of blood cells and blood platelets. In some bone marrow diseases, normal bone marrow architecture can be displaced by infections (e.g., tuberculosis) or malignancies, which in turn can lead to the decrease in production of blood cells and blood platelets. Non-limiting examples of bone marrow diseases include leukemias, bacterial infections (e.g., tuberculosis), radiation sickness or poisoning, apnocytopenia, anemia, multiple myeloma and the like.

T cells and Activated T cells (CD3⁺): T cells (also referred to as T lymphocytes) belong to a group of white blood cells referred to as lymphocytes. Lymphocytes generally are involved in cell-mediated immunity. The "T" in "T cells" refers to cells derived from or whose maturation is influenced by the thymus. T cells can be distinguished from other lymphocytes types such as B cells and Natural Killer (NK) cells by the presence of cell surface proteins known as T cell receptors. The term "activated T cells" as used herein, refers to T cells that have been stimulated to produce an immune response (e.g., clonal expansion of activated T cells) by recognition of an antigenic determinant presented in the context of a Class II major histocompatibility (MHC) marker. T cells are activated by the presence of an antigenic determinant, cytokines and/or lymphokines and cluster of differentiation cell surface proteins (e.g., CD3, CD4, CD8, the like and combinations thereof). Cells that express a cluster of differential protein often are said to be "positive" for expression of that protein on the surface of T cells (e.g., cells positive for CD3 or CD4 expression are referred to as CD3⁺ or CD4⁺). CD3 and CD4 proteins are cell surface receptors or co-receptors that may be directly and/or indirectly involved in signal transduction in T cells.

Peripheral blood: The term "peripheral blood" as used herein, refers to cellular components of blood (e.g., red blood cells, white blood cells and platelets), which are obtained or prepared from the circulating pool of blood and not sequestered within the lymphatic system, spleen, liver or bone marrow.

Umbilical cord blood: Umbilical cord blood is distinct from peripheral blood and blood sequestered within the lymphatic system, spleen, liver or bone marrow. The terms "umbilical cord blood", "umbilical blood" or "cord blood", which can be used interchangeably, refers to blood that remains in the placenta and in the attached umbilical cord after child birth. Cord blood often contains stem cells including hematopoietic cells.

By "obtained or prepared" as, for example, in the case of cells, is meant that the cells or cell culture are isolated, purified, or partially purified from the source, where the source may be, for example, umbilical cord blood, bone marrow, or peripheral blood. The terms may also apply to the case where the original source, or a cell culture, has been cultured and the cells have replicated, and where the progeny cells are now derived from the original source.

By "kill" or "killing" as in a percent of cells killed, is meant the death of a cell through apoptosis, as measured using any method known for measuring apoptosis. The term may also refer to cell ablation.

Donor T cell: The term "donor T cell" as used here refers to T cells that often are administered to a recipient to confer anti-viral and/or anti-tumor immunity following allogeneic stem cell transplantation. Donor T cells often are utilized to inhibit marrow graft rejection and increase the success of alloengraftment, however the same donor T cells can cause an alloaggressive response against host antigens, which in turn can result in graft versus host disease (GvHD). Certain activated donor T cells can cause a higher or lower GvHD response than other activated T cells. Donor T cells may also be reactive against recipient tumor cells, causing a beneficial graft vs. tumor effect.

Function-conservative variants are proteins or enzymes in which a given amino acid residue has been changed without altering overall conformation and function of the protein or enzyme, including, but not limited to, replacement of an amino acid with one having similar properties, including polar or non-polar character, size, shape and charge. Conservative amino acid substitutions for many of the commonly known non-genetically encoded amino acids are well known in the art. Conservative substitutions for other non-encoded amino acids can be determined based on their physical properties as compared to the properties of the genetically encoded amino acids.

Amino acids other than those indicated as conserved may differ in a protein or enzyme so that the percent protein or amino acid sequence similarity between any two proteins of similar function may vary and can be, for example, at least 70%, at least 80%, at least 90%, and, for example, at least 95%, as determined according to an alignment scheme. As referred to herein, "sequence similarity" means the extent to which nucleotide or protein sequences are related. The extent of similarity between two sequences can be based on percent sequence identity and/or conservation. "Sequence identity" herein means the extent to which two nucleotide or amino acid sequences are invariant. "Sequence alignment" means the process of lining up two or more sequences to achieve maximal levels of identity (and, in the case of amino acid sequences, conservation) for the purpose of assessing the degree of similarity. Numerous methods for aligning sequences and assessing similarity/identity are known in the art such as, for example, the Cluster Method, wherein similarity is based on the MEGALIGN algorithm, as well as BLASTN, BLASTP, and FASTA. When using any of these programs, the settings used are those that results in the highest sequence similarity.

Mesenchymal stromal cell: The terms "mesenchymal stromal cell" or "bone marrow derived mesenchymal stromal cell" as used herein, refer to multipotent stem cells that can differentiate ex vivo, in vitro and in vivo into adipocytes, osteoblasts and chondroblasts, and may be further defined as a fraction of mononuclear bone marrow cells that adhere to plastic culture dishes in standard culture conditions, are negative for hematopoietic lineage markers and are positive for CD73, CD90 and CD105.

Inducible pluripotent stem cell: The terms "inducible pluripotent stem cell" or "induced pluripotent stem cell" as used herein refers to adult, or differentiated cells, that are "reprogrammed" or induced by genetic (e.g., expression of genes that in turn activates pluripotency), biological (e.g., treatment viruses or retroviruses) and/or chemical (e.g., small molecules, peptides and the like) manipulation to generate cells that are capable of differentiating into many if not all cell types, like embryonic stem cells. Inducible pluripotent stem cells are distinguished from embryonic stem cells in that they achieve an intermediate or terminally differentiated state (e.g., skin cells, bone cells, fibroblasts, and the like) and then are induced to dedifferentiate, thereby regaining some or all of the ability to generate multipotent or pluripotent cells.

CD34⁺ cell: The term "CD34⁺ cell" as used herein refers to a cell expressing the CD34 protein on its cell surface. "CD34" as used herein refers to a cell surface glycoprotein (e.g., sialomucin protein) that often acts as a cell-cell adhesion factor and is involved in T cell entrance into lymph nodes, and is a member of the "cluster of differentiation" gene family. CD34 also may mediate the attachment of stem cells to bone marrow, extracellular matrix or directly to stromal cells. CD34⁺ cells often are found in the umbilical cord and bone marrow as hematopoietic cells, a subset of mesenchymal stem cells, endothelial progenitor cells, endothelial cells of blood vessels but not lymphatics (except pleural lymphatics), mast cells, a sub-population of dendritic cells (which are factor Xllla negative) in the interstitium and around the adnexa of dermis of skin, as well as cells in certain soft tissue tumors (e.g., alveolar soft part sarcoma, pre-B acute lymphoblastic leukemia (Pre-B-ALL), acute myelogenous leukemia (AML) , AML-M7, dermatofibrosarcoma protuberans, gastrointestinal stromal tumors, giant cell fibroblastoma, granulocytic sarcoma, Kaposi's sarcoma, liposarcoma, malignant fibrous histiocytoma, malignant peripheral nerve sheath tumors, mengingeal hemangiopericytomas, meningiomas, neurofibromas, schwannomas, and papillary thyroid carcinoma).

Tumor infiltrating lymphocytes (TILs) refer to T cells having various receptors which infiltrate tumors and kill tumor cells in a targeted manor. Regulating the activity of the TILs using the methods of the present application would allow for more direct control of the elimination of tumor cells.

Gene expression vector: The terms "gene expression vector", "nucleic acid expression vector", or "expression vector" as used herein, which can be used interchangeably throughout the document, generally refers to a nucleic acid molecule (e.g., a plasmid, phage, autonomously replicating sequence (ARS), artificial chromosome, yeast artificial chromosome (e.g., YAC)) that can be replicated in a host cell and be utilized to introduce a gene or genes into a host cell. The genes introduced on the expression vector can be endogenous genes (e.g., a gene normally found in the host cell or organism) or heterologous genes (e.g., genes not normally found in the genome or on extra-chromosomal nucleic acids of the host cell or organism). The genes introduced into a cell by an expression vector can be native genes or genes that have been modified or engineered. The gene expression vector also can be engineered to contain 5' and 3' untranslated regulatory sequences that sometimes can function as enhancer sequences, promoter regions and/or terminator sequences that can facilitate or enhance efficient transcription of the gene or genes carried on the expression vector. A gene expression vector sometimes also is engineered for replication and/or expression functionality (e.g., transcription and translation) in a particular cell type, cell location, or tissue type. Expression vectors sometimes include a selectable marker for maintenance of the vector in the host or recipient cell.

Developmentally regulated promoter: The term "developmentally regulated promoter" as used herein refers to a promoter that acts as the initial binding site for RNA polymerase to transcribe a gene which is expressed under certain conditions that are controlled, initiated by or influenced by a developmental program or pathway. Developmentally regulated promoters often have additional control regions at or near the promoter region for binding activators or repressors of transcription that can influence transcription of a gene that is part of a development program or pathway. Developmentally regulated promoters sometimes are involved in transcribing genes whose gene products influence the developmental differentiation of cells.

Developmentally differentiated cells: The term "developmentally differentiated cells", as used herein refers to cells that have undergone a process, often involving expression of specific developmentally regulated genes, by which the cell evolves from a less specialized form to a more specialized form in order to perform a specific function. Non-limiting examples of developmentally differentiated cells are liver cells, lung cells, skin cells, nerve cells, blood cells, and the like. Changes in developmental differentiation generally involve changes in gene expression (e.g., changes in patterns of gene expression), genetic re-organization (e.g., remodeling or chromatin to hide or expose genes that will be silenced or expressed, respectively), and occasionally involve changes in DNA sequences (e.g., immune diversity differentiation). Cellular differentiation during development can be understood as the result of a gene regulatory network. A regulatory gene and its cis-regulatory modules are nodes in a gene regulatory network that receive input (e.g., protein expressed upstream in a development pathway or program) and create output elsewhere in the network (e.g., the expressed gene product acts on other genes downstream in the developmental pathway or program).

The term "hyperproliferative disease" is defined as a disease that results from a hyperproliferation of cells. Exemplary hyperproliferative diseases include, but are not limited to cancer or autoimmune diseases. Other hyperproliferative diseases may include vascular occlusion, restenosis, atherosclerosis, or inflammatory bowel disease.

In some embodiments, the nucleic acid is contained within a viral vector. In certain embodiments, the viral vector is an adenoviral vector, or a retroviral or lentiviral vector. It is understood that in some embodiments, the cell is contacted with the viral vector ex vivo, and in some embodiments, the cell is contacted with the viral vector in vivo.

In certain embodiments, the cell is also contacted with an antigen. Often, the cell is contacted with the antigen ex vivo. Sometimes, the cell is contacted with the antigen in vivo. In some embodiments, the cell is in a subject and an immune response is generated against the antigen. Sometimes, the immune response is a cytotoxic T-lymphocyte (CTL) immune response. Sometimes, the immune response is generated against a tumor antigen. In certain embodiments, the cell is activated without the addition of an adjuvant.

In some embodiments, the cell is transduced with the nucleic acid ex vivo and administered to the subject by intradermal administration. In some embodiments, the cell is transduced with the nucleic acid ex vivo and administered to the subject by subcutaneous administration. Sometimes, the cell is transduced with the nucleic acid ex vivo. Sometimes, the cell is transduced with the nucleic acid in vivo.

The cell in some embodiments is contacted with an antigen, sometimes ex vivo. In certain embodiments the cell is in a subject and an immune response is generated against the antigen, such as a cytotoxic T-lymphocyte (CTL) immune response. In certain embodiments, an immune response is generated against a tumor antigen (e.g., PSMA). In some embodiments, the nucleic acid is prepared ex vivo and administered to the subject by intradermal administration or by subcutaneous administration, for example. Sometimes the cell is transduced or transfected with the nucleic acid ex vivo or in vivo.

In some embodiments, the nucleic acid comprises a promoter sequence operably linked to the polynucleotide sequence. Alternatively, the nucleic acid comprises an ex vivo-transcribed RNA, containing the protein-coding region of the chimeric protein.

By "reducing tumor size" or "inhibiting tumor growth" of a solid tumor is meant a response to treatment, or stabilization of disease, according to standard guidelines, such as, for example, the Response Evaluation Criteria in Solid Tumors (RECIST) criteria. For example, this may include a reduction in the diameter of a solid tumor of about 5%, 10%, 20%, 30%, 40%, 50%, 60%, 70%, 80%, 90%, or 100%, or the reduction in the number of tumors, circulating tumor cells, or tumor markers, of about 5%, 10%, 20%, 30%, 40%, 50%, 60%, 70%, 80%, 90%, or 100%. The size of tumors may be analyzed by any method, including, for example, CT scan, MRI, for example, CT-MRI, chest X-ray (for tumors of the lung), or molecular imaging, for example, PET scan, such as, for example, a PET scan after administering an iodine 123-labelled PSA, for example, PSMA ligand, such as, for example, where the inhibitor is TROFEX^{™}/MIP-1072/1095, or molecular imaging, for example, SPECT, or a PET scan using PSA, for example, PSMA antibody, such as, for example, capromad pendetide (Prostascint), a 111-iridium labeled PSMA antibody.

By "reducing, slowing, or inhibiting tumor vascularization" is meant a reduction in tumor vascularization of about 5%, 10%, 20%, 30%, 40%, 50%, 60%, 70%, 80%, 90%, or 100%, or a reduction in the appearance of new vasculature of about 5%, 10%, 20%, 30%, 40%, 50%, 60%, 70%, 80%, 90%, or 100%, when compared to the amount of tumor vascularization before treatment. The reduction may refer to one tumor, or may be a sum or an average of the vascularization in more than one tumor. Methods of measuring tumor vascularization include, for example, CAT scan, MRI, for example, CT-MRI, or molecular imaging, for example, SPECT, or a PET scan, such as, for example, a PET scan after administering an iodine 123-labelled PSA, for example, PSMA ligand, such as, for example, where the inhibitor is TROFEX^{™}/MIP-1072/1095, or a PET scan using PSA, for example, PSMA antibody, such as, for example, capromad pendetide (Prostascint), a 111-iridium labeled PSMA antibody.

A tumor is classified, or named as part of an organ, such as a prostate cancer tumor when, for example, the tumor is present in the prostate gland, or has derived from or metastasized from a tumor in the prostate gland, or produces PSA. A tumor has metastasized from a tumor in the prostate gland, when, for example, it is determined that the tumor has chromosomal breakpoints that are the same as, or similar to, a tumor in the prostate gland of the subject.

For hematological malignancies, by "reducing, slowing, or inhibiting a hematological malignancy" is meant a reduction, slowing or inhibition of the amount or concentration of malignant cells, for example as measured in a sample obtained from the subject, of about 5%, 10%, 20%, 30%, 40%, 50%, 60%, 70%, 80%, 90%, or 100%, when compared to the amount or concentration of malignant cells before treatment. Methods for measuring the amount or concentration of malignant cells, or the tumor load include, for example, qRT-PCR and genome wide sequencing.

For hematological tumors, by "reducing, slowing, or inhibiting a tumor load" is meant a reduction, slowing or inhibition of the amount or concentration of tumor cells, for example as measured in a sample obtained from the subject, of about 5%, 10%, 20%, 30%, 40%, 50%, 60%, 70%, 80%, 90%, or 100%, when compared to the amount or concentration of tumor cells before treatment. Methods for measuring the amount or concentration of tumor cells, include, for example, qRT-PCR and genome wide sequencing.

### Engineering Expression Constructs

Expression constructs that express the present TCRs or chimeric polypeptides comprise the TCR or polypeptide coding region and a promoter sequence, all operatively linked. In general, the term "operably linked" is meant to indicate that the promoter sequence is functionally linked to a second sequence, wherein the promoter sequence initiates and mediates transcription of the DNA corresponding to the second sequence.

In certain examples, the polynucleotide coding for the TCR or other polypeptide is included in the same vector, such as, for example, a viral or plasmid vector, as a polynucleotide coding for the second polypeptide. This second polypeptide may be, for example, a caspase polypeptide, as discussed herein, or a marker polypeptide. In these examples, the construct may be designed with one promoter operably linked to a nucleic acid comprising a polynucleotide coding for the two polypeptides, linked by a cleavable 2A polypeptide or by the internal ribosome entry sequence (IRES). In these examples, the first and second polypeptides are separated during translation, resulting in a TCR and an additional polypeptide. In other examples, the two polypeptides may be expressed separately from the same vector, where each nucleic acid comprising a polynucleotide coding for one of the polypeptides is operably linked to a separate promoter. In yet other examples, one promoter may be operably linked to the two polynucleotides, directing the production of two separate RNA transcripts, and thus two polypeptides; in one example, the promoter may be bi-directional, and the coding regions may be in opposite directions 5'-3'. Therefore, the expression constructs discussed herein may comprise at least one, or at least two promoters.

In yet other examples, two polypeptides, such as, for example, the TCR and a caspase polypeptide may be expressed by the cell using two separate vectors. The cells may be co-transfected or co-transformed with the vectors, or the vectors may be introduced to the cells at different times.

The polypeptides may vary in their order, from the amino terminus to the carboxy terminus. The order of the various domains may be assayed using methods such as, for example, those discussed herein, to obtain the optimal expression and activity.

### Selectable Markers

In certain embodiments, the expression constructs contain nucleic acid constructs whose expression is identified in vitro or in vivo by including a marker in the expression construct. Such markers would confer an identifiable change to the cell permitting easy identification of cells containing the expression construct. Usually the inclusion of a drug selection marker aids in cloning and in the selection of transformants. For example, genes that confer resistance to neomycin, puromycin, hygromycin, DHFR, GPT, zeocin and histidinol are useful selectable markers. Alternatively, enzymes such as Herpes Simplex Virus thymidine kinase (tk) are employed. Immunologic surface markers containing the extracellular, non-signaling domains or various proteins (e.g. CD34, CD19, low affinity nerve growth factor receptor (LNGFR)) also can be employed, permitting a straightforward method for magnetic or fluorescence antibody-mediated sorting. The selectable marker employed is not believed to be important, so long as it is capable of being expressed simultaneously with the nucleic acid encoding a gene product. Further examples of selectable markers include, for example, reporters such as GFP, EGFP, β-gal or chloramphenicol acetyltransferase (CAT). In certain embodiments, the marker protein, such as, for example, CD19 is used for selection of the cells for transfusion, such as, for example, in immunomagnetic selection. As discussed herein, a CD19 marker is distinguished from an anti-CD19 antibody, or, for example, an scFv, TCR, or other antigen recognition moiety that binds to CD19.

In certain embodiments, the marker polypeptide is linked to the inducible chimeric signaling molecule. For example, the marker polypeptide may be linked to the inducible chimeric signaling molecule via a polypeptide sequence, such as, for example, a cleavable 2A-like sequence. The marker polypeptide may be, for example, CD19, ΔCD19, or may be, for example, a heterologous protein, selected to not affect the activity of the inducible chimeric signaling molecule.

2A-like sequences, or "peptide bond-skipping" 2A sequences, are derived from, for example, many different viruses, including, for example, from *Thosea asigna.* These sequences are sometimes also known as "peptide skipping sequences." When this type of sequence is placed within a cistron, between two peptides that are intended to be separated, the ribosome appears to skip a peptide bond, in the case of *Thosea asigna* sequence; the bond between the Gly and Pro amino acids at the carboxy terminal "P-G-P" is omitted. This leaves two to three polypeptides, in this case the co-stimulating polypeptide cytoplasmic region and the marker polypeptide. When this sequence is used, the peptide that is encoded 5' of the 2A sequence may end up with additional amino acids at the carboxy terminus, including the Gly residue and any upstream residues in the 2A sequence. The peptide that is encoded 3' of the 2A sequence may end up with additional amino acids at the amino terminus, including the Pro residue and any downstream residues following the 2A sequence.

In some embodiments, a polypeptide may be included in the expression vector to aid in sorting cells. For example, the CD34 minimal epitope may be incorporated into the vector. In some embodiments, the expression vectors used to express the TCRs provided herein further comprise a polynucleotide that encodes the 16 amino acid CD34 minimal epitope. In some embodiments, such as certain embodiments provided in the examples herein, the CD34 minimal epitope is incorporated at the amino terminal position of the CD8 stalk.

### Ligand-binding Regions

Ligand binding regions may be included in the chimeric polypeptides discussed herein, for example, as part of the inducible caspase polypeptides. The ligand-binding ("dimerization") domain of the expression construct can be any convenient domain that will allow for induction using a natural or unnatural ligand, for example, an unnatural synthetic ligand. The multimerizing region or ligand-binding domain can be internal or external to the cellular membrane, depending upon the nature of the construct and the choice of ligand. A wide variety of ligand-binding proteins, including receptors, are known, including ligand-binding proteins associated with the cytoplasmic regions indicated above. As used herein the term "ligand-binding domain can be interchangeable with the term "receptor". Of particular interest are ligand-binding proteins for which ligands (for example, small organic ligands) are known or may be readily produced. These ligand-binding domains or receptors include the FKBPs and cyclophilin receptors, the steroid receptors, the tetracycline receptor, the other receptors indicated above, and the like, as well as "unnatural" receptors, which can be obtained from antibodies, particularly the heavy or light chain subunit, mutated sequences thereof, random amino acid sequences obtained by stochastic procedures, combinatorial syntheses, and the like. In certain embodiments, the ligand-binding region is selected from the group consisting of FKBP ligand-binding region, cyclophilin receptor ligand-binding region, steroid receptor ligand-binding region, cyclophilin receptors ligand-binding region, and tetracycline receptor ligand-binding region. Often, the ligand-binding region comprises an FᵥFᵥₗₛ sequence. Sometimes, the F_{v'}Fᵥₗₛ sequence further comprises an additional Fv' sequence. The FKBP12 region may have, for example, an amino acid substitution at position 36, for example, amino acids substitutions selected from the group consisting of valine, leucine, isoleucine and alanine. Examples include, for example, those discussed in Kopytek, S.J., et al., Chemistry & Biology 7:313-321 (2000) and in Gestwicki, J.E., et al., Combinatorial Chem. & High Throughput Screening 10:667-675 (2007); Clackson, T. (2006) Chem Biol Drug Des 67:440-2; Clackson, T., in Chemical Biology: From Small Molecules to Systems Biology and Drug Design (Schreiber, s., et al., eds., Wiley, 2007)). For example, amino acid sequence SEQ ID NO: 77 represents one example of a sequence wherein there is an amino acid substitution of valine at position 36 (provided as the 35^{th} amino acid in the sequence herein). Amino acid sequence SEQ ID NO: 84 represents an amino acid sequence for FKBP12 having a wild type phenylalanine at position 36 (provided as the 35^{th} amino acid in the sequence herein).

For the most part, the ligand-binding domains or receptor domains will be at least about 50 amino acids, and fewer than about 350 amino acids, usually fewer than 200 amino acids, either as the natural domain or truncated active portion thereof. The binding domain may, for example, be small (<25 kDa, to allow efficient transfection in viral vectors), monomeric, nonimmunogenic, have synthetically accessible, cell permeable, nontoxic ligands that can be configured for dimerization.

The receptor domain can be intracellular or extracellular depending upon the design of the expression construct and the availability of an appropriate ligand. For hydrophobic ligands, the binding domain can be on either side of the membrane, but for hydrophilic ligands, particularly protein ligands, the binding domain will usually be external to the cell membrane, unless there is a transport system for internalizing the ligand in a form in which it is available for binding. For an intracellular receptor, the construct can encode a signal peptide and transmembrane domain 5' or 3' of the receptor domain sequence or may have a lipid attachment signal sequence 5' of the receptor domain sequence. Where the receptor domain is between the signal peptide and the transmembrane domain, the receptor domain will be extracellular.

The portion of the expression construct encoding the receptor can be subjected to mutagenesis for a variety of reasons. The mutagenized protein can provide for higher binding affinity, allow for discrimination by the ligand of the naturally occurring receptor and the mutagenized receptor, provide opportunities to design a receptor-ligand pair, or the like. The change in the receptor can involve changes in amino acids known to be at the binding site, random mutagenesis using combinatorial techniques, where the codons for the amino acids associated with the binding site or other amino acids associated with conformational changes can be subject to mutagenesis by changing the codon(s) for the particular amino acid, either with known changes or randomly, expressing the resulting proteins in an appropriate prokaryotic host and then screening the resulting proteins for binding.

Antibodies and antibody subunits, e.g., heavy or light chain, particularly fragments, more particularly all or part of the variable region, or fusions of heavy and light chain to create high-affinity binding, can be used as the binding domain. Antibodies that are contemplated include ones that are an ectopically expressed human product, such as an extracellular domain that would not trigger an immune response and generally not expressed in the periphery (i.e., outside the CNS/brain area). Such examples, include, but are not limited to low affinity nerve growth factor receptor (LNGFR), and embryonic surface proteins (i.e., carcinoembryonic antigen).

Yet further, antibodies can be prepared against haptenic molecules, which are physiologically acceptable, and the individual antibody subunits screened for binding affinity. The cDNA encoding the subunits can be isolated and modified by deletion of the constant region, portions of the variable region, mutagenesis of the variable region, or the like, to obtain a binding protein domain that has the appropriate affinity for the ligand. In this way, almost any physiologically acceptable haptenic compound can be employed as the ligand or to provide an epitope for the ligand. Instead of antibody units, natural receptors can be employed, where the binding domain is known and there is a useful ligand for binding.

### Oligomerization

The transduced signal will normally result from ligand-mediated oligomerization of the chimeric protein molecules, i.e., as a result of oligomerization following ligand-binding, although other binding events, for example allosteric activation, can be employed to initiate a signal. The construct of the chimeric protein will vary as to the order of the various domains and the number of repeats of an individual domain.

For multimerizing the caspase-9 polypeptide, the ligand for the ligand-binding domains/receptor domains of the chimeric inducible caspase-9 polypeptides will usually be multimeric in the sense that it will have at least two binding sites, with each of the binding sites capable of binding to the ligand receptor domain. By "multimeric ligand binding region" is meant a ligand binding region that binds to a multimeric ligand. The term "multimeric ligands" include dimeric ligands. A dimeric ligand will have two binding sites capable of binding to the ligand receptor domain. Desirably, the subject ligands will be a dimer or higher order oligomer, usually not greater than about tetrameric, of small synthetic organic molecules, the individual molecules typically being at least about 150 Da and less than about 5 kDa, usually less than about 3 kDa. A variety of pairs of synthetic ligands and receptors can be employed. For example, in embodiments involving natural receptors, dimeric FK506 can be used with an FKBP12 receptor, dimerized cyclosporin A can be used with the cyclophilin receptor, dimerized estrogen with an estrogen receptor, dimerized glucocorticoids with a glucocorticoid receptor, dimerized tetracycline with the tetracycline receptor, dimerized vitamin D with the vitamin D receptor, and the like. Alternatively higher orders of the ligands, e.g., trimeric can be used. For embodiments involving unnatural receptors, e.g., antibody subunits, modified antibody subunits, single chain antibodies comprised of heavy and light chain variable regions in tandem, separated by a flexible linker domain, or modified receptors, and mutated sequences thereof, and the like, any of a large variety of compounds can be used. A significant characteristic of these ligand units is that each binding site is able to bind the receptor with high affinity and they are able to be dimerized chemically. Also, methods are available to balance the hydrophobicity/hydrophilicity of the ligands so that they are able to dissolve in serum at functional levels, yet diffuse across plasma membranes for most applications.

In certain embodiments, the present methods utilize the technique of chemically induced dimerization (CID) to produce a conditionally controlled protein or polypeptide. In addition to this technique being inducible, it also is reversible, due to the degradation of the labile dimerizing agent or administration of a monomeric competitive inhibitor.

The CID system uses synthetic bivalent ligands to rapidly crosslink signaling molecules that are fused to ligand-binding domains. This system has been used to trigger the oligomerization and activation of cell surface (Spencer, D. M., et al., Science, 1993. 262: p. 1019-1024; Spencer D. M. et al., Curr Biol 1996, 6:839-847; Blau, C. A. et al., Proc Natl Acad.Sci. USA 1997, 94:3076-3081), or cytosolic proteins (Luo, Z. et al., Nature 1996,383:181-185; MacCorkle, R. A. et al., Proc Natl Acad Sci USA 1998, 95:3655-3660), the recruitment of transcription factors to DNA elements to modulate transcription (Ho, S. N. et al., Nature 1996, 382:822-826; Rivera, V. M. et al., Nat.Med. 1996, 2:1028-1032) or the recruitment of signaling molecules to the plasma membrane to stimulate signaling (Spencer D. M. et al., Proc.Natl.Acad.Sci. USA 1995, 92:9805-9809; Holsinger, L. J. et al., Proc.Natl.Acad.Sci. USA 1995, 95:9810-9814).

The CID system is based upon the notion that surface receptor aggregation effectively activates downstream signaling cascades. In the simplest embodiment, the CID system uses a dimeric analog of the lipid permeable immunosuppressant drug, FK506, which loses its normal bioactivity while gaining the ability to crosslink molecules genetically fused to the FK506-binding protein, FKBP12. By fusing one or more FKBPs and a myristoylation sequence to the cytoplasmic signaling domain of a target receptor, one can stimulate signaling in a dimerizer drug-dependent, but ligand and ectodomain-independent manner. This provides the system with temporal control, reversibility using monomeric drug analogs, and enhanced specificity. The high affinity of third-generation AP20187/AP1903 CIDs for their binding domain, FKBP12 permits specific activation of the recombinant receptor in vivo without the induction of nonspecific side effects through endogenous FKBP12. FKBP12 variants having amino acid substitutions and deletions, such as FKBP12ᵥ36, that bind to a dimerizer drug, may also be used. In addition, the synthetic ligands are resistant to protease degradation, making them more efficient at activating receptors in vivo than most delivered protein agents.

The ligands used are capable of binding to two or more of the ligand-binding domains. The chimeric proteins may be able to bind to more than one ligand when they contain more than one ligand-binding domain. The ligand is typically a non-protein or a chemical. Exemplary ligands include, but are not limited to dimeric FK506 (e.g., FK1012).

Other ligand binding regions may be, for example, dimeric regions, or modified ligand binding regions with a wobble substitution, such as, for example, FKBP12(V36): The human 12 kDa FK506-binding protein with an F36 to V substitution, the complete mature coding sequence (amino acids 1-107), provides a binding site for synthetic dimerizer drug AP1903 (Jemal, A. et al., CA Cancer J. Clinic. 58, 71-96 (2008); Scher, H.I. and Kelly, W.K., Journal of Clinical Oncology 11, 1566-72 (1993)). Two tandem copies of the protein may also be used in the construct so that higher-order oligomers are induced upon cross-linking by AP1903.

F36V'-FKBP: F36V'-FKBP is a codon-wobbled version of F36V-FKBP. It encodes the identical polypeptide sequence as F36V-FKPB but has only 62% homology at the nucleotide level. F36V'-FKBP was designed to reduce recombination in retroviral vectors (Schellhammer, P.F. et al., J. Urol. 157, 1731-5 (1997)). F36V'-FKBP was constructed by a PCR assembly procedure. The transgene contains one copy of F36V'-FKBP linked directly to one copy of F36V-FKBP.

In some embodiments, the ligand is a small molecule. The appropriate ligand for the selected ligand-binding region may be selected. Often, the ligand is dimeric, sometimes, the ligand is a dimeric FK506 or a dimeric FK506 analog. In certain embodiments, the ligand is AP1903 (INN: rimiducid, CAS Index Name: 2-Piperidinecarboxylic acid, 1-[(2S)-1-oxo-2-(3, 4,5-trimethoxyphenyl)butyl]-, 1,2-ethanediylbis [imino(2-oxo-2,1-ethanediyl)oxy-3,1-phenylene[(1R)-3-(3,4-dimethoxyphenyl)propylidene]] ester, [2S-[1(R*),2R*[S*[S*[1(R*),2R*]]]]]-(9CI)CAS Registry Number: 195514-63-7; Molecular Formula: C78H98N4O20 Molecular Weight: 1411.65). In certain embodiments, the ligand is AP20187. In certain embodiments, the ligand is an AP20187 analog, such as, for example, AP1510. In some embodiments, certain analogs will be appropriate for the FKBP12, and certain analogs appropriate for the mutant (V36) version of FKBP12. In certain embodiments, one ligand binding region is included in the chimeric protein. In other embodiments, two or more ligand binding regions are included. Where, for example, the ligand binding region is FKBP12, where two of these regions are included, one may, for example, be the wobbled version.

Other dimerization systems contemplated include the coumermycin/DNA gyrase B system. Coumermycin-induced dimerization activates a modified Raf protein and stimulates the MAP kinase cascade. See Farrar et al., 1996.

AP1903 API is manufactured by Alphora Research Inc. and AP1903 Drug Product for Injection is made by AAI Pharma Services Corp. It is formulated as a 5 mg/mL solution of AP1903 in a 25% solution of the non-ionic solubilizer Solutol HS 15 (250 mg/mL, BASF). At room temperature, this formulation is a clear solution. Upon refrigeration, this formulation undergoes a reversible phase transition on extended storage, resulting in a milky solution. This phase transition is reversed upon re-warming to room temperature. The fill is 8 mL in a 10 mL glass vial (~40 mg AP1903 for Injection total per vial).

For use, the AP1903 will be warmed to room temperature and diluted prior to administration. For subjects over 50 kg, the AP1903 is administered via i.v. infusion at a dose of 40 mg diluted in 100 mL physiological saline over 2 hours at a rate of 50 mL per hour using a DEHP-free saline bag and solution set. Subjects less than 50 kg receive 0.4 mg/kg AP1903.

All study medication is maintained at a temperature between 2 degrees C and 8 degrees C, protected from excessive light and heat, and stored in a locked area with restricted access.

Upon determining a need to administer AP1903 and activate caspase-9 in order to induce apoptosis of the engineered TCR-expressing T cells, patients may be, for example, administered a single fixed dose of AP1903 for Injection (0.4 mg/kg) via IV infusion over 2 hours, using a non-DEHP, non-ethylene oxide sterilized infusion set. The dose of AP1903 is calculated individually for all patients, and is not be recalculated unless body weight fluctuates by ≥10%. The calculated dose is diluted in 100 mL in 0.9% normal saline before infusion.

In a previous Phase I study of AP1903, 24 healthy volunteers were treated with single doses of AP1903 for Injection at dose levels of 0.01, 0.05, 0.1, 0.5 and 1.0 mg/kg infused IV over 2 hours. AP1903 plasma levels were directly proportional to dose, with mean Cmax values ranging from approximately 10 - 1275 ng/mL over the 0.01 - 1.0 mg/kg dose range. Following the initial infusion period, blood concentrations demonstrated a rapid distribution phase, with plasma levels reduced to approximately 18, 7, and 1% of maximal concentration at 0.5, 2 and 10 hours post-dose, respectively. AP1903 for Injection was shown to be safe and well tolerated at all dose levels and demonstrated a favorable pharmacokinetic profile. luliucci JD, et al., J Clin Pharmacol. 41: 870-9, 2001.

The fixed dose of AP1903 for injection used, for example, may be 0.4 mg/kg intravenously infused over 2 hours. The amount of AP1903 needed in vitro for effective signaling of cells is about 10 - 100 nM (MW: 1412 Da). This equates to 14 - 140 µg/L or ~0.014 - 0.14 mg/kg (1.4 - 140 µg/kg). The dosage may vary according to the application, and may, in certain examples, be more in the range of 0.1-10 nM, or in the range of 50-150 nM, 10-200 nM, 75-125 nM, 100-500 nM, 100-600 nM, 100-700 nM, 100-800 nM, or 100-900 nM. Doses up to 1 mg/kg were well-tolerated in the Phase I study of AP1903 described above.

### Membrane-targeting

A membrane-targeting sequence provides for transport of the chimeric protein to the cell surface membrane, where the same or other sequences can encode binding of the chimeric protein to the cell surface membrane. Molecules in association with cell membranes contain certain regions that facilitate the membrane association, and such regions can be incorporated into a chimeric protein molecule to generate membrane-targeted molecules. For example, some proteins contain sequences at the N-terminus or C-terminus that are acylated, and these acyl moieties facilitate membrane association. Such sequences are recognized by acyltransferases and often conform to a particular sequence motif. Certain acylation motifs are capable of being modified with a single acyl moiety (often followed by several positively charged residues (e.g. human c-Src: M-G-S-N-K-S-K-P-K-D-A-S-Q-R-R-R) to improve association with anionic lipid head groups) and others are capable of being modified with multiple acyl moieties. For example the N-terminal sequence of the protein tyrosine kinase Src can comprise a single myristoyl moiety. Dual acylation regions are located within the N-terminal regions of certain protein kinases, such as a subset of Src family members (e.g., Yes, Fyn, Lck) and G-protein alpha subunits. Such dual acylation regions often are located within the first eighteen amino acids of such proteins, and conform to the sequence motif Met-Gly-Cys-Xaa-Cys, where the Met is cleaved, the Gly is N-acylated and one of the Cys residues is S-acylated. The Gly often is myristoylated and a Cys can be palmitoylated. Acylation regions conforming to the sequence motif Cys-Ala-Ala-Xaa (so called "CAAX boxes"), which can modified with C15 or C10 isoprenyl moieties, from the C-terminus of G-protein gamma subunits and other proteins (e.g., World Wide Web address ebi.ac.uk/interpro/DisplaylproEntry?ac=IPR001230) also can be utilized. These and other acylation motifs include, for example, those discussed in Gauthier-Campbell et al., Molecular Biology of the Cell 15: 2205-2217 (2004); Glabati et al., Biochem. J. 303: 697-700 (1994) and Zlakine et al., J. Cell Science 110: 673-679 (1997), and can be incorporated in chimeric molecules to induce membrane localization. In certain embodiments, a native sequence from a protein containing an acylation motif is incorporated into a chimeric protein. For example, in some embodiments, an N-terminal portion of Lck, Fyn or Yes or a G-protein alpha subunit, such as the first twenty-five N-terminal amino acids or fewer from such proteins (e.g., about 5 to about 20 amino acids, about 10 to about 19 amino acids, or about 15 to about 19 amino acids of the native sequence with optional mutations), may be incorporated within the N-terminus of a chimeric protein. In certain embodiments, a C-terminal sequence of about 25 amino acids or less from a G-protein gamma subunit containing a CAAX box motif sequence (e.g., about 5 to about 20 amino acids, about 10 to about 18 amino acids, or about 15 to about 18 amino acids of the native sequence with optional mutations) can be linked to the C-terminus of a chimeric protein.

In some embodiments, an acyl moiety has a log p value of +1 to +6, and sometimes has a log p value of +3 to +4.5. Log p values are a measure of hydrophobicity and often are derived from octanol/water partitioning studies, in which molecules with higher hydrophobicity partition into octanol with higher frequency and are characterized as having a higher log p value. Log p values are published for a number of lipophilic molecules and log p values can be calculated using known partitioning processes (e.g., Chemical Reviews, Vol. 71, Issue 6, page 599, where entry 4493 shows lauric acid having a log p value of 4.2). Any acyl moiety can be linked to a peptide composition discussed above and tested for antimicrobial activity using known methods and those discussed hereafter. The acyl moiety sometimes is a C1-C20 alkyl, C2-C20 alkenyl, C2-C20 alkynyl, C3-C6 cycloalkyl, C1-C4 haloalkyl, C4-C12 cyclalkylalkyl, aryl, substituted aryl, or aryl (C1-C4) alkyl, for example. Any acyl-containing moiety sometimes is a fatty acid, and examples of fatty acid moieties are propyl (C3), butyl (C4), pentyl (C5), hexyl (C6), heptyl (C7), octyl (C8), nonyl (C9), decyl (C10), undecyl (C11), lauryl (C12), myristyl (C14), palmityl (C16), stearyl (C18), arachidyl (C20), behenyl (C22) and lignoceryl moieties (C24), and each moiety can contain 0, 1, 2, 3, 4, 5, 6, 7 or 8 unsaturations (i.e., double bonds). An acyl moiety sometimes is a lipid molecule, such as a phosphatidyl lipid (e.g., phosphatidyl serine, phosphatidyl inositol, phosphatidyl ethanolamine, phosphatidyl choline), sphingolipid (e.g., shingomyelin, sphingosine, ceramide, ganglioside, cerebroside), or modified versions thereof. In certain embodiments, one, two, three, four or five or more acyl moieties are linked to a membrane association region. Any membrane-targeting sequence can be employed that is functional in the host and may, or may not, be associated with one of the other domains of the chimeric protein. In some embodiments, such sequences include, but are not limited to myristoylation-targeting sequence, palmitoylation-targeting sequence, prenylation sequences (i.e., farnesylation, geranyl-geranylation, CAAX Box), protein-protein interaction motifs or transmembrane sequences (utilizing signal peptides) from receptors. Examples include those discussed in, for example, ten Klooster JP et al, Biology of the Cell (2007) 99, 1-12, Vincent, S., et al., Nature Biotechnology 21:936-40, 1098 (2003).

Additional protein domains exist that can increase protein retention at various membranes. For example, an ~ 120 amino acid pleckstrin homology (PH) domain is found in over 200 human proteins that are typically involved in intracellular signaling. PH domains can bind various phosphatidylinositol (PI) lipids within membranes (e.g. PI (3, 4,5)-P3, PI (3,4)-P2, PI (4,5)-P2) and thus play a key role in recruiting proteins to different membrane or cellular compartments. Often the phosphorylation state of PI lipids is regulated, such as by PI-3 kinase or PTEN, and thus, interaction of membranes with PH domains are not as stable as by acyl lipids.

### Control Regions

### 1. Promoters

The particular promoter employed to control the expression of a polynucleotide sequence of interest is not believed to be important, so long as it is capable of directing the expression of the polynucleotide in the targeted cell. Thus, where a human cell is targeted the polynucleotide sequence-coding region may, for example, be placed adjacent to and under the control of a promoter that is capable of being expressed in a human cell. Generally speaking, such a promoter might include either a human or viral promoter.

In various embodiments, the human cytomegalovirus (CMV) immediate early gene promoter, the SV40 early promoter, the Rous sarcoma virus long terminal repeat, β-actin, rat insulin promoter and glyceraldehyde-3-phosphate dehydrogenase can be used to obtain high-level expression of the coding sequence of interest. The use of other viral or mammalian cellular or bacterial phage promoters which are well known in the art to achieve expression of a coding sequence of interest is contemplated as well, provided that the levels of expression are sufficient for a given purpose. By employing a promoter with well-known properties, the level and pattern of expression of the protein of interest following transfection or transformation can be optimized.

Selection of a promoter that is regulated in response to specific physiologic or synthetic signals can permit inducible expression of the gene product. For example in the case where expression of a transgene, or transgenes when a multicistronic vector is utilized, is toxic to the cells in which the vector is produced in, it is desirable to prohibit or reduce expression of one or more of the transgenes. Examples of transgenes that are toxic to the producer cell line are pro-apoptotic and cytokine genes. Several inducible promoter systems are available for production of viral vectors where the transgene products are toxic (add in more inducible promoters).

The ecdysone system (Invitrogen, Carlsbad, CA) is one such system. This system is designed to allow regulated expression of a gene of interest in mammalian cells. It consists of a tightly regulated expression mechanism that allows virtually no basal level expression of the transgene, but over 200-fold inducibility. The system is based on the heterodimeric ecdysone receptor of Drosophila, and when ecdysone or an analog such as muristerone A binds to the receptor, the receptor activates a promoter to turn on expression of the downstream transgene high levels of mRNA transcripts are attained. In this system, both monomers of the heterodimeric receptor are constitutively expressed from one vector, whereas the ecdysone-responsive promoter, which drives expression of the gene of interest, is on another plasmid. Engineering of this type of system into the gene transfer vector of interest would therefore be useful. Cotransfection of plasmids containing the gene of interest and the receptor monomers in the producer cell line would then allow for the production of the gene transfer vector without expression of a potentially toxic transgene. At the appropriate time, expression of the transgene could be activated with ecdysone or muristeron A.

Another inducible system that may be useful is the Tet-Off^{™} or Tet-On^{™} system (Clontech, Palo Alto, CA) originally developed by Gossen and Bujard (Gossen and Bujard, Proc. Natl. Acad. Sci. USA, 89:5547-5551, 1992; Gossen et al., Science, 268:1766-1769, 1995). This system also allows high levels of gene expression to be regulated in response to tetracycline or tetracycline derivatives such as doxycycline. In the Tet-On^{™} system, gene expression is turned on in the presence of doxycycline, whereas in the Tet-Off^{™} system, gene expression is turned on in the absence of doxycycline. These systems are based on two regulatory elements derived from the tetracycline resistance operon of E. coli. The tetracycline operator sequence to which the tetracycline repressor binds and the tetracycline repressor protein. The gene of interest is cloned into a plasmid behind a promoter that has tetracycline-responsive elements present in it. A second plasmid contains a regulatory element called the tetracycline-controlled transactivator, which is composed, in the Tet-Off^{™} system, of the VP16 domain from the herpes simplex virus and the wild-type tetracycline repressor. Thus in the absence of doxycycline, transcription is constitutively on. In the Tet-On^{™} system, the tetracycline repressor is not wild type and in the presence of doxycycline activates transcription. For gene therapy vector production, the Tet-Off^{™} system may be used so that the producer cells could be grown in the presence of tetracycline or doxycycline and prevent expression of a potentially toxic transgene, but when the vector is introduced to the patient, the gene expression would be constitutively on.

In some circumstances, it is desirable to regulate expression of a transgene in a gene therapy vector. For example, different viral promoters with varying strengths of activity are utilized depending on the level of expression desired. In mammalian cells, the CMV immediate early promoter is often used to provide strong transcriptional activation. The CMV promoter is reviewed in Donnelly, J.J., et al., 1997. Annu. Rev. Immunol. 15:617-48. Modified versions of the CMV promoter that are less potent have also been used when reduced levels of expression of the transgene are desired. When expression of a transgene in hematopoietic cells is desired, retroviral promoters such as the LTRs from MLV or MMTV are often used. Other viral promoters that are used depending on the desired effect include SV40, RSV LTR, HIV-1 and HIV-2 LTR, adenovirus promoters such as from the E1A, E2A, or MLP region, AAV LTR, HSV-TK, and avian sarcoma virus.

Similarly tissue specific promoters are used to effect transcription in specific tissues or cells so as to reduce potential toxicity or undesirable effects to non-targeted tissues. These promoters may result in reduced expression compared to a stronger promoter such as the CMV promoter, but may also result in more limited expression, and immunogenicity. (Bojak, A., et al.,2002. Vaccine. 20:1975-79; Cazeaux, N., et al., 2002. Vaccine 20:3322-31). For example, tissue specific promoters such as the PSA associated promoter or prostate-specific glandular kallikrein, or the muscle creatine kinase gene may be used where appropriate.

Examples of tissue specific or differentiation specific promoters include, but are not limited to, the following: B29/CD79b (B cells); CD14 (monocytic cells); CD43 (leukocytes and platelets); CD45 (hematopoietic cells); CD68 (macrophages); desmin (muscle); elastase-1 (pancreatic acinar cells); endoglin (endothelial cells); fibronectin (differentiating cells, healing tissues); and Flt-1 (endothelial cells); GFAP (astrocytes).

In certain indications, it is desirable to activate transcription at specific times after administration of the gene therapy vector. This is done with such promoters as those that are hormone or cytokine regulatable. Cytokine and inflammatory protein responsive promoters that can be used include K and T kininogen (Kageyama et al., (1987) J. Biol. Chem., 262,2345-2351), c-fos, TNF-α, C-reactive protein (Arcone, et al., (1988) Nucl. Acids Res., 16(8), 3195-3207), haptoglobin (Oliviero et al., (1987) EMBO J., 6, 1905-1912), serum amyloid A2, C/EBP α, IL-1, IL-6 (Poli and Cortese, (1989) Proc. Nat'l Acad. Sci. USA, 86,8202-8206), Complement C3 (Wilson et al., (1990) Mol. Cell. Biol., 6181-6191), IL-8, α-1 acid glycoprotein (Prowse and Baumann, (1988) Mol Cell Biol, 8,42-51), α-1 antitrypsin, lipoprotein lipase (Zechner et al., Mol. Cell. Biol., 2394-2401, 1988), angiotensinogen (Ron, et al., (1991) Mol. Cell. Biol., 2887-2895), fibrinogen, c-jun (inducible by phorbol esters, TNF-α, UV radiation, retinoic acid, and hydrogen peroxide), collagenase (induced by phorbol esters and retinoic acid), metallothionein (heavy metal and glucocorticoid inducible), Stromelysin (inducible by phorbol ester, interleukin-1 and EGF), α-2 macroglobulin and α-1 anti-chymotrypsin. Other promoters include, for example, SV40, MMTV, Human Immunodeficiency Virus (MV), Moloney virus, ALV, Epstein Barr virus, Rous Sarcoma virus, human actin, myosin, hemoglobin, and creatine.

It is envisioned that any of the above promoters alone or in combination with another can be useful depending on the action desired. Promoters, and other regulatory elements, are selected such that they are functional in the desired cells or tissue. In addition, this list of promoters should not be construed to be exhaustive or limiting; other promoters that are used in conjunction with the promoters and methods disclosed herein.

### 2. Enhancers

Enhancers are genetic elements that increase transcription from a promoter located at a distant position on the same molecule of DNA. Early examples include the enhancers associated with immunoglobulin and T cell receptors that both flank the coding sequence and occur within several introns. Many viral promoters, such as CMV, SV40, and retroviral LTRs are closely associated with enhancer activity and are often treated like single elements. Enhancers are organized much like promoters. That is, they are composed of many individual elements, each of which binds to one or more transcriptional proteins. The basic distinction between enhancers and promoters is operational. An enhancer region as a whole stimulates transcription at a distance and often independent of orientation; this need not be true of a promoter region or its component elements. On the other hand, a promoter has one or more elements that direct initiation of RNA synthesis at a particular site and in a particular orientation, whereas enhancers lack these specificities. Promoters and enhancers are often overlapping and contiguous, often seeming to have a very similar modular organization. A subset of enhancers includes locus-control regions (LCRs) that can not only increase transcriptional activity, but (along with insulator elements) can also help to insulate the transcriptional element from adjacent sequences when integrated into the genome.

Any promoter/enhancer combination (as per the Eukaryotic Promoter Data Base EPDB) can be used to drive expression of the gene, although many will restrict expression to a particular tissue type or subset of tissues. (Reviewed in, for example, Kutzler, M.A., and Weiner, D.B., 2008. Nature Reviews Genetics 9:776-88). Examples include, but are not limited to, enhancers from the human actin, myosin, hemoglobin, muscle creatine kinase, sequences, and from viruses CMV, RSV, and EBV. Appropriate enhancers may be selected for particular applications. Eukaryotic cells can support cytoplasmic transcription from certain bacterial promoters if the appropriate bacterial polymerase is provided, either as part of the delivery complex or as an additional genetic expression construct.

### 3. Polyadenylation Signals

Where a cDNA insert is employed, one will typically desire to include a polyadenylation signal to effect proper polyadenylation of the gene transcript. The nature of the polyadenylation signal is not believed to be crucial to the successful practice of the present methods, and any such sequence is employed such as human or bovine growth hormone and SV40 polyadenylation signals and LTR polyadenylation signals. One non-limiting example is the SV40 polyadenylation signal present in the pCEP3 plasmid (Invitrogen, Carlsbad, California). Also contemplated as an element of the expression cassette is a terminator. These elements can serve to enhance message levels and to minimize read through from the cassette into other sequences. Termination or poly(A) signal sequences may be, for example, positioned about 11-30 nucleotides downstream from a conserved sequence (AAUAAA) at the 3' end of the mRNA. (Montgomery, D.L., et al., 1993. DNA Cell Biol. 12:777-83; Kutzler, M.A., and Weiner, D.B., 2008. Nature Rev. Gen. 9:776-88).

### 4. Initiation Signals and Internal Ribosome Binding Sites

A specific initiation signal also may be required for efficient translation of coding sequences. These signals include the ATG initiation codon or adjacent sequences. Exogenous translational control signals, including the ATG initiation codon, may need to be provided. The initiation codon is placed in-frame with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments, the use of internal ribosome entry sites (IRES) elements is used to create multigene, or polycistronic messages. IRES elements are able to bypass the ribosome-scanning model of 5' methylated cap-dependent translation and begin translation at internal sites (Pelletier and Sonenberg, Nature, 334:320-325, 1988). IRES elements from two members of the picornavirus family (polio and encephalomyocarditis) have been discussed (Pelletier and Sonenberg, 1988), as well an IRES from a mammalian message (Macejak and Sarnow, Nature, 353:90-94, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819)

### Sequence Optimization

Protein production may also be increased by optimizing the codons in the transgene. Species specific codon changes may be used to increase protein production. Also, codons may be optimized to produce an optimized RNA, which may result in more efficient translation. By optimizing the codons to be incorporated in the RNA, elements such as those that result in a secondary structure that causes instability, secondary mRNA structures that can, for example, inhibit ribosomal binding, or cryptic sequences that can inhibit nuclear export of mRNA can be removed. (Kutzler, M.A., and Weiner, D.B., 2008. Nature Rev. Gen. 9:776-88; Yan, J. et al., 2007. Mol. Ther. 15:411-21; Cheung, Y.K., et al., 2004. Vaccine 23:629-38; Narum, D.L., et al., 2001. 69:7250-55; Yadava, A., and Ockenhouse, C.F., 2003. Infect. Immun. 71:4962-69; Smith, J.M., et al., 2004. AIDS Res. Hum. Retroviruses 20:1335-47; Zhou, W., et al., 2002. Vet. Microbiol. 88:127-51; Wu, X., et al., 2004. Biochem. Biophys. Res. Commun. 313:89-96; Zhang, W., et al., 2006. Biochem. Biophys. Res. Commun. 349:69-78; Deml, L.A., et al., 2001. J. Virol. 75:1099-11001; Schneider, R. M., et al., 1997. J. Virol. 71:4892-4903; Wang, S.D., et al., 2006. Vaccine 24:4531-40; zur Megede, J., et al., 2000. J. Virol. 74:2628-2635). For example, the FKBP12 or other multimerizing region polypeptide, Caspase-9 polypeptide, and the TCR polypeptide-encoding polynucleotide sequences may be optimized by changes in the codons.

### Leader Sequences

Leader sequences may be added to enhance the stability of mRNA and result in more efficient translation. The leader sequence is usually involved in targeting the mRNA to the endoplasmic reticulum. Examples include the signal sequence for the HIV-1 envelope glycoprotein (Env), which delays its own cleavage, and the IgE gene leader sequence (Kutzler, M.A., and Weiner, D.B., 2008. Nature Rev. Gen. 9:776-88; Li, V., et al., 2000. Virology 272:417-28; Xu, Z.L., et al. 2001. Gene 272:149-56; Malin, A.S., et al., 2000. Microbes Infect. 2:1677-85; Kutzler, M.A., et al., 2005. J. Immunol. 175:112-125; Yang, J.S., et al., 2002. Emerg. Infect. Dis. 8:1379-84; Kumar, S., et al., 2006. DNA Cell Biol. 25:383-92; Wang, S., et al, 2006. Vaccine 24:4531-40). The IgE leader may be used to enhance insertion into the endoplasmic reticulum (Tepler, I, et al. (1989) J. Biol. Chem. 264:5912).

Expression of the transgenes may be optimized and/or controlled by the selection of appropriate methods for optimizing expression. These methods include, for example, optimizing promoters, delivery methods, and gene sequences, (for example, as presented in Laddy, D.J., et al., 2008. PLoS.ONE 3 e2517; Kutzler, M.A., and Weiner, D.B., 2008. Nature Rev. Gen. 9:776-88).

### Nucleic Acids

A "nucleic acid" as used herein generally refers to a molecule (one, two or more strands) of DNA, RNA or a derivative or analog thereof, comprising a nucleobase. A nucleobase includes, for example, a naturally occurring purine or pyrimidine base found in DNA (e.g., an adenine "A," a guanine "G," a thymine "T" or a cytosine "C") or RNA (e.g., an A, a G, an uracil "U" or a C). The term "nucleic acid" encompasses the terms "oligonucleotide" and "polynucleotide," each as a subgenus of the term "nucleic acid." Nucleic acids may be, be at least, be at most, or be about 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides, or any range derivable therein, in length.

Nucleic acids herein provided may have regions of identity or complementarity to another nucleic acid. It is contemplated that the region of complementarity or identity can be at least 5 contiguous residues, though it is specifically contemplated that the region is, is at least, is at most, or is about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 contiguous nucleotides.

As used herein, "hybridization", "hybridizes" or "capable of hybridizing" is understood to mean forming a double or triple stranded molecule or a molecule with partial double or triple stranded nature. The term "anneal" as used herein is synonymous with "hybridize." The term "hybridization", "hybridize(s)" or "capable of hybridizing" encompasses the terms "stringent condition(s)" or "high stringency" and the terms "low stringency" or "low stringency condition(s)."

As used herein "stringent condition(s)" or "high stringency" are those conditions that allow hybridization between or within one or more nucleic acid strand(s) containing complementary sequence(s), but preclude hybridization of random sequences. Stringent conditions tolerate little, if any, mismatch between a nucleic acid and a target strand. Such conditions are known, and are often used for applications requiring high selectivity. Non-limiting applications include isolating a nucleic acid, such as a gene or a nucleic acid segment thereof, or detecting at least one specific mRNA transcript or a nucleic acid segment thereof, and the like.

Stringent conditions may comprise low salt and/or high temperature conditions, such as provided by about 0.02 M to about 0.5 M NaCl at temperatures of about 42 degrees C to about 70 degrees C. It is understood that the temperature and ionic strength of a desired stringency are determined in part by the length of the particular nucleic acid(s), the length and nucleobase content of the target sequence(s), the charge composition of the nucleic acid(s), and the presence or concentration of formamide, tetramethylammonium chloride or other solvent(s) in a hybridization mixture.

It is understood that these ranges, compositions and conditions for hybridization are mentioned by way of non-limiting examples only, and that the desired stringency for a particular hybridization reaction is often determined empirically by comparison to one or more positive or negative controls. Depending on the application envisioned varying conditions of hybridization may be employed to achieve varying degrees of selectivity of a nucleic acid towards a target sequence. In a non-limiting example, identification or isolation of a related target nucleic acid that does not hybridize to a nucleic acid under stringent conditions may be achieved by hybridization at low temperature and/or high ionic strength. Such conditions are termed "low stringency" or "low stringency conditions," and non-limiting examples of low stringency include hybridization performed at about 0.15 M to about 0.9 M NaCl at a temperature range of about 20 degrees C. to about 50 degrees C. The low or high stringency conditions may be further modified to suit a particular application.

"Function-conservative variants" are proteins or enzymes in which a given amino acid residue has been changed without altering overall conformation and function of the protein or enzyme, including, but not limited to, replacement of an amino acid with one having similar properties, including polar or non-polar character, size, shape and charge. Conservative amino acid substitutions for many of the commonly known non-genetically encoded amino acids are well known in the art. Conservative substitutions for other non-encoded amino acids can be determined based on their physical properties as compared to the properties of the genetically encoded amino acids.

Amino acids other than those indicated as conserved may differ in a protein or enzyme so that the percent protein or amino acid sequence similarity between any two proteins of similar function may vary and can be, for example, at least 70%, at least 80%, at least 90%, and, for example, at least 95%, as determined according to an alignment scheme. As referred to herein, "sequence similarity" means the extent to which nucleotide or protein sequences are related. The extent of similarity between two sequences can be based on percent sequence identity and/or conservation. "Sequence identity" herein means the extent to which two nucleotide or amino acid sequences are invariant. "Sequence alignment" means the process of lining up two or more sequences to achieve maximal levels of identity (and, in the case of amino acid sequences, conservation) for the purpose of assessing the degree of similarity. Numerous methods for aligning sequences and assessing similarity/identity are known in the art such as, for example, the Cluster Method, wherein similarity is based on the MEGALIGN algorithm, as well as BLASTN, BLASTP, and FASTA. When using any of these programs, the settings used are those that results in the highest sequence similarity.

### Nucleic Acid Modification

Any of the modifications discussed below may be applied to a nucleic acid. Examples of modifications include alterations to the RNA or DNA backbone, sugar or base, and various combinations thereof. Any suitable number of backbone linkages, sugars and/or bases in a nucleic acid can be modified (e.g., independently about 5%, 10%, 15%, 20%, 25%, 30%, 35%, 40%, 45%, 50%, 55%, 60%, 65%, 70%, 75%, 80%, 85%, 90%, 95%, up to 100%). An unmodified nucleoside is any one of the bases adenine, cytosine, guanine, thymine, or uracil joined to the 1' carbon of β-D-ribo-furanose.

A modified base is a nucleotide base other than adenine, guanine, cytosine and uracil at a 1' position. Non-limiting examples of modified bases include inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (e. g., 5-methylcytidine), 5-alkyluridines (e. g., ribothymidine), 5-halouridine (e. g., 5-bromouridine) or 6-azapyrimidines or 6-alkylpyrimidines (e. g. 6- methyluridine), propyne, and the like. Other non-limiting examples of modified bases include nitropyrrolyl (e.g., 3-nitropyrrolyl), nitroindolyl (e.g., 4-, 5-, 6-nitroindolyl), hypoxanthinyl, isoinosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, difluorotolyl, 4-fluoro-6-methylbenzimidazole, 4-methylbenzimidazole, 3-methyl isocarbostyrilyl, 5-methyl isocarbostyrilyl, 3-methyl-7-propynyl isocarbostyrilyl, 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenyl, tetracenyl, pentacenyl and the like.

In some embodiments, for example, a nucleid acid may comprise modified nucleic acid molecules, with phosphate backbone modifications. Non-limiting examples of backbone modifications include phosphorothioate, phosphorodithioate, methylphosphonate, phosphotriester, morpholino, amidate carbamate, carboxymethyl, acetamidate, polyamide, sulfonate, sulfonamide, sulfamate, formacetal, thioformacetal, and/or alkylsilyl modifications. In certain instances, a ribose sugar moiety that naturally occurs in a nucleoside is replaced with a hexose sugar, polycyclic heteroalkyl ring, or cyclohexenyl group. In certain instances, the hexose sugar is an allose, altrose, glucose, mannose, gulose, idose, galactose, talose, or a derivative thereof. The hexose may be a D-hexose, glucose, or mannose. In certain instances, the polycyclic heteroalkyl group may be a bicyclic ring containing one oxygen atom in the ring. In certain instances, the polycyclic heteroalkyl group is a bicyclo[2.2.1]heptane, a bicyclo[3.2.1]octane, or a bicyclo[3.3.1]nonane.

Nitropyrrolyl and nitroindolyl nucleobases are members of a class of compounds known as universal bases. Universal bases are those compounds that can replace any of the four naturally occurring bases without substantially affecting the melting behavior or activity of the oligonucleotide duplex. In contrast to the stabilizing, hydrogen-bonding interactions associated with naturally occurring nucleobases, oligonucleotide duplexes containing 3-nitropyrrolyl nucleobases may be stabilized solely by stacking interactions. The absence of significant hydrogen-bonding interactions with nitropyrrolyl nucleobases obviates the specificity for a specific complementary base. In addition, 4-, 5- and 6-nitroindolyl display very little specificity for the four natural bases. Procedures for the preparation of 1-(2'-O-methyl-.β.-D-ribofuranosyl)-5-nitroindole are discussed in Gaubert, G.; Wengel, J. Tetrahedron Letters 2004, 45, 5629. Other universal bases include hypoxanthinyl, isoinosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, and structural derivatives thereof.

Difluorotolyl is a non-natural nucleobase that functions as a universal base. Difluorotolyl is an isostere of the natural nucleobase thymine. But unlike thymine, difluorotolyl shows no appreciable selectivity for any of the natural bases. Other aromatic compounds that function as universal bases are 4-fluoro-6-methylbenzimidazole and 4-methylbenzimidazole. In addition, the relatively hydrophobic isocarbostyrilyl derivatives 3-methyl isocarbostyrilyl, 5-methyl isocarbostyrilyl, and 3-methyl-7-propynyl isocarbostyrilyl are universal bases which cause only slight destabilization of oligonucleotide duplexes compared to the oligonucleotide sequence containing only natural bases. Other non-natural nucleobases include 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenyl, tetracenyl, pentacenyl, and structural derivatives thereof. For a more detailed discussion, including synthetic procedures, of difluorotolyl, 4-fluoro-6-methylbenzimidazole, 4-methylbenzimidazole, and other non-natural bases mentioned above, see: Schweitzer et al., J. Org. Chem., 59:7238-7242 (1994);

In addition, chemical substituents, for example cross-linking agents, may be used to add further stability or irreversibility to the reaction. Non-limiting examples of cross-linking agents include, for example, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl) dithio]propioimidate.

A nucleotide analog may also include a "locked" nucleic acid. Certain compositions can be used to essentially "anchor" or "lock" an endogenous nucleic acid into a particular structure. Anchoring sequences serve to prevent disassociation of a nucleic acid complex, and thus not only can prevent copying but may also enable labeling, modification, and/or cloning of the endogenous sequence. The locked structure may regulate gene expression (i.e. inhibit or enhance transcription or replication), or can be used as a stable structure that can be used to label or otherwise modify the endogenous nucleic acid sequence, or can be used to isolate the endogenous sequence, i.e. for cloning.

Nucleic acid molecules need not be limited to those molecules containing only RNA or DNA, but further encompass chemically modified nucleotides and non-nucleotides. The percent of non-nucleotides or modified nucleotides may be from 1% to 100% (e.g., about 5, 10, 15, 20, 25, 30, 35, 40, 45, 50, 55, 60, 65, 70, 75, 80, 85, 90 or 95%).

### Nucleic Acid Preparation

In some embodiments, a nucleic acid is provided for use as a control or standard in an assay, or therapeutic, for example. A nucleic acid may be made by any technique known in the art, such as for example, chemical synthesis, enzymatic production or biological production. Nucleic acids may be recovered or isolated from a biological sample. The nucleic acid may be recombinant or it may be natural or endogenous to the cell (produced from the cell's genome). It is contemplated that a biological sample may be treated in a way so as to enhance the recovery of small nucleic acid molecules. Generally, methods may involve lysing cells with a solution having guanidinium and a detergent.

Nucleic acid synthesis may also be performed according to standard methods. Non-limiting examples of a synthetic nucleic acid (e.g., a synthetic oligonucleotide), include a nucleic acid made by in vitro chemical synthesis using phosphotriester, phosphite, or phosphoramidite chemistry and solid phase techniques or via deoxynucleoside H-phosphonate intermediates. Various different mechanisms of oligonucleotide synthesis have been disclosed elsewhere.

Nucleic acids may be isolated using known techniques. In particular embodiments, methods for isolating small nucleic acid molecules, and/or isolating RNA molecules can be employed. Chromatography is a process used to separate or isolate nucleic acids from protein or from other nucleic acids. Such methods can involve electrophoresis with a gel matrix, filter columns, alcohol precipitation, and/or other chromatography. If a nucleic acid from cells is to be used or evaluated, methods generally involve lysing the cells with a chaotropic (e.g., guanidinium isothiocyanate) and/or detergent (e.g., N-lauroyl sarcosine) prior to implementing processes for isolating particular populations of RNA.

Methods may involve the use of organic solvents and/or alcohol to isolate nucleic acids. In some embodiments, the amount of alcohol added to a cell lysate achieves an alcohol concentration of about 55% to 60%. While different alcohols can be employed, ethanol works well. A solid support may be any structure, and it includes beads, filters, and columns, which may include a mineral or polymer support with electronegative groups. A glass fiber filter or column is effective for such isolation procedures.

A nucleic acid isolation processes may sometimes include: a) lysing cells in the sample with a lysing solution comprising guanidinium, where a lysate with a concentration of at least about 1 M guanidinium is produced; b) extracting nucleic acid molecules from the lysate with an extraction solution comprising phenol; c) adding to the lysate an alcohol solution for form a lysate/alcohol mixture, wherein the concentration of alcohol in the mixture is between about 35% to about 70%; d) applying the lysate/alcohol mixture to a solid support; e) eluting the nucleic acid molecules from the solid support with an ionic solution; and, f) capturing the nucleic acid molecules. The sample may be dried down and resuspended in a liquid and volume appropriate for subsequent manipulation.

### Methods of Gene Transfer

In order to mediate the effect of the transgene expression in a cell, it will be necessary to transfer the expression constructs into a cell. Such transfer may employ viral or non-viral methods of gene transfer. This section provides a discussion of methods and compositions of gene transfer.

A transformed cell comprising an expression vector is generated by introducing into the cell the expression vector. Suitable methods for polynucleotide delivery for transformation of an organelle, a cell, a tissue or an organism for use with the current methods include virtually any method by which a polynucleotide (e.g., DNA) can be introduced into an organelle, a cell, a tissue or an organism.

A host cell can, and has been, used as a recipient for vectors. Host cells may be derived from prokaryotes or eukaryotes, depending upon whether the desired result is replication of the vector or expression of part or all of the vector-encoded polynucleotide sequences. Numerous cell lines and cultures are available for use as a host cell, and they can be obtained through the American Type Culture Collection (ATCC), which is an organization that serves as an archive for living cultures and genetic materials. In specific embodiments, the host cell is a T cell, a tumor-infiltrating lymphocyte, a natural killer cell, or a natural killer T cell.

An appropriate host may be determined. Generally this is based on the vector backbone and the desired result. A plasmid or cosmid, for example, can be introduced into a prokaryote host cell for replication of many vectors. Bacterial cells used as host cells for vector replication and/or expression include DH5α, JM109, and KC8, as well as a number of commercially available bacterial hosts such as SURE^{®} Competent cells and SOLOPACK Gold Cells (STRATAGENE^{®}, La Jolla, CA). Alternatively, bacterial cells such as E. coli LE392 could be used as host cells for phage viruses. Eukaryotic cells that can be used as host cells include, but are not limited to yeast, insects and mammals. Examples of mammalian eukaryotic host cells for replication and/or expression of a vector include, but are not limited to, HeLa, NIH3T3, Jurkat, 293, COS, CHO, Saos, and PC12. Examples of yeast strains include, but are not limited to, YPH499, YPH500 and YPH501.

Nucleic acid vaccines may include, for example, non-viral DNA vectors, "naked" DNA and RNA, and viral vectors. Methods of transforming cells with these vaccines, and for optimizing the expression of genes included in these vaccines are known and are also discussed herein.

### Examples of Methods of Nucleic Acid or Viral Vector Transfer

Any appropriate method may be used to transfect or transform the cells, for example, the T cells, or to administer the nucleotide sequences or compositions of the present methods. Certain examples are presented herein, and further include methods such as delivery using cationic polymers, lipid like molecules, and certain commercial products such as, for example, IN-VIVO-JET PEI.

### 1. Ex vivo Transformation

Various methods are available for transfecting vascular cells and tissues removed from an organism in an ex vivo setting. For example, canine endothelial cells have been genetically altered by retroviral gene transfer in vitro and transplanted into a canine (Wilson et al., Science, 244:1344-1346, 1989). In another example, Yucatan minipig endothelial cells were transfected by retrovirus in vitro and transplanted into an artery using a double-balloon catheter (Nabel et al., Science, 244(4910):1342-1344, 1989). Thus, it is contemplated that cells or tissues may be removed and transfected ex vivo using the polynucleotides presented herein. In particular aspects, the transplanted cells or tissues may be placed into an organism. For example, T cells may be obtained from an animal, the cells transfected or transformed with the expression vector and then administered back to the animal.

### 2. Injection

In certain embodiments, a cell or a nucleic acid or viral vector may be delivered to an organelle, a cell, a tissue or an organism via one or more injections (i.e., a needle injection), such as, for example, subcutaneous, intradermal, intramuscular, intravenous, intraprotatic, intratumor, intraperitoneal, etc. Methods of injection include, for example, injection of a composition comprising a saline solution. Further embodiments include the introduction of a polynucleotide by direct microinjection. The amount of the expression vector used may vary upon the nature of the antigen as well as the organelle, cell, tissue or organism used.

Intradermal, intranodal, or intralymphatic injections are some of the more commonly used methods of DC administration. Intradermal injection is characterized by a low rate of absorption into the bloodstream but rapid uptake into the lymphatic system. The presence of large numbers of Langerhans dendritic cells in the dermis will transport intact as well as processed antigen to draining lymph nodes. Proper site preparation is necessary to perform this correctly (i.e., hair is clipped in order to observe proper needle placement). Intranodal injection allows for direct delivery of antigen to lymphoid tissues. Intralymphatic injection allows direct administration of DCs.

### 3. Electroporation

In certain embodiments, a polynucleotide is introduced into an organelle, a cell, a tissue or an organism via electroporation. Electroporation involves the exposure of a suspension of cells and DNA to a high-voltage electric discharge. In some variants of this method, certain cell wall-degrading enzymes, such as pectin-degrading enzymes, are employed to render the target recipient cells more susceptible to transformation by electroporation than untreated cells (U.S. Patent No. 5,384,253 ).

Transfection of eukaryotic cells using electroporation has been quite successful. Mouse pre-B lymphocytes have been transfected with human κ-immunoglobulin genes (Potter et al., (1984) Proc. Nat'l Acad. Sci. USA, 81, 7161-7165), and rat hepatocytes have been transfected with the chloramphenicol acetyltransferase gene (Tur-Kaspa et al., (1986) Mol. Cell Biol., 6,716-718) in this manner.

*In vivo* electroporation for vaccines, or eVac, is clinically implemented through a simple injection technique. A DNA vector encoding tumor antigen is injected intradermally in a patient. Then electrodes apply electrical pulses to the intradermal space causing the cells localized there, especially resident dermal dendritic cells, to take up the DNA vector and express the encoded tumor antigen. These tumor antigen-expressing dendritic cells activated by local inflammation can then migrate to lymph-nodes, presenting tumor antigens and priming tumor antigen-specific T cells. A nucleic acid is electroporetically administered when it is administered using electroporation, following, for example, but not limited to, injection of the nucleic acid or any other means of administration where the nucleic acid may be delivered to the cells by electroporation

Methods of electroporation are discussed in, for example, Sardesai, N.Y., and Weiner, D.B., Current Opinion in Immunotherapy 23:421-9 (2011) and Ferraro, B. et al., Human Vaccines 7:120-127 (2011).

### 4. Calcium Phosphate

In other embodiments, a polynucleotide is introduced to the cells using calcium phosphate precipitation. Human KB cells have been transfected with adenovirus 5 DNA (Graham and van der Eb, (1973) Virology, 52,456-467) using this technique. Also in this manner, mouse L(A9), mouse C127, CHO, CV-1, BHK, NIH3T3 and HeLa cells were transfected with a neomycin marker gene (Chen and Okayama, Mol. Cell Biol., 7(8):2745-2752, 1987), and rat hepatocytes were transfected with a variety of marker genes (Rippe et al., Mol. Cell Biol., 10:689-695, 1990).

### 5. DEAE-Dextran

In another embodiment, a polynucleotide is delivered into a cell using DEAE-dextran followed by polyethylene glycol. In this manner, reporter plasmids were introduced into mouse myeloma and erythroleukemia cells (Gopal, T.V., Mol Cell Biol. 1985 May;5(5):1188-90).

### 6. Sonication Loading

Additional embodiments include the introduction of a polynucleotide by direct sonic loading. LTK- fibroblasts have been transfected with the thymidine kinase gene by sonication loading (Fechheimer et al., (1987) Proc. Nat'l Acad. Sci. USA, 84, 8463-8467).

### 7. Liposome-Mediated Transfection

In a further embodiment, a polynucleotide may be entrapped in a lipid complex such as, for example, a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo selfrearrangement before the formation of closed structures and entrap water and dissolved solutes between the lipid bilayers (Ghosh and Bachhawat, (1991) In: Liver Diseases, Targeted Diagnosis and Therapy Using Specific Receptors and Ligands. pp. 87-104). Also contemplated is a polynucleotide complexed with Lipofectamine (Gibco BRL) or Superfect (Qiagen).

### 8. Receptor-Mediated Transfection

Still further, a polynucleotide may be delivered to a target cell via receptor-mediated delivery vehicles. These take advantage of the selective uptake of macromolecules by receptor-mediated endocytosis that will be occurring in a target cell. In view of the cell type-specific distribution of various receptors, this delivery method adds another degree of specificity. Certain receptor-mediated gene targeting vehicles comprise a cell receptor-specific ligand and a polynucleotide-binding agent. Others comprise a cell receptor-specific ligand to which the polynucleotide to be delivered has been operatively attached. Several ligands have been used for receptor-mediated gene transfer (Wu and Wu, (1987) J. Biol. Chem., 262, 4429-4432; Wagner et al., Proc. Natl. Acad. Sci. USA, 87(9):3410-3414, 1990; Perales et al., Proc. Natl. Acad. Sci. USA, 91:4086-4090, 1994; Myers, EPO 0273085), which establishes the operability of the technique. Specific delivery in the context of another mammalian cell type has been discussed (Wu and Wu, Adv. Drug Delivery Rev., 12:159-167, 1993).

In certain aspects, a ligand is chosen to correspond to a receptor specifically expressed on the target cell population.

In other embodiments, a polynucleotide delivery vehicle component of a cell-specific polynucleotide-targeting vehicle may comprise a specific binding ligand in combination with a liposome. The polynucleotide(s) to be delivered are housed within the liposome and the specific binding ligand is functionally incorporated into the liposome membrane. The liposome will thus specifically bind to the receptor(s) of a target cell and deliver the contents to a cell. Such systems have been shown to be functional using systems in which, for example, epidermal growth factor (EGF) is used in the receptor-mediated delivery of a polynucleotide to cells that exhibit upregulation of the EGF receptor.

In still further embodiments, the polynucleotide delivery vehicle component of a targeted delivery vehicle may be a liposome itself, which may, for example, comprise one or more lipids or glycoproteins that direct cell-specific binding. For example, lactosyl-ceramide, a galactoseterminal asialoganglioside, have been incorporated into liposomes and observed an increase in the uptake of the insulin gene by hepatocytes (Nicolau et al., (1987) Methods Enzymol., 149,157-176). It is contemplated that the tissue-specific transforming constructs may be specifically delivered into a target cell in a similar manner.

### 9. Microprojectile Bombardment

Microprojectile bombardment techniques can be used to introduce a polynucleotide into at least one, organelle, cell, tissue or organism (U.S. Patent No. 5,550,318; U.S. Patent No. 5,538,880; U.S. Patent No. 5,610,042; and PCT Application WO 94/09699).

This method depends on the ability to accelerate DNA-coated microprojectiles to a high velocity allowing them to pierce cell membranes and enter cells without killing them (Klein et al., (1987) Nature, 327, 70-73). There are a wide variety of microprojectile bombardment techniques known in the art, many of which are applicable to the present methods.

In this microprojectile bombardment, one or more particles may be coated with at least one polynucleotide and delivered into cells by a propelling force. Several devices for accelerating small particles have been developed. One such device relies on a high voltage discharge to generate an electrical current, which in turn provides the motive force (Yang et al., (1990) Proc. Nat'l Acad. Sci. USA, 87, 9568-9572). The microprojectiles used have consisted of biologically inert substances such as tungsten or gold particles or beads. Exemplary particles include those comprised of tungsten, platinum, and, in certain examples, gold, including, for example, nanoparticles. It is contemplated that in some instances DNA precipitation onto metal particles would not be necessary for DNA delivery to a recipient cell using microprojectile bombardment. However, it is contemplated that particles may contain DNA rather than be coated with DNA. DNA-coated particles may increase the level of DNA delivery via particle bombardment but are not necessary.

### 10. Transposon-mediated Transfer

Transposon-mediated transfer methods may also be employed using, for example, the piggy/Bac gene transfer system. Sato, M., et al., Biotechnol J. 2014 Oct 24. doi: 10.1002/biot.201400283. [Epub ahead of print].

### Examples of Methods of Viral Vector-Mediated Transfer

Any viral vector suitable for administering nucleotide sequences, or compositions comprising nucleotide sequences, to a cell or to a subject, such that the cell or cells in the subject may express the genes encoded by the nucleotide sequences may be employed in the present methods. In certain embodiments, a transgene is incorporated into a viral particle to mediate gene transfer to a cell. Typically, the virus simply will be exposed to the appropriate host cell under physiologic conditions, permitting uptake of the virus. The present methods are advantageously employed using a variety of viral vectors, as discussed below.

### 1. Adenovirus

Adenovirus is particularly suitable for use as a gene transfer vector because of its mid-sized DNA genome, ease of manipulation, high titer, wide target-cell range, and high infectivity. The roughly 36 kb viral genome is bounded by 100-200 base pair (bp) inverted terminal repeats (ITR), in which are contained cis-acting elements necessary for viral DNA replication and packaging. The early (E) and late (L) regions of the genome that contain different transcription units are divided by the onset of viral DNA replication.

The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression, and host cell shut off (Renan, M. J. (1990) Radiother Oncol., 19, 197-218). The products of the late genes (L1, L2, L3, L4 and L5), including the majority of the viral capsid proteins, are expressed only after significant processing of a single primary transcript issued by the major late promoter (MLP). The MLP (located at 16.8 map units) is particularly efficient during the late phase of infection, and all the mRNAs issued from this promoter possess a 5' tripartite leader (TL) sequence, which makes them useful for translation.

In order for adenovirus to be optimized for gene therapy, it is necessary to maximize the carrying capacity so that large segments of DNA can be included. It also is very desirable to reduce the toxicity and immunologic reaction associated with certain adenoviral products. The two goals are, to an extent, coterminous in that elimination of adenoviral genes serves both ends. By practice of the present methods, it is possible to achieve both these goals while retaining the ability to manipulate the therapeutic constructs with relative ease.

The large displacement of DNA is possible because the cis elements required for viral DNA replication all are localized in the inverted terminal repeats (ITR) (100-200 bp) at either end of the linear viral genome. Plasmids containing ITR's can replicate in the presence of a nondefective adenovirus (Hay, R.T., et al., J Mol Biol. 1984 Jun 5;175(4):493-510). Therefore, inclusion of these elements in an adenoviral vector may permit replication.

In addition, the packaging signal for viral encapsulation is localized between 194-385 bp (0.5-1.1 map units) at the left end of the viral genome (Hearing et al., J. (1987) Virol., 67, 2555-2558). This signal mimics the protein recognition site in bacteriophage lambda DNA where a specific sequence close to the left end, but outside the cohesive end sequence, mediates the binding to proteins that are required for insertion of the DNA into the head structure. E1 substitution vectors of Ad have demonstrated that a 450 bp (0-1.25 map units) fragment at the left end of the viral genome could direct packaging in 293 cells (Levrero et al., Gene, 101:195-202, 1991).

Previously, it has been shown that certain regions of the adenoviral genome can be incorporated into the genome of mammalian cells and the genes encoded thereby expressed. These cell lines are capable of supporting the replication of an adenoviral vector that is deficient in the adenoviral function encoded by the cell line. There also have been reports of complementation of replication deficient adenoviral vectors by "helping" vectors, e.g., wild-type virus or conditionally defective mutants.

Replication-deficient adenoviral vectors can be complemented, in trans, by helper virus. This observation alone does not permit isolation of the replication-deficient vectors, however, since the presence of helper virus, needed to provide replicative functions, would contaminate any preparation. Thus, an additional element was needed that would add specificity to the replication and/or packaging of the replication-deficient vector. That element derives from the packaging function of adenovirus.

It has been shown that a packaging signal for adenovirus exists in the left end of the conventional adenovirus map (Tibbetts et. al. (1977) Cell, 12,243-249). Later studies showed that a mutant with a deletion in the E1A (194-358 bp) region of the genome grew poorly even in a cell line that complemented the early (E1A) function (Hearing and Shenk, (1983) J. Mol. Biol. 167,809-822). When a compensating adenoviral DNA (0-353 bp) was recombined into the right end of the mutant, the virus was packaged normally. Further mutational analysis identified a short, repeated, position-dependent element in the left end of the Ad5 genome. One copy of the repeat was found to be sufficient for efficient packaging if present at either end of the genome, but not when moved toward the interior of the Ad5 DNA molecule (Hearing et al., J. (1987) Virol., 67, 2555-2558).

By using mutated versions of the packaging signal, it is possible to create helper viruses that are packaged with varying efficiencies. Typically, the mutations are point mutations or deletions. When helper viruses with low efficiency packaging are grown in helper cells, the virus is packaged, albeit at reduced rates compared to wild-type virus, thereby permitting propagation of the helper. When these helper viruses are grown in cells along with virus that contains wild-type packaging signals, however, the wild-type packaging signals are recognized preferentially over the mutated versions. Given a limiting amount of packaging factor, the virus containing the wild-type signals is packaged selectively when compared to the helpers. If the preference is great enough, stocks approaching homogeneity may be achieved.

To improve the tropism of ADV constructs for particular tissues or species, the receptor-binding fiber sequences can often be substituted between adenoviral isolates. For example the Coxsackie-adenovirus receptor (CAR) ligand found in adenovirus 5 can be substituted for the CD46-binding fiber sequence from adenovirus 35, making a virus with greatly improved binding affinity for human hematopoietic cells. The resulting "pseudotyped" virus, Ad5f35, has been the basis for several clinically developed viral isolates. Moreover, various biochemical methods exist to modify the fiber to allow re-targeting of the virus to target cells, such as, for example, T cells. Methods include use of bifunctional antibodies (with one end binding the CAR ligand and one end binding the target sequence), and metabolic biotinylation of the fiber to permit association with customized avidin-based chimeric ligands. Alternatively, one could attach ligands (e.g. anti-CD205 by heterobifunctional linkers (e.g. PEG-containing), to the adenovirus particle.

### 2. Retrovirus

The retroviruses are a group of single-stranded RNA viruses characterized by an ability to convert their RNA to double-stranded DNA in infected cells by a process of reversetranscription (Coffin, (1990) In: Virology, ed., New York: Raven Press, pp. 1437-1500). The resulting DNA then stably integrates into cellular chromosomes as a provirus and directs synthesis of viral proteins. The integration results in the retention of the viral gene sequences in the recipient cell and its descendants. The retroviral genome contains three genes - gag, pol and env - that code for capsid proteins, polymerase enzyme, and envelope components, respectively. A sequence found upstream from the gag gene, termed psi, functions as a signal for packaging of the genome into virions. Two long terminal repeat (LTR) sequences are present at the 5' and 3' ends of the viral genome. These contain strong promoter and enhancer sequences and also are required for integration in the host cell genome (Coffin, 1990). Thus, for example, the present technology includes, for example, cells whereby the polynucleotide used to transduce the cell is integrated into the genome of the cell.

In order to construct a retroviral vector, a nucleic acid encoding a promoter is inserted into the viral genome in the place of certain viral sequences to produce a virus that is replication-defective. In order to produce virions, a packaging cell line containing the gag, pol and env genes but without the LTR and psi components is constructed (Mann et al., (1983) Cell, 33,153-159). When a recombinant plasmid containing a human cDNA, together with the retroviral LTR and psi sequences is introduced into this cell line (by calcium phosphate precipitation for example), the psi sequence allows the RNA transcript of the recombinant plasmid to be packaged into viral particles, which are then secreted into the culture media (Nicolas, J.F., and Rubenstein, J.L.R., (1988) In: Vectors: a Survey of Molecular Cloning Vectors and Their Uses, Rodriquez and Denhardt, Eds.). Nicolas and Rubenstein; Temin et al., (1986) In: Gene Transfer, Kucherlapati (ed.), New York: Plenum Press, pp. 149-188; Mann et al., 1983). The media containing the recombinant retroviruses is collected, optionally concentrated, and used for gene transfer. Retroviral vectors are able to infect a broad variety of cell types. However, integration and stable expression of many types of retroviruses require the division of host cells (Paskind et al., (1975) Virology, 67,242-248).

An approach designed to allow specific targeting of retrovirus vectors recently was developed based on the chemical modification of a retrovirus by the chemical addition of galactose residues to the viral envelope. This modification could permit the specific infection of cells such as hepatocytes via asialoglycoprotein receptors, may this be desired.

A different approach to targeting of recombinant retroviruses was designed, which used biotinylated antibodies against a retroviral envelope protein and against a specific cell receptor. The antibodies were coupled via the biotin components by using streptavidin (Roux et al., (1989) Proc. Nat'l Acad. Sci. USA, 86, 9079-9083). Using antibodies against major histocompatibility complex class I and class II antigens, the infection of a variety of human cells that bore those surface antigens was demonstrated with an ecotropic virus in vitro (Roux et al., 1989).

### 3. Lentivirus

Lentiviral vectors used in the present methods may be derived from any appropriate lentivirus. Lentiviral vectors are a type of retroviral vector, including both primate and non-primate groups. Examples of lentiviral vectors are discussed in, for example, Coffin et al. (1997) "Retroviruses" Cold Spring Harbor Laboratory Press Eds: J M Coffin, S M Hughes, H E Varmus pp 758-763). Examples of primate lentiviruses include but are not limited to: the human immunodeficiency virus (HIV), the causative agent of human auto-immunodeficiency syndrome (AIDS), and the simian immunodeficiency virus (SIV). The non-primate lentiviral group includes the prototype "slow virus" visna/maedi virus (VMV), caprine arthritis-encephalitis virus (CAEV), equine infectious anaemia virus (EIAV) and feline immunodeficiency virus (FIV) and bovine immunodeficiency virus (BIV). Lentiviruses are capable of infecting both dividing and nondividing cells (Lewis et al. (1992); Lewis and Emerman (1994)).

A lentiviral vector, as used herein, is a vector which comprises at least one component part, wherein the component part is involved in the biological mechanisms by which the vector infects cells, expresses genes or is replicated, derivable from a lentivirus.

The basic structure of retrovirus and lentivirus genomes share many common features such as a 5' LTR and a 3' LTR, between or within which are located a packaging signal to enable the genome to be packaged, a primer binding site, integration sites to enable integration into a host cell genome and gag, pol and env genes encoding the packaging components. Lentiviruses also comprise additional features, such as rev and RRE sequences in HIV, which enable the efficient export of RNA transcripts of the integrated provirus from the nucleus to the cytoplasm of an infected target cell.

In the provirus, the viral genes are flanked at both ends by regions called long terminal repeats (LTRs). The LTRs are responsible for proviral integration, and transcription. LTRs also serve as enhancer-promoter sequences and can control the expression of the viral genes.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different viruses.

In examples of the lentiviral vectors discussed herein, at least part of one or more protein coding regions essential for replication may be removed from the virus. This makes the viral vector replication-defective. Portions of the viral genome may also be replaced by an NOI in order to generate a vector comprising an NOI which is capable of transducing a target nondividing host cell and/or integrating its genome into a host genome.

In one embodiment the retroviral vectors are non-integrating vectors as discussed in WO 2007/071994, WO 2007/072056, U.S. Patent Serial Number 9,169,491, U.S. Patent Serial Number 8,084,249, or U.S. Patent Number 7,531,648. In some examples, the lentiviral vector is a self-inactivating retroviral vector, wherein the transcriptional enhancers, or the enhancers and promoter in the U3 region of the 3' LTR have been deleted (see, for example, Yu et al. (1986) Proc. Natl. Acad. Sci. 83:3194-3198; Dougherty and Temin (1987) Proc. Natl. Acad. Sci. 84:1197-1201; Hawley et al. (1987) Proc. Natl. Acad. Sci. 84:2406-2410; Yee et al. (1987) Proc. Natl. Acad. Sci. 91:9564-9568).

The lentiviral plasmid vector used to produce the viral genome within a host cell/packaging cell will also include transcriptional regulatory control sequences operably linked to the lentiviral genome to direct transcription of the genome in a host cell/packaging cell. These regulatory sequences may be the natural sequences associated with the transcribed lentiviral sequence, i.e. the 5' U3 region, or they may be a heterologous promoter such as another viral promoter, for example the CMV promoter.

### 4. Adeno-associated Virus

AAV utilizes a linear, single-stranded DNA of about 4700 base pairs. Inverted terminal repeats flank the genome. Two genes are present within the genome, giving rise to a number of distinct gene products. The first, the cap gene, produces three different virion proteins (VP), designated VP-1, VP-2 and VP-3. The second, the rep gene, encodes four non-structural proteins (NS). One or more of these rep gene products is responsible for transactivating AAV transcription.

The three promoters in AAV are designated by their location, in map units, in the genome. These are, from left to right, p5, p19 and p40. Transcription gives rise to six transcripts, two initiated at each of three promoters, with one of each pair being spliced. The splice site, derived from map units 42-46, is the same for each transcript. The four non-structural proteins apparently are derived from the longer of the transcripts, and three virion proteins all arise from the smallest transcript.

AAV is not associated with any pathologic state in humans. Interestingly, for efficient replication, AAV requires "helping" functions from viruses such as herpes simplex virus I and II, cytomegalovirus, pseudorabies virus and, of course, adenovirus. The best characterized of the helpers is adenovirus, and many "early" functions for this virus have been shown to assist with AAV replication. Low-level expression of AAV rep proteins is believed to hold AAV structural expression in check, and helper virus infection is thought to remove this block.

The terminal repeats of the AAV vector can be obtained by restriction endonuclease digestion of AAV or a plasmid such as p201, which contains a modified AAV genome (Samulski et al., J. Virol., 61:3096-3101 (1987)), or by other methods, including but not limited to chemical or enzymatic synthesis of the terminal repeats based upon the published sequence of AAV. It can be determined, for example, by deletion analysis, the minimum sequence or part of the AAV ITRs which is required to allow function, i.e., stable and site-specific integration. It can also be determined which minor modifications of the sequence can be tolerated while maintaining the ability of the terminal repeats to direct stable, site-specific integration.

AAV-based vectors have proven to be safe and effective vehicles for gene delivery in vitro, and these vectors are being developed and tested in pre-clinical and clinical stages for a wide range of applications in potential gene therapy, both ex vivo and in vivo (Carter and Flotte, (1995) Ann. N.Y. Acad. Sci., 770; 79-90; Chatteijee, et al., (1995) Ann. N.Y. Acad. Sci., 770,79-90; Ferrari et al., (1996) J. Virol., 70,3227-3234; Fisher et al., (1996) J. Virol., 70,520-532; Flotte et al., Proc. Nat'l Acad. Sci. USA, 90,10613-10617, (1993); Goodman et al. (1994), Blood, 84,1492-1500; Kaplitt et al., (1994) Nat'l Genet., 8,148-153; Kaplitt, M.G., et al., Ann Thorac Surg. 1996 Dec;62(6):1669-76; Kessler et al., (1996) Proc. Nat'l Acad. Sci. USA, 93,14082-14087; Koeberl et al., (1997) Proc. Nat'l Acad. Sci. USA, 94,1426-1431; Mizukami et al., (1996) Virology, 217,124-130).

AAV-mediated efficient gene transfer and expression in the lung has led to clinical trials for the treatment of cystic fibrosis (Carter and Flotte, 1995; Flotte et al., Proc. Nat'l Acad. Sci. USA, 90, 10613-10617, (1993)). Similarly, the prospects for treatment of muscular dystrophy by AAV-mediated gene delivery of the dystrophin gene to skeletal muscle, of Parkinson's disease by tyrosine hydroxylase gene delivery to the brain, of hemophilia B by Factor IX gene delivery to the liver, and potentially of myocardial infarction by vascular endothelial growth factor gene to the heart, appear promising since AAV-mediated transgene expression in these organs has recently been shown to be highly efficient (Fisher et al., (1996) J. Virol., 70,520-532; Flotte et al., 1993; Kaplitt et al., 1994; 1996; Koeberl et al., 1997; McCown et al., (1996) Brain Res., 713,99-107; Ping et al., (1996) Microcirculation, 3,225-228; Xiao et al., (1996) J. Virol., 70,8098-8108).

### 5. Other Viral Vectors

Other viral vectors are employed as expression constructs in the present methods and compositions. Vectors derived from viruses such as vaccinia virus (Ridgeway, (1988) In: Vectors: A survey of molecular cloning vectors and their uses, pp. 467-492; Baichwal and Sugden, (1986) In, Gene Transfer, pp. 117-148; Coupar et al., Gene, 68:1-10, 1988) canary poxvirus, and herpes viruses are employed. These viruses offer several features for use in gene transfer into various mammalian cells.

Once the construct has been delivered into the cell, the nucleic acid encoding the transgene are positioned and expressed at different sites. In certain embodiments, the nucleic acid encoding the transgene is stably integrated into the genome of the cell. This integration is in the cognate location and orientation via homologous recombination (gene replacement) or it is integrated in a random, non-specific location (gene augmentation). In yet further embodiments, the nucleic acid is stably maintained in the cell as a separate, episomal segment of DNA. Such nucleic acid segments or "episomes" encode sequences sufficient to permit maintenance and replication independent of or in synchronization with the host cell cycle. How the expression construct is delivered to a cell and where in the cell the nucleic acid remains is dependent on the type of expression construct employed.

### Methods for engineering T cells, and evaluation of the modified T cells

Examples of methods for engineering T cells and evaluation of the modified T cells are provided herein.

### Retrovirus transduction

For the transient production of retrovirus, 293T cells are transfected with the chimeric polypeptide constructs, along with plasmids encoding gag-pol and RD 114 envelope using GeneJuice transfection reagent (Novagen, Madison, WI). Virus is harvested 48 to 72 hours after transfection, snap frozen, and stored at ~80°C until use. For the transient production of lentivirus, 293T cells are transfected with the constructs along with the plasmids pLP1 (gag/pol), pLP2 (rev) and pLP/VSVG (VSVG env) using GeneJuice. Virus is harvested 48 to 72 hours after transfection, snap frozen, and stored at -80°C until use. For large-scale retrovirus production, a stable FLYRD 18-derived retroviral producer line is generated by multiple transductions with VSV-G pseudotyped transient retroviral supernatant. FLYRD18 cells with highest transgene expression are single-cell sorted, and the clone that produces the highest virus titer is expanded and used to produce virus for lymphocyte transduction. The transgene expression, function, and retroviral titer of this clone is maintained during continuous culture for more than 8 weeks. Non-tissue culture-treated 24-well plates are coated with 7 µg/ml Retronectin (Takara Bio, Otsu, Shiga, Japan) for 1 hour at 37°C or overnight at 4°C. The wells are washed with phosphate-buffered saline (PBS) then coated with retroviral supernatant by incubating the plate with 1.5 ml of supernatant for 30 minutes at 37°C. Subsequently, T cell blasts are plated at 5 ×10⁵ cells per well in viral supernatant supplemented with 100 U/ml IL-2. Transduction is performed over a 60-hour period. Following transduction, cells are harvested and phenotyped for CD19 or GFP expression by flow cytometry.

### Cytotoxicity of transduced T cells

The cytotoxic activity of each transduced T cell line is evaluated in a standard 4-hour 51Cr release assay, as previously presented. T cells transduced with the retrovirus or lentivirus and compared against Cr51-labeled target cells, including autologous phytohaemagglutinin (PHA) stimulated lymphocytes (PHA blasts), LNCaP, PC3 or DU145 and A549 cancer cell lines, and transgenic A549 expressing human PSMA (A549-PSMA). Target cells incubated in complete medium or 1% Triton X-100 (Sigma, St Louis, MO) are used to determine spontaneous and maximum 51Cr release, respectively. The mean percentage of specific lysis of triplicate wells was calculated as 100 X (experimental release - spontaneous release) / (maximal release - spontaneous release). In addition to chromium-release assays, co-culture experiments with are performed. Here, the cell lines LNCaP, PC3, DU145, A549 and A549-PSMA are transduced to express fluorescent mOrange and used as target cells. mOrange-expressing tumor cells are co-cultured with non-transduced or modified T cells at a ratio of 1:10 tumor cells to T cells in the presence of IL-2 (50 U/ml) in complete media. After 24 hours, T cells are stimulated with 100 nM AP1903. After 72 hours, cells are collected, counted and labeled with CD3 to detect T cells and percentage of mOrange tumor cells is analyzed by flow cytometry (LSRII; BD).

### Phenotyping and activation status of transduced T cells

Cell surface phenotype of transduced T cells is investigated using the following monoclonal antibodies: CD3, CD4, CD8, CD19, CD25, CD27, CD28, CD44, CD45RA, CD45RO, CD62L, CD80, CD83, CD86, CD95, CD127, CD134, CD137, HLA-ABC and HLA-DR. Phenotyping is performed with and without 100 nM AP1903. Appropriate matched isotype controls are used in each experiment and cells are analyzed with a LSRII flow cytometer (BD). The chimeric polypeptide expression is assessed using anti-F(ab')2 (Jackson ImmunoResearch Laboratories, West Grove, PA).

### Analysis of cytokine production of transduced T cells

The concentration of interferon-y (IFN-y), IL-2, IL-4, IL-5, IL-10, and tumor necrosis factor. α(TNFα) in T cell culture supernatants before and after (24 hours) 100 nM AP1903 stimulation is measured using the Human Th1/Th2 cytokine cytometric Bead Array (BD Pharmin¬gen). Induced cytokine production in the culture supernatants is validated by enzyme-linked immunosorbent assay (ELISA; R&D Systems, Minneapolis, MN) according to the instructions of the manufacturer.

### Proliferation of transduced T cells

The proliferative effect of AP1903-induced activation is evaluated by measuring cell growth of transduced and non-transduced T cells following exposure to AP1903. T cells are labeled with 10 µM carboxyfluorescein diacetate, succinimidyl ester (CFSE) for 10 minutes at 37°C. After incubation, cells are washed in PBS and then resuspended in Cellgenix DC media. 1×10⁶ CFSE-labeled modified or non-transduced T cells are subsequently cultured in Cellgenix DC media alone, or stimulated with 100 nM AP1903. After 5 days, cells are harvested and labeled with CD3-PerCP.Cy5.5 and CD19-PE and analyzed by flow cytometry for CFSE dilution.

To evaluate whether soluble immunoglobulins affect the proliferation and expansion of the transduced T lymphocytes, cells are cultured at 1 × 10⁵ cells/well either with serial dilution of human plasma obtained from healthy donors or serial dilution of purified human immunoglobulins (Jackson ImmunoResearch) without any addition of exogenous cytokines. After 72 hours, the cells are pulsed with 1 µCi (0.037 MBq) methyl-3[H]thymidine (Amersham Pharmacia Biotech, Piscataway, NJ) and cultured for additional 15 hours. The cells were then harvested onto filters and dried, and counts per minute are measured in a β-scintittation counter (TriCarb 2500 TR; Packard BioScience, Meridien, CT). The experiments are performed in triplicate. In other experiments, control and modified T lymphocytes are cultured either with media alone or with media in which serial dilution of plasma or purified immunoglobulins are added every second day. Cells are then counted every third day using trypan blue exclusion.

### Activation of T cells ex vivo and administration to a human subject

Presented in this example are methods of using modified T cells, such as PRAME-specific recombinant TCR -modified T cells, which may or may not also comprise polynucleotides encoding additional chimeric polypeptides, such as the chimeric Caspase-9 polypeptides discussed herein, for human therapy.

### Materials and Methods

### Large-scale generation of gene-modified T cells

T cells are generated from healthy volunteers, using standard methods. Briefly, peripheral blood mononuclear cells (PBMCs) from healthy donors or cancer patients are activated for expansion and transduction using soluble αCD3 and αCD28 (Miltenyi Biotec, Auburn, CA). PBMCs are resuspended in Cellgenix DC media supplemented with 100 U/ml IL-2 (Cellgenix) at 1×10⁶ cells/ml and stimulated with 0.2 µg/ml αCD3 and 0.5 µg/ml αCD28 soluble antibody.

Cells are then cultured at 37°C, 5% CO2 for 4 days. On day four, 1 ml of fresh media containing IL-2 is added. On day 7, cells are harvested and resuspended in Cellgenix DC media for transduction.

### Plasmid and retrovirus

The compositions and methods of the present example may be modified to include PRAME-specific recombinant TCR--encoding lentiviral vectors as discussed herein. The SFG plasmid consists of inducible chimeric polypeptide linked, via a cleavable 2A-like sequence, to truncated human CD19. The inducible chimeric polypeptide consists of a human FK506-binding protein (FKBP12; GenBank AH002 818) with an F36V mutation, connected via a Ser-Gly-Gly-Gly-Ser-Gly linker to human chimeric polypeptide. The F36V mutation increases the binding affinity of FKBP12 to the synthetic homodimerizer, AP20187 or AP1903. The 2A-like sequence, "T2A", encodes an 20 amino acid peptide from Thosea asigna insect virus, which mediates >99% cleavage between a glycine and terminal proline residue, resulting in 19 extra amino acids in the C terminus of the inducible chimeric polypeptide, and one extra proline residue in the N terminus of CD19. CD19 consists of full-length CD19 (GenBank NM 001770) truncated at amino acid 333 (TDPTRRF), which shortens the intracytoplasmic domain from 242 to 19 amino acids, and removes all conserved tyrosine residues that are potential sites for phosphorylation.

A stable PG13 clone producing Gibbon ape leukemia virus (Gal-V) pseudotyped retrovirus is made by transiently transfecting Phoenix Eco cell line (ATCC product #SD3444; ATCC, Manassas, VA) with the SFG plasmid. This produces Eco-pseudotyped retrovirus. The PG13 packaging cell line (ATCC) is transduced three times with Eco-pseudotyped retrovirus to generate a producer line that contained multiple SFG plasmid proviral integrants per cell. Single cell cloning is performed, and the PG13 clone that produced the highest titer is expanded and used for vector production.

### Retroviral transduction

Culture medium for T cell activation and expansion is serum-free Cellgenix DC medium (Cellgenix) supplemented by 100 U/ml IL-2 (Cellgenix). T cells are activated by soluble anti-CD3 and anti-CD28 (Miltenyi Biotec) for 7 days before transduction with retroviral vector. Immunomagnetic selection of ΔCD19, if necessary, is performed on day 4 after transduction; the positive fraction was expanded for a further 2 days and cryopreserved.

### Scaling-up production of gene-modified allodepleted cells

Scale-up of the transduction process for clinical application use non-tissue culture-treated T75 flasks (Nunc, Rochester, NY), which are coated with 10 ml of anti-CD3 0.5micrograms/ml and anti-CD28 0.2 µg/ml or 10ml of fibronectin 7micrograms/ml at 4°C overnight. Fluorinated ethylene propylene bags corona-treated for increased cell adherence (2PF-0072AC, American Fluoroseal Corporation, Gaithersburg, MD) are also used. PBMCs are seeded in anti-CD3, anti-CD28 -coated flasks at 1×10⁶ cells/ml in media supplemented with 100U/ml IL-2. For retroviral transduction, retronectin-coated flasks or bags are loaded once with 10ml of retrovirus-containing supernatant for 2 to 3 hours. Activated T cells are seeded at 1×10⁶ cells/ml in fresh retroviral vector-containing medium and T cell culture medium at a ratio of 3:1, supplemented with 100U/ml IL-2. Cells are harvested the following morning and expanded in tissue-culture treated T75 or T175 flasks in culture medium supplemented with 100U/ml IL-2 at a seeding density of between about 5×10⁵ cells/ ml to 8×10⁵ cells/ ml.

### CD19 immunomagnetic selection

In the present example, the modified cells express a CD19 marker protein; it is understood that the modified cells may be selected using markers other than CD19, or by other methods. Immunomagnetic selection for CD19 may be performed, in one example, 4 days after transduction. Cells are labeled with paramagnetic microbeads conjugated to monoclonal mouse anti-human CD19 antibodies (Miltenyi Biotech, Auburn, CA) and selected on MS or LS columns in small scale experiments and on a CliniMacs Plus automated selection device in large scale experiments. CD19-selected cells are expanded for a further 4 days and cryopreserved on day 8 post transduction. These cells are referred to as "gene-modified cells".

### Immunophenotyping and pentamer analysis

Flow cytometric analysis (FACSCalibur and CellQuest software; Becton Dickinson) is performed using the following antibodies: CD3, CD4, CD8, CD19, CD25, CD27, CD28, CD45RA, CD45RO, CD56 and CD62L. CD19-PE (Clone 4G7; Becton Dickinson) is found to give optimum staining and was used in all subsequent analysis. A non-transduced control is used to set the negative gate for CD19.

### Statistical analysis

Paired, 2-tailed Student's t test is used to determine the statistical significance of differences between samples. All data are represented as mean ± 1 standard deviation.

### Methods for Treating a Disease

Described herein are methods of treatment or prevention of a disease where administration of cells by, for example, infusion, may be beneficial.

Cells, such as, for example, T cells, tumor infiltrating lymphocytes, natural killer cells, natural killer T cells, or progenitor cells, such as, for example, hematopoietic stem cells, mesenchymal stromal cells, stem cells, pluripotent stem cells, and embryonic stem cells may be used for cell therapy. The cells may be from a donor, or may be cells obtained from the patient. The cells may, for example, be used in regeneration, for example, to replace the function of diseased cells. The cells may also be modified to express a heterologous gene so that biological agents may be delivered to specific microenvironments such as, for example, diseased bone marrow or metastatic deposits. Mesenchymal stromal cells have also, for example, been used to provide immunosuppressive activity, and may be used in the treatment of graft versus host disease and autoimmune disorders. The cells provided in the present application contain a safety switch that may be valuable in a situation where following cell therapy, the activity of the therapeutic cells needs to be increased, or decreased. For example, where T cells that express a T cell receptor, such as a PRAME-targeted TCR, are provided to the patient, in some situations there may be an adverse event, such as off-target toxicity. Ceasing the administration of the ligand would return the therapeutic T cells to a non-activated state, remaining at a low, non-toxic, level of expression. Or, for example, the therapeutic cell may work to decrease the tumor cell, or tumor size, and may no longer be needed. In this situation, administration of the ligand may cease, and the therapeutic cells would no longer be activated. If the tumor cells return, or the tumor size increases following the initial therapy, the ligand may be administered again, in order to further activate the TCR-expressing T cells, and re-treat the patient.

By "therapeutic cell" is meant a cell used for cell therapy, that is, a cell administered to a subject to treat or prevent a condition or disease.

The term "unit dose" as it pertains to the inoculum refers to physically discrete units suitable as unitary dosages for mammals, each unit containing a predetermined quantity of pharmaceutical composition calculated to produce the desired immune-stimulating effect in association with the required diluent. The specifications for the unit dose of an inoculum are dictated by and are dependent upon the unique characteristics of the pharmaceutical composition and the particular immunologic effect to be achieved.

An effective amount of the pharmaceutical composition, such as the multimeric ligand presented herein, would be the amount that achieves this selected result of inducing apoptosis in the caspase-9-expressing cells T cells, such that over 60%, 70%, 80%, 85%, 90%, 95%, or 97%, or that under 80%, 70%, 60%, 50%, 40%, 30%, 20%, or 10% of the therapeutic cells are killed. The term is also synonymous with "sufficient amount." The effective amount where the pharmaceutical composition is the modified T cell may also be the amount that achieves the desired therapeutic response, such as, the reduction of tumor size, the decrease in the level of tumor cells, or the decrease in the level of leukemic cells, compared to the time before the ligand inducer is administered.

The effective amount for any particular application can vary depending on such factors as the disease or condition being treated, the particular composition being administered, the size of the subject, and/or the severity of the disease or condition. One can empirically determine the effective amount of a particular composition presented herein without necessitating undue experimentation.

The terms "contacted" and "exposed," when applied to a cell, tissue or organism, are used herein to describe the process by which the pharmaceutical composition and/or another agent, such as for example a chemotherapeutic or radiotherapeutic agent, are delivered to a target cell, tissue or organism or are placed in direct juxtaposition with the target cell, tissue or organism. To achieve cell killing or stasis, the pharmaceutical composition and/or additional agent(s) are delivered to one or more cells in a combined amount effective to kill the cell(s) or prevent them from dividing.

The administration of the pharmaceutical composition may precede, be concurrent with and/or follow the other agent(s) by intervals ranging from minutes to weeks. In examples where the pharmaceutical composition and other agent(s) are applied separately to a cell, tissue or organism, one would generally ensure that a significant period of time did not expire between the times of each delivery, such that the pharmaceutical composition and agent(s) would still be able to exert an advantageously combined effect on the cell, tissue or organism. For example, in such instances, it is contemplated that one may contact the cell, tissue or organism with two, three, four or more modalities substantially simultaneously (i.e., within less than about a minute) with the pharmaceutical composition. In other examples, one or more agents may be administered within of from substantially simultaneously, about 1 minute, to about 24 hours to about 7 days to about 1 to about 8 weeks or more, and any range derivable therein, prior to and/or after administering the expression vector. Yet further, various combination regimens of the pharmaceutical composition presented herein and one or more agents may be employed.

### Optimized and Personalized Therapeutic Treatment

The dosage and administration schedule of the modified cells may be optimized by determining the level of the disease or condition to be treated. For example, the size of any remaining solid tumor, or the level of targeted cells such as, for example, tumor cells or leukemic cells, which remain in the patient, may be determined.

For example, determining that a patient has clinically relevant levels of tumor cells, or a solid tumor, after initial therapy, provides an indication to a clinician that it may be necessary to administer the modified T cells. In another example, determining that a patient has a reduced level of tumor cells or reduced tumor size after treatment with the modified cells may indicate to the clinician that no additional dose of the modified cells is needed. Similarly, after treatment with the modified cells, determining that the patient continues to exhibit disease or condition symptoms, or suffers a relapse of symptoms may indicate to the clinician that it may be necessary to administer at least one additional dose of modified cells.

The term "dosage" is meant to include both the amount of the dose and the frequency of administration, such as, for example, the timing of the next dose. The term "dosage level" refers to the amount of the modified cells administered in relation to the body weight of the subject.

In certain embodiments the cells are in an animal, such as human, non-human primate, cow, horse, pig, sheep, goat, dog, cat, or rodent. The subject may be, for example, an animal, such as a mammal, for example, a human, non-human primate, cow, horse, pig, sheep, goat, dog, cat, or rodent. The subject may be, for example, human, for example, a patient suffering from an infectious disease, and/or a subject that is immunocompromised, or is suffering from a hyperproliferative disease.

Thus, for example, in certain examples, the methods comprise determining the presence or absence of a tumor size increase and/or increase in the number of tumor cells in a subject relative to the tumor size and/or the number of tumor cells following administration of the modified cells or nucleic acid, and administering an additional dose of the modified cells or nucleic acid to the subject in the event the presence of a tumor size increase and/or increase in the number of tumor cells is determined. The methods also comprise, for example, determining the presence or absence of an increase in leukemic cells in the subject relative to the level of leukemic cells following administration of the modified cells or nucleic acid, and administering an additional dose of the modified cells or nucleic acid to the subject in the event the presence of an increase in leukemic cells in the subject is determined. In these examples, for example, the patient is initially treated with the therapeutic cells or nucleic acid according to the methods provided herein. Following the initial treatment, the size of the tumor, the number of tumor cells, or the number of leukemic cells, for example, may decrease relative to the time prior to the initial treatment. At a certain time after this initial treatment, the patient is again tested, or the patient may be continually monitored for disease symptoms. If it is determined that the size of the tumor, the number of tumor cells, or the number of leukemic cells, for example, is increased relative to the time just after the initial treatment, then the modified cells or nucleic acid may be administered for an additional dose. This monitoring and treatment schedule may continue while noting that the therapeutic cells that express the PRAME-targeted T cell receptors remain in the patient.

In other examples, following administration of the modified cells or nucleic acid, wherein the modified cells or nucleic acid express the inducible caspase-9 polypeptide, in the event of a need to reduce the number of modified cells or in vivo modified cells, the multimeric ligand may be administered to the patient. In these examples, the methods comprise determining the presence or absence of a negative symptom or condition, such as Graft vs Host Disease, or off target toxicity, and administering a dose of the multimeric ligand. The methods may further comprise monitoring the symptom or condition and administering an additional dose of the multimeric ligand in the event the symptom or condition persists. This monitoring and treatment schedule may continue while the therapeutic cells that express the PRAME-targeted TCRs remain in the patient.

An indication of adjusting or maintaining a subsequent drug dose, such as, for example, a subsequent dose of the modified cells or nucleic acid, and/or the subsequent drug dosage, can be provided in any convenient manner. An indication may be provided in tabular form (e.g., in a physical or electronic medium) in some embodiments. For example, the size of the tumor cell, or the number or level of tumor cells in a sample may be provided in a table, and a clinician may compare the symptoms with a list or table of stages of the disease. The clinician then can identify from the table an indication for subsequent drug dose. In certain embodiments, an indication can be presented (e.g., displayed) by a computer, after the symptoms are provided to the computer (e.g., entered into memory on the computer). For example, this information can be provided to a computer (e.g., entered into computer memory by a user or transmitted to a computer via a remote device in a computer network), and software in the computer can generate an indication for adjusting or maintaining a subsequent drug dose, and/or provide the subsequent drug dose amount.

Once a subsequent dose is determined based on the indication, a clinician may administer the subsequent dose or provide instructions to adjust the dose to another person or entity. The term "clinician" as used herein refers to a decision maker, and a clinician is a medical professional in certain embodiments. A decision maker can be a computer or a displayed computer program output in some embodiments, and a health service provider may act on the indication or subsequent drug dose displayed by the computer. A decision maker may administer the subsequent dose directly (e.g., infuse the subsequent dose into the subject) or remotely (e.g., pump parameters may be changed remotely by a decision maker).

Methods as disclosed herein include without limitation the delivery of an effective amount of an activated cell, a nucleic acid, or an expression construct encoding the same. An "effective amount" of the activated cell, nucleic acid, or expression construct, generally, is defined as that amount sufficient to detectably and repeatedly to achieve the stated desired result, for example, to ameliorate, reduce, minimize or limit the extent of the disease or its symptoms. Other more rigorous definitions may apply, including elimination, eradication or cure of disease. In some embodiments there may be a step of monitoring the biomarkers, or other disease symptoms such as tumor size or tumor antigen expression, to evaluate the effectiveness of treatment and to control toxicity.

In further embodiments, the expression construct and/or expression vector can be utilized as a composition or substance that activates cells. Such a composition that "activates cells" or "enhances the activity of cells" refers to the ability to stimulate one or more activities associated with cells. For example, a composition, such as the expression construct or vector of the present methods, can stimulate upregulation of co-stimulating molecules on cells, induce nuclear translocation of NF-κB in cells, activate toll- like receptors in cells, or other activities involving cytokines or chemokines.

The expression construct, expression vector and/or transduced cells can enhance or contribute to the effectiveness of a vaccine by, for example, enhancing the immunogenicity of weaker antigens such as highly purified or recombinant antigens, reducing the amount of antigen required for an immune response, reducing the frequency of immunization required to provide protective immunity, improving the efficacy of vaccines in subjects with reduced or weakened immune responses, such as newborns, the aged, and immunocompromised individuals, and enhancing the immunity at a target tissue, such as mucosal immunity, or promote cell-mediated or humoral immunity by eliciting a particular cytokine profile.

In certain embodiments, the cell is also contacted with an antigen. Often, the cell is contacted with the antigen ex vivo. Sometimes, the cell is contacted with the antigen in vivo. In some embodiments, the cell is in a subject and an immune response is generated against the antigen. Sometimes, the immune response is a cytotoxic T-lymphocyte (CTL) immune response. Sometimes, the immune response is generated against a tumor antigen. In certain embodiments, the cell is activated without the addition of an adjuvant.

In certain examples, the cell is transduced with the nucleic acid ex vivo and administered to the subject by intravenous administration. In other examples, the cell is administered using intradermal administration. In some examples, the cell is transduced with the nucleic acid ex vivo and administered to the subject by subcutaneous administration. Sometimes, the cell is transduced with the nucleic acid ex vivo. Sometimes, the cell is transduced with the nucleic acid in vivo.

In certain examples the cell is transduced with the nucleic acid ex vivo and administered to the subject by intradermal administration, and sometimes the cell is transduced with the nucleic acid ex vivo and administered to the subject by subcutaneous administration. The antigen may be a tumor antigen, and the CTL immune response can be induced by migration of the cell to a draining lymph node. A tumor antigen is any antigen such as, for example, a peptide or polypeptide, that triggers an immune response in a host. The tumor antigen may be a tumorassociated antigen, which is associated with a neoplastic tumor cell.

In some embodiments, an immunocompromised individual or subject is a subject that has a reduced or weakened immune response. Such individuals may also include a subject that has undergone chemotherapy or any other therapy resulting in a weakened immune system, a transplant recipient, a subject currently taking immunosuppressants, an aging individual, or any individual that has a reduced and/or impaired CD4 T helper cells. It is contemplated that the present methods can be utilized to enhance the amount and/or activity of CD4 T helper cells in an immunocompromised subject.

### Antigens

When assaying T cell activation in vitro or ex vivo, target antigens may be obtained or isolated from various sources. The target antigen, as used herein, is an antigen or immunological epitope on the antigen, which is crucial in immune recognition and ultimate elimination or control of the disease-causing agent or disease state in a mammal. The immune recognition may be cellular and/or humoral. In the case of intracellular pathogens and cancer, immune recognition may, for example, be a T lymphocyte response. The target antigen herein may comprise, for example, PRAME. The T cell receptors may bind to an epitope, or polypeptide derived from PRAME alone, or in the context of a peptide-MHC complex, for example a peptide molecule presented as part of an MHC complex with an HLA Class I molecule. Major histocompatibility complex (MHC) molecules bind to TCRs and CD4/CD8 co-receptors on T lymphocytes; the MHC molecules also present a polypeptide fragment, or epitope, that interacts with the TCR. In general, MHC Class I molecules interact with the CD8 receptor and class II molecules interact with the CD4 receptor. Thus, in the present application, by "specifically binds to PRAME," such as, for example, where CDR3 regions of a TCR, or a TCR specifically binds to PRAME or a target antigen, it is understood that the CDR3 regions alone, as part of a TCR, as part of a recombinant TCR comprising heterologous polypeptide sequences, or as part of a heterologous polypeptide, may specifically bind to PRAME, an epitope or polypeptide derived from PRAME, or the epitope or polypeptide derived from PRAME as part of a peptide-MHC complex, for example, an HLA Class I complex, or, for example, as part of a peptide-MHC complex including an HLA Class I A2.01 molecule. The CDR3 regions of the alpha and beta polypeptides together recognize, or bind to, the peptide-MHC complex, it is understood that other TCR regions, or other polypeptides may contribute to this binding. Thus, the TCR CDR3 regions discussed herein that "specifically bind" to PRAME, have specific binding affinities for PRAME peptide-MHC complexes, wherein the MHC, for example, is an HLA Class I molecule, for example, an HLA Class I A2.01 molecule.

The target antigen may be derived or isolated from, for example, a pathogenic microorganism such as viruses including HIV, (Korber et al, eds HIV Molecular Immunology Database, Los Alamos National Laboratory, Los Alamos, N. Mex. 1977) influenza, Herpes simplex, human papilloma virus (U.S. Pat. No. 5,719,054), Hepatitis B (U.S. Pat. No. 5,780,036), Hepatitis C (U.S. Pat. No. 5,709,995), EBV, Cytomegalovirus (CMV) and the like. Target antigen may be derived or isolated from pathogenic bacteria such as, for example, from Chlamydia (U.S. Pat. No. 5,869,608), Mycobacteria, Legionella, Meningiococcus, Group A Streptococcus, Salmonella, Listeria, Hemophilus influenzae (U.S. Pat. No. 5,955,596) and the like).

Target antigen may be derived or isolated from, for example, pathogenic yeast including Aspergillus, invasive Candida (U.S. Pat. No. 5,645,992), Nocardia, Histoplasmosis, Cryptosporidia and the like.

Target antigen may be derived or isolated from, for example, a pathogenic protozoan and pathogenic parasites including but not limited to Pneumocystis carinii, Trypanosoma, Leishmania (U.S. Pat. No. 5,965,242), Plasmodium (U.S. Pat. No. 5,589,343) and Toxoplasma gondii.

Target antigen includes an antigen associated with a preneoplastic or hyperplastic state. Target antigen may also be associated with, or causative of cancer. In certain embodiments, the target antigen is PRAME. Such target antigen may be, for example, tumor specific antigen, tumor associated antigen (TAA) or tissue specific antigen, epitope thereof, and epitope agonist thereof. Such target antigens include but are not limited to carcinoembryonic antigen (CEA) and epitopes thereof such as CAP-1, CAP-1-6D and the like (GenBank Accession No. M29540), MART-1 (Kawakarni et al, J. Exp. Med. 180:347-352, 1994), MAGE-1 (U.S. Pat. No. 5,750,395), MAGE-3, GAGE (U.S. Pat. No. 5,648,226), GP-100 (Kawakami et al Proc. Nat'l Acad. Sci. USA 91:6458-6462, 1992), MUC-1, MUC-2, point mutated ras oncogene, normal and point mutated p53 oncogenes (Hollstein et al Nucleic Acids Res. 22:3551-3555, 1994), PSMA (Israeli et al Cancer Res. 53:227-230, 1993), tyrosinase (Kwon et al PNAS 84:7473-7477, 1987) TRP-1 (gp75) (Cohen et al Nucleic Acid Res. 18:2807-2808, 1990; U.S. Pat. No. 5,840,839), NY-ESO-1 (Chen et al PNAS 94: 1914-1918, 1997), TRP-2 (Jackson et al EMBOJ, 11:527-535, 1992), TAG72, KSA, CA-125, PSA, HER-2/neu/c-erb/B2, (U.S. Pat. No. 5,550,214), BRC-I, BRC-II, bcr-abl, pax3-fkhr, ews-fli-1, modifications of TAAs and tissue specific antigen, splice variants of TAAs, epitope agonists, and the like. Other TAAs may be identified, isolated and cloned by methods known in the art such as those disclosed in U.S. Pat. No. 4,514,506. Target antigen may also include one or more growth factors and splice variants of each.

An antigen may be expressed more frequently in cancer cells than in non-cancer cells. The antigen may result from contacting the modified cell with a prostate specific membrane antigen, for example, a prostate specific membrane antigen (PSMA) or fragment thereof.

Prostate antigen (PA001) is a recombinant protein consisting of the extracellular portion of PSMA antigen. PSMA is a ~ 100 kDa (84kDa before glycosylation, ~ 180kDa as dimer) type II membrane protein with neuropeptidase and folate hydrolase activities, but the true function of PSMA is currently unclear. Carter RE, et al., Proc Natl Acad Sci USA. 93: 749-53, 1996; Israeli RS, et al., Cancer Res. 53: 227-30, 1993; Pinto JT, et al., Clin Cancer Res. 2: 1445-51, 1996.

The cell may be contacted with tumor antigen, such as PSA, for example, PSMA polypeptide, by various methods, including, for example, pulsing immature DCs with unfractionated tumor lysates, MHC-eluted peptides, tumor-derived heat shock proteins (HSPs), tumor associated antigens (TAAs (peptides or proteins)), or transfecting DCs with bulk tumor mRNA, or mRNA coding for TAAs (reviewed in Gilboa, E. & Vieweg, J., Immunol Rev 199, 251-63 (2004); Gilboa, E, Nat Rev Cancer 4, 401-11 (2004)).

For organisms that contain a DNA genome, a gene encoding a target antigen or immunological epitope thereof of interest is isolated from the genomic DNA. For organisms with RNA genomes, the desired gene may be isolated from cDNA copies of the genome. If restriction maps of the genome are available, the DNA fragment that contains the gene of interest is cleaved by restriction endonuclease digestion by routine methods. In instances where the desired gene has been previously cloned, the genes may be readily obtained from the available clones. Alternatively, if the DNA sequence of the gene is known, the gene can be synthesized by any of the conventional techniques for synthesis of deoxyribonucleic acids.

Genes encoding an antigen of interest can be amplified, for example, by cloning the gene into a bacterial host. For this purpose, various prokaryotic cloning vectors can be used. Examples are plasmids pBR322, pUC and pEMBL.

The genes encoding at least one target antigen or immunological epitope thereof can be prepared for insertion into the plasmid vectors designed for recombination with a virus by standard techniques. In general, the cloned genes can be excised from the prokaryotic cloning vector by restriction enzyme digestion. In most cases, the excised fragment will contain the entire coding region of the gene. The DNA fragment carrying the cloned gene can be modified as needed, for example, to make the ends of the fragment compatible with the insertion sites of the DNA vectors used for recombination with a virus, then purified prior to insertion into the vectors at restriction endonuclease cleavage sites (cloning sites).

Antigen loading of cells, such as, for example, dendritic cells, with antigens, such as, for example, a PRAME epitope polypeptide, may be achieved, for example, by contacting cells, such as, for example, dendritic cells or progenitor cells with an antigen, for example, by incubating the cells with the antigen. Loading may also be achieved, for example, by incubating DNA (naked or within a plasmid vector) or RNA that code for the antigen; or with antigen-expressing recombinant bacterium or viruses (e.g., vaccinia, fowlpox, adenovirus or lentivirus vectors). Prior to loading, the antigen may be covalently conjugated to an immunological partner that provides T cell help (e.g., a carrier molecule). Alternatively, a dendritic cell may be pulsed with a non-conjugated immunological partner, separately or in the presence of the polypeptide. Antigens from cells or MHC molecules may be obtained by acidelution or other methods (see Zitvogel L, et al., J Exp Med 1996. 183:87-97). The cells may be transduced or transfected with the chimeric protein-encoding nucleotide sequence according to the present methods before, after, or at the same time as the cells are loaded with antigen. In particular embodiments, antigen loading is subsequent to transduction or transfection.

In further embodiments, the transduced cell is transfected with tumor cell mRNA. The transduced transfected cell is administered to an animal to effect cytotoxic T lymphocytes and natural killer cell anti-tumor antigen immune response and regulated using dimeric FK506 and dimeric FK506 analogs. The tumor cell mRNA may be, for example, mRNA from a prostate tumor cell.

In some embodiments, the transduced cell may be loaded by pulsing with tumor cell lysates. The pulsed transduced cells are administered to an animal to effect cytotoxic T lymphocytes and natural killer cell anti-tumor antigen immune response and regulated using dimeric FK506 and dimeric FK506 analogs. The tumor cell lysate may be, for example, a prostate tumor cell lysate.

### Immune Cells and Cytotoxic T Lymphocyte Response

T-lymphocytes may be activated by contact with the cell that comprises the expression vector discussed herein, where the cell has been challenged, transfected, pulsed, or electrofused with an antigen.

T cells express a unique antigen binding receptor on their membrane (T cell receptor), which can only recognize antigen in association with, or in the context of, major histocompatibility complex (MHC) molecules on the surface of other cells. There are several populations of T cells, such as T helper cells and T cytotoxic cells. T helper cells and T cytotoxic cells are primarily distinguished by their display of the membrane bound glycoproteins CD4 and CD8, respectively. T helper cells secret various lymphokines, which are crucial for the activation of B cells, T cytotoxic cells, macrophages and other cells of the immune system. In contrast, a naïve CD8 T cell that recognizes an antigen-MHC complex proliferates and differentiates into an effector cell called a cytotoxic CD8 T lymphocyte (CTL). CTLs eliminate cells of the body displaying antigen, such as virus-infected cells and tumor cells, by producing substances that result in cell lysis.

Modified cells transduced or transfected with the nucleic acids, vectors, or compositions herein include, for example, T helper cells, T cytotoxic cells, CD8+ T cells, CD4+ T cells, NK T cells, and NK cells. A modified cell as provided herein is typically a collection of modified cells.

CTL activity can be assessed by methods discussed herein, for example. For example, CTLs may be assessed in freshly isolated peripheral blood mononuclear cells (PBMC), in a phytohaemaglutinin-stimulated IL-2 expanded cell line established from PBMC (Bernard et al., AIDS, 12(16):2125-2139, 1998) or by T cells isolated from a previously immunized subject and restimulated for 6 days with DC infected with an adenovirus vector containing the antigen using standard 4 hour 51Cr release microtoxicity assays. One type of assay uses cloned T cells. Cloned T cells have been tested for their ability to mediate both perforin and Fas liganddependent killing in redirected cytotoxicity assays (Simpson et al., Gastroenterology, 115(4):849-855, 1998). The cloned cytotoxic T lymphocytes displayed both Fas- and perforindependent killing. Recently, an in vitro dehydrogenase release assay has been developed that takes advantage of a new fluorescent amplification system (Page, B., et al., Anticancer Res. 1998 Jul-Aug;18(4A):2313-6). This approach is sensitive, rapid, and reproducible and may be used advantageously for mixed lymphocyte reaction (MLR). It may easily be further automated for large-scale cytotoxicity testing using cell membrane integrity, and is thus considered. In another fluorometric assay developed for detecting cell-mediated cytotoxicity, the fluorophore used is the non-toxic molecule AlamarBlue (Nociari et al., J. Immunol. Methods, 213(2): 157-167, 1998). The AlamarBlue is fluorescently quenched (i.e., low quantum yield) until mitochondrial reduction occurs, which then results in a dramatic increase in the AlamarBlue fluorescence intensity (i.e., increase in the quantum yield). This assay is reported to be extremely sensitive, specific and requires a significantly lower number of effector cells than the standard ⁵¹Cr release assay.

Other immune cells that can be induced by the present methods include natural killer cells (NK). NKs are lymphoid cells that lack antigen-specific receptors and are part of the innate immune system. Typically, infected cells are usually destroyed by T cells alerted by foreign particles bound to the cell surface MHC. However, virus-infected cells signal infection by expressing viral proteins that are recognized by antibodies. These cells can be killed by NKs. In tumor cells, if the tumor cells lose expression of MHC I molecules, then it may be susceptible to NKs.

### Formulations and Routes for Administration to Patients

Where clinical applications are contemplated, it will be necessary to prepare pharmaceutical compositions-expression constructs, expression vectors, fused proteins, transduced cells, activated T cells, transduced and loaded T cells--in a form appropriate for the intended application. Generally, this will entail preparing compositions that are essentially free of pyrogens, as well as other impurities that could be harmful to humans or animals.

The multimeric ligand, such as, for example, AP1903, may be delivered, for example at doses of about 0.01 to 1 mg/kg subject weight, of about 0.05 to 0.5 mg/kg subject weight, 0.1 to 2 mg/kg subject weight, of about 0.05 to 1.0 mg/kg subject weight, of about 0.1 to 5 mg/kg subject weight, of about 0.2 to 4 mg/kg subject weight, of about 0.3 to 3 mg/kg subject weight, of about 0.3 to 2 mg/kg subject weight, or about 0.3 to 1 mg/kg subject weight, for example, about 0.1, 0.2, 0.3, 0.4, 0.5, 0.6, 0.7, 0.8, 0.9, 1.0, 1.5, 2, 2.5, 3, 3.5, 4, 4.5, 5, 6, 7, 8, 9, or 10 mg/kg subject weight. In some embodiments, the ligand is provided at 0.4mg/kg per dose, for example at a concentration of 5mg/mL. Vials or other containers may be provided containing the ligand at, for example, a volume per vial of about 0.25 ml to about 10 ml, for example, about 0.25, 0.5, 1, 1.5, 2, 2.5, 3, 3.5, 4, 4.5, 5, 5.5, 6, 6.5, 7, 7.5, 8, 8.5, 9, 9.5, or 10 ml, for example, about 2 ml.

One may generally desire to employ appropriate salts and buffers to render delivery vectors stable and allow for uptake by target cells. Buffers also may be employed when recombinant cells are introduced into a patient. Aqueous compositions comprise an effective amount of the vector to cells, dissolved or dispersed in a pharmaceutically acceptable carrier or aqueous medium. Such compositions also are referred to as inocula. The phrase "pharmaceutically or pharmacologically acceptable" refers to molecular entities and compositions that do not produce adverse, allergic, or other untoward reactions when administered to an animal or a human. A pharmaceutically acceptable carrier includes any and all solvents, dispersion media, coatings, antibacterial and antifungal agents, isotonic and absorption delaying agents and the like. The use of such media and agents for pharmaceutically active substances is known. Except insofar as any conventional media or agent is incompatible with the vectors or cells, its use in therapeutic compositions is contemplated. Supplementary active ingredients also can be incorporated into the compositions.

The active compositions may include classic pharmaceutical preparations. Administration of these compositions will be via any common route so long as the target tissue is available via that route. This includes, for example, oral, nasal, buccal, rectal, vaginal or topical.

Alternatively, administration may be by orthotopic, intradermal, subcutaneous, intramuscular, intraperitoneal or intravenous injection. Such compositions would normally be administered as pharmaceutically acceptable compositions, discussed herein.

The pharmaceutical forms suitable for injectable use include sterile aqueous solutions or dispersions and sterile powders for the extemporaneous preparation of sterile injectable solutions or dispersions. In all cases the form is sterile and is fluid to the extent that easy syringability exists. It is stable under the conditions of manufacture and storage and is preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In certain examples, isotonic agents, for example, sugars or sodium chloride may be included. Prolonged absorption of the injectable compositions can be brought about by the use in the compositions of agents delaying absorption, for example, aluminum monostearate and gelatin.

For oral administration, the compositions may be incorporated with excipients and used in the form of non-ingestible mouthwashes and dentifrices. A mouthwash may be prepared incorporating the active ingredient in the required amount in an appropriate solvent, such as a sodium borate solution (Dobell's Solution). Alternatively, the active ingredient may be incorporated into an antiseptic wash containing sodium borate, glycerin and potassium bicarbonate. The active ingredient also may be dispersed in dentifrices, including, for example: gels, pastes, powders and slurries. The active ingredient may be added in a therapeutically effective amount to a paste dentifrice that may include, for example, water, binders, abrasives, flavoring agents, foaming agents, and humectants.

The compositions may be formulated in a neutral or salt form. Pharmaceutically acceptable salts include, for example, the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Upon formulation, solutions will be administered in a manner compatible with the dosage formulation and in such amount as is therapeutically effective. For example, a therapeutically effective amount of modified cells that express the PRAME TCRs discussed herein may be an amount that reduces the amount or concentration of a PRAME-expressing cell in a subject, as measured by, for examples, assays discussed heren in vivo in a subject, or in a mouse or other animal model. A therapeutically effective amount therefore, may be an amount sufficient to reduce the percent or amount of PRAME-expressing cells by 10, 20, 30, 40, 50, 60, 70, 80, 90, or 95%; a therapeutically effective amount may also, for example, be sufficient to result in stable disease, that is, the number or concentration of PRAME-expressing cells does not significantly increase. The formulations are easily administered in a variety of dosage forms such as injectable solutions, drug release capsules and the like. For parenteral administration in an aqueous solution, for example, the solution may be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media can be employed. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations may meet sterility, pyrogenicity, and general safety and purity standards as required by FDA Office of Biologics standards.

The administration schedule may be determined as appropriate for the patient and may, for example, comprise a dosing schedule where the PRAME TCR modified cells are administered at week 0, followed by administration of additional cells when needed to obtain an effective therapeutic result or, for example, at 2, 4, 6, 8, 10, 12, 14, 16, 18, 20 intervals thereafter for a total of, for example, 2, 4, 6, 8, 10, 12, 14, 16, 18, 20, 22, 24, 26, 28, or 30, 40, 50, 60, 70, 80, 90, or 100 weeks.

If needed, the method may further include additional leukaphereses to obtain more cells to be used in treatment.

The modified cells of the present application may be delivered in a single administration or multiple administrations of a total number of cells of, for example, 0.25 ×10⁶ (±20%) cells/kg, 0.5 ×10⁵ (±20%) cells/kg, 0.75 ×10⁵ (±20%) cells/kg, 1 ×10⁵ (±20%) cells/kg, 0.3 ×10⁶ (±20%) cells/kg, 0.625 ×10⁶ (±20%) cells/kg, 1.25 ×10⁶ (±20%) cells/kg, 2.5 ×10⁶ (±20%) cells/kg, 5 ×10⁶ (±20%) cells/kg, 7.5 ×10⁶ (±20%) cells/kg, 1 ×10⁷ (±20%) cells/kg, 2.5 ×10⁷ (±20%) cells/kg, 5 ×10⁷ (±20%) cells/kg, 7.5 ×10⁷ (±20%) cells/kg, or 1 ×10⁸ (±20%) cells/kg subject body weight.

Where activation, or inductionof the caspase-9 switch is needed, the administration of AP1903 or other chemical inducer may be determined as appropriate for the patient and may, for example, comprise a dosing schedule where the first dose is administered at week 0 of the start of CID therapy, followed by administration of additional chemical inducer when needed to obtain an effective therapeutic result or, for example, at 2, 4, 6, 8, 10, 12, 14, 16, 18, 20 intervals thereafter for a total of, for example, 2, 4, 6, 8, 10, 12, 14, 16, 18, 20, 22, 24, 26, 28, or 30, 40, 50, 60, 70, 80, 90, or 100 weeks.

### Methods for Treating a Disease

Described herein are methods of treatment or prevention of a disease caused by a hyperproliferative disease.

Preneoplastic or hyperplastic states which may be treated or prevented using the pharmaceutical composition (transduced T cells, expression vector, expression construct, etc.) include but are not limited to preneoplastic or hyperplastic states such as colon polyps, Crohn's disease, ulcerative colitis, breast lesions and the like.

Cancers, including solid tumors, which may be treated using the pharmaceutical composition include, but are not limited to primary or metastatic melanoma, adenocarcinoma, squamous cell carcinoma, adenosquamous cell carcinoma, thymoma, uveal melanoma, lymphoma, sarcoma, lung cancer, liver cancer, non-Hodgkin's lymphoma, Hodgkin's lymphoma, leukemias, uterine cancer, breast cancer, prostate cancer, ovarian cancer, pancreatic cancer, colon cancer, multiple myeloma, neuroblastoma, NPC, bladder cancer, cervical cancer and the like.

Other hyperproliferative diseases, including solid tumors, that may be treated using the T cell and other therapeutic cell activation system presented herein include, but are not limited to rheumatoid arthritis, inflammatory bowel disease, osteoarthritis, leiomyomas, adenomas, lipomas, hemangiomas, fibromas, vascular occlusion, restenosis, atherosclerosis, preneoplastic lesions (such as adenomatous hyperplasia and prostatic intraepithelial neoplasia), carcinoma in situ, oral hairy leukoplakia, or psoriasis.

In the method of treatment, the administration of the pharmaceutical composition (expression construct, expression vector, fused protein, transduced cells, and activated T cells, transduced and loaded T cells) may be for either "prophylactic" or "therapeutic" purpose. When provided prophylactically, the pharmaceutical composition is provided in advance of any symptom. The prophylactic administration of pharmaceutical composition serves to prevent or ameliorate any subsequent infection or disease. When provided therapeutically, the pharmaceutical composition is provided at or after the onset of a symptom of infection or disease. Thus the compositions presented herein may be provided either prior to the anticipated exposure to a disease-causing agent or disease state or after the initiation of the infection or disease. Thus provided herein are methods for prophylactic treatment of solid tumors such as those found in cancer, or for example, but not limited to, prostate cancer, using the nucleic acids and cells discussed herein. For example, methods are provided of prophylactically preventing or reducing the size of a tumor in a subject comprising administering a the nucleic acids or cells discussed herein, whereby the nucleic acids or cells are administered in an amount effect to prevent or reduce the size of a tumor in a subject.

Solid tumors from any tissue or organ may be treated using the present methods, including, for example, any tumor expressing PSA, for example, PSMA, in the vasculature, for example, solid tumors present in, for example, lungs, bone, liver, prostate, or brain, and also, for example, in breast, ovary, bowel, testes, colon, pancreas, kidney, bladder, neuroendocrine system, soft tissue, boney mass, and lymphatic system. Other solid tumors that may be treated include, for example, glioblastoma, and malignant myeloma.

The term "unit dose" as it pertains to the inoculum refers to physically discrete units suitable as unitary dosages for mammals, each unit containing a predetermined quantity of pharmaceutical composition calculated to produce the desired immunogenic effect in association with the required diluent. The specifications for the unit dose of an inoculum are dictated by and are dependent upon the unique characteristics of the pharmaceutical composition and the particular immunologic effect to be achieved.

An effective amount of the pharmaceutical composition would be the amount that achieves this selected result of enhancing the immune response, and such an amount could be determined. For example, an effective amount of for treating an immune system deficiency could be that amount necessary to cause activation of the immune system, resulting in the development of an antigen specific immune response upon exposure to antigen. The term is also synonymous with "sufficient amount."

The effective amount for any particular application can vary depending on such factors as the disease or condition being treated, the particular composition being administered, the size of the subject, and/or the severity of the disease or condition. One can empirically determine the effective amount of a particular composition presented herein without necessitating undue experimentation. Thus, for example, in one example, t he transduced T cells or other cells are administered to a subject in an amount effective to, for example, induce an immune response, or, for example, to reduce the size of a tumor or reduce the amount of tumor vasculature.

### A. Genetic Based Therapies

In certain embodiments, a cell is provided with an expression construct capable of providing a recombinant TCR polypeptide, such as, for example, PRAME specific recombinant TCR polypeptides, in a T cell. The discussion of expression vectors and the genetic elements employed therein is incorporated into this section by reference. In certain examples, the expression vectors may be viral vectors, such as adenovirus, adeno-associated virus, herpes virus, vaccinia virus lentivirus, and retrovirus. In another example, the vector may be a lysosomal-encapsulated expression vector.

Gene delivery may be performed in both in vivo and ex vivo situations. For viral vectors, one generally will prepare a viral vector stock. Examples of viral vector-mediated gene delivery ex vivo and in vivo are presented in the present application. For in vivo delivery, depending on the kind of virus and the titer attainable, one will deliver, for example, about 1, 2, 3, 4, 5, 6, 7, 8, or 9 × 10⁴, 1, 2, 3, 4, 5, 6, 7, 8, or 9 × 10⁵, 1, 2, 3, 4, 5, 6, 7, 8, or 9 × 10⁶, 1, 2, 3, 4, 5, 6, 7, 8, or 9 × 10⁷, 1, 2, 3, 4, 5, 6, 7, 8, or 9 × 10⁸, 1, 2, 3, 4, 5, 6, 7, 8, or 9 × 10⁹, 1, 2, 3, 4, 5, 6, 7, 8, or 9 × 10¹⁰, 1, 2, 3, 4, 5, 6, 7, 8, or 9 × 10¹¹ or 1, 2, 3, 4, 5, 6, 7, 8, or 9 × 10¹² infectious particles to the patient. Similar figures may be extrapolated for liposomal or other non-viral formulations by comparing relative uptake efficiencies. Formulation as a pharmaceutically acceptable composition is discussed below. The multimeric ligand, such as, for example, AP1903, may be delivered, for example at doses of about 0.1, 0.2, 0.3, 0.4, 0.5, 0.6, 0.7, 0.8, 0.9, 1.0, 1.5, 2, 2.5, 3, 3.5, 4, 4.5, 5, 6, 7, 8, 9, or 10 mg/kg subject weight.

### B. Cell based Therapy

Another therapy that is contemplated is the administration of engineered T cells, such as, for example, the administration of transduced T cells. The T cells may be engineered in vitro. Formulation as a pharmaceutically acceptable composition is discussed herein.

In cell based therapies, the engineered cells may be, for example, transduced with retroviral or lentiviral vectors coding for target antigen nucleic acids or transfected with target antigen nucleic acids, such as mRNA or DNA or proteins; pulsed with cell lysates, proteins or nucleic acids; or electrofused with cells. The cells, proteins, cell lysates, or nucleic acid may derive from cells, such as tumor cells or other pathogenic microorganism, for example, viruses, bacteria, protozoa, etc.

### C. Combination Therapies

In order to increase the effectiveness of the expression vectors presented herein, it may be desirable to combine these compositions and methods with an agent effective in the treatment of the disease.

In certain examples, anti-cancer agents may be used in combination with the present methods. An "anti-cancer" agent is capable of negatively affecting cancer in a subject, for example, by killing one or more cancer cells, inducing apoptosis in one or more cancer cells, reducing the growth rate of one or more cancer cells, reducing the incidence or number of metastases, reducing a tumor's size, inhibiting a tumor's growth, reducing the blood supply to a tumor or one or more cancer cells, promoting an immune response against one or more cancer cells or a tumor, preventing or inhibiting the progression of a cancer, or increasing the lifespan of a subject with a cancer. Anti-cancer agents include, for example, chemotherapy agents (chemotherapy), radiotherapy agents (radiotherapy), a surgical procedure (surgery), immune therapy agents (immunotherapy), genetic therapy agents (gene therapy), hormonal therapy, other biological agents (biotherapy) and/or alternative therapies.

In further examples antibiotics can be used in combination with the pharmaceutical composition to treat and/or prevent an infectious disease. Such antibiotics include, but are not limited to, amikacin, aminoglycosides (e.g., gentamycin), amoxicillin, amphotericin B, ampicillin, antimonials, atovaquone sodium stibogluconate, azithromycin, capreomycin, cefotaxime, cefoxitin, ceftriaxone, chloramphenicol, clarithromycin, clindamycin, clofazimine, cycloserine, dapsone, doxycycline, ethambutol, ethionamide, fluconazole, fluoroquinolones, isoniazid, itraconazole, kanamycin, ketoconazole, minocycline, ofloxacin), para-aminosalicylic acid, pentamidine, polymixin definsins, prothionamide, pyrazinamide, pyrimethamine sulfadiazine, quinolones (e.g., ciprofloxacin), rifabutin, rifampin, sparfloxacin, streptomycin, sulfonamides, tetracyclines, thiacetazone, trimethaprim-sulfamethoxazole, viomycin or combinations thereof. More generally, such an agent would be provided in a combined amount with the expression vector effective to kill or inhibit proliferation of a cancer cell and/or microorganism. This process may involve contacting the cell(s) with an agent(s) and the pharmaceutical composition at the same time or within a period of time wherein separate administration of the pharmaceutical composition and an agent to a cell, tissue or organism produces a desired therapeutic benefit. This may be achieved by contacting the cell, tissue or organism with a single composition or pharmacological formulation that includes both the pharmaceutical composition and one or more agents, or by contacting the cell with two or more distinct compositions or formulations, wherein one composition includes the pharmaceutical composition and the other includes one or more agents.

The terms "contacted" and "exposed," when applied to a cell, tissue or organism, are used herein to describe the process by which the pharmaceutical composition and/or another agent, such as for example a chemotherapeutic or radiotherapeutic agent, are delivered to a target cell, tissue or organism or are placed in direct juxtaposition with the target cell, tissue or organism. To achieve cell killing or stasis, the pharmaceutical composition and/or additional agent(s) are delivered to one or more cells in a combined amount effective to kill the cell(s) or prevent them from dividing.

The administration of the pharmaceutical composition may precede, be concurrent with and/or follow the other agent(s) by intervals ranging from minutes to weeks. In examples where the pharmaceutical composition and other agent(s) are applied separately to a cell, tissue or organism, one would generally ensure that a significant period of time did not expire between the times of each delivery, such that the pharmaceutical composition and agent(s) would still be able to exert an advantageously combined effect on the cell, tissue or organism. For example, in such instances, it is contemplated that one may contact the cell, tissue or organism with two, three, four or more modalities substantially simultaneously (i.e., within less than about a minute) with the pharmaceutical composition. In other examples, one or more agents may be administered within of from substantially simultaneously, about 1 minute, to about 24 hours to about 7 days to about 1 to about 8 weeks or more, and any range derivable therein, prior to and/or after administering the expression vector. Yet further, various combination regimens of the pharmaceutical composition presented herein and one or more agents may be employed.

In some embodiments, the chemotherapeutic agent may be a lymphodepleting chemotherapeutic. In other examples, the chemotherapeutic agent may be Taxotere (docetaxel), or another taxane, such as, for example, cabazitaxel. The chemotherapeutic may be administered before, during, or after treatment with the cells and inducer. For example, the chemotherapeutic may be administered about 1 year, 11, 10, 9, 8, 7, 6, 5, or 4 months, or 18, 17, 16, 15, 14, 13, 12,11, 10, 9, 8, 7, 6, 5, 4, 3, 2, weeks or 1 week prior to administering the first dose of activated nucleic acid. Or, for example, the chemotherapeutic may be administered about 1 week or 2, 3, 4, 5, 6, 7, 8, 9, 10, 11, 12, 13, 14, 15, 16, 17, or 18 weeks or 4, 5, 6, 7, 8, 9, 10, or 11 months or 1 year after administering the first dose of cells or inducer.

Administration of a chemotherapeutic agent may comprise the administration of more than one chemotherapeutic agent. For example, cisplatin may be administered in addition to Taxotere or other taxane, such as, for example, cabazitaxel.

Methods as presented herein include without limitation the delivery of an effective amount of an activated cell, a nucleic acid, or an expression construct encoding the same. An "effective amount" of the pharmaceutical composition, generally, is defined as that amount sufficient to detectably and repeatedly to achieve the stated desired result, for example, to ameliorate, reduce, minimize or limit the extent of the disease or its symptoms. Other more rigorous definitions may apply, including elimination, eradication or cure of disease. In some embodiments there may be a step of monitoring the biomarkers to evaluate the effectiveness of treatment and to control toxicity.

An effective amount of the modified cell may be determined by a physician, considering the individual patient. Factors to be considered may include, for example, the extent of the disease or condition, tumor size, extent of infection, metastasis, age, and weight. The dosage and number of administrations may be determined by the physician, or other clinician, by monitoring the patient for disease or condition symptoms, and for responses to previous dosages, for example, by monitoring tumor size, or the level or concentration of tumor antigen. In certain examples, the modified cells may be administered at a dosage of 10⁴ to 10⁹ modified cells/kg body weight, 10⁵ to 10⁶, 10⁹-10¹¹, or 10¹⁰-10¹¹ modified cells/kg body weight.

### D. Dose Escalation Study Evaluating Safety and Feasiblity of PRAME TCR-expressing T cells in Patients with Relapsed or Refractory Myeloid Neoplasms

PRAME TCR-expressing T cells that comprise a chimeric caspase-9-encoding nucleic acid (Cell A) may be administered to subjects having a condition or disease associated with expression of the PRAME antigen, for example, in subjects having a myeloid neoplasm. The caspase-9 safety switch (iCasp9) is included in the design of Cell A, providing a method of controlling the level of treatment, or stopping treatment, with the PRAME TCR-expressing T cells.

Phase I. open-label, non-randomized, feasibilty, safety and dose finding study. During Phase I MTD stage, subject receives one dose of Cell A on Day 0. The design consists of 5 cohorts of Cell A consisting of 3-6 subjects per cohort who is treated with Cell A following a 3+3 dose escalation/de-escalation schema. During the Phase Ib expansion stage, subjects receives highest tolerated dose of Cell A on Day 0. Uncontrolled toxicity will trigger the use of the dimerizer drug, rimiducid, which activates the CaspaCIDe suicide switch, thus eliminating the Cell A PRAME reactive T cells.

### Dosing design:

- If 1/3 experiences a DLT at the starting dose (Cohort 3); another three subjects are enrolled and treated in Cohort 3. The initial 3 patients are enrolled sequentially, with a 3 week followup after each patient before enrolling any further patients. All subsequent cohorts may be enrolled simultaneously.
- If 0/3 or 1/6 subjects experience a DLT in Cohort 3, (starting dose), then the subsequent subjects are enrolled in Cohort 4. If ≥2/6 subjects experience a DLT in Cohort 3; then the subsequent subjects are enrolled in Cohort 2.
- If 0/3 or 1/6 subjects experience a DLT in Cohort 4, then the subsequent subjects are enrolled in Cohort 5. If ≥2/6 subjects experience a DLT in Cohort 4 then Cohort 3 are declared the MTD and if only 3 subjects have been treated in Cohort 3, then an additional 3 subjects are enrolled to confirm MTD.
- If 0/3 or 1/6 subjects experience a DLT in Cohort 5, then Cohort 5 are declared the MTD if only 3 subjects have been treatedin Cohort 5, then an additional 3 subjects are enrolled to confirm MTD. If ≥2/6 subjects experience a DLT in Cohort 5 then Cohort 4 are declared the MTD if only 3 subjects have been treated in Cohort 4, then an additional 3 subjects are enrolled to confirm MTD.
- If 0/3 or 1/6 subjects experience a DLT in Cohort 2, then Cohort 2 are declared the MTD; if only 3 subjects have been treated in Cohort 2, then an additional 3 subjects are enrolled to confirm MTD. If ≥2/6 subjects experience a DLT in Cohort 2; the subsequent subjects are enrolled in Cohort 1.
- If 0/3 or 1/6 subjects experience a DLT in Cohort 1, then Cohort 1 are declared the MTD. If ≥2/6 subjects experience a DLT in Cohort 1; then the study is halted and the data evaluated by the Clinical Study Team including the sponsor and investigatiors.

### DLTs for Cell A treatment are defined as below:

New adverse events occuring in the first 21 days related to the Cell A infusion
- ≥ Grade 5 hematological toxicity related to Cell A;
- ≥ Grade 3 non-hematological toxicity including hypersensitivity reactions and autoimmune reactions related to Cell A infusion

Treatment effects are calulated compared to baseline using international working group criteria.

### Examples of Inclusion Criteria for a Dosing Study

1. Acute leukemia: Patients with refractory or relapsed AML, other than acute promyelocytic leukemia (APL)
2. Patients with a monosomal or complex karyotype may enroll at the time of day 14 biopsy after induction chemotherapy, if residual disease is identified.
3. Patients must express HLA-A2.01 and myeloid blasts must express PRAME.
4. Absolute lymphocyte count (ALC) > 300/mm3 or CD3+ >150 cells/ mm3.
5. Patients who have relapsed and are greater than 100 days after a stem cell transplant are eligible unless they have active GVHD requiring systemic immunosuppressive therapy.
6. Relapsed or refractory AML or MDS
   - AML patients must have > 5% bone marrow blasts at study entry, without alternative causality (e.g. bone marrow regeneration)
      - Relapsed or refractory AML according to the Modified International Working Group Criteria for AML
   - IPSS INT-2 or High Grade MDS (RAEB-2) with 10-19% blasts, not responding to hypomethylation therapy or IPSS INT-1, Int-2 or high grade MDS with recurrence after initial response.
7. Age >18 years.
8. Life expectancy of at least 2 months.
9. ECOG performance status: ≤2
10. Off previous cancer therapy for at least 14 days for prior cytotoxic agents of previously administered drug, prior to first study treatment administration, except hydroxyurea given only when needed for control of hyperleukocytosis. Persistent clinically significant toxicities from prior chemotherapy must not be greater than grade 1 at the time of enrollment. Chemotherapeutic agents may be given up to 5 days prior to T cell reinfusion if necessary to control rapidly growing disease.
11. Able to meet institutional criteria for T cell apheresis collection
12. Renal function:
   1. All patients must have a calculated creatinine clearance >40 mL/min according to Cockraft-Gault.
   2. Routine urinalysis must show no clinically significant abnormalities.
13. Adequate LFTs: Total bilirubin ≤3.0 × the institutional upper normal limits (ULN) with direct bilirubin < 1.6 x ULN.
14. ALT/AST and Alkaline Phosphatase <5× ULN
15. Acceptable coagulation status:
   - INR/PT ≤1.5 times upper limit of normal (ULN)
   - PTT <1.5 times ULN

**Table 1: Cell A Dose Level in Each Cohort Based on Subject's Actual Body Weight**

| Cohort | **Cell A** |
|---|---|
| 1 | 0.3 ×10⁶ (±20%) cells/kg |
| 2 | 0.625 ×10⁶ (±20%) cells/kg |
| 3 (starting dose) | 1.25 ×10⁶ (±20%) cells/kg |
| 4 | 2.5 ×10⁶ (±20%) cells/kg |
| 5 | 5 ×10⁶ (±20%) cells/kg |

### LIST OF ABBREVIATIONS AND TERMS

- ABW: Adjusted body weight
- AE: Adverse event
- APC: Antigen presenting cells
- BM: Bone marrow
- CR: Complete remission
- CRF: Case report forms
- CRO: Contract research organization
- CTL: Cytotoxic T lymphocytes
- DFS: Disease-free survival
- DLT: Dose limiting toxicity
- eCRF: Electronic case report form
- EDTA: Ethylenediaminetetraacetic acid
- EKG: Electrocardiogram
- FDA: Food and Drug Administration
- GCP: Good clinical practice
- G-CSF: Granulocyte-colony stimulating factor
- GMP: Good medical practice
- HAMA: Human anti-mouse antibody
- HIPAA: (US) Health Insurance Portability and Accountability Act
- HIV: Human immunodeficiency virus
- HSCT: Hematopoietic stem cell transplant
- ICF: Informed consent form
- ICH: International Conference on Harmonization
- IMP: Investigational medicinal product
- IRB: Institutional review board
- IV: Intravenous
- KPS: Karnofsky performance status
- LN2: Liquid nitrogen
- LVEF: Left ventricular ejection fraction
- MACS: Magnetically activated cell sorting
- MDS: Myelodysplastic syndrome
- MHC: Major histocompatibility complex
- MRD: Minimal residual disease
- MTD: Maximum tolerated dose
- NCI CTCAE: National Cancer Institute Common Terminology for Adverse Events
- NK: Natural killer
- NR: No response
- NRM: Non-relapse mortality
- OS: Overall survival
- PAgs: Non-peptide phosphoantigens
- PBMC: Peripheral blood mononuclear cell
- PBSC: Peripheral blood stem cell
- PD: Progressive disease
- PR: Partial response
- RCR: Replication competent retrovirus
- SAE: Serious adverse event
- SDV: Source data verification
- TCR: T-cell receptor
- TNC: Total nuclear cell
- TNF: Tumor necrosis factor
- TPHA: Treponema pallidum haemagglutination test
- WHO: World Health Organization

### Summary

Relapsed or Refractory Acute Myeloid Leukemia is a challenging clinical situation for patient survival with no clear path toward determining optimum management. Even with aggressive management, overall survival of refractory AML at 4 years is consistently poor: SWOG - 3% at 4 years for patients who are over age 60, HOVON-SAKK 7% overall survival at 5 years for patients under the age of 60 and 4% at 2 years in patients over the age of 60. A retrospective analysis study of 594 patients with AML undergoing second salvage therapy with standard therapies including stem cell transplant reported a median survival of 1.5 months with a one year survival of 8%. Standard algorithms recommend HSCT or "clinical trial with novel agent" in MDS patients with progressive disease (Sekeres, 2014). Clearly, there is an unmet clinical need for patients with myeloid neoplasms to bring their disease under control in order to qualify for potentially curative transplant.

PRAME is expressed in AML at a high frequency and at a level much higher than that detected in normal tissues. Since any off-tumor/on-target side effects should be addressed by the activation of the CaspaCIDe suicide switch and elimination of the Cell A T cells, TCR immunotherapy is a potential treatment strategy which should be evaluated in "no-option" patients with relapsed or refractory myeloid neoplasms.

In this dose-finding protocol, patients with relapsed or refractory AML or advanced hypomethylating agent-resistant MDS will have autologous T cells collected via apheresis and modified using a retrovirus to express a transgenic T-cell-receptor (TCR) that targets PRAME in context of a known class I HLA restricting element, HLA A2.01. The cells may be reinfused according to a dose-finding schedule after the patient has been identified as having adequate lymphopenia to provide for homeostatic expansion of the adoptively transferred, engineered T cell therapeutic product.

### Primary Refractory AML

For patients initially treated with aggressive induction chemotherapy (1 or 2 cycles), between 20-40% will not obtain a remission and are considered to have primary refractory disease (Cheson 2004). Primary refractory disease is more common identified in patients with a complex or monosomal karyotype, or a secondary treatment related AML or AML arising from antecedent hematologic disorders but can be seen across all AML disease risk stratifications. Approximately 10% of patients with AML under age 60 are identified with a monosomal karyotype within their leukemic clones while this molecular subtype is increasingly identified with age with ~ 20% of patients over age 60 expressing this resistant disease phenotype. With aggressive induction chemotherapy, in patients under the age of 60, the remission rate is approximately 14-50%, while in patients over the age of 60, the remission rate diminishes to 13-34% (Breems, 2008; Lowenberg, 2009; Medeiros 2010).

### Relapsed AML

Similarly disappointing are outcomes for patients with relapsed AML. Although varied in cytogenetic and biologic risk groups, 30-60% of patients with relapsed AML are able to obtain a second remission with salvage chemotherapy (Mangan, 2011) and data from MD Anderson Cancer Center demonstrate a median survival of 5.6 months for chemotherapy alone vs. 11.7 months in those patients who went on to a stem cell transplant (Armistead, 2009). If patients require a second salvage therapy, outcomes are extremely poor. A retrospective analysis study of 594 patients with AML undergoing second salvage therapy with standard therapies including stem cell transplant reported a median survival of 1.5 months with a one year survival of 8%. When the subgroup (13%) of those attaining a complete remission (CR) was analyzed, the median CR duration was 7 months. On multivariate analysis, a number of poor prognostic features were identified including age > 60, initial CR duration < 12 months, and second CR duration < 6 months (Giles, 2005). Importantly, standard therapies do not appear to favorably impact patients who relapse in < 12 months (Kumar, 2011).

### MDS

Patients with myelodysplastic syndrome (MDS) who have been treated with hypomethylating agents and have had progression of disease, or whose disease has failed to respond have a very poor prognosis with no evidence of benefit of any therapy over supportive care. Standard algorithms recommend "clinical trial with novel agent" in this situation (Sekeres, 2014). PRAME expression and overexpression has been identified in MDS by several groups. PRAME expression in MDS has been studied as a marker for higher risk disease, and worse outcomes have been noted, though this has not been validated in larger cohorts of patients. (Andrews, 2014).

### Treatment Options in Relapsed or Refractory Myeloid Neoplasms

### Transplantation

In patients with AML persistence or recurrence, the leukemia is considered not to be chemotherapy sensitive and alternative management strategies are required. Currently, the universal goal for these patients is to pursue allogeneic hematopoietic stem cell transplant (HSCT), assuming that they are not frail and can withstand the rigors of the procedure. If a patient with refractory, relapsed AML and is not able to obtain a clinical remission, and that patient has a suitable HLA-matched donor, a good performance status (>90% KPS) and no circulating blasts, long term overall survival after transplant is 20-30% (Craddock 2011, Duvall 2010). Without transplantation, there is no meaningful long term survival.

Elderly patients with acute leukemia, not eligible for allogeneic HSCT, generally have poor outcomes and thus, remain a significant therapeutic challenge. Although 30-40% of elderly patients with AML will achieve a CR with standard induction, the median survival in this population approximates 10 months (Oran 2012). The worse outcomes in this population have been attributed to high risk cytogenetics, higher rates of secondary leukemia, increased multidrug resistance, decreased performance status, significant co-morbidities, as well as poorly defined benefits of post remission consolidation and a high treatment related mortality (Oran 2012). Thus, a change in clinical strategy over the past decade has been to expand HSCT eligibility to attempt to include elderly patients (Hegenbart, 2006).

### Cell A: PRAME TCR Immunotherapy

As a therapeutic modality, adoptive T cell therapy has been shown to be an effective tool in managing relapse after allogeneic transplantation (Collins, 1997; Kolb, 1995; Porter, 2005). Unprimed donor derived, T cells adoptively transferred in the absence of immune suppression can be effective, particularly when combined with chemotherapy, and recapturing clinical remissions that can be sustained. Ex vivo expanded T cells that are restricted on HLA antigens and particular tumor peptides have been demonstrated to have benefit in small institutional series, using targets such as WT1 or PR1. Recently, the emergence of chimeric antigen receptor modified T cells, introduced into the autologous T cell product by lentivirus vectors, has been shown to be highly effective at targeting known lineage specific antigens in various disease states, with the greatest experience currently in the B-Cell malignancies (Porter, 2011; Grupp, 2013). These most recent advances have been rapidly commercialized as they have been able to be associated with standard manufacturing and generalizable to a variety of patients with malignancies expressing common antigens. However, therapies for AML and MDS have been elusive as CAR-T cell would, if efficacious, likely be associated with prolonged cytopenias.

Another peptide antigen of interest is derived from preferentially expressed antigen of melanoma (PRAME), which is a tumor antigen expressed on malignant cells in several tumor types, including AML and MDS. PRAME has a role in the regulation of retinoic acid signaling, a pathway which can be disrupted and is known to be critical in leukemogenesis.

PRAME is a member of the cancer-testis antigens family and is expressed at high levels in germinal tissues as well as some malignancies. It was initially identified in melanoma tissue, but has subsequently been detected in multiple cancers, including both lymphoid and myeloid malignancies. It has been identified by multiple groups on acute myeloid leukemia cells, and is considered a leukemia-associated antigen (LAA). PRAME is naturally immunogenic and exvivo expanded T cells are able to target, through the T cell receptor, PRAME peptide in context of HLA restricting elements, suggesting that this may be a target that can be harnessed for adoptive cell therapy.

PRAME over-expression has been studied and is present in approximately 32% of newly diagnosed AML patients (Goswami, 2014), 35% in a study by van Baren et al. (van Baren, 1998) and 55.4% in a study by Qin et al. (Qin, 2009). High expression was associated with t(8;21) and t(15;17) leukemia (40.7%) in a study by Ding et al. (Ding, 2012). Qin et al identified 74.4% and 56.6% expression and overexpression on bone marrow samples in patients with MDS (Qin 2009).

Targeting of the HLA restricted PRAME antigenic determinant may be similarly targeted for adoptive T cell therapy as an alternative cellular therapy for patients with refractory AML and MDS.

### PRAME Targeted TCR immunotherapy

There are safety risks associated with TCR immunotherapy, related to both on-target and off-target reactivity against healthy normal tissues. For example, the high-affinity TCRs generated for immunotherapy could pose a risk for on-target/off-tumor toxicity, as reported in a clinical trial of TCR T cells specific for MART-1, necessitating treatment for some patients. Therefore, it is important to select a target antigen whose expression in cancer and normal tissues is well understood. Because the PRAME antigen has been identified with expression in the kidney epithelial cells at low levels, careful monitoring of renal function with imaging and laboratory testing are performed.

The inclusion of a "safety switch" to remove the gene-modified T cells in the event of uncontrollable off-tumor/on target T cell toxicity would improve clinical safety. Cell A, a TCRbased therapy targeting the PRAME antigen is desgned to incorporatea CaspaCIDe suicide switch technology for the treatment of AML. The TCR expressed in the Cell A T cell product recognizes and binds to a PRAME peptide bound to HLA-A2 on a cancer cell surface, resulting in apoptosis in the tumor cell.

The TCR which has been developed has natural high affinitiy, but has not been further affinity enhanced. In addition, activation of the CaspaCIDe safety switch in response to administration of rimiducid results in activation of the iCaspase-9 cascade and the elimination of the Cell A T cells *in vivo.* This safety switch allows the patient to receive the benefits of adoptive T-cell therapy, while mitigating associated health risks.

T cells expressing the TCR expressed by CELL A showed high reactivity against a panel of PRAME-positive tumor cell lines and against primary AML cells, and no reactivity against normal cell types, with the exception of low reactivity against proximal tubular epithelial cells and intermediate reactivity against mature dendritic cells. The gene for the TCR α and β chains were sequenced and inserted, along with the iCasp9 suicide gene, in a retroviral vector which is used to transduce patient T cells to produce the Cell A TCR T cell immunotherapy product.

High affinity PRAME specific T cells were isolated from an AML patient after allogeneic stem cell transplantation (Amir et al CCR 2011). The T cells were found to recognize the PRAMEderived peptide SLLQHLIGL (SLL) described previously by Kessler et al (Kessler, 2001). Further analysis of these high affinity PRAME specific T cell clones showed high reactivity against a panel of PRAME-positive tumor cell lines and against metastatic melanoma, sarcomas, and primary AML cells, and no reaactivity against normal cell types, with the exception of low reactivity against proximal tubular epithelial cells and intermediate reactivity against mature dendritic cells. The gene for the TCR α and β chains of the PRAME specific T cell clone HSS1 (also called AAV54) were sequenced, and inserted along with the iCasp9 suicide gene in a retroviral vector to be used to transduce T cells to produce the Cell A TCR T cell immunotherapy product.

### In Vivo Preclinical Studies

Although immunodeficient mouse models have historically not been useful in predicting toxicities associated with unexpected, on target/off tumor toxicities, the use of in vivo mouse models with the Cell A PRAME TCR are useful in demonstrating both the efficacy of the killing of the PRAME-directed TCR, and the functional elimination of the Cell A T cells by rimiducid activation of the CaspaCIDe switch. Two *in vivo* studies were conducted, both in the immunedeficient NSG (NOD.CgPrkdcscid II2rgtm1WjI/SzJ; NSG) mouse model, using Cell A, HLA-A2.01-restricted, human T cells transduced with a γ-retrovirus encoding iCasp9 and the PRAME αβTCR . The Cell A construct contains the same cysteine-modification amino acid sequence as the cysteine-modified TCR HSS1 which was designed to minimize mispairing (Amir, 2011).

### Cell A Efficacy against PRAME -Expressing Tumor

Normal donor HLA-A2.01-restricted, human T cells were activated and transduced with the pSFG-iC9.2A.PRAME-derived Cell A vector. Cell A T cells and non-transduced (NT) control T cells were analyzed by flow cytometry for expression of Vβ1, the variable domain segment of the β chain of the Cell A TCR. U266 myeloma tumors were established in ten NSG mice by tail-vein injection of 2×10⁶ U266-EGFP-luciferase (U266-luc) tumor cells per animal. On day 24 post-tumor-engraftment, 5 mice received either 1×10⁷ non-transduced (NT) T cells or 1×10⁷ T cells transduced with Cell A via i.v. injection. IVIS imaging was performed on a weekly basis to measure tumor size.

Cell A demonstrated tumor elimination to background levels following treatment after a single i.v. injection of Cell A in less than two weeks, whereas injection of NT T cells did not control tumor growth (Figure 21). Using average radiance as a measure of tumor burden, tumors in mice treated with NT T cells had ~800-fold more cells than mice treated with Cell A T cells. These data indicate that Cell A, at a dose of 1×10⁷ T cells administered via i.v. injection, is effective against U266 myeloma tumors *in vivo.*

### In vivo Cell A Response to Rimiducid

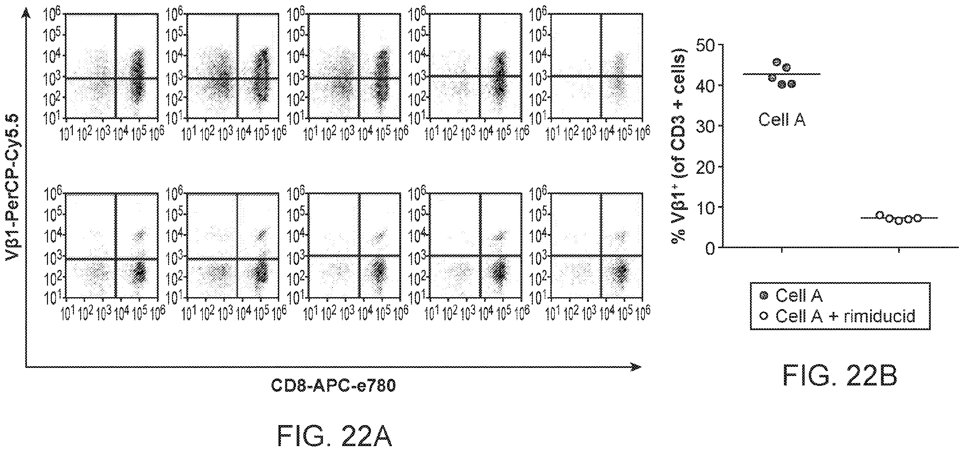

16 mice received an i.v. injection of 1×10⁷ Cell A T cells on Day 0 as outlined above. Forty eight hours later, eight of the mice were injected i.p. with rimiducid at 5 mg/kg to evaluate activation of iCasp9 and apoptosis of the Cell A T cells; the other eight mice were left untreated. Flow cytometric analysis of the spleens 24 hours after rimiducid administration demonstrated that, although non-transduced human T cells were detected in five animals in each group of eight, rimiducid had effectively eliminated the majority of transduced Cell A T cells in the treated animals: 42 ± 1.1% of the human T cells were Vβ1+ in untreated mice, compared to 7.3 ± 0.2% in the rimiducid-treated animals (p<0.0001) (Figure 22, Top row: untreated animals; bottom row: rimiducid-treated mice). Thus, the iCasp9 encoded within Cell A represents a functioning safety switch that can be triggered *in vivo* to eliminate Cell A cells.

### CaspaCIDe (iCasp9) switch remains sensitive in Cell A T cells after 51 days in vivo

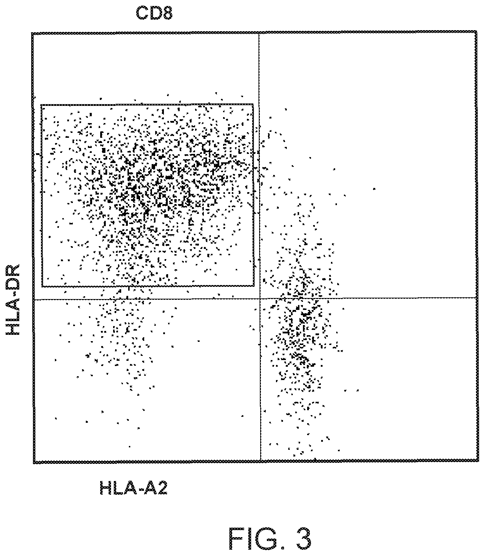

Spleen and bone marrow cells were harvested from mice treated either with NT- or Cell A-modified T cells on day 51 after T cell injection (day 74 post-tumor implantation), counted, and analyzed by flow cytometry for Vβ1 expression on CD4⁺ and CD8⁺ T cells. Even in animals who exhibited effective tumor elimination, Cell A T cells persisted in the spleen and bone marrow (Figure 3). There were ~600-fold more total CD8+ T cells and ~130-fold more Vβ1+CD8+ T cells in the spleens of mice that received Cell A T cells than in the mice that received non-transduced T cells.

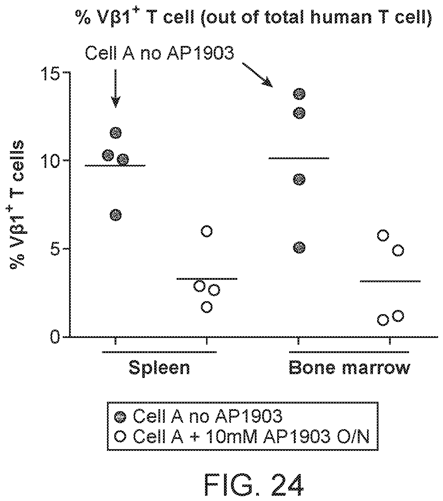

A fraction of spleen and bone marrow cells were isolated from the Cell A-treated animals as analyzed above, and were cultured overnight with or without 10 nM rimiducid in order to determine the sensitivity of the suicide switch. Rimiducid significantly reduced the fraction of Vβ1+ T cells recovered from both spleen and bone marrow (p<0.05), confirming that the persisting Cell A cells retain a functional iCasp9 suicide gene (Figure 24).

### Preclinical Conclusions

Following tail-vein injection into NSG mice, Cell A T cells can be detected in the spleen as early as 24 hours later, and in increased numbers at 48 hours post-injection. Cell A T cells show measureable persistence and antitumor efficacy against U266 myeloma cells when administered i.v. in NSG mice. Cell A-modified T cells, at a dose of 1×10⁷ T cells, eliminated U266 myeloma tumors in less than 2 weeks after administration, and controlled tumor growth for over 50 days.The iCasp9 encoded within Cell A functions in vivo as a safety switch that can be triggered by rimiducid administration to eliminate Cell A cells by inducing apoptosis. Despite the lack of detectable tumor by luciferase bioluminescence (measured by IVIS), Cell A T cells could be recovered from both the spleen and bone marrow at 51 days; these cells remained sensitive to subsequent rimiducid treatment.

These data suggest that the Cell A is effective against tumor cells expressing the PRAME/HLA-A2 antigen, and that the iCasp9 switch is effective in eliminating the transduced T cells following rimiducid treatment.

### Clinical Evaluation of Rimiducid Safety and Functionality

### Safety of TCR Cells Therapy in Humans

In general, one safety concern for TCR cell therapy is the risk of on-target off-tumor toxicity resulting from T-cell activation in normal tissues expression of the tumor antigen. Short term toxicities potentially consist of acute infusion reactions, anaphylaxis and cardiac arrest, on-target toxicities such as tumor lysis syndromes and cytokine release syndromes (CRS).

### Rimiducid / AP1903 Dimerizer Drug

AP1903 is a member of a new class of lipid-permeable compounds termed activating, or dimerizer, drugs that act by inducing clustering of engineered proteins inside cells. AP1903-inducible activation of the Caspase 9 suicide gene is achieved by expressing a chimeric protein (iCasp9), fused to a drug-binding domain derived from human FK506-binding protein (FKBP). This chimeric protein is quiescent inside cells until administration of AP1903, which cross-links the FKBP domains, initiating iCasp9 signaling. This signaling induces apoptosis of the gene modified cells. AP1903 is formulated at a concentration of 5 mg/ml in 25% Solutol HS 15 (a non-ionic solubilizer).

### Rimiducid in Human Volunteers

A Phase I, single blind, placebo controlled, ascending single intravenous dose study was performed in adult healthy subjects to determine the safety, tolerability and pharmacokinetics of rimiducid/(AP1903) (luliucci, 2001). Twenty-eight (28) subjects were enrolled into 5 treatment groups in which five dose levels of AP1903 were investigated (i.e., 0.01, 0.05, 0.1, 0.5 and 1.0 mg/kg). Within each group, 4 subjects received AP1903, 1 subject received placebo and 1 subject received normal saline. The only exception was in the 0.5 mg/kg treatment group in which 3 subjects received AP1903 and 1 subject received normal saline. Within each treatment group, the dose volumes for AP1903, placebo, and normal saline were equivalent on a body weight basis. All treatments were administered as intravenous infusions over a period of 2 hours. Clinical assessments included vital signs (supine blood pressure, supine pulse rate and oral body temperature) that were measured pre-dose and at 10 min, 20 min, 40 min, 1 hr., 1 hr. 20 min, 1 hr. 40 min, 2, 4, 8, 12 and 24 hours. after the start of the infusion and again on Day 7. A 12-lead resting ECG was performed pre-dose, 3 hours. and 24 hours after the start of the infusion and on Day 7. Continuous cardiac monitoring occurred from approximately 1 hr. prior to the start of the infusion and continued until 3 hours after the start of the infusion. AP1903 for Injection was shown to be safe and well tolerated at all dose levels and demonstrated a favorable pharmacokinetic profile.

### Clinical Functionality of AP1903 (rimiducid)

Ten subjects in the CASPALLO trial were treated with T cells containing the iCaspase-9 suicide switch and four subjects developed acute GVHD which was rapidly abrogated after administration of AP1903, with selective elimination of allo-reactive T cells through apoptosis. AP1903 resulted in elimination of ≥90% of inducible caspase-9 expressing T cells 30 minutes from the end of a 2 hour intravenous infusion. No adverse events were reported in association with the AP1903 infusion.

The DOTTI trial is a follow-up study to the CASPALLO trial. Three subjects subsequently developed Grade I & II acute GVHD and were treated with a single dose of the dimerizing drug AP1903. Four subjects are alive at a median of 476 days after transplant (range 278-674 days), and who had received AP1903. There were no immediate or delayed adverse events associated with AP1903.

### Study Design

This is a single arm, dose escalation/ de-escalation, single US center, phase 1 study to determine the safety and efficacy of autologous T cells genetically modified with a retroviral construct consisting of a/b T cell receptor reacting with PRAME peptide in context of restriction element HLA A2.01. The use of escalation de-escalation design is supported by the absence of effective therapy for patients with relapsed or refractory AML or MDS, and recognizing that to start at subtherapeutic dosing would likely lead to rapid leukemic progression and inability to determine if any efficacy signal was present. The study will enroll approximately up to 36 patients patients, which will allow 24 patients to be treated and assessed for efficacy. After assessment of eligibility, patients who qualify for the study are enrolled and plan to pursue T cell apheresis. Best practice efforts are used to treat the patients' relapsed and refractory leukemia or MDS, but generally will include a purine analog, incorporated within a standard AML or MDS salvage regimen, which will also duly accomplish the task of lymphodepletion, prior to activation of study treatment at the time of manufactured T-cell infusion.

Safety is monitored throughout the trial. Based upon established guidelines (FDA) for gene therapy products for advanced therapy products that utilize integrating vectors (e.g. retrovirus constructs), all patients treated with Cell A must be monitored for specific toxicities for a total of up to 15 years, irrespective of their response to the treatment agent. All patients are monitored in this trial for five years, followed by annual safety assessments in the separate long-term safety follow-up protocol. The purpose is to assess the risk of acute & delayed adverse events associated with the cellular therapy as well as to monitor RCR (replication competent retrovirus).

The efficacy of the treatment agent may be evaluated through the secondary endpoint of disease control, including the assessment of molecular, cytogenetic and hematologic complete and partial responses. A measure of clinical response is to determine the percent of patients who proceeded to HSCT for which they otherwise would not have been eligible.

### Dosing design:

- If 1/3 experiences a DLT at the starting dose (Cohort 3); another three subjects are enrolled and treated in Cohort 3. The initial 3 patients are enrolled sequentially, with a 3 week followup after each patient before enrolling any further patients. All subsequent cohorts may be enrolled simultaneously.
- If 0/3 or 1/6 subjects experience a DLT in Cohort 3, (starting dose), then the subsequent subjects are enrolled in Cohort 4. If ≥2/6 subjects experience a DLT in Cohort 3; then the subsequent subjects are enrolled in Cohort 2.
- If 0/3 or 1/6 subjects experience a DLT in Cohort 4, then the subsequent subjects are enrolled in Cohort 5. If ≥2/6 subjects experience a DLT in Cohort 4 then Cohort 3 may be declared the MTD and if only 3 subjects have been treated in Cohort 3, then an additional 3 subjects are enrolled to confirm MTD.
- If 0/3 or 1/6 subjects experience a DLT in Cohort 5, then Cohort 5 may be declared the MTD if only 3 subjects have been treatedin Cohort 5, then an additional 3 subjects are enrolled to confirm MTD. If ≥2/6 subjects experience a DLT in Cohort 5 then Cohort 4 may be declared the MTD if only 3 subjects have been treated in Cohort 4, then an additional 3 subjects are enrolled to confirm MTD.
- If 0/3 or 1/6 subjects experience a DLT in Cohort 2, then Cohort 2 may be declared the MTD; if only 3 subjects have been treated in Cohort 2, then an additional 3 subjects are enrolled to confirm MTD. If ≥2/6 subjects experience a DLT in Cohort 2; the subsequent subjects are enrolled in Cohort 1.
- If 0/3 or 1/6 subjects experience a DLT in Cohort 1, then Cohort 1 may be declared the MTD. If ≥2/6 subjects experience a DLT in Cohort 1; then the study are halted and the data evaluated by the Clinical Study Team including the sponsor and investigatiors.

**Table 2: Cell A Dose Level in Each Cohort Based on Subject's Actual Body Weight**

| Cohort | Cell A |
|---|---|
| 1 | 0.3 ×10⁶ (±20%) cells/kg |
| 2 | 0.625 ×10⁶ (±20%) cells/kg |
| 3 (starting dose) | 1.25 ×10⁶ (±20%) cells/kg |
| 4 | 2.5 ×10⁶ (±20%) cells/kg |
| 5 | 5 ×10⁶ (±20%) cells/kg |

The safety of dosing may be evaluated by the Clinical Study Team, which is comprised of the Sponsor (Responsible Medical Officer), Study Medical Monitor, and Investigators. The Clinical Study Team will review the emerging safety data from each cohort to determine if dose escalation or de-escalation will occur. Alternatively, a dose level intermediate between the nontolerated dose level and the previously tolerated dose level may be explored and declared the MTD if <2 out of 6 subjects experience a DLT at that dose.

If there are inadequate cells to meet the target dose for a given subject, the subject may be given available cells, but not be considered evaluable for determination of MTD.

Due to frequent co-morbidities and concurrent medications in the population under study, attribution of AEs to a particular drug is challenging. Therefore, all AEs that cannot clearly be determined to be unrelated to Cell A T cells may be considered relevant to determining DLTs and may be reviewed by the Clinical Study Team.

Prior to the non-mobilized T cell leukapheresis, the subject's blood count and differential may be collected and recorded. Infectious disease monitoring per the established regulatory guidelines may be performed. Subject must meet institutional criteria for CBC and platelets prior to intiation of leukapheresis. The leukocyte fraction may be collected using standardized continuous flow centrifugation. The subject may be monitored during apheresis. A standard apheresis procedure of up to approximately 3-4 blood volumes may be processed per institutional standard procedures, including precautions for leukemic patients. The volume processed and the duration of leukapheresis may be documented and recorded. If less than 5 × 10⁹ mononuclear cells are collected, the Medical Monitor must be consulted.

### PRAME-TCR cell (Cell A) Manufacturing Process

The starting material for Cell A production is a patient derived PBMC collection which is shipped fresh overnight to a centralized GMP manufacturing facility or from which the mononuclear cells have been previously selected and cryopreserved. The ficolled collection is cryopreserved in multiple aliquots, which are shipped to the centralized GMP manufacturing facility. Upon receipt and after verification of the acceptability of the starting material, an aliquot is transferred to the manufacturing cleanroom and rapidly thawed. The cells are washed and placed into culture media so that the T-cells will proliferate until achieving a target cell number for transduction. The expanded T cells are transduced with the Cell A retroviral construct. The cells are formulated with the cryopreservation media (CryoStor CS10) and cryopreserved. The final Cell A product is stored cryopreserved in the vapor phase of liquid nitrogen (LN2) until release testing is complete. The Cell A product may be shipped in the vapor phase of LN2 in validated cryoshippers. It is estimated that it will take approximately 2 to 4 weeks to manufacture and release Cell A for treatment.

Packaging and Formulation: Cell A T cells are cryopreserved in 10-15mL freezing medium (Cryostor, BioLife) and are stored frozen in cryostorage bags in the vapor phase of liquid nitrogen.

Shipping and Storage: Cryopreserved Cell A may be shipped in the vapor phase of LN2 to clinical sites in a validated shipping container. The receiving cell processing laboratory will store the product in vapor phase of LN2 until time of infusion. At that time, the product may be thawed at 37°C ± 2°C per instructions for infusion to the recipient. Depending on the day of shipment timing of shipping may be longer than 1 day.

### Chemotherapy and Lymphodepletion Prior to Cell A T cell InfusionChemotherapy Prior to Cell A T cell Infusion

Prior to treatment with the modified T cells, patients will receive bridging and simultaneously lymphodepleting chemotherapy with one of the following regimens based on clinician assessment of their disease biology and comorbidities, and in order to provide temporary disease control.
- FLAG-Ida (fludarabine, cytarabine, GCSF, idarubicin)
- FLAG (fludarabine, cytarabine, GCSF)
- single agent cladribine
- single agent cyclophosphamide as below

### Lymphodepletion Prior to Cell A T cell Infusion

If the subject's absolute lymphocyte count is below 1000 / ul then no lymphodepletion is required prior to the Cell A infusion. If the subject's ALC is above 1000 cells/ul, then lymphodepleting chemotherapy with either bendamustine 90 mg/m2 or fludaribine 90mg / m2 over 3 days with cyclophosphamide 750 mg/M2 may be administered no later than 3 days prior to infusion.

Additional lymphodepleting agents such as fludarabine could be considered for subsequent subjects at the discretion of the Investigator.

### Cell A T Cell Infusion

The Cell A cells may be thawed in a 37 °C water bath, diluted with 50mL Plasmalyte, 35mL initially, then 15mL to "rinse" the bag, as instructed in the Study Procedures Manual and administered over 15-30 minutes.

Subjects may be admitted in the hospital overnight for the administration of Cell A on Day 0. The maximum expected inpatient stay is 1 night unless unexpected AEs occur. The subject may be pretreated with acetaminophen and diphenhydramine per institutional standards for T cell products.

Monitoring may be undertaken according to institutional standards. Blood samples may be collected for biomarker monitoring prior to infusion, and at 4 hours post infusion. Biomarker monitoring may be batched and evaluated. The AE monitoring will continue for a minimum of 4 hours after infusion.

### Tumor evaluation and Biomarkers

### Tumor evaluation

Serial blood and bone marrow sampling to determine tumor response to treatment based on modified International Working Group (IWG) Response Criteria in AML (Cheson, 2003) and in MDS (Cheson, 2006).

Bone marrow biopsies may be completed at the following time points:
- within 7 days prior to enrollment
- within 7 days prior to T cell infusion
- day 4 after T cell infusion
- 28 days (+/- 1 day) after T cell infusion
- as clinically indicated

Biomarkers Serial blood sampling may be collected and cryopreserved for future analysis of selective cytokines and biomarkers.

### Rimiducid (AP1903) Dimerizer Drug Packaging, Labeling and Storage

Packaging and Formulation: The AP1903 for Injection is packaged in either a 10 mL or 2mL Type 1 clear glass serum vials. The contents of each vial is composed of the labeled content (40 mg or 10mg respectively) of AP1903 drug substance dissolved in a sterile, endotoxin free, 25% Solutol HS 15/Water for Injection solution at an AP1903 concentration of 5 mg/mL and at pH 5.0 - 7.5. Each vial is stoppered with a Teflon^{©} coated serum stopper and a flip-off seal.

Labeling: The primary product label (applied directly to the vial) for the AP1903 for Injection may contain the following information: product name, AP1903 for Injection; the manufacturer's lot number; product concentration, 5 mg/mL; volume of solution available in the vial; total AP1903 contents of the vial (40 mg or 10mg); a statement, "For IV Administration, contains no preservatives" and the IND notation, "Caution: New Drug-Limited by Federal Law to Investigational Use". The product may be labeled according to the requirement of each competent authority.

Storage: The AP1903 for Injection vials must be stored at 5°C ± 3°C (41°F ± 5°F) in a limited access, qualified refrigerator, and may be stored without light.

Preparation for Treatment: For use, the AP1903 may be diluted prior to administration. The AP1903 is administered via IV infusion at the target dose of 0.4 mg/kg diluted in normal saline with volume to be administered over 2 hours, using a DEHP-free saline bag and solution set. Details are included in the pharmacy manual.

### Suicide induction of PRAME-TCR with rimiducid

A dose of 0.4mg/kg of rimiducid (AP1903) may be infused. Acetaminophen, H1 and H2 blockers and any other standard institutional pretreatment/prophylaxis, are required prior to the infusion of rimiducid, as prophylaxis for potential anaphylactoid infusion reactions (potentially seen with Kolliphor products such as Solutol). The infusion of rimiducid shall be performed using non-DEHP saline bags and details can be found in rimiducid pharmacy manual. Blood samples may be collected for biomarker monitoring prior to infusion, and at 4 hours post infusion. Biomarker monitoring may be batched and evaluated. The AE monitoring will continue for a minimum of 4 hours after infusion. The maximum expected inpatient stay is 1 night unless unexpected AEs occur.

### On Target - Off Tumor

In the circumstance that the Cell A PRAME-TCR T cells react with a non-tumor target, then the patients should receive supportive care, and administration of rimiducid as determined by the Investigator.

### Other Investigational Agents

Other investigational agents or investigational biologics may not be administered 3 months after Cell A unless the patient's disease state is worsening or non-responsive. The subject may proceed to HSCT.

### CLINICAL EVALUATION

### Clinical Aassessment

- A clinical assessment of the subjects may be performed.
- Renal status may be assessed by evaluation of sequential Creatinine testing, and additional assessments as clinically indicated.
- Serial blood and bone marrow sampling to determine response to treatment based on modified International Working Group (IWG) Response Criteria in AML (Cheson, 2003) and in MDS (Cheson, 2006).
- Bone marrow biopsies may be completed at the following time points:
   ▪ -within 7 days prior to enrollment
   ▪ -within 7 days prior to T cell infusion
   ▪ -day 4 after T cell infusion
   ▪ -28 days (+/- 1 day) after T cell infusion
   ▪ -as clinically indicated
- Testing for minimal residual disease may be performed with each bone marrow aspirate beginning on day 4 using 2 methods as patients may have MRD that is detectable by only one methodology.
   ∘ Molecular testing for previously identified molecular abnormalities (Klco, 2015)
   ∘ Flow cytometry using 8 color flow cytometry (Grimwade, 2014)

### Cell A T Cell Functional Assays

Persistence of genetically modified T cells in peripheral blood, tumor tissues and any other cellular specimens as available may be performed by qPCR and or flow cytometry assay per the schedule in Table 2. In addition, pre-and post-Cell A infusion, PBMC samples may be analyzed by a PRAME-specific cytotoxic activity.

### General Statistical Approach

Descriptive statistics may be utilized to summarize demographic and baseline characteristics. All summary tables for quantitative parameters will display mean, standard deviation, median, range (minimum and maximum), as well as number of missing data, where relevant. All summary tables for qualitative parameters will display counts, percentages, and, number of missing data, where relevant.

### Dose-escalation algorithm

In the MTD stage, the design consists of 5 cohorts (Table 1). Cohorts consisting 3-6 subjects per cohort may be treated with the Cell A T cells following a 3+3 dose escalation/de-escalation schema. Subject will receive one dose of Cell A on Day 0.

The enrollment will start from Cohort 3. Subjects may be evaluated for dose liminting toxicities (DLTs) after the infusion of Cell A. DLTs observed may be used to determined if additional subjects shall be enrolled at the same dose level, higher dose level or lower dose level using the rules outline below:
- If 1/3 experiences a DLT at the starting dose (Cohort 3); another three subjects may be enrolled in Cohort 3. The initial 3 patients may be enrolled sequentially, with a 3 week followup after each patient before enrolling any further patients. All subsequent cohorts may be enrolled simultaneously.
- If 0/3 or 1/6 subjects experience a DLT in Cohort 3, (starting dose), then the following subjects may be enrolled in Cohort 4. If ≥2/6 subjects experience a DLT in Cohort 3; then the following subjects may be enrolled in Cohort 2.
- If 0/3 or 1/6 subjects experience a DLT in Cohort 4, then the following subjects may be enrolled in Cohort 5. If ≥2/6 subjects experience a DLT in Cohort 4 then Cohort 3 may be declared the MTD and if only 3 subjects have been enrolled in Cohort 3, then an additional 3 subjects may be enrolled to confirm MTD.
- If 0/3 or 1/6 subjects experience a DLT in Cohort 5, then Cohort 5 may be declared the MTD if only 3 subjects have been enrolled in Cohort 5, then an additional 3 subjects may be enrolled to confirm MTD. If ≥2/6 subjects experience a DLT in Cohort 5 then Cohort 4 may be declared the MTD if only 3 subjects have been enrolled in Cohort 4, then an additional 3 subjects may be enrolled to confirm MTD.
- If 0/3 or 1/6 subjects experience a DLT in Cohort 2, then Cohort 2 may be declared the MTD; if only 3 subjects have been enrolled in Cohort 2, then an additional 3 subjects may be enrolled to confirm MTD. If ≥2/6 subjects experience a DLT in Cohort 2; the following subjects may be enrolled in Cohort 1.
- If 0/3 or 1/6 subjects experience a DLT in Cohort 1, then Cohort 1 may be declared the MTD. If ≥2/6 subjects experience a DLT in Cohort 1; then the study may be halted and the data evaluated by the Clinical Study Team.

DLTs are defined as below:
Cell A: DLTs may be based on new adverse events occuring in the first 28 days of therapy and the adverse events must be drug related (i.e. definitely, probably or possibly):
- ≥ Grade 3 CRS related toxicity or other Grade 3 organ toxicity

### REFERENCES

Amir AL, van der Steen DM, van Loenen MM, Hagedoom RS, de Boer R, Kester MDG, de Ru AH, Lugthart G-J, van Kooten C, Heimstra PS, Jedema I, Griffoen M, van Veelen PA, Falkenburg JHF, Heemskerk MHM. PRAME-Specific Allo-HLA-Restricted T Cells with Potent Antitumor Reactivity Useful for therapeutic T-Cell Receptor Gene Transfer. Clin Cancer Res. 17:5615-5625, 2011.

Andrews RK, Gardiner EE et al. Lymphomania. Blood. 2014 May 15;123(20):3057-8.

Armistead PM, de Lima M et al. Quantifying the survival benefit for allogeneic hematopoietic stem cell transplantation in relapsed acute myelogenous leukemia. Biol Blood Marrow Transplant. 2009; 15: 1431-38.

Breems DA et al. Monosomal karyotype in acute myeloid leukemia: a better indicator of poor prognosis than a complex karyotype. J Clin Oncol. 2008; 26(29):4791-7.

Chen Y, Cortes J, Estrov Z, et al. Persistence of cytogenetic abnormalities at complete remission after induction in patients with acute myeloid leukemia: prognostic significance and the potential role of allogeneic stem-cell transplantation. J Clin Oncol. 2011; 29:2507.

Cheson BD, Bennett JM, Kopecky KJ, et al. Revised recommendations of the International Working Group for Diagnosis, Standardization of Response Criteria, Treatment Outcomes, and Reporting Standards for Therapeutic Trials in Acute Myeloid Leukemia. J Clin Oncol. 2003; 21:4642.

Cheson BD. Overview of the revised response criteria for acute myelogenous leukemia. Clin Adv Hematol Oncol. 2004;2(5):277-9.

Cheson BD, Greenberg PL et al. Clinical application and proposal for modification of the International Working Group (IWG) response criteria in myelodysplasia. Blood. 2006 Jul 15;108(2):419-25.

Collins RH Jr, Shpilberg O et al. Donor leukocyte infusions in 140 patients with relapsed malignancy after allogeneic bone marrow transplantation. J Clin Oncol. 1997 Feb;15(2):433-44.

Craddock C, Labopin M, et al. Fac- tors predicting outcome after unrelated donor stem cell transplantation in primary refractory acute myeloid leukaemia. Leukemia. 2011; 25:808-813.

de Greef GE, van Putten WL, Boogaerts M, et al. Criteria for defining a complete remission in acute myeloid leukaemia revisited. An analysis of patients treated in HOVON-SAKK cooperative group studies. Br J Haematol. 2005; 128:184.

Ding K, Wang XM et al. PRAME Gene Expression in Acute Leukemia and Its Clinical Significance. Cancer Biol Med. 2012 Mar;9(1):73-6.

Craddock C, Labopin M, et al. Fac- tors predicting outcome after unrelated donor stem cell transplantation in primary refractory acute myeloid leukaemia. Leukemia. 2011; 25:808-813. Duval M, Klein JP et al. Hematopoietic stem-cell transplantation for acute leukemia in relapse or primary induction failure. J Clin Oncol. 2010; 28:3730-38.

Epping, MT, Wang, L et al. The human tumor antigen PRAME is a dominant repressor of retinoic acid receptor signaling. Cell. 2005;122: 835-847.

Giles F et al. Outcome of patients with acute myelogenous leukemia after second salvage therapy. Cancer. 2005;104(3):547-54.

Goswami M, Hensel N et al. Expression of putative targets of immunotherapy in acute myeloid leukemia and healthy tissues. Leukemia. 2014 May;28(5):1167-70.

Grimwade D, Freeman SD et al. Defining minimal residual disease in acute myeloid leukemia: which platforms are ready for "prime time"? Blood. 2014 Nov 27;124(23):3345-55.

Grupp SA, Kalos M et al. Chimeric antigen receptor-modified T cells for acute lymphoid leukemia. N Engl J Med. 2013 Apr 18;368(16):1509-18.

Hegenbart U, Niederwieser et al. Treatment for acute myelogenous leukemia by low-dose, total-body, irradiation-based conditioning and hematopoietic cell transplantation from related and unrelated donors. J Clin Oncol. 2006 Jan 20;24(3):444-53.

Ikeda, H, Lethe, B et al. Characterization of an antigen that is recognized on a melanoma showing partial HLA loss by CTL expressing an NK inhibitory receptor. Immunity. 1997; 6:199-208.

luliucci JD, Oliver SD et al. Intravenous safety and pharmacokinetics of a novel dimerizer drug, AP1903, in healthy volunteers. J Clin Pharmacol. 2001 Aug;41(8):870-9.

Kessler JH, Beekman NJ, Bres-Vloemans SA, Verdijk P, van Veelen PA, Kloosterman-Joosten AM, et al. Efficient identification of novel HLA-A(*)0201-presented cytotoxic T lymphocyte epitopes in the widely expressed tumor antigen PRAME by proteasome-mediated digestion analysis. J Exp Med. 2001;193:73-88.

Klco JM, Miller CA et al. Association Between Mutation Clearance After Induction Therapy and Outcomes in Acute Myeloid Leukemia. JAMA. 2015 Aug 25;314(8):811-22.

Kolb HJ, Schattenberg A et al. Graft-versus-leukemia effect of donor lymphocyte transfusions in marrow grafted patients. Blood. 1995 Sep 1;86(5):2041-50.

Kumar CC. Genetic abnormalities and challenges in the treatment of acute myeloid leukemia. Genes Cancer. 2011 Feb;2(2):95-107.

Löwenberg B, Ossenkoppele GJ et al. High-dose daunorubicin in older patients with acute myeloid leukemia. N Engl J Med. 2009;361(13):1235-48.

Mangan J and Luger S. Salvage therapy for relapsed or refractory acute myeloid leukemia. Ther Adv Hematol. 2011; Apr; 2(2): 73-82.

Marcucci G, Mrózek K, Ruppert AS, et al. Abnormal cytogenetics at date of morphologic complete remission predicts short overall and disease-free survival, and higher relapse rate in adult acute myeloid leukemia: results from cancer and leukemia group B study 8461. J Clin Oncol. 2004; 22:2410.

Medeiros BC, Othus M et al. Prognostic impact of monosomal karyotype in young adult and elderly acute myeloid leukemia: the Southwest Oncology Group (SWOG) experience. Blood. 2010 Sep 30;116(13):2224-28.

Oran B and Weisdorf DJ. Survival for older patients with acute myeloid leukemia: a population-based study. Haematologica. 2012 Dec; 97(12): 1916-24.

Porter DL, June CH et al. T-cell reconstitution and expansion after hematopoietic stem cell transplantation: 'T' it up! Bone Marrow Transplant. 2005 May;35(10):935-42.

Porter DL, Levine BL et al. Chimeric antigen receptor-modified T cells in chronic lymphoid leukemia. N Engl J Med. 2011 Aug 25;365(8):725-33.

Qin Y, Zhu H et al. Expression patterns of WT1 and PRAME in acute myeloid leukemia patients and their usefulness for monitoring minimal residual disease. Leuk Res. 2009 Mar;33(3):384-90.

Sekeres M and Cutler C. How we treat higher-risk myelodysplastic syndromes. Blood. 2014;123(6): 829-36.

van Baren N, Chambost H et al. PRAME, a gene encoding an antigen recognized on a human melanoma by cytolytic T cells, is expressed in acute leukaemia cells. Brit. J. Haemat. 1998;102: 1376-79.

Complete remission in AML has been defined using the following criteria developed by an International Working Group (Dohner et al, 2010; Cheson et al, 2001; de Greef et al, 2005):
- Normal values for absolute neutrophil count (>1000/microL) and platelet count (>100,000/microL), and independence from red cell transfusion.
- A bone marrow biopsy that reveals no clusters or collections of blast cells. Extramedullary leukemia (eg, central nervous system or soft tissue involvement) must be absent.
- A bone marrow aspiration reveals normal maturation of all cellular components (ie, erythrocytic, granulocytic, and megakaryocytic series). There is no requirement for bone marrow cellularity.
- Less than 5 percent blast cells are present in the bone marrow, and none can have a leukemic phenotype (eg, Auer rods). The persistence of dysplasia is worrisome as an indicator of residual AML but has not been validated as a criterion for remission status.
- The absence of a previously detected clonal cytogenetic abnormality (ie, complete cytogenetic remission, CRc) confirms the morphologic diagnosis of CR but is not currently a required criterion. However, conversion from an abnormal to a normal karyotype at the time of first CR is an important prognostic indicator, supporting the use of CRc as a criterion for CR in AML (Cheson, 2003; Marcucci et al, 2004; Chen et al, 2011).
- CR with incomplete platelet recovery (CRp): All CR criteria except for residual thrombocytopenia (platelet counts <100 x 109/L [100,000/µL])
- CR with incomplete recovery (CRi): All CR criteria except for residual neutropenia (absolute neutrophil count <1.0 x 109/L [1000/µL])

### Modified Caspase-9 Polypeptides with Lower Basal Activity and Minimal Loss of Ligand IC50

Basal signaling, signaling in the absence of agonist or activating agent, is prevalent in a multitude of biomolecules. For example, it has been observed in more than 60 wild-type G protein coupled receptors (GPCRs) from multiple subfamilies [1], kinases, such as ERK and abl [2], surface immunoglobulins [3], and proteases. Basal signaling has been hypothesized to contribute to a vast variety of biological events, from maintenance of embryonic stem cell pluripotency, B cell development and differentiation [4-6], T cell differentiation [2, 7], thymocyte development [8], endocytosis and drug tolerance [9], autoimmunity [10], to plant growth and development [11]. While its biological significance is not always fully understood or apparent, defective basal signaling can lead to serious consequences. Defective basal Gₛ protein signaling has led to diseases, such as retinitis pigmentosa, color blindness, nephrogenic diabetes insipidus, familial ACTH resistance, and familial hypocalciuric hypercalcemia [12, 13].

Even though homo-dimerization of wild-type initiator Caspase-9 is energetically unfavorable, making them mostly monomers in solution [14-16], the low-level inherent basal activity of unprocessed Caspase-9 [15, 17] is enhanced in the presence of the Apaf-1-based "apoptosome", its natural allosteric regulator [6]. Moreover, supra-physiological expression levels and/or co-localization could lead to proximity-driven dimerization, further enhancing basal activation. The modified cells of the present application may comprise nucleic acids coding for a chimeric Caspase-9 polypeptide having lower basal signaling activity. Examples of Caspase-9 mutants with lower basal signaling are provided in the table below. Polynucleotides comprising Caspase-9 mutants with lower basal signaling may be expressed in the modified cells used for cell therapy herein. In these examples, the modified cells may include a safety switch, comprising a polynucleotide encoding a lower basal signaling chimeric Caspase-9 polypeptide. In the event of an adverse event following administration of the modified cells comprising the chimeric stimulating molecules or chimeric antigen receptors herein, Caspase-9 activity may be induced by administering the dimerizer to the patient, thus inducing apoptosis and clearance of some, or all of the modified cells. In some examples, the amount of dimerizer administered may be determined as an amount designed to remove the highest amount, at least 80% or 90% of the modified cells. In other examples, the amount of dimerizer administered may be determined as an amount designed to remove only a portion of the modified cells, in order to alleviate negative symptoms or conditions, while leaving a sufficient amount of therapeutic modified cells in the patient, in order to continue therapy. Methods for using chimeric Caspase-9 polypeptides to induce apoptosis are discussed in PCT Publication Number WO 2011/146862 by Malcolm K. Brenner et al., titled Methods for Inducing Selective Apoptosis, filed May 20, 2011, and in United States Patent Publication Number US 2011/0286980 by Malcolm K. Brenner et al., titled Methods for Inducing Selective Apoptosis, filed May 20, 2011, issued July 28, 2015 as U.S. Patent Serial Number 9,089,520. Chimeric caspase polypeptides having modified basal activity are discussed in PCT Publication Number WO2014/164348 by David Spencer et al., titled Modified Caspase Polypeptides and Uses Thereof, filed March 7, 2014, published October 9, 2014 and in United States Patent Publication Number US2014/0255360 by David Spencer et al., titled Modified Caspase Polypeptides and Uses Thereof, filed March 7, 2014; and in U.S. Patent Publication Number US 2015/0328292 by Spencer et al., filed March 6, 2015. Methods for inducing partial apoptosis of the therapeutic modified cells are discussed in PCT Publication Number WO 2014/197638 by Kevin Slawin et al., titled Methods for Inducing Partial Apoptosis Using Caspase Polypeptides, filed June 4, 2014, published December 11, 2014
and in United States Patent Publication Number US 2016/0151465 by Kevin Slawin et al., titled Methods for Inducing Partial Apoptosis Using Caspase Polypeptides, filed June 4, 2014.

**Table 3: Caspase Mutant Classes and Basal Activity**

| **Basal Activity** | **Homodimerization domain** | **Cleavage sites & XIAP Interaction** | **Phosphor ylation** | **Double mutants, Misc.** | **Total mutants** |
|---|---|---|---|---|---|
| **Decreased basal and similar IC₅₀** | | | S144A | | 80 |
| | | | S144D | | ^{∗} , predicted |
| | | T317S | S196D | | |
| **Decreased basal but higher IC₅₀** | **N405Q** | **D330A** | S183A | D330A-N405Q | **Bold,** Tested in T cells |
| | ⁴⁰²GCFNF⁴⁰¹ISAQT (Casp-10) | **D330E** | S195A | D330A-5144A | |
| | **F404Y** | **D330G** | S196A | D330A-S144D | |
| | F406A | **D330N** | | D330A-S183A | |
| | **F406W** | **D330S** | | D330A-S196A | |
| | **F406Y** | **D330V** | | N405Q-S144A | |
| | **N405Qco** | L329E | | N405Q-S144D | |
| | | T317A | | N405Q-S196D | |
| | | | | N405Q-T317S | |
| | | | | *^{∗}N405Q-S144Aco* | |
| | | | | *^{∗}N405Q-T317Sco* | |
| **Decreased basal but much higher IC₅₀** | F404T | D315A | Y153A | | |
| | F404W | A316G | Y153F | | |
| | N405F | F319W | S307A | | |
| | **F406T** | | | | |
| **Similar basal and IC₅₀** | C403A | ³¹⁶ATPF³¹⁹AVPI (SMAC/Diablo) | | | |
| | C4035 | T317C | | | |
| | C403T | P318A | | | |
| | N405A | F319A | | | |
| **Increased basal** | N405T | T317E | | D330A-N405T | |
| | | F326K | | | |
| | | D327G | | | |
| | | D327K | | | |
| | | D327R | | | |
| | | Q328K | | | |
| | | Q328R | | | |
| | | L329G | | | |
| | | L329K | | | |
| | | A331K | | | |
| **Catalytically dead** | ⁴⁰²GCFNF⁴⁰⁶AAAAA | | | C285A | |
| | ⁴⁰²GCFNF⁴⁰⁶YCSTL (Casp-2) | | | D315A-D330A | |
| | ⁴⁰²GCFNF⁴⁰⁶CIVSM (Casp-3) | | | D330A-Y153A | |
| | ⁴⁰²GCFNF⁴⁰⁶QPTFT (Casp-8) | | | D330A-Y153F | |
| | G402A | | | D330A-T317E | |
| | G402I | | | | |
| | G402Q | | | | |
| | G402Y | | | | |
| | C403P | | | | |
| | F404A | | | | |
| | F404S | | | | |
| | F406L | | | | |

### Generation of the Retroviral Vector Construct

A human TCR specific for the PRAME-derived peptide SLLQLIGL (PRAME) discussed in the present application was designed using a codon-optimized and cysteine-modified TCR-AV1S1 and TCR-BV1 S1 linked by the T2A sequence, following isolation and identification of a T cell clone that recognizes the SLLQLIGL (Heemskerk 2001; Heemskerk, 2004; van Loenen, 2011) polypeptide. The TCRalpha and TCR betachain-encoding nucleotide sequences were codon optimized and cysteine modified to prevent mixed TCR dimer formation and to optimize TCR expression.

The retroviral construct SFG.iCasp9.2A.SLL-TCR (also called SFG.iCasp9.2A.PRAME) encodes a synthetic ligand-inducible human caspase-9 cDNA (iCasp9) linked to the alpha and beta chains of a human TCR specific for the PRAME-derived peptide SLLQLIGL (PRAME 425-433).

The functional components important for activity are:
- 5' LTR - Retroviral long terminal repeat at 5' end of vector (functions as promoter sequence)
- ψ - Retroviral encapsidation signal (psi; necessary for packaging of RNA into virion particles)
- SA - splice acceptor site
- iCasp9 - the inducible caspase-9 expression cassette. iCasp9 consists of the human FK506-binding protein (FKBP12) with an F36V mutation, connected via a 6 amino-acid Gly-Ser linker to a modified CARD domain-deleted human caspase-9
- FKBP12-F36V -an engineered FK506-binding protein containing F36V mutation to optimize binding affinity for AP1903. The FKBP12-F36V protein domain serves as the drug-binding/oligomerization domain of linked therapeutic proteins. FKBP12-F36V functions as a regulator of caspase-9: in the absence of AP1903, iCasp9 has minimal activity; AP1903 binding to FKBP12-F36V promotes dimerization and brings two caspase-9 molecules into apposition to initiate apoptosis. Thus, the FKBP12-F36V moiety functionally replaces the endogenous dimerization/ activation module (Caspase Activation and Recruitment Domain; CARD) of caspase-9 that mediates Apaf-1-associated oligomerization.
- Linker - synthetic Ser-Gly-Gly-Gly-Ser-Gly peptide linker used to fuse switch-regulator sequences to caspase-9.
- Caspase-9 - Human caspase-9 cDNA sequence (critical pro-apoptotic regulator) and therapeutic component of construct (regulated suicide gene). The endogenous dimerization/activation module (Caspase Activation and Recruitment Domain; CARD) was deleted to reduce spontaneous Apaf1-binding and hence background killing.
- 2A - encodes a synthetic 20 amino acid peptide from *Thosea Asigna* insect virus, which functions as a cleavable linker between the caspase-9 protein and TCR proteins.
- TCR- The human TCR specific for the PRAME-derived SLLQLIGL peptide. It consists of an α and β chain.
- TCRβ- sequence of the beta chain of the PRAME-TCR, belonging to Vbeta1 family. It consists of a variable and a constant region, in which a S57C modification has been inserted to increase the TCR chain pairing.
- 2A - encodes a synthetic 20 amino acid peptide from *Thosea Asigna* insect virus, which functions as a cleavable linker between the caspase-9 protein and TCR proteins. The native sequence has been codon optimized to reduce recombination events with the first 2A sequence.
- TCRα- sequence of the beta chain of the PRAME-TCR, belonging to Valfa8 family. It consists of a variable and a constant region, in which a T48C modification has been inserted to increase the TCR chain pairing.
- 3' LTR - Retroviral long terminal repeat at 3' end of vector (functions as terminator/ polyadenylation sequences).

### Vector Diagram

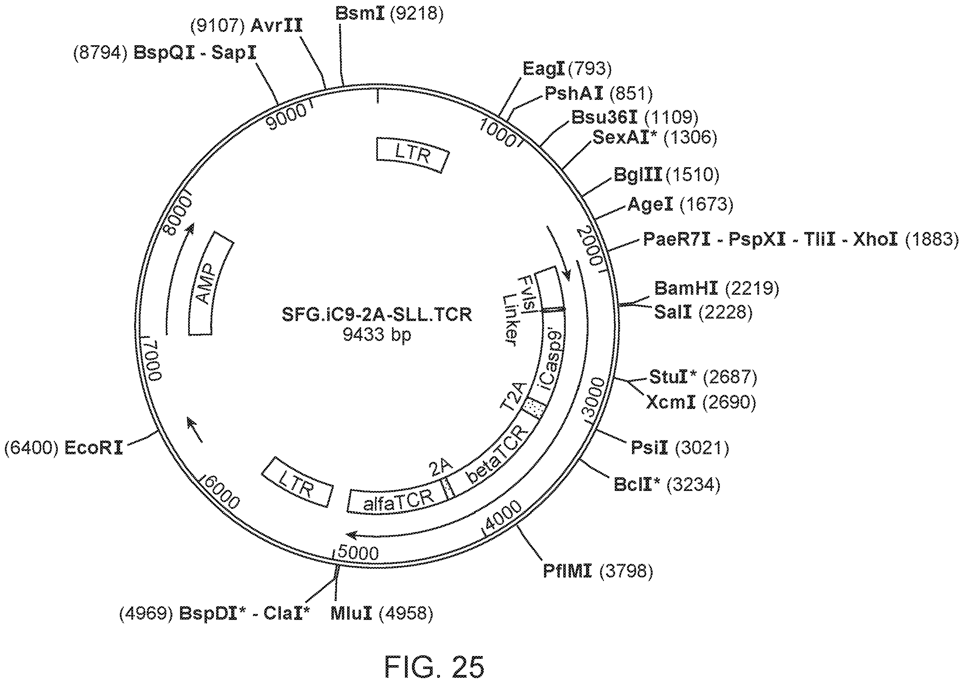

The plasmid map of the retroviral shuttle vector (SFG.iCasp9.2A.SLL-TCR) used in product manufacture is shown in Figure 25. The unique restriction sites and transgene features are illustrated.

The functional elements of the construct are defined in Table 4 below.

**Table 4**

| Component | Start | End |
|---|---|---|
| LTR | 1 | 590 |
| iCasp9 cassette | 1880 | 3091 |
| FKBP12-F36V | 1880 | 2209 |
| Linker peptide | 2210 | 2227 |
| Casp9 | 2234 | 3091 |
| 2A peptide | 3092 | 3145 |
| TCRβ | 3146 | 4078 |
| 2A peptide | 4079 | 4132 |
| TCRa | 4133 | 4954 |
| LTR | 5158 | 5707 |
| AmpR marker | 7002 | 7862 |

A reference electronic vector sequence was assembled by combining the DNA sequence files for each component of the vector construct. Since the retroviral genome is RNA-based, sequence analysis was performed on the plasmid DNA used for transfection into the 293VEC cell line (initial step in retroviral product preparation). Bi-directional sequencing was performed at Ospedale Pediatrico Bambino Gesù (OPBG) (Rome, Italy) on the entire vector. Sequencing runs were assembled using SnapGene software. No mismatched bases compared to the theoretical reference electronic sequence were identified. TCRβ S57C and TCRa-T48C were introduced to increase the pairing between the α- and β-TCR chains. Thus, these differences are not mutations, but were actually engineered into the parental construct.

A nucleotide deviation from the reference sequence occurred within the Caspase-9 coding domain (nt 2493), which substitutes a glutamine for an arginine. This reflects a naturally occurring common polymorphism in human Caspase-9 and was present in the initial Caspase-9 cDNA that acted as the template for iCaspase-9. Therefore, this was also not a mutation.

A SFG.iCasp9.2A SLL-TCR retroviral vector was derived from a parental SFG.iCasp9.2A.CAR. SFG.iCasp9.2A.CAR was derived from MSCV.IRES.GFP-based construct by cloning the expression insert (termed F-Casp9 and renamed iCasp9) from the MCSV backbone into the SFG retroviral backbone. The iCasp9 moiety was moved from the MCSV backbone into the SFG backbone to (i) eliminate the co-expressed IRES-GFP in the MCSV construct and (ii) to utilize the SFG backbone. Furthermore, SFG retroviral constructs have demonstrated stability for up to 9 years. In the SFG backbone, the iCasp9 cassette was joined to the TCR sequence via a 2A-like cleavable linker. The 2A-like sequence encodes a 20 amino acid peptide from *Thosea Asigna* insect virus, which mediates >99% cleavage between a glycine and terminal proline residue, resulting in 19 extra amino acids in the C terminus of iCasp9, and 1 extra proline residue in the N-terminus of TCR beta chain. The TCR element consists of the full-length human TCRβ (variable and constant regions) and the full-length human TCRa (variable and constant regions), linked by a second, codon optimized, T2A.

The vector cassette including the unrelated CAR sequence has been removed through the enzymatic digestion with PSI-1 (present in the sequence of iCasp9) and MLU-1 (present after the codon stop of the unrelated CAR sequence). A construct has been synthesized by SIGMA Aldrich Company, with the iCasp9 sequence present after the PSI-1 site, in frame with 2A peptide, TCRβ, a second 2A peptide and the TCRα, and shipped to OPBG center into the expression vector PUC. The relevant gene cassette present in the PUC vector has been obtained after the enzymatic digestion with PSI-1 and MLU-1 and used for the ligation in the vector backbone, prepared as described above.

The SFG.iCasp9.2A.SLL-TCR retroviral vector was manufactured at the Ospedale Pediatrico Bambino Gesù, Cell and Gene Therapy for Pediatric Tumor Laboratory. The packaging cell line for producing the retrovirus was generated in the research environment using a dedicated Laminar Flow Hoods and CO2 incubator. The SFG.iCasp9.2A.SLL-TCR retroviral vector is generated from a cGMP-banked 293VEC RD114 producer clone.

The BioVec 293Vec-RD114 Packaging Cell Line is a human embryonic kidney HEK293- based packaging cell line. The 293Vec-RD114 Cell Line was developed using zeocin and puromycin resistance genes to stably express Moloney murine leukemia (MLV) gag-pol and RD114 envelope (env) viral proteins. Vectors produced by 293Vec-RD114 cells can infect a broad range of mammalian cells.

For the first stage of production of SFG.iCasp9.2A.SLL-TCR retroviral vector, the 293VEC GALV producer cell line was used to generate a transient retroviral supernatant, that was subsequently employed to stably transduce 293VEC RD114 producer cell line. The Cell and Gene Therapy for Pediatric Tumor Laboratory at OPBG received a vial of 293VEC GALV and 293VEC RD114, which was subsequently expanded and used in the research lab to generate a Working Cell Bank (WCB), using pharmaceutical grade fetal bovine serum.

A vial of the WCB 293VEC GALV cell line was expanded in the Translational Research Laboratories using all dedicated material and instrumentation. The 293VEC GALV were transiently transfected using lipofectamine 2000 reagent and 5µg of BPZ-701 retroviral vector.

Two ml of the supernatant from the transfection was applied to 293VEC RD114 cells in the presence of polybrene. Single-cell cloning was performed, and the 293VEC RD114 clone that produced the highest titer (using PCR analysis for vector presence in the supernatant) was expanded, banked in the cGMP facility of OPBG and used for retrovirus production under cGMP conditions, after testing for sterility and mycoplasma.

Producer lines are grown in DMEM - Dulbecco's Modified Eagle Medium, Iscove's Modified Dulbecco's Medium (IMDM), with 10% of pharma grade, gamma-irradiated fetal bovine serum. Supernatant from the 293VEC producer cell line is filtered through 0.20µm filters to remove contaminating 293VEC cells.

The primary T cells are cultured in a serum-free, xeno-free, defined medium (Cellgenix). Patient-derived PBMC are cultured and activated. Recombinant interleukin 2 (IL-2; Cellgenix) is added and T cells are selectively expanded. RetroNectin (Takara Bio incorporated, Japan), a chimeric peptide of human fibronectin fragments, is used to facilitate retroviral transduction of T cells. The transduced cells are again cultured in media supplemented with IL-2 for and cryopreserved in a defined freezing medium (Cryostor CS10, Biolife Solutions) using a controlled-rate freezer.

Autologous T cells are the target for the Cell A genetic modification. Peripheral blood mononuclear cells will be transduced after expansion under T-cell specific conditions. Since the cells must provide the CD3 signal for adequate TCR function, only the T cells will be functional in the final product.

### PRAME TCR-iCasp9 Vector Nucleotide Sequences

The following sequence SEQ ID NO: 85 includes LTR, linker, T2A, and other non-iCasp9 or PRAME TCR coding sequences. It is understood that variants and modifications to this sequence may be used without affecting the function of the vector. Certain coding sequences, in order, are: 5'LTR; FKBP12v; GS linker; dCaspase9; T2A; TCRbeta; 2A; TCRalpha; 3'LTR.
SEQ ID NO: 85: SFG.iC9-2A-SLL.TCR iCasp9-PRAME TCR Coding Sequence 
TGAAAGACCCCACCTGTAGGTTTGGCAAGCTAGCTTAAGTAACGCCATTTTGCAAGGCATG
GAAAAATACATAACTGAGAATAGAAAAGTTCAGATCAAGGTCAGGAACAGATGGAACAGCT
GAATATGGGCCAAACAGGATATCTGTGGTAAGCAGTTCCTGCCCCGGCTCAGGGCCAAGA
ACAGATGGAACAGCTGAATATGGGCCAAACAGGATATCTGTGGTAAGCAGTTCCTGCCCC
GGCTCAGGGCCAAGAACAGATGGTCCCCAGATGCGGTCCAGCCCTCAGCAGTTTCTAGA
 
GAACCATCAGATGTTTCCAGGGTGCCCCAAGGACCTGAAATGACCCTGTGCCTTATTTGAA
CTAACCAATCAGTTCGCTTCTCGCTTCTGTTCGCGCGCTTATGCTCCCCGAGCTCAATAAA
AGAGCCCACAACCCCTCACTCGGGGCGCCAGTCCTCCGATTGACTGAGTCGCCCGGGTA
CCCGTGTATCCAATAAACCCTCTTGCAGTTGCATCCGACTTGTGGTCTCGCTGTTCCTTGG
GAGGGTCTCCTCTGAGTGATTGACTACCCGTCAGCGGGGGTCTTTCATTTGGGGGCTCGT
CCGGGATCGGGAGACCCCTGCCCAGGGACCACCGACCCACCACCGGGAGGTAAGCTGG
CCAGCAACTTATCTGTGTCTGTCCGATTGTCTAGTGTCTATGACTGATTTTATGCGCCTGC
GTCGGTACTAGTTAGCTAACTAGCTCTGTATCTGGCGGACCCGTGGTGGAACTGACGAGT
TCGGAACACCCGGCCGCAACCCTGGGAGACGTCCCAGGGACTTCGGGGGCCGTTTTTGT
GGCCCGACCTGAGTCCTAAAATCCCGATCGTTTAGGACTCTTTGGTGCACCCCCCTTAGA
GGAGGGATATGTGGTTCTGGTAGGAGACGAGAACCTAAAACAGTTCCCGCCTCCGTCTGA
ATTTTTGCTTTCGGTTTGGGACCGAAGCCGCGCCGCGCGTCTTGTCTGCTGCAGCATCGT
TCTGTGTTGTCTCTGTCTGACTGTGTTTCTGTATTTGTCTGAAAATATGGGCCCGGGCTAG
CCTGTTACCACTCCCTTAAGTTTGACCTTAGGTCACTGGAAAGATGTCGAGCGGATCGCTC
ACAACCAGTCGGTAGATGTCAAGAAGAGACGTTGGGTTACCTTCTGCTCTGCAGAATGGC
CAACCTTTAACGTCGGATGGCCGCGAGACGGCACCTTTAACCGAGACCTCATCACCCAGG
TTAAGATCAAGGTCTTTTCACCTGGCCCGCATGGACACCCAGACCAGGTGGGGTACATCG
TGACCTGGGAAGCCTTGGCTTTTGACCCCCCTCCCTGGGTCAAGCCCTTTGTACACCCTA
AGCCTCCGCCTCCTCTTCCTCCATCCGCCCCGTCTCTCCCCCTTGAACCTCCTCGTTCGA
CCCCGCCTCGATCCTCCCTTTATCCAGCCCTCACTCCTTCTCTAGGCGCCCCCATATGGC
CATATGAGATCTTATATGGGGCACCCCCGCCCCTTGTAAACTTCCCTGACCCTGACATGAC
AAGAGTTACTAACAGCCCCTCTCTCCAAGCTCACTTACAGGCTCTCTACTTAGTCCAGCAC
GAAGTCTGGAGACCTCTGGCGGCAGCCTACCAAGAACAACTGGACCGACCGGTGGTACC
TCACCCTTACCGAGTCGGCGACACAGTGTGGGTCCGCCGACACCAGACTAAGAACCTAGA
ACCTCGCTGGAAAGGACCTTACACAGTCCTGCTGACCACCCCCACCGCCCTCAAAGTAGA
CGGCATCGCAGCTTGGATACACGCCGCCCACGTGAAGGCTGCCGACCCCGGGGGTGGA
CCATCCTCTAGACTGCCATGCTCGAGGGAGTGCAGGTGGAAACCATCTCCCCAGGAGAC
GGGCGCACCTTCCCCAAGCGCGGCCAGACCTGCGTGGTGCACTACACCGGGATGCTTGA
AGATGGAAAGAAAGTTGATTCCTCCCGGGACAGAAACAAGCCCTTTAAGTTTATGCTAGGC
AAGCAGGAGGTGATCCGAGGCTGGGAAGAAGGGGTTGCCCAGATGAGTGTGGGTCAGAG
AGCCAAACTGACTATATCTCCAGATTATGCCTATGGTGCCACTGGGCACCCAGGCATCATC
CCACCACATGCCACTCTCGTCTTCGATGTGGAGCTTCTAAAACTGGAATCTGGCGGTGGA
TCCGGAGTCGACGGATTTGGTGATGTCGGTGCTCTTGAGAGTTTGAGGGGAAATGCAGAT
TTGGCTTACATCCTGAGCATGGAGCCCTGTGGCCACTGCCTCATTATCAACAATGTGAACT
TCTGCCGTGAGTCCGGGCTCCGCACCCGCACTGGCTCCAACATCGACTGTGAGAAGTTG
CGGCGTCGCTTCTCCTCGCTGCATTTCATGGTGGAGGTGAAGGGCGACCTGACTGCCAA
GAAAATGGTGCTGGCTTTGCTGGAGCTGGCGCAGCAGGACCACGGTGCTCTGGACTGCT
 
GCGTGGTGGTCATTCTCTCTCACGGCTGTCAGGCCAGCCACCTGCAGTTCCCAGGGGCT
GTCTACGGCACAGATGGATGCCCTGTGTCGGTCGAGAAGATTGTGAACATCTTCAATGGG
ACCAGCTGCCCCAGCCTGGGAGGGAAGCCCAAGCTCTTTTTCATCCAGGCCTGTGGTGG
GGAGCAGAAAGACCATGGGTTTGAGGTGGCCTCCACTTCCCCTGAAGACGAGTCCCCTG
GCAGTAACCCCGAGCCAGATGCCACCCCGTTCCAGGAAGGTTTGAGGACCTTCGACCAG
CTGGACGCCATATCTAGTTTGCCCACACCCAGTGACATCTTTGTGTCCTACTCTACTTTCC
CAGGTTTTGTTTCCTGGAGGGACCCCAAGAGTGGCTCCTGGTACGTTGAGACCCTGGACG
ACATCTTTGAGCAGTGGGCTCACTCTGAAGACCTGCAGTCCCTCCTGCTTAGGGTCGCTA
ATGCTGTTTCGGTGAAAGGGATTTATAAACAGATGCCTGGTTGCTTTAATTTCCTCCGGAA
AAAACTTTTCTTTAAAACATCAGCTAGCAGAGCCGAGGGCAGGGGAAGTCTTCTAACATGC
GGGGACGTGGAGGAAAATCCCGGCCCATGGGCTTCCGGCTGCTGTGCTGCGTGGCCTT
TTGTCTGCTGGGAGCCGGCCCTGTGGATAGCGGCGTGACCCAGACCCCCAAGCACCTGA
TCACCGCCACCGGCCAGAGAGTGACCCTGCGCTGCAGCCCTAGAAGCGGCGACCTGAGC
GTGTACTGGTATCAGCAGAGCCTCGACCAGGGCCTGCAGTTCCTGATCCAGTACTACAAC
GGCGAGGAACGGGCCAAGGGCAACATCCTGGAACGGTTCAGCGCCCAGCAGTTCCCCGA
TCTGCACAGCGAGCTGAACCTGAGCAGCCTGGAACTGGGCGACAGCGCCCTGTACTTCT
GCGCCAGCGCCAGATGGGATAGAGGCGGCGAGCAGTACTTCGGCCCTGGCACCAGACT
GACCGTGACCGAGGACCTGAAGAACGTGTTCCCCCCAGAGGTGGCCGTGTTTGAGCCCA
GCGAGGCCGAGATCAGCCACACCCAGAAAGCCACCCTGGTGTGCCTGGCCACCGGCTTC
TACCCCGACCACGTGGAGCTGTCTTGGTGGGTGAACGGCAAAGAGGTGCACAGCGGCGT
CTGCACCGACCCCCAGCCCCTGAAAGAGCAGCCCGCCCTGAACGACAGCCGGTACTGCC
TGAGCAGCAGACTGCGGGTGTCCGCCACCTTCTGGCAGAACCCCCGGAACCACTTCCGG
TGCCAGGTGCAGTTCTACGGCCTGAGCGAGAACGACGAGTGGACCCAGGACAGAGCCAA
GCCTGTGACCCAGATCGTGTCTGCCGAAGCCTGGGGCAGAGCCGACTGCGGCTTCACCA
GCGAGAGCTACCAGCAGGGCGTGCTGAGCGCCACCATCCTGTACGAGATCCTGCTGGGC
AAGGCCACACTGTACGCCGTGCTGGTGTCCGCTCTGGTGCTGATGGCCATGGTGAAGCG
GAAGGACAGCAGAGGCGAGGGCAGAGGAAGTCTTCTAACATGCGGTGACGTGGAGGAGA
ATCCCGGCCCTATGCTGCTGCTGCTGGTGCCCGTGCTGGAAGTGATCTTCACCCTGGGC
GGCACCAGAGCCCAGAGCGTGACACAGCTGGGCAGCCACGTGTCCGTGTCTGAGAGGG
CCCTGGTGCTGCTGAGATGCAACTACTCTTCTAGCGTGCCCCCCTACCTGTTTTGGTACGT
GCAGTACCCCAACCAGGGGCTGCAGCTGCTCCTGAAGTACACCAGCGCCGCCACACTGG
TGAAGGGCATCAACGGCTTCGAGGCCGAGTTCAAGAAGTCCGAGACAAGCTTCCACCTGA
CCAAGCCCAGCGCCCACATGTCTGACGCCGCCGAGTACTTCTGTGCCGTGAGCGGCCAG
ACCGGCGCCAACAACCTGTTCTTCGGCACCGGCACCCGGCTGACAGTGATCCCTTACATC
CAGAACCCCGACCCCGCCGTGTACCAGCTGCGGGACAGCAAGAGCAGCGACAAGAGCGT
GTGCCTGTTCACCGACTTCGACAGCCAGACCAACGTGTCCCAGAGCAAGGACAGCGACG
TGTACATCACCGATAAGTGCGTGCTGGACATGCGGAGCATGGACTTCAAGAGCAACAGCG
 
CCGTGGCCTGGTCCAACAAGAGCGACTTCGCCTGCGCCAACGCCTTCAACAACAGCATCA
TCCCCGAGGACACATTCTTCCCAAGCCCCGAGAGCAGCTGCGACGTGAAGCTGGTGGAG
AAGTCCTTCGAGACAGACACCAACCTGAACTTCCAGAACCTGTCCGTGATCGGCTTCAGA
ATCCTGCTGCTGAAAGTGGCCGGCTTCAACCTGCTGATGACCCTGCGGCTGTGGTCCAGC
TGAACGCGTCATCATCGATCCGGATTAGTCCAATTTGTTAAAGACAGGATATCAGTGGTCC
AGGCTCTAGTTTTGACTCAACAATATCACCAGCTGAAGCCTATAGAGTACGAGCCATAGAT
AAAATAAAAGATTTTATTTAGTCTCCAGAAAAAGGGGGGAATGAAAGACCCCACCTGTAGG
TTTGGCAAGCTAGCTTAAGTAACGCCATTTTGCAAGGCATGGAAAAATACATAACTGAGAA
TAGAGAAGTTCAGATCAAGGTCAGGAACAGATGGAACAGCTGAATATGGGCCAAACAGGA
TATCTGTGGTAAGCAGTTCCTGCCCCGGCTCAGGGCCAAGAACAGATGGAACAGCTGAAT
ATGGGCCAAACAGGATATCTGTGGTAAGCAGTTCCTGCCCCGGCTCAGGGCCAAGAACAG
ATGGTCCCCAGATGCGGTCCAGCCCTCAGCAGTTTCTAGAGAACCATCAGATGTTTCCAG
GGTGCCCCAAGGACCTGAAATGACCCTGTGCCTTATTTGAACTAACCAATCAGTTCGCTTC
TCGCTTCTGTTCGCGCGCTTCTGCTCCCCGAGCTCAATAAAAGAGCCCACAACCCCTCAC
TCGGGGCGCCAGTCCTCCGATTGACTGAGTCGCCCGGGTACCCGTGTATCCAATAAACCC
TCTTGCAGTTGCATCCGACTTGTGGTCTCGCTGTTCCTTGGGAGGGTCTCCTCTGAGTGAT
TGACTACCCGTCAGCGGGGGTCTTTCACACATGCAGCATGTATCAAAATTAATTTGGTTTTT
TTTCTTAAGTATTTACATTAAATGGCCATAGTACTTAAAGTTACATTGGCTTCCTTGAAATAA
ACATGGAGTATTCAGAATGTGTCATAAATATTTCTAATTTTAAGATAGTATCTCCATTGGCTT
TCTACTTTTTCTTTTATTTTTTTTTGTCCTCTGTCTTCCATTTGTTGTTGTTGTTTGTTTG
TTTGTTTGTTGGTTGGTTGGTTAATTTTTTTTTAAAGATCCTACACTATAGTTCAAGCTAGAC
TATTAGCTACTCTGTAACCCAGGGTGACCTTGAAGTCATGGGTAGCCTGCTGTTTTAGCCT
TCCCACATCTAAGATTACAGGTATGAGCTATCATTTTTGGTATATTGATTGATTGATTGATTG
ATGTGTGTGTGTGTGATTGTGTTTGTGTGTGTGACTGTGAAAATGTGTGTATGGGTGTGTG
TGAATGTGTGTATGTATGTGTGTGTGAGTGTGTGTGTGTGTGTGCATGTGTGTGTGT
GTGACTGTGTCTATGTGTATGACTGTGTGTGTGTGTGTGTGTGTGTGTGTGTGTGTGTG
TGTGTGTGTTGTGAAAAAATATTCTATGGTAGTGAGAGCCAACGCTCCGGCTCAGGTGTCA
GGTTGGTTTTTGAGACAGAGTCTTTCACTTAGCTTGGAATTCACTGGCCGTCGTTTTACAA
CGTCGTGACTGGGAAAACCCTGGCGTTACCCAACTTAATCGCCTTGCAGCACATCCCCCT
TTCGCCAGCTGGCGTAATAGCGAAGAGGCCCGCACCGATCGCCCTTCCCAACAGTTGCG
CAGCCTGAATGGCGAATGGCGCCTGATGCGGTATTTTCTCCTTACGCATCTGTGCGGTATT
TCACACCGCATATGGTGCACTCTCAGTACAATCTGCTCTGATGCCGCATAGTTAAGCCAGC
CCCGACACCCGCCAACACCCGCTGACGCGCCCTGACGGGCTTGTCTGCTCCCGGCATCC
GCTTACAGACAAGCTGTGACCGTCTCCGGGAGCTGCATGTGTCAGAGGTTTTCACCGTCA
TCACCGAAACGCGCGATGACGAAAGGGCCTCGTGATACGCCTATTTTTATAGGTTAATGTC
ATGATAATAATGGTTTCTTAGACGTCAGGTGGCACTTTTCGGGGAAATGTGCGCGGAACCC
CTATTTGTTTATTTTTCTAAATACATTCAAATATGTATCCGCTCATGAGACAATAACCCTGAT
 
AAATGCTTCAATAATATTGAAAAAGGAAGAGTATGAGTATTCAACATTTCCGTGTCGCCCTT
ATTCCCTTTTTTGCGGCATTTTGCCTTCCTGTTTTTGCTCACCCAGAAACGCTGGTGAAAGT
AAAAGATGCTGAAGATCAGTTGGGTGCACGAGTGGGTTACATCGAACTGGATCTCAACAG
CGGTAAGATCCTTGAGAGTTTTCGCCCCGAAGAACGTTTTCCAATGATGAGCACTTTTAAA
GTTCTGCTATGTGGCGCGGTATTATCCCGTATTGACGCCGGGCAAGAGCAACTCGGTCGC
CGCATACACTATTCTCAGAATGACTTGGTTGAGTACTCACCAGTCACAGAAAAGCATCTTA
CGGATGGCATGACAGTAAGAGAATTATGCAGTGCTGCCATAACCATGAGTGATAACACTG
CGGCCAACTTACTTCTGACAACGATCGGAGGACCGAAGGAGCTAACCGCTTTTTTGCACA
ACATGGGGGATCATGTAACTCGCCTTGATCGTTGGGAACCGGAGCTGAATGAAGCCATAC
CAAACGACGAGCGTGACACCACGATGCCTGTAGCAATGGCAACAACGTTGCGCAAACTAT
TAACTGGCGAACTACTTACTCTAGCTTCCCGGCAACAATTAATAGACTGGATGGAGGCGGA
TAAAGTTGCAGGACCACTTCTGCGCTCGGCCCTTCCGGCTGGCTGGTTTATTGCTGATAAA
TCTGGAGCCGGTGAGCGTGGGTCTCGCGGTATCATTGCAGCACTGGGGCCAGATGGTAA
GCCCTCCCGTATCGTAGTTATCTACACGACGGGGAGTCAGGCAACTATGGATGAACGAAA
TAGACAGATCGCTGAGATAGGTGCCTCACTGATTAAGCATTGGTAACTGTCAGACCAAGTT
TACTCATATATACTTTAGATTGATTTAAAACTTCATTTTTAATTTAAAAGGATCTAGGTGAAG
ATCCTTTTTGATAATCTCATGACCAAAATCCCTTAACGTGAGTTTTCGTTCCACTGAGCGTC
AGACCCCGTAGAAAAGATCAAAGGATCTTCTTGAGATCCTTTTTTTCTGCGCGTAATCTGCT
GCTTGCAAACAAAAAAACCACCGCTACCAGCGGTGGTTTGTTTGCCGGATCAAGAGCTAC
CAACTCTTTTTCCGAAGGTAACTGGCTTCAGCAGAGCGCAGATACCAAATACTGTCCTTCT
AGTGTAGCCGTAGTTAGGCCACCACTTCAAGAACTCTGTAGCACCGCCTACATACCTCGCT
CTGCTAATCCTGTTACCAGTGGCTGCTGCCAGTGGCGATAAGTCGTGTCTTACCGGGTTG
GACTCAAGACGATAGTTACCGGATAAGGCGCAGCGGTCGGGCTGAACGGGGGGTTCGTG
CACACAGCCCAGCTTGGAGCGAACGACCTACACCGAACTGAGATACCTACAGCGTGAGCA
TTGAGAAAGCGCCACGCTTCCCGAAGGGAGAAAGGCGGACAGGTATCCGGTAAGCGGCA
GGGTCGGAACAGGAGAGCGCACGAGGGAGCTTCCAGGGGGAAACGCCTGGTATCTTTAT
AGTCCTGTCGGGTTTCGCCACCTCTGACTTGAGCGTCGATTTTTGTGATGCTCGTCAGGG
GGGCGGAGCCTATGGAAAAACGCCAGCAACGCGGCCTTTTTACGGTTCCTGGCCTTTTGC
TGGCCTTTTGCTCACATGTTCTTTCCTGCGTTATCCCCTGATTCTGTGGATAACCGTATTAC
CGCCTTTGAGTGAGCTGATACCGCTCGCCGCAGCCGAACGACCGAGCGCAGCGAGTCAG
TGAGCGAGGAAGCGGAAGAGCGCCCAATACGCAAACCGCCTCTCCCCGCGCGTTGGCCG
ATTCATTAATGCAGCTGGCACGACAGGTTTCCCGACTGGAAAGCGGGCAGTGAGCGCAAC
GCAATTAATGTGAGTTAGCTCACTCATTAGGCACCCCAGGCTTTACACTTTATGCTTCCGG
CTCGTATGTTGTGTGGAATTGTGAGCGGATAACAATTTCACACAGGAAACAGCTATGACCA
TGATTACGCCAAGCTTTGCTCTTAGGAGTTTCCTAATACATCCCAAACTCAAATATATAAAG
CATTTGACTTGTTCTATGCCCTAGGGGGCGGGGGGAAGCTAAGCCAGCTTTTTTTAACATT
TAAAATGTTAATTCCATTTTAAATGCACAGATGTTTTTATTTCATAAGGGTTTCAATGTGCAT
 
GAATGCTGCAATATTCCTGTTACCAAAGCTAGTATAAATAAAAATAGATAAACGTGGAAATT
ACTTAGAGTTTCTGTCATTAACGTTTCCTTCCTCAGTTGACAACATAAATGCGCTGCTGAGC
AAGCCAGTTTGCATCTGTCAGGATCAATTTCCCATTATGCCAGTCATATTAATTACTAGTCA
ATTAGTTGATTTTTATTTTTGACATATACATGTGAA
SEQ ID NO: 86: Nucleotide sequence coding for Caspase-9 polypeptide in above SEQ ID NO: 85 
GTCGACGGATTTGGTGATGTCGGTGCTCTTGAGAGTTTGAGGGGAAATGCAGATTTGGCT
TACATCCTGAGCATGGAGCCCTGTGGCCACTGCCTCATTATCAACAATGTGAACTTCTGCC
GTGAGTCCGGGCTCCGCACCCGCACTGGCTCCAACATCGACTGTGAGAAGTTGCGGCGT
CGCTTCTCCTCGCTGCATTTCATGGTGGAGGTGAAGGGCGACCTGACTGCCAAGAAAATG
GTGCTGGCTTTGCTGGAGCTGGCGCAGCAGGACCACGGTGCTCTGGACTGCTGCGTGGT
GGTCATTCTCTCTCACGGCTGTCAGGCCAGCCACCTGCAGTTCCCAGGGGCTGTCTACGG
CACAGATGGATGCCCTGTGTCGGTCGAGAAGATTGTGAACATCTTCAATGGGACCAGCTG
CCCCAGCCTGGGAGGGAAGCCCAAGCTCTTTTTCATCCAGGCCTGTGGTGGGGAGCAGA
AAGACCATGGGTTTGAGGTGGCCTCCACTTCCCCTGAAGACGAGTCCCCTGGCAGTAACC
CCGAGCCAGATGCCACCCCGTTCCAGGAAGGTTTGAGGACCTTCGACCAGCTGGACGCC
ATATCTAGTTTGCCCACACCCAGTGACATCTTTGTGTCCTACTCTACTTTCCCAGGTTTTGT
TTCCTGGAGGGACCCCAAGAGTGGCTCCTGGTACGTTGAGACCCTGGACGACATCTTTGA
GCAGTGGGCTCACTCTGAAGACCTGCAGTCCCTCCTGCTTAGGGTCGCTAATGCTGTTTC
GGTGAAAGGGATTTATAAACAGATGCCTGGTTGCTTTAATTTCCTCCGGAAAAAACTTTTCT
TTAAAACATCAGCTAGCAGAGCC
SEQ ID NO: 87: Nucleotide sequence coding for TCR beta in above SEQ ID NO: 85 
ATGGGCTTCCGGCTGCTGTGCTGCGTGGCCTTTTGTCTGCTGGGAGCCGGCCCTGTGGA
TAGCGGCGTGACCCAGACCCCCAAGCACCTGATCACCGCCACCGGCCAGAGAGTGACCC
TGCGCTGCAGCCCTAGAAGCGGCGACCTGAGCGTGTACTGGTATCAGCAGAGCCTCGAC
CAGGGCCTGCAGTTCCTGATCCAGTACTACAACGGCGAGGAACGGGCCAAGGGCAACAT
CCTGGAACGGTTCAGCGCCCAGCAGTTCCCCGATCTGCACAGCGAGCTGAACCTGAGCA
GCCTGGAACTGGGCGACAGCGCCCTGTACTTCTGCGCCAGCGCCAGATGGGATAGAGGC
GGCGAGCAGTACTTCGGCCCTGGCACCAGACTGACCGTGACCGAGGACCTGAAGAACGT
GTTCCCCCCAGAGGTGGCCGTGTTTGAGCCCAGCGAGGCCGAGATCAGCCACACCCAGA
AAGCCACCCTGGTGTGCCTGGCCACCGGCTTCTACCCCGACCACGTGGAGCTGTCTTGG
TGGGTGAACGGCAAAGAGGTGCACAGCGGCGTCTGCACCGACCCCCAGCCCCTGAAAGA
GCAGCCCGCCCTGAACGACAGCCGGTACTGCCTGAGCAGCAGACTGCGGGTGTCCGCCA
 
CCTTCTGGCAGAACCCCCGGAACCACTTCCGGTGCCAGGTGCAGTTCTACGGCCTGAGC
GAGAACGACGAGTGGACCCAGGACAGAGCCAAGCCTGTGACCCAGATCGTGTCTGCCGA
AGCCTGGGGCAGAGCCGACTGCGGCTTCACCAGCGAGAGCTACCAGCAGGGCGTGCTG
AGCGCCACCATCCTGTACGAGATCCTGCTGGGCAAGGCCACACTGTACGCCGTGCTGGT
GTCCGCTCTGGTGCTGATGGCCATGGTGAAGCGGAAGGACAGCAGAGGC
SEQ ID NO: 88: Nucleotide sequence coding for TCR alpha in above SEQ ID NO: 85

ATGCTGCTGCTGCTGGTGCCCGTGCTGGAAGTGATCTTCACCCTGGGCGGCACCAGAGC
CCAGAGCGTGACACAGCTGGGCAGCCACGTGTCCGTGTCTGAGAGGGCCCTGGTGCTGC
TGAGATGCAACTACTCTTCTAGCGTGCCCCCCTACCTGTTTTGGTACGTGCAGTACCCCAA
CCAGGGGCTGCAGCTGCTCCTGAAGTACACCAGCGCCGCCACACTGGTGAAGGGCATCA
ACGGCTTCGAGGCCGAGTTCAAGAAGTCCGAGACAAGCTTCCACCTGACCAAGCCCAGC
GCCCACATGTCTGACGCCGCCGAGTACTTCTGTGCCGTGAGCGGCCAGACCGGCGCCAA
CAACCTGTTCTTCGGCACCGGCACCCGGCTGACAGTGATCCCTTACATCCAGAACCCCGA
CCCCGCCGTGTACCAGCTGCGGGACAGCAAGAGCAGCGACAAGAGCGTGTGCCTGTTCA
CCGACTTCGACAGCCAGACCAACGTGTCCCAGAGCAAGGACAGCGACGTGTACATCACC
GATAAGTGCGTGCTGGACATGCGGAGCATGGACTTCAAGAGCAACAGCGCCGTGGCCTG
GTCCAACAAGAGCGACTTCGCCTGCGCCAACGCCTTCAACAACAGCATCATCCCCGAGGA
CACATTCTTCCCAAGCCCCGAGAGCAGCTGCGACGTGAAGCTGGTGGAGAAGTCCTTCGA
GACAGACACCAACCTGAACTTCCAGAACCTGTCCGTGATCGGCTTCAGAATCCTGCTGCT
GAAAGTGGCCGGCTTCAACCTGCTGATGACCCTGCGGCTGTGGTCCAGC

### Literature References Cited or Providing Additional Support to the Present Example

1. Seifert, R. and K. Wenzel-Seifert, Constitutive activity of G-protein-coupled receptors: cause of disease and common property of wild-type receptors. Naunyn Schmiedebergs Arch Pharmacol, 2002. 366(5): p. 381-416.
2. Roose, J.P., et al., T cell receptor-independent basal signaling via Erk and Abl kinases suppresses RAG gene expression. PLoS Biol, 2003. 1(2): p. E53.
3. Tze, L.E., et al., Basal immunoglobulin signaling actively maintains developmental stage in immature B cells. PLoS Biol, 2005. 3(3): p. e82.
4. Schram, B.R., et al., B cell receptor basal signaling regulates antigen-induced Ig light chain rearrangements. J Immunol, 2008. 180(7): p. 4728-41.
5. Randall, K.L., et al., Dock8 mutations cripple B cell immunological synapses, germinal centers and long-lived antibody production. Nat Immunol, 2009. 10(12): p. 1283-91.
6. Kouskoff, V., et al., B cell receptor expression level determines the fate of developing B lymphocytes: receptor editing versus selection. Proc Natl Acad Sci U S A, 2000. 97(13): p. 7435-9.
7. Hong, T., et al., A simple theoretical framework for understanding heterogeneous differentiation of CD4+ T cells. BMC Syst Biol, 2012. 6: p. 66.
8. Rudd, M.L., A. Tua-Smith, and D.B. Straus, Lck SH3 domain function is required for T-cell receptor signals regulating thymocyte development. Mol Cell Biol, 2006. 26(21): p. 7892-900.
9. Sorkin, A. and M. von Zastrow, Endocytosis and signaling: intertwining molecular networks. Nat Rev Mol Cell Biol, 2009. 10(9): p. 609-22.
10. Luning Prak, E.T., M. Monestier, and R.A. Eisenberg, B cell receptor editing in tolerance and autoimmunity. Ann N YAcad Sci, 2011. 1217: p. 96-121.
11. Boss, W.F., et al., Basal signaling regulates plant growth and development. Plant Physiol, 2010. 154(2): p. 439-43.
12. Tao, Y.X., Constitutive activation of G protein-coupled receptors and diseases: insights into mechanisms of activation and therapeutics. Pharmacol Ther, 2008. 120(2): p. 129-48.
13. Spiegel, A.M., Defects in G protein-coupled signal transduction in human disease. Annu Rev Physiol, 1996. 58: p. 143-70.
14. Shiozaki, E.N., et al., Mechanism of XIAP-mediated inhibition of Caspase-9. Mol Cell, 2003. 11(2): p. 519-27.
15. Renatus, M., et al., Dimer formation drives the activation of the cell death protease Caspase-9. Proc Natl Acad Sci U S A, 2001. 98(25): p. 14250-5.
16. Shi, Y., Mechanisms of Caspase activation and inhibition during apoptosis. Mol Cell, 2002. 9(3): p. 459-70.
17. Shiozaki, E.N., J. Chai, and Y. Shi, Oligomerization and activation of Caspase-9, induced by Apaf-1 CARD. Proc Natl Acad Sci U S A, 2002. 99(7): p. 4197-202.
18. Straathof, K.C., et al., An inducible Caspase-9 safety switch for T-cell therapy. Blood, 2005. 105(11): p. 4247-54.
19. MacCorkle, R.A., K.W. Freeman, and D.M. Spencer, Synthetic activation of Caspases: artificial death switches. Proc Natl Acad Sci U S A, 1998. 95(7): p. 3655-60.
20. Di Stasi, A., et al., Inducible apoptosis as a safety switch for adoptive cell therapy. N Engl J Med, 2011. 365(18): p. 1673-83.
21. Chang, W.C., et al., Modifying ligand-induced and constitutive signaling of the human 5-HT4 receptor. PLoS One, 2007. 2(12): p. e1317.
22. Bloom, J.D. and F.H. Arnold, In the light of directed evolution: pathways of adaptive protein evolution. Proc Natl Acad Sci U S A, 2009. 106 Suppl 1: p. 9995-10000.
23. Boatright, K.M. and G.S. Salvesen, Mechanisms of Caspase activation. Curr Opin Cell Biol, 2003. 15(6): p. 725-31.
24. Boatright, K.M., et al., A unified model for apical Caspase activation. Mol Cell, 2003. 11(2): p. 529-41.
25. Chao, Y., et al., Engineering a dimeric Caspase-9: a re-evaluation of the induced proximity model for Caspase activation. PLoS Biol, 2005. 3(6): p. e183.
26. Stennicke, H.R., et al., Caspase-9 can be activated without proteolytic processing. J Biol Chem, 1999. 274(13): p. 8359-62.
27. Brady, S.C., L.A. Allan, and P.R. Clarke, Regulation of Caspase-9 through phosphorylation by protein kinase C zeta in response to hyperosmotic stress. Mol Cell Biol, 2005. 25(23): p. 10543-55.
28. Martin, M.C., et al., Protein kinase A regulates Caspase-9 activation by Apaf-1 downstream of cytochrome c. J Biol Chem, 2005. 280(15): p. 15449-55.
29. Cardone, M.H., et al., Regulation of cell death protease Caspase-9 by phosphorylation. Science, 1998. 282(5392): p. 1318-21.
30. Raina, D., et al., c-Abl tyrosine kinase regulates Caspase-9 autocleavage in the apoptotic response to DNA damage. J Biol Chem, 2005. 280(12): p. 11147-51.
31. Papworth, C., Bauer, J. C., Braman, J. and Wright, D. A. , Site-directed mutagenesis in one day with >80% efficiency. Strategies, 1996. 9(3): p. 3-4.
32. Spencer, D.M., et al., Functional analysis of Fas signaling in vivo using synthetic inducers of dimerization. Curr Biol, 1996. 6(7): p. 839-47.
33. Hsiao, E.C., et al., Constitutive Gs activation using a single-construct tetracycline-inducible expression system in embryonic stem cells and mice. Stem Cell Res Ther, 2011. 2(2): p. 11.
34. Waldner, C., et al., Double conditional human embryonic kidney cell line based on FLP and PhiC31 mediated transgene integration. BMC Res Notes, 2011. 4: p. 420.

### Examples

The examples set forth below illustrate certain embodiments and do not limit the technology.

### Example 1: PRAME-Specific T cells

### Isolation and analysis of PRAME specific T cells

All studies were conducted with approval of the institutional review board at Leiden University Medical Center (LUMC). After informed consent, peripheral blood mononuclear cells (PBMCs) were collected from a patient suffering from AML who experienced acute GVHD after single HLA-A2 mismatched SCT and subsequent DLI. Based on a cross-over, the patient was HLA-A*0201 positive and the sibling donor was HLA-A*0201 negative, whereas all other HLA class I and II molecules were completely matched. Patient PBMCs collected during GVHD were stained with anti-HLA-A2-FITC (Pharmingen), anti-HLA-DR-APC (Pharmingen) and anti-CD8-PE (BD) for 30 min at 4°C, and activated (HLA-DRpos), donor derived (HLA-A2neg) CD8+ T cells were isolated by cell sorting (FACSAria).Since PBMCs were limited, the sorted T cells were first expanded with anti-CD3/CD28 and irradiated autologous PBMCs (0.5x106/ml) in T cell medium. T cell medium consists of Iscove's Modified Dulbecco's Medium (IMDM; Lonza) with 10% human serum (HS), IL-2 (120 IU/ml; Proleukin) and IL-15 (20 ng/ml; Peprotech). T cells were stimulated non-specifically using irradiated allogeneic PBMCs (0.5×10⁶ /ml), IL-2 (120 IU/ml), and phytohemagglutinin (PHA, 0.8 µg/ml; Murex Biotec Limited). After 14 days of culture, T cells were labeled with anti-CD8-APC (BD) and PE-conjugated HLA-A2 tetramers specific for the different TAA peptides (1-4): for PRAME were tested: VLDGLDVLL (VLD), SLYSFPEPEA (SLY), ALYVDSLFFL (ALY), and SLLQHLIGL (SLL), for WT-1: RMFPNAPYL, for Pr-1: VLQELNVTV. For single cell sorting, T cells were stained with APC conjugated tetramers in combination with TCR VB repertoire kit staining (Beckman Coulter) for 1 h at 4°C, and SLL tetramer⁺VB1⁺ and SLL tetramer⁺ VB3⁺ CD8⁺ T cells were sorted and stimulated non-specifically using irradiated allogeneic PBMCs (0.5×10⁶ /ml), IL-2 (120 IU/ml), and phytohemagglutinin (PHA, 0.8 µg/ml; Murex Biotec Limited). Self-restricted PRAME specific T cell clones were isolated from an HLA-A*0201 patient that was transplanted with a fully HLA-identical donor graft. PBMCs derived from the patient after SCT were labeled with PE-conjugated SLL tetramer for 1 h at 4 °C. Tetramer positive T cells were isolated by MACS using anti-PE coated magnetic beads (Miltenyi Biotec) and were expanded for 10 days with anti-CD3/CD28 beads as provided above. For subsequent sorting, T cells were stained with PE-conjugated SLL tetramer and anti-CD8 APC for 1 h at 4°C, and tetramer positive CD8⁺ T cells were sorted single cell per well and expanded. Three PRAME-SLL tetramer positive T cell clones AAV54 (also called clone 54 or HSS1), AAV46 (also called HSS3), and DMG16 were selected, and used for further analysis. In addition, the PRAME specific clone DSK3 was selected and the specificity of this T cell clone was determined by peptide elution studies.

### Functional reactivity of the PRAME specific T cell clones

Stimulation assays were performed with 5,000 T cells and 20,000 targets in 96-well plates in Iscoves Dulbecco Modified Medium (IMDM), supplemented with 10% human serum (HS) and 100 IU/ml interleukin 2 (IL-2). The different malignant and non-malignant cells were collected and prepared. Stable Epstein-Barr virus (EBV)-transformed B cell-lines (EBV-LCLs) were generated using standard procedures, and cultured in IMDM and 10% FBS. K562, COS, T2, renal cell carcinoma cell-lines (RCC 1257, RCC 1774, RCC 1851), lung carcinoma cell-lines (A549, NCI-H292), melanoma cell-lines (518A2, FM3, FM6, SK2.3, MI-3046, BML, 1.14), cervix carcinoma cell-lines (SIHA, HELA, CASKI), breast carcinoma cell-lines (MCF7, BT549, MDA231) and colon carcinoma cell-lines (SW480, HCT116, LS411, LS180) were cultured in IMDM and 10% FBS. K562, COS, H292, A549, SIHA and HELA not expressing HLA-A2 were transduced with a retroviral vector encoding for HLA-A2 as previously discussed⁵ .

In addition, melanoma cells were freshly isolated from an HLA-A2 positive patient with lymph node metastatic melanoma by ficol isolation of minced tumor cells and subsequent FACS sort of the CD45, CD3, CD19, CD14, CD56 negative cells. For selected experiments COS-A2 cells were transfected with pcDNA3.1 expression vector encoding for wild-type human PRAME. Peripheral blood of HLA-A2 positive patients with primary AML cells (>80% blasts) were cultured for 1 day in IMDM and 10% FCS and used as stimulator cells. Primary AML cells were activated for 1 day with GM-CSF (100 ng /ml; Novartis), TNFa (10 ng/ml; R&D Systems), IL-1b (10 ng/ml; Immunex), IL-6 (10 ng/ml; Cellgenix), PGE-2 (1 µg/ml; Sigma-Aldrich), and IFNγ (500 IU/ml; Immukine, Boehringer Ingelheim). HLA-A2 positive ALL cell-lines were generated as previously discussed⁶. B cells were isolated from PBMCs by MACS using anti-CD19 coated magnetic beads (Miltenyi Biotec). ALL cell-lines and freshly isolated B cells were activated by culturing the cells for 48 h at a concentration of 10⁶ cells/ml in 24-well plates in the presence of IL-4 (500 U/ml; Schering-Plough), CpG oligodeoxynucleotide (10 µg/ml; Eurogentec) and 1×10⁵/ml murine fibroblasts transfected with the human CD40 ligand⁷ 7). In-vivo activated B cells were derived from inflamed tonsils. T-cell blasts were generated by stimulation of PBMCs with PHA and IL-2 (120 IU/ml) for 7 days.

Monocytes were isolated from PBMCs by MACS using anti-CD14 coated magnetic beads (Miltenyi Biotec). Macrophages (MØ) were generated by culturing CD14⁺cells for 6 days in IMDM and 10% HS at a concentration of 0.5×10⁶cells/ml in 24-well plates. Pro-inflammatory macrophages (MØ1) cells were obtained by culture in the presence of GM-CSF (5 ng/ml) and anti-inflammatory macrophages (MØ2) cells were cultured with M-CSF (5 ng/ml, Cetus Corporation). Monocyte derived DCs were generated by culturing CD14⁺ cells for 48 h in IMDM and 10% HS at a concentration of 0.5×10⁶ cells/ml in 24-well plates in the presence of IL-4 (500 U/ml) and GM-CSF(100 ng/ml). For maturation of the CD14 DCs, cells were cultured for another 48 h in IMDM and 10% HS supplemented with GM-CSF (100 ng /ml), TNFa (10 ng/ml), IL-1b (10 ng/ml), IL-6 (10 ng/ml), PGE-2 (1 µg/ml), and IFNγ (500 IU/ml).

CD34⁺ cells were isolated from peripheral blood stem cell grafts by MACS using anti-CD34 coated magnetic beads (Miltenyi Biotec). CD34 DCs were generated by culturing CD34 cells for 4 days in IMDM and 10% HS at a concentration of 0.25×10⁶ cells/ml in 24-well plates in the presence of GM-CSF (100 ng/ml), SCF (20 ng/ml; kindly provided by Amgen), and TNFa (2 ng/ml), and subsequently for 3 days with additionally IL-4 (500 IU/ml). To maturate the CD34 DCs, the cells were cultured for another 48 h in IMDM and 10% HS supplemented with GM-CSF (100 ng /ml), SCF (20 ng/ml), TNFa (10 ng/ml), IL-1b (10 ng/ml), IL-6 (10 ng/ml), PGE-2 (1 ug/ml), and IFNγ (500 IU/ml). For the isolation of blood derived myeloid DCs (MDCs) and plasmacytoid DCs (PDCs), PBMCs were stained with anti-BDCA1-PE (Biolegend) or anti-BDCA2-PE mAbs (Miltenyi Biotec), respectively, and the BDCA1-PE or BDCA2-PE positive cells were isolated by MACS using anti-PE coated magnetic beads. The MACS isolated cells were stained with FITC conjugated anti-CD3, anti-CD14, anti-CD19 and anti-CD56 mAbs (BD) and the MDCs and PDCs were selected by cell sorting on bases of BDCA1 or BDCA2 positivity and lineage marker negative cells. To maturate the MDCs and PDCs, cells were cultured for 24 h in IMDM and 10% HS supplemented with either poly-IC (Amersham) or CpG (10 µg/ml) and IL-3 (50 ng/ml; kindly provided by Novartis), respectively. Fibroblasts were cultured from skin biopsies in Dulbecco's modified Eagle medium (DMEM; Lonza) with 1g/l glucose (BioWhittaker) and 10% FBS.

Keratinocytes were cultured from skin biopsies in keratinocyte serum free medium supplemented with 30 µg/ml bovine pituitary extract and 2 ng/ml epithelial growth factor (EGF) (all components were purchased from Invitrogen). Fibroblasts and keratinocytes were cultured for 3 days in the presence or absence of IFNγ (200 IU/ml). Primary bronchial epithelial cells (PBEC) were derived and cultured as previously discussed⁴.

Mesenchymal stromal cells (MSCs) were derived from bone marrow of healthy donors as previously discussed⁵ and cultured in DMEM and 10% FBS. Colon epithelial cells were cultured in DMEM F12 (Lonza) and 10% FCS, and supplemented with EGF (10 ng/ml; Promega), T3 hormone (2nmol/l; Sigma), hydrocortisone (0,4ug/ml; Pharmacy LUMC), and insulin (5 ng/ml; Sigma). Hepatocytes and intrahepatic biliary epithelial cells (IHBEC) (both purchased from ScienCell) were cultured in RPMI (Lonza) and 10% FBS. Proximal tubular epithelial cells (PTEC) were isolated and cultured as previously discussed⁶.

For peptide titrations, T2 cells were preincubated for 1 h with different concentrations of peptide, and washed. After 18 h of stimulation, supernatant was harvested and IFNγ production was measured by standard ELISA. In the cytotoxicity assays, T cells were tested at different effector-target ratios against 1,000 ⁵¹Cr labeled targets in 96-well plates in a standard 4h ⁵¹Cr - release assay. In these experiments a control HLA-A2 restricted T cell clone HSS12 specific for the USP11 gene was included.

### Peptide elution, reverse phase high performance liquid chromatography (RP-HPLC) and mass spectrometry (MS)

Peptide elution, RP-HPLC and MS were performed as previously discussed⁸. Briefly, 3×10¹⁰ Epstein Barr Virus transformed B-cells (EBV-LCLs) were lysed and the peptide-HLA-A2 complexes were purified by affinity chromatography using HLA-A2 specific BB7.2 monoclonal antibody (mAb). Subsequently, peptides were eluted from HLA-A2 molecules, and separated from the HLA monomers and β2-microglobulin by size filtration. After freeze drying, the peptide mixture was subjected to a first round of RP-C18-HPLC using a water/acetonitrile/TFA, and fractions were collected. A small sample of each fraction was loaded on T2 cells and tested for recognition by the T cell clones. The recognized fraction was subjected to a second and a third round of RP-C18-HPLC fractionation. In the second fractionation a water/isopropanol/TFA gradient was used, and in the third fractionation a water/methanol/formic acid gradient was used. After the third fractionation, the peptide masses present in the recognized fractions and in the adjacent non-recognized fractions were determined by MS. Peptides of which the abundance correlated with the recognition pattern of the T cell clone were selected for tandem mass spectrometry and their sequences determined.

### PRAME expression by quantitative real-time PCR, and inhibition of PRAME expression by silencing RNA

PRAME expression was quantified by real-time PCR (TaqMan). Inhibition of PRAME was performed using retroviral vectors encoding for short hairpin (sh) RNA sequences specific for PRAME in combination with the puromycin resistance gene that were kindly provided by Dr. R. Bernards, NKI, Amsterdam, The Netherlands⁹. Retrovirally transduced cells were cultured with different concentrations of puromycin for at least 1 week before testing. Proximal tubular epithelial cells (PTECs) were cultured at 3 µg/ml, renal cell carcinoma cell line RCC1257 at 4 µg/ml, and CD34⁺derived dendritic cells (CD34DCs) at 0.4 µg/ml. CD34DCs were generated as provided herein, and were transduced on day 1 of culture.

### TCR gene transfer

The TCRAV and TCRBV gene usage of clone AAV54 (also called clone 54 or HSS1) was determined using reverse transcriptase (RT)-PCR and sequencing⁵. HSS1 expressed TCRAV1S1 (IMGT: TRAV8-4*04 and TCR-BV1S1 (IMGT: TRBV9*01). A retroviral vector was constructed with a codon optimized and cysteine modified TCRα and TCRβ chain linked by the T2A sequence in combination with the truncated nerve growth factor receptor (ΔNGF-R)^{10,11}. Cytomegalovirus (CMV)-IE1 specific HLA-B8 restricted T cells were sorted using CMV-IE1 tetramers, stimulated for 2 days with PHA and irradiated allogeneic PBMCs and transduced with PRAME-TCR or mock. Transduced T cells were sorted based on positivity for ΔNGF-R, and tested for functional reactivity.

### PRAME expression by quantitative real-time (RT) PCR

PRAME expression was quantified by RT- PCR (TaqMan). Total RNA was isolated from cells using a RNeasy mini kit (Qiagen) or the micro RNaqueous kit (Ambion). First strand cDNA synthesis was performed with oligo dT primers using M-MLV reverse transcriptase (Invitrogen) or with the Transcriptor reverse transcriptase (Roche). Samples were run on a 7900HT RT-PCR System of Applied Biosystems. The following PRAME primers were used, sense 5' CGTTTGTGGGGTTCCATTC 3', anti-sense 5' GCTCCCTGGGCAGCAAC 3' and for the anti-sense probe 5' CCTGCCAGCTCCACAAGTCTCCGTG 3'. The Probe used VIC as dye and TAMRA as quencher; both primers were chosen over an intron/exon boundary. Each sample was run in duplicate with cDNA from 50 ng total RNA. The Porphobilinogen Deaminase (PBGD) gene was measured as housekeeping gene to ensure good quality of the cDNA.

### CD34 cell proliferation assay

In the CD34 cell proliferation inhibition assay, CD34 cells were labeled with carboxyfluorescein diacetate succinimidyl ester (CFSE) as previously discussed, and resuspended in progenitor cell culture medium¹².

### Reference List

1. Kessler,J.H., N.J.Beekman, S.A.Bres-Vloemans, P.Verdijk, P.A.van Veelen, A.M.Kloosterman-Joosten, D.C.Vissers, G.J.ten Bosch, M.G.Kester, A.Sijts, D.J.Wouter, F.Ossendorp, R.Offringa, and C.J.Melief. 2001. Efficient identification of novel HLA-A(*)0201-presented cytotoxic T lymphocyte epitopes in the widely expressed tumor antigen PRAME by proteasome-mediated digestion analysis. J.Exp.Med. 193:73-88.
2. Molldrem,J., S.Dermime, K.Parker, Y.Z.Jiang, D.Mavroudis, N.Hensel, P.Fukushima, and A.J.Barrett. 1996. Targeted T-cell therapy for human leukemia: cytotoxic T lymphocytes specific for a peptide derived from proteinase 3 preferentially lyse human myeloid leukemia cells. Blood 88:2450-2457.
3. Inoue,K., H.Ogawa, Y.Sonoda, T.Kimura, H.Sakabe, Y.Oka, S.Miyake, H.Tamaki, Y.Oji, T.Yamagami, T.Tatekawa, T.Soma, T.Kishimoto, and H.Sugiyama. 1997. Aberrant overexpression of the Wilms tumor gene (WT1) in human leukemia. Blood 89:1405-1412.
4. Gao,L., I.Bellantuono, A.Elsasser, S.B.Marley, M.Y.Gordon, J.M.Goldman, and H.J.Stauss. 2000. Selective elimination of leukemic CD34(+) progenitor cells by cytotoxic T lymphocytes specific for WT1. Blood 95:2198-2203.
5. Heemskerk,M.H., R.A.de Paus, E.G.Lurvink, F.Koning, A.Mulder, R.Willemze, J.J.van Rood, and J.H.Falkenburg. 2001. Dual HLA class I and class II restricted recognition of alloreactive T lymphocytes mediated by a single T cell receptor complex. Proc.Natl.Acad.Sci.U.S.A 98:6806-6811.
6. Nijmeijer,B.A., K.Szuhai, H.M.Goselink, M.L.van Schie, B.M.van der, J.D.de, E.W.Marijt, O.G.Ottmann, R.Willemze, and J.H.Falkenburg. 2009. Long-term culture of primary human lymphoblastic leukemia cells in the absence of serum or hematopoietic growth factors. Exp.Hematol. 37:376-385.
7. Hoogendoorn,M., J.O.Wolbers, W.M.Smit, M.R.Schaafsma, R.M.Barge, R.Willemze, and J.H.Falkenburg. 2004. Generation of B-cell chronic lymphocytic leukemia (B-CLL)-reactive T-cell lines and clones from HLA class I-matched donors using modified B-CLL cells as stimulators: implications for adoptive immunotherapy. Leukemia 18:1278-1287.
8. van Bergen,C.A., M.G.Kester, I.Jedema, M.H.Heemskerk, S.A.Luxemburg-Heijs, F.M.Kloosterboer, W.A.Marijt, A.H.de Ru, M.R.Schaafsma, R.Willemze, P.A.van Veelen, and J.H.Falkenburg. 2007. Multiple myeloma-reactive T cells recognize an activation-induced minor histocompatibility antigen encoded by the ATP-dependent interferon-responsive (ADIR) gene. Blood 109:4089-4096.
9. Epping,M.T., L.Wang, M.J.Edel, L.Carlee, M.Hernandez, and R.Bernards. 2005. The human tumor antigen PRAME is a dominant repressor of retinoic acid receptor signaling. Cell 122:835-847.
10. van Loenen,M.M., B.R.de, R.S.Hagedoorn, E.H.van Egmond, J.H.Falkenburg, and M.H.Heemskerk. 2011. Optimization of the HA-1-specific T-cell receptor for gene therapy of hematologic malignancies. Haematologica 96:477-481.
11. van Loenen,M.M., R.de Boer, A.L.Amir, R.S.Hagedoorn, G.L.Volbeda, R.Willemze, J.J.van Rood, J.H.Falkenburg, and M.H.Heemskerk. 2010. Mixed T cell receptor dimers harbor potentially harmful neoreactivity. Proc.Natl.Acad.Sci.U.S.A 107:10972-10977.
12. Jedema,I., N.M.van der Werff, R.M.Barge, R.Willemze, and J.H.Falkenburg. 2004. New CFSE-based assay to determine susceptibility to lysis by cytotoxic T cells of leukemic precursor cells within a heterogeneous target cell population. Blood 103:2677-2682.

### Isolation of high affinity PRAME specific TCRs from the allo-HLA repertoire

T cells from the allo-HLA repertoire that are specific for the clinically relevant antigen Preferentially Expressed Antigen of Melanoma (PRAME) were isolated and assayed essentially as discussed in Amir et al Clin Cancer Res 2011. Allo-HLA-restricted TAA-specific T cells were analyzed in a patient who had received a single HLA-A2-mismatched stem cell transplant. The HLA-A2⁺ patient was treated with chemotherapy and radiation, which reduced the level of malignant cells and the patient's normal hematopoietic system. Next, the patient received an HLA-mismatched (HLA-A2-) stem cell transplant (SCT); following the SCT, the patient's level of malignant cells increased, as did the presence of a normal immune system. The patient then received an HLA-A2- donor cell lymphocyte infusion, which provided a beneficial graft-versus tumor (GVT) response, reducing the level of malignant cells, but which also resulted in graft-versus-host disease (GVHD). Activated tumor-reactive CD8⁺ T cells were obtained from the patient (Figure 3). Fifty anti-HLA-A2 reactive T cell clones were identified, all expressing different T cell receptors (TCRs). The TCR-specificity was analyzed by isolating HLA-bound peptides, multidimensional HPLC fractionation, and mass spectrometry. T cells that were specific for PRAME were identified that exerted highly single-peptide-specific reactivity.

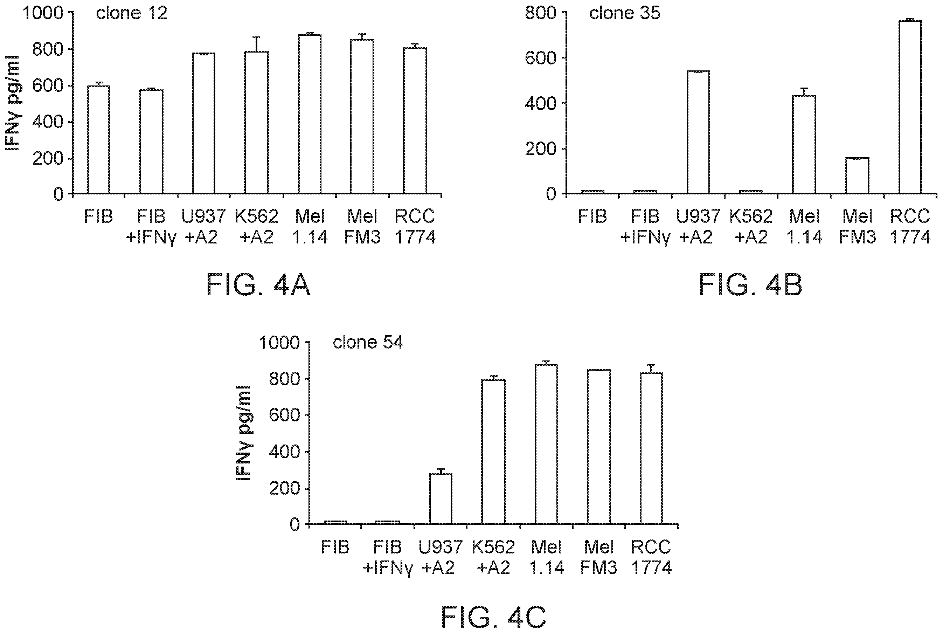

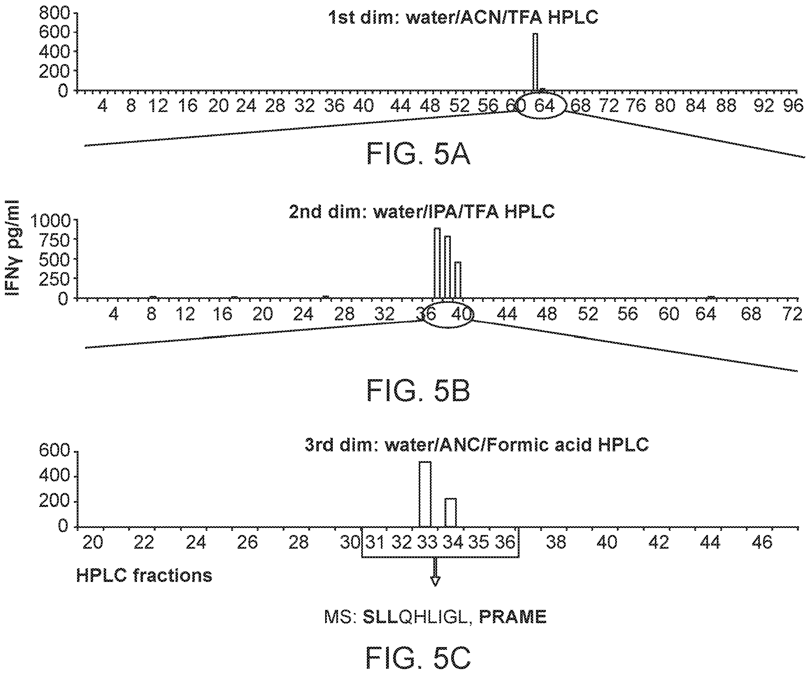

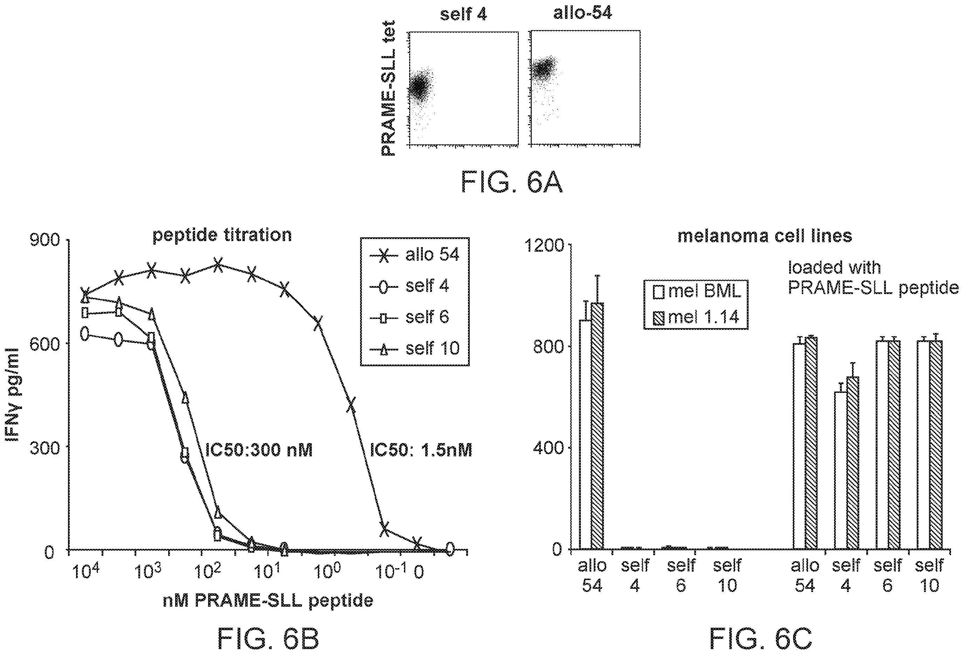

The identified PRAME specific allo-HLA restricted T cell clones were highly reactive against a panel of PRAME positive tumor cell lines as well as freshly isolated (PRAME-positive) metastatic melanoma and primary leukemic cells (Figure 4). For example, T cell clone 54 was determined to be PRAME specific (Figure 5). Interestingly, comparing the antigen sensitivity of PRAME specific T cells derived from the allo-HLA repertoire with PRAME-specific T cells obtained from an HLA-A2-expressing individual revealed that the PRAME specific T cells from the allo-HLA repertoire required a 200 fold lower peptide concentration for T cell activation. Furthermore, only the allo-HLA restricted T cells were capable of recognition of tumor cell lines and leukemic cells (Figure 6). These data suggest that T cell tolerance may cap the affinity of tumor-specific T cells that can be obtained from the patient repertoire.

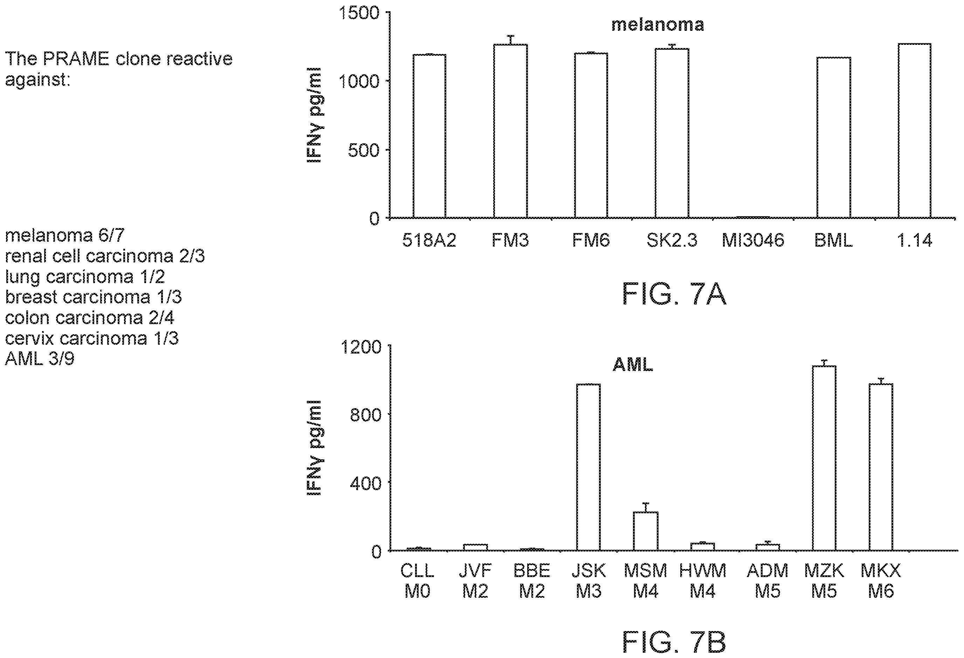

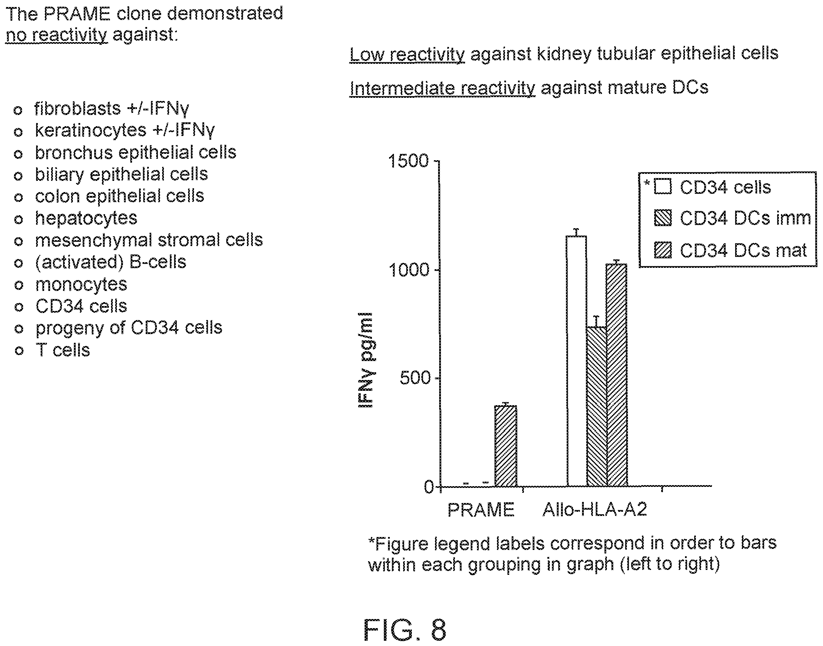

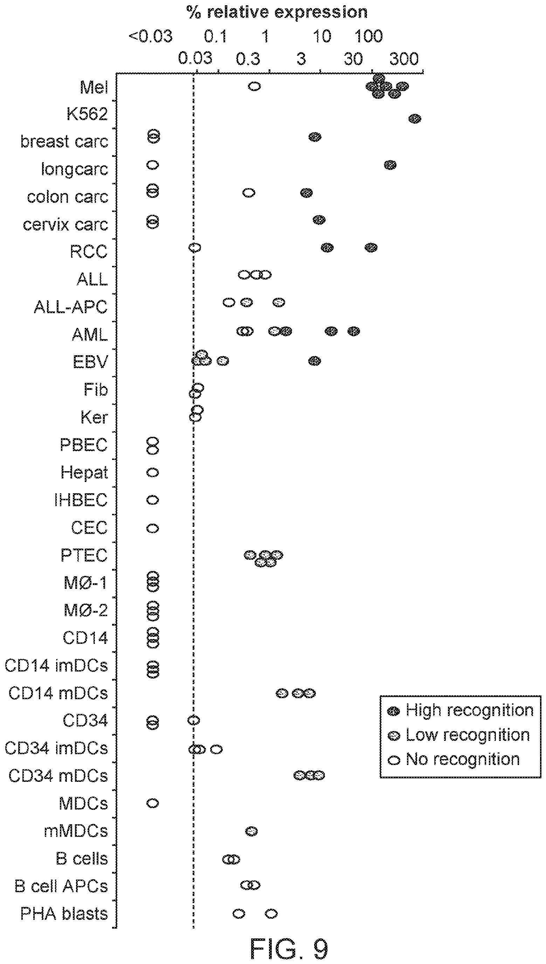

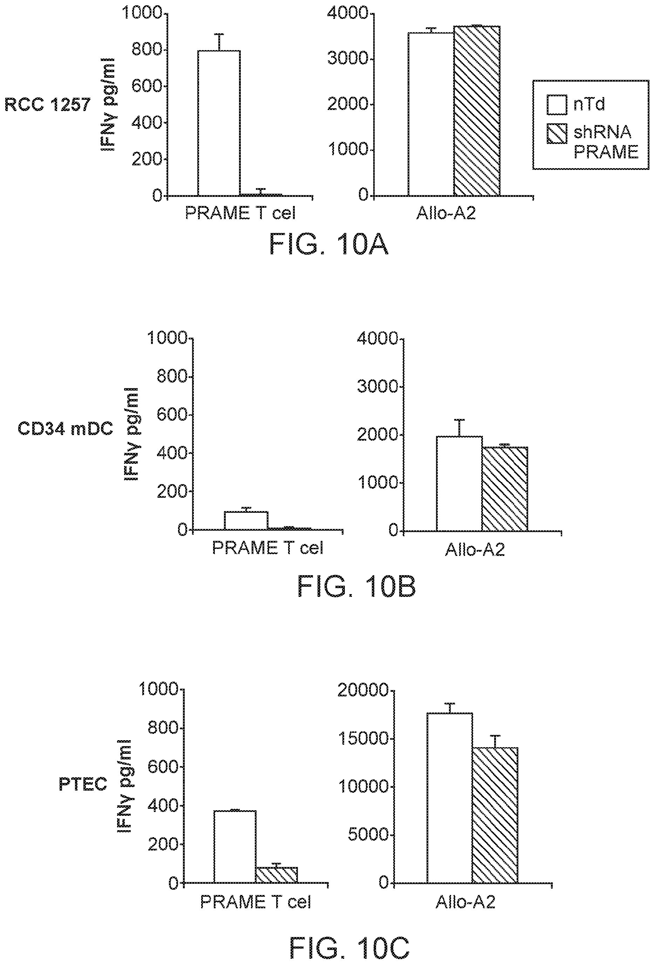

After determining the high affinity of the PRAME-specific T cells derived from the allo-HLA repertoire, the safety signature of the PRAME specific T cells was characterized. The T cells were extensively tested against a large panel of non-malignant cells. This panel of non-malignant cells consisted of epithelial cells derived from different tissues, e.g. skin, lung, colon, biliary tract, kidney, liver, as well as fibroblasts, mesenchymal stromal cells and all different hematopoietic lineages including hematopoietic stem cells (Figures 7 and 8). The T cells had low reactivity against healthy cells. None of the non-malignant cell types were recognized with the exception of low reactivity against proximal tubular epithelial cells (PTEC) and intermediate reactivity against mature dendritic cells (CD34-mDCs). Reactivity strictly correlated with PRAME expression as analyzed by quantitative RT-PCR as well as by PRAME specific shRNA transduction (Figure 9). (Amir et al Clin Can Res 2011). The single peptide specificity of the allo-HLA restricted PRAME specific T cell clones was demonstrated by down-regulation of the expression of the recognized antigens using silencing shRNA (Figure 10).

### Cloning of PRAME-specific TCRs

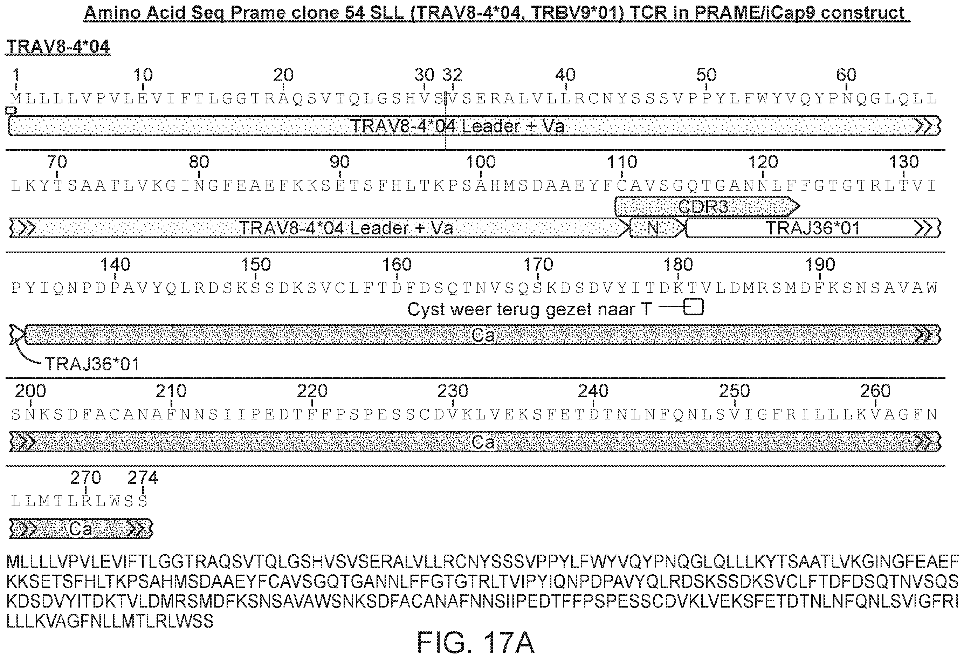

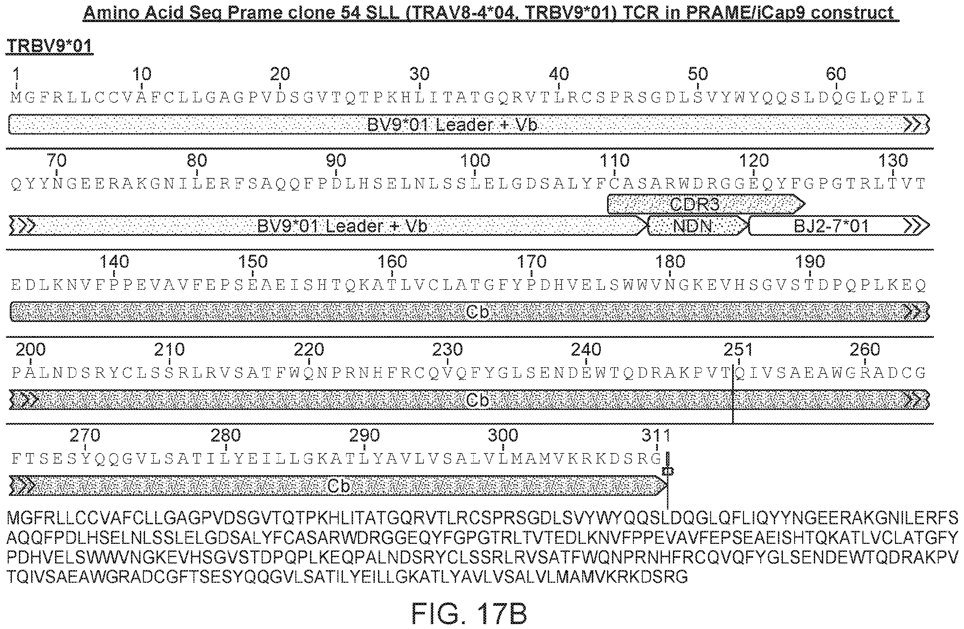

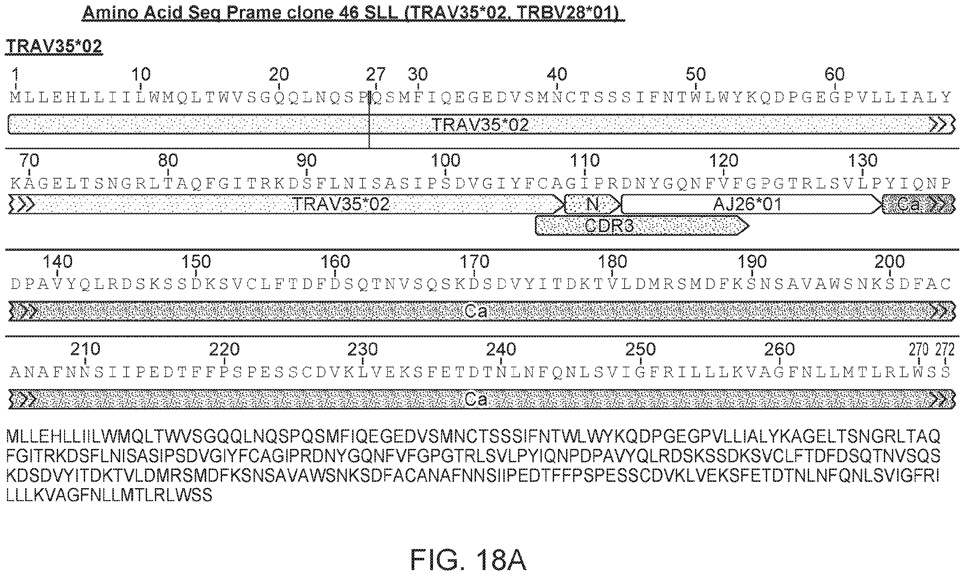

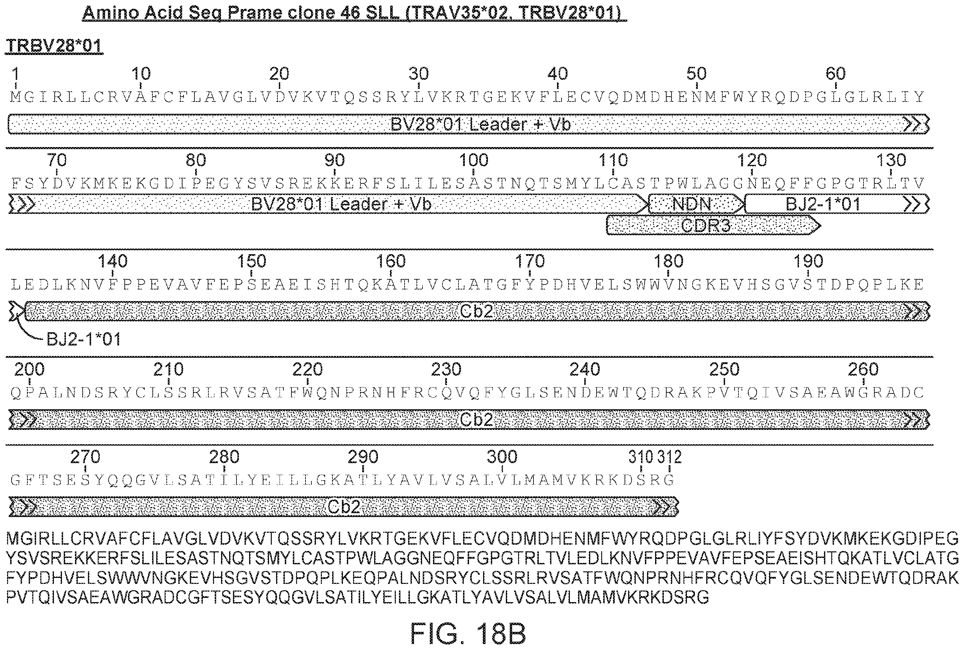

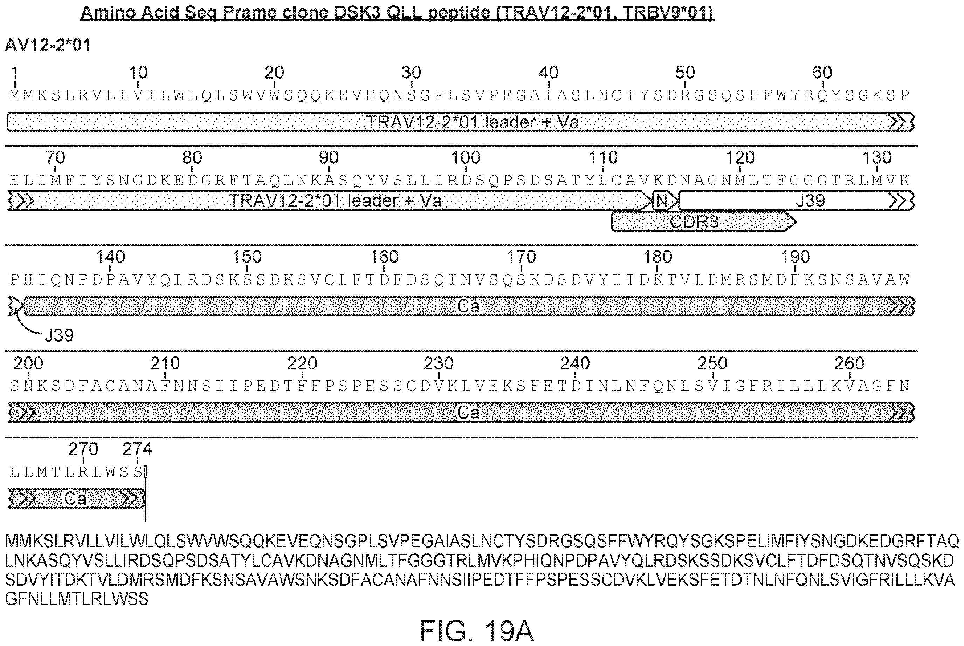

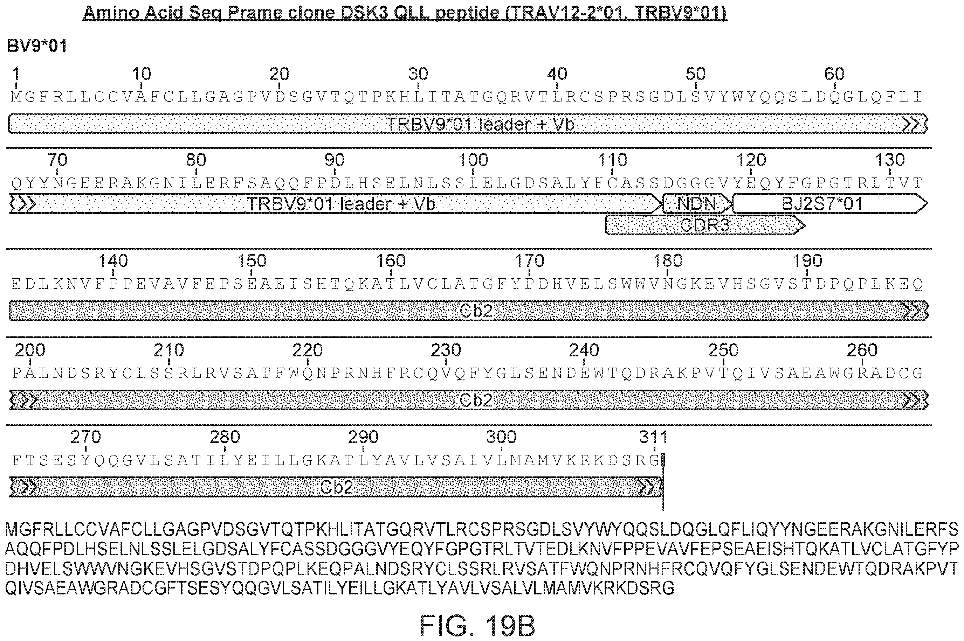

The T cell receptors expressed by clones AAV54 SLL (also called allo 54 or HSS1), AAV46 SLL (also called HSS3), and DSK3 QLL clones were sequenced. The sequences of the two different high affinity TCRs specific for the SLLQHLIGL peptide of PRAME (AAV54 and AAV46) are shown in Figures 17 and 18. In addition, a high affinity TCR directed against the QLLALLPSL epitope of PRAME, this TCR sequence of clone DSK3 is shown in Figure 19. Retroviral vectors were constructed that encoded the PRAME-specific HLA-A2 restricted TCRs, and used to transduce peripheral blood T cells. The PRAME-specific TCR transduced CD8⁺ T cells derived from peripheral blood have a PRAME-specific recognition patterns.

### Characterization of PRAME-Specific TCR-expressing Cells

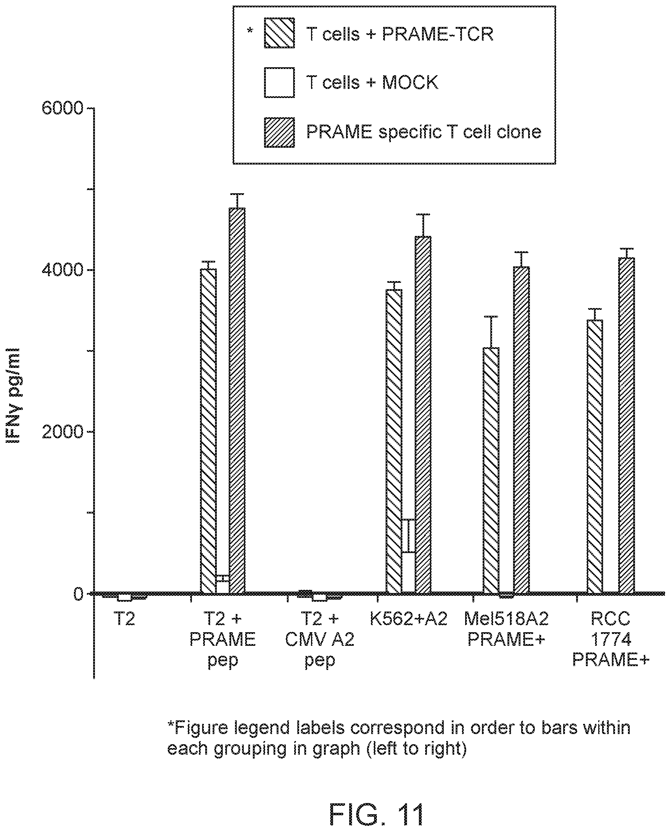

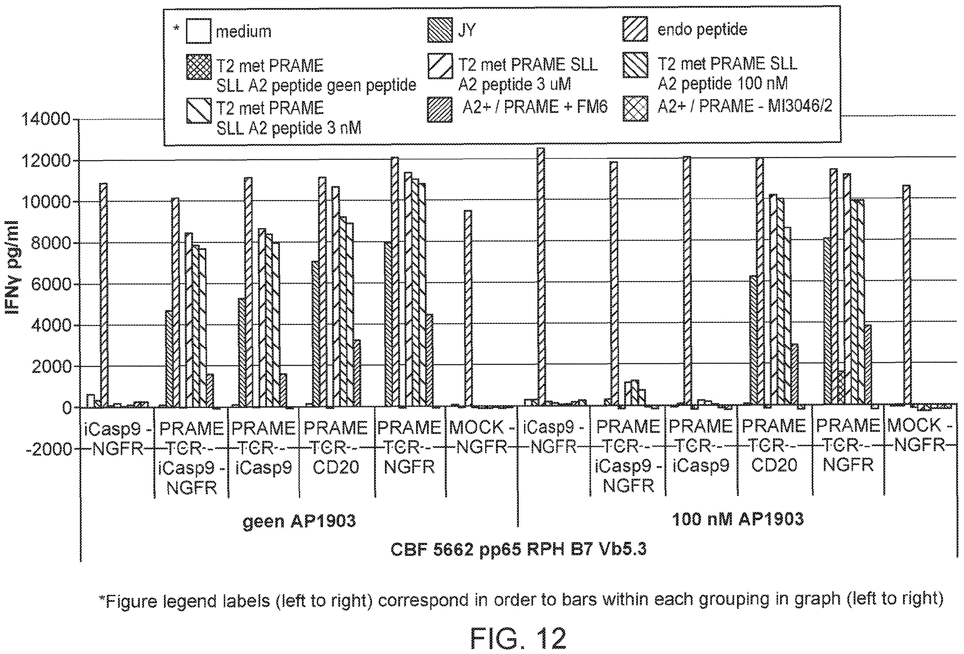

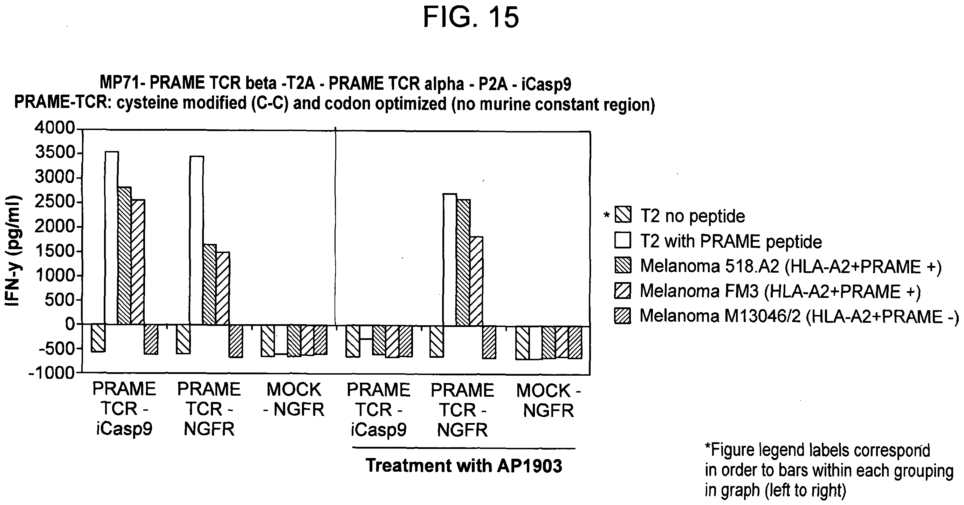

TCR transduced CD8⁺ T cells stained with the PRAME specific tetramer, demonstrated a similar recognition pattern compared to the corresponding original T cell clones (Figure 11; also Amir et al Clin Can Res 2011). Retroviral constructs were generated in which the PRAME-TCR is linked to the iCasp9 suicide switch. The functionality of the T cells transduced with the MP71-PRAME-TCR-iCasp9 and MP71-PRAME-TCR-iCasp9-NGFR was similar to the T cells transduced with the MP71-PRAME-TCR-CD20 and the MP71-PRAME-TCR-NGFR, and after overnight incubation with AP1903 the PRAME specific functional activity of the T cells was abrogated, indicating the functional expression of the iCasp9 (Figure 12 and Figure 15).

Figure 12, Different retroviral vectors encoding for the PRAME-TCR were transduced into virus specific T cells and the reactivity is measured against target cells loaded with different concentrations of the PRAME peptide and against two melanoma cell-lines: FM6 positive for HLA-A2 and PRAME, and MI3046/2 positive for HLA-A2 but negative for PRAME. The reactivity of the transduced T cells against JY, an EBV-LCL that is positive for HLA-A*0201 and has intermediate expression for PRAME, was also assayed. After treatment for 18h with 100nM of AP1903 the PRAME reactivity was abrogated in the T cells transduced with the PRAME-TCR in combination with the iCasp9 (right part of the Figure).

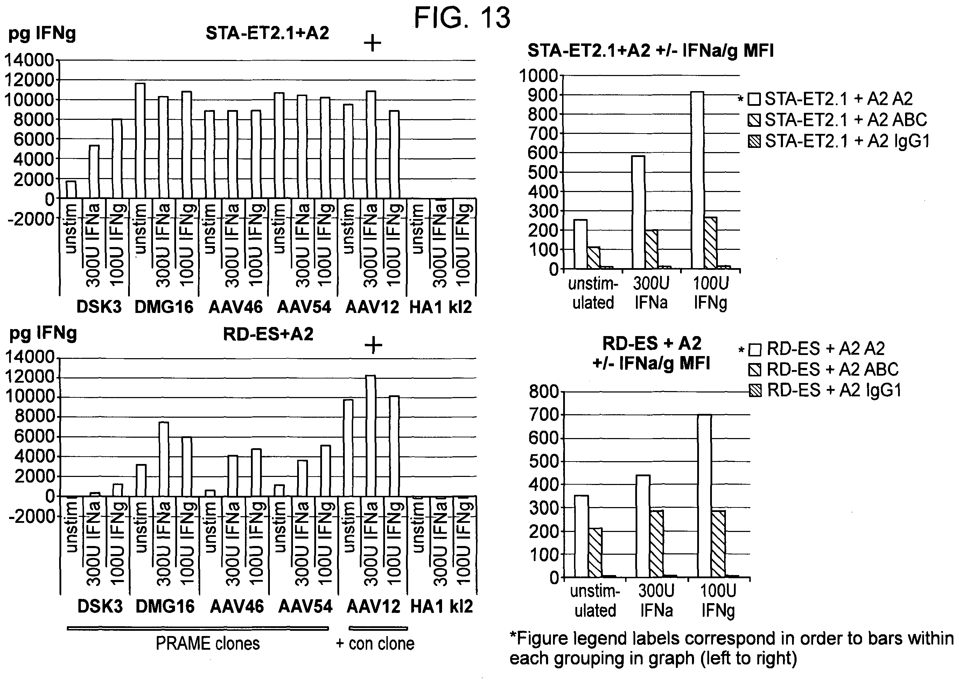

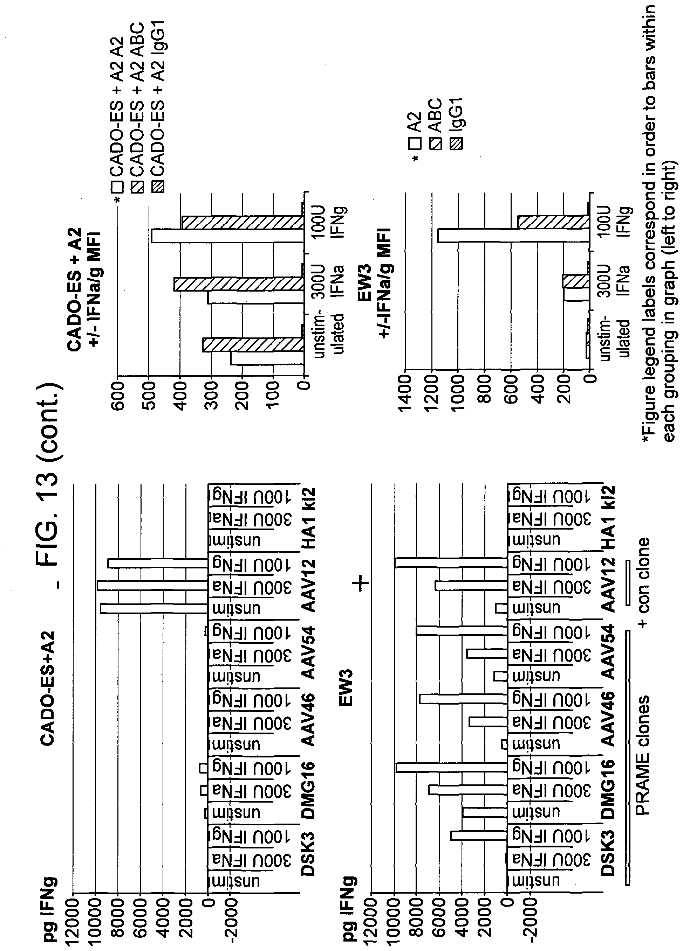

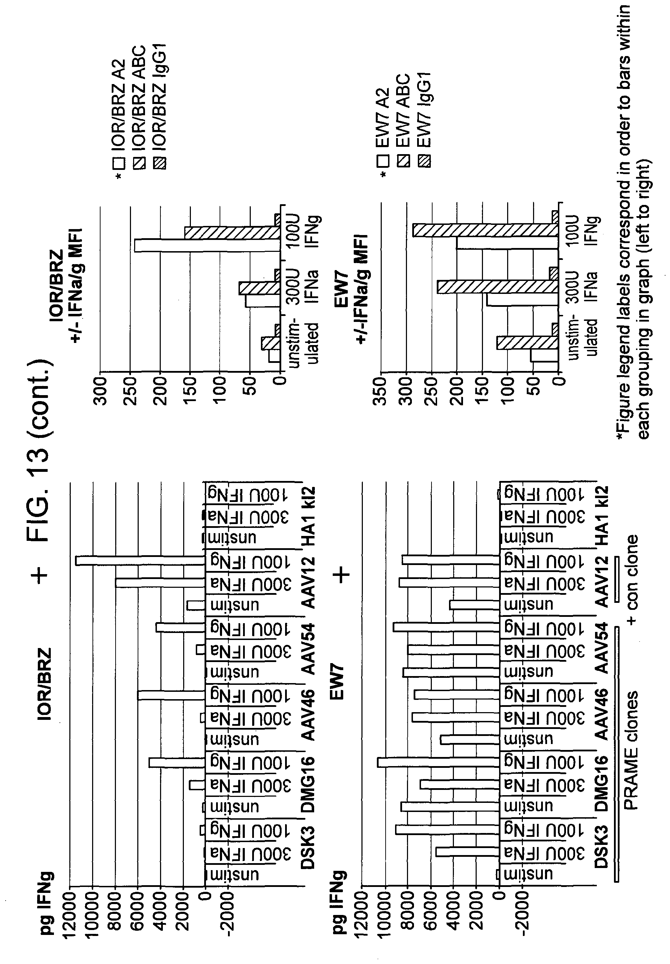

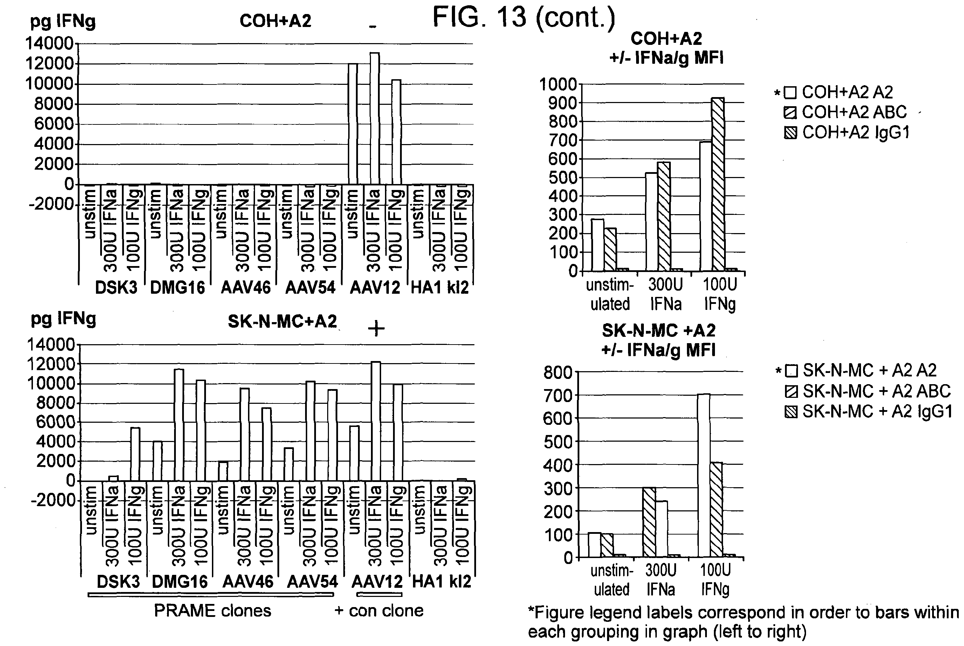

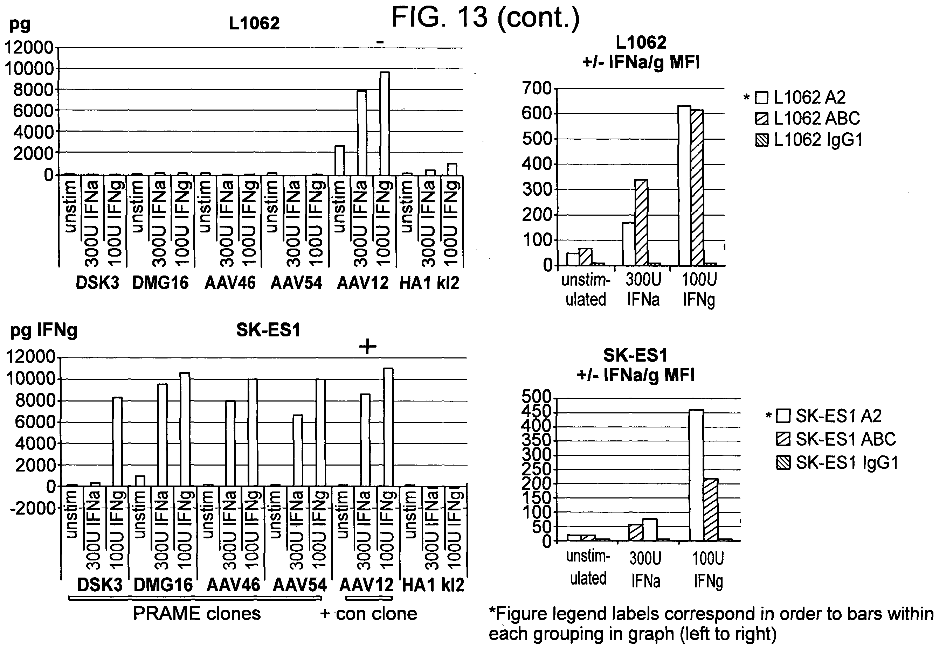

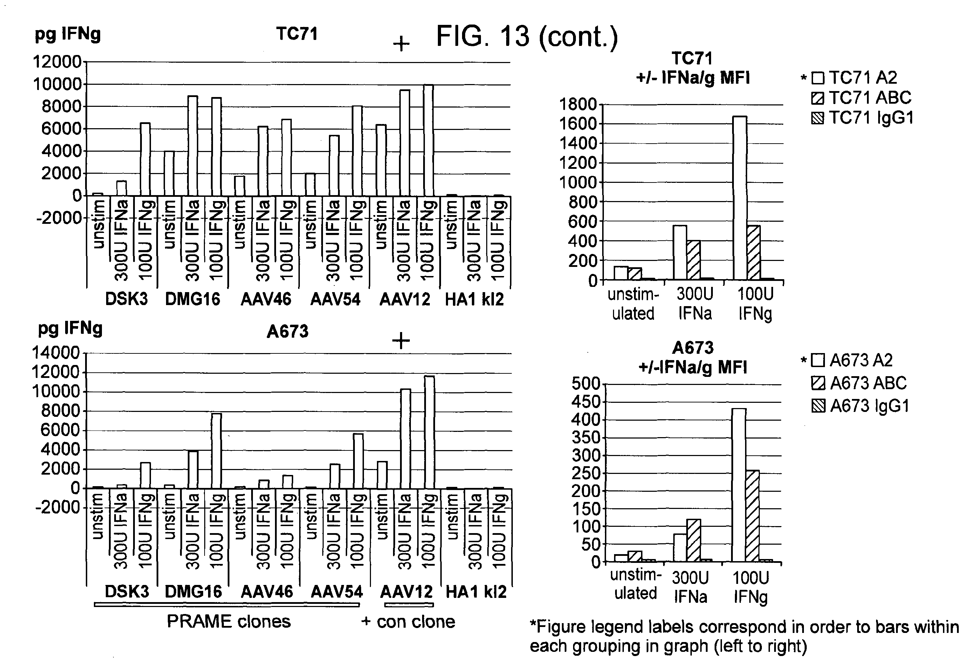

Figure 13. 4 different PRAME specific T cell clones were tested for reactivity directed against Ewing sarcoma cell lines. DSK3 is the QLL specific T cell clone, DMG16 and AAV54 are 2 identical T cell clones (identical TCR alpha and beta sequence), and AAV46 is also directed against the SLL epitope, however the clone expresses a different TCR. AAV12 is used as a positive control T cell clone recognizing a peptide of the USP11 gene. Clone HA1k4 is a negative control clone. The Ewing sarcoma cell lines were treated for 48h with either 300 IU/ml of IFN-alpha or 100 IU/ml IFN-gamma, washed, and added to the different T cell clones. After 18h of coculture the supernatant was harvested and the IFN-gamma production of the T cell clones was measured. The results indicate that 8 out of 12 Ewing sarcoma cell lines express PRAME. Treatment with IFN-alpha and IFN-gamma increased the HLA-class I expression on the cell surface leading to better recognition of the Ewing cell lines by the different T cell clones.

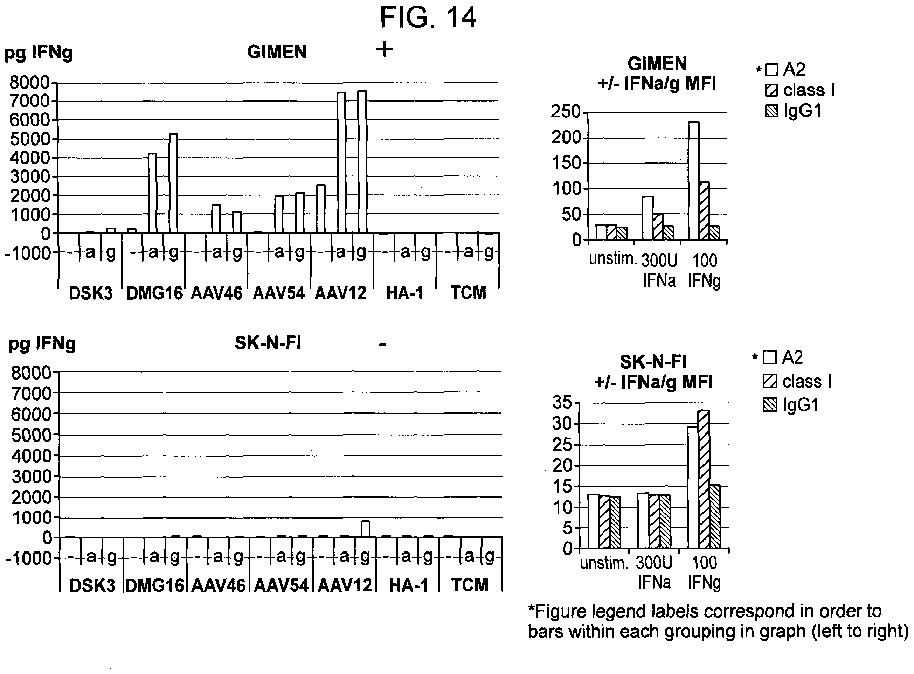

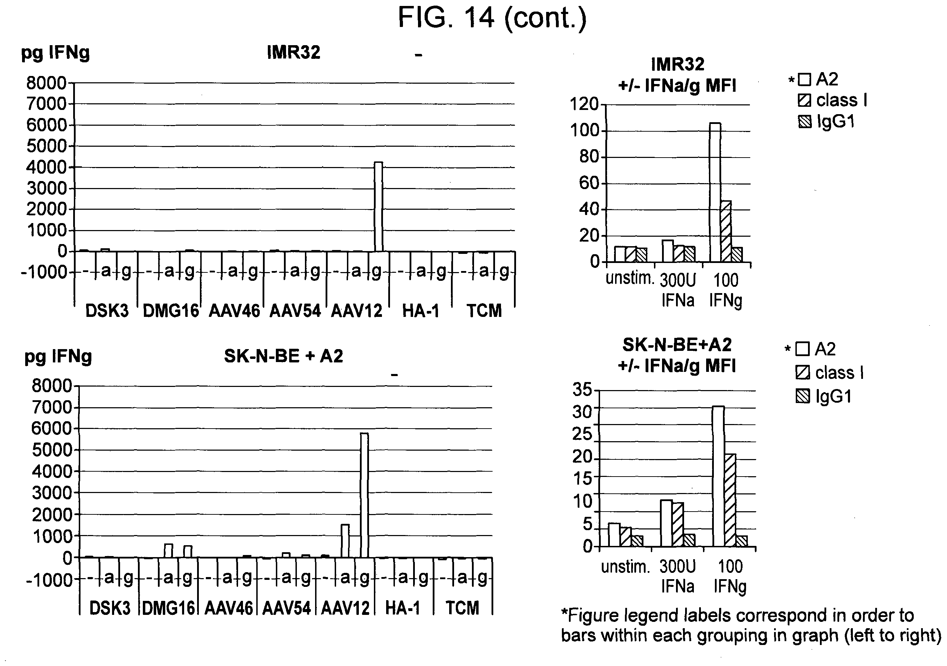

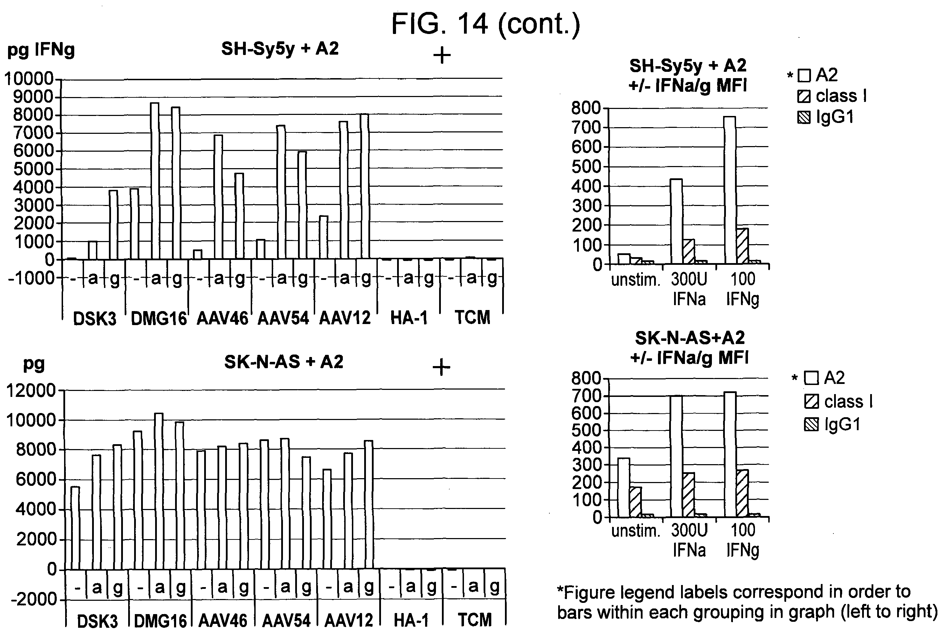

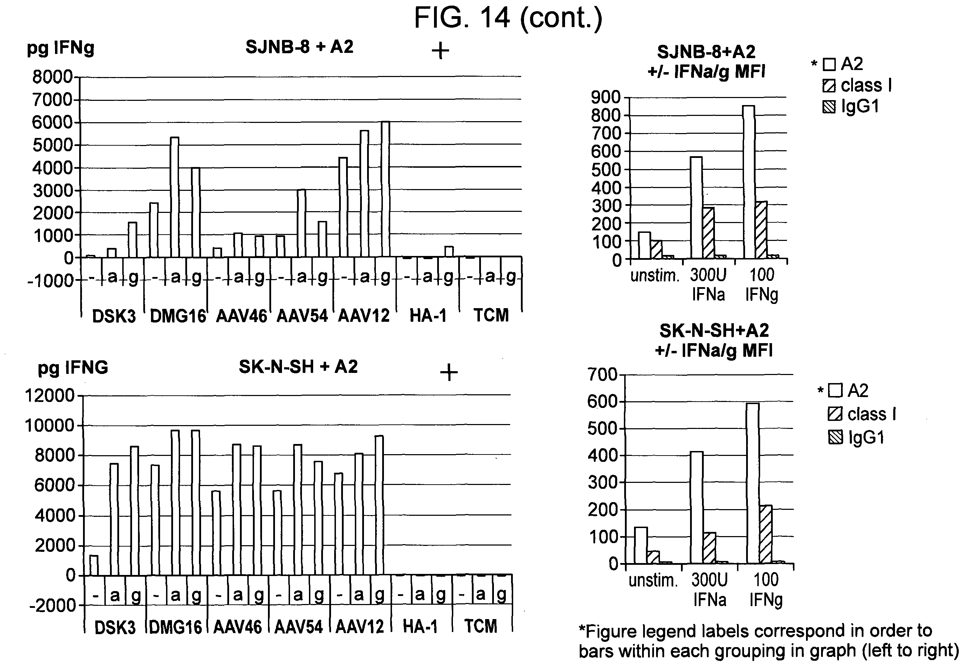

Figure 14. 4 different PRAME specific T cell clones were tested for reactivity directed against neuroblastoma (NB) cell lines. DSK3 (QLL specific), DMG16 and AAV54 (SLL specific, same TCR usage), and AAV46 (SLL). AAV12 was used as a positive control recognizing a peptide of USP11. Clone HA1k4 was a negative control. The NB cell lines were transduced with HLA-A2 (+ A2) treated for 48h with either 300 IU/ml of IFN-a or 100 IU/ml IFN-y, washed, and added to the different T cell clones. After 18h of coculture the supernatant was harvested and the IFN-γ production of the T cell clones was measured. The results indicate that 5 out of 7 NB cell lines express PRAME (for 1NB cell line (SK-N-F1) the PRAME expression is unknown, due to low class I expression even after treatment with IFN-a and IFN-y). Treatment with IFN-α and IFN-γ increased the HLA-class I expression on the cell surface, leading to better recognition of the NB cell lines by the different T cell clones.

By quantitative RT-PCR, the PRAME expression was determined in both the Ewing sarcoma cell lines and in the neuroblastoma cell lines, and correlated with recognition by the T cell clones.

Figure 15 Different retroviral vectors encoding for the PRAME-TCR were transduced into virus specific T cells and the reactivity was measured against target cells (T2 cells) loaded with PRAME peptide and two PRAME positive and HLA-A2 positive melanoma cell-lines: 518.A2 and FM6, and one PRAME negative but HLA-A2 positive melanoma cell line MI3046/2. After treatment for 18h with 100nM of AP1903 the PRAME reactivity was abrogated in the T cells transduced with the PRAME-TCR in combination with the iCasp9.

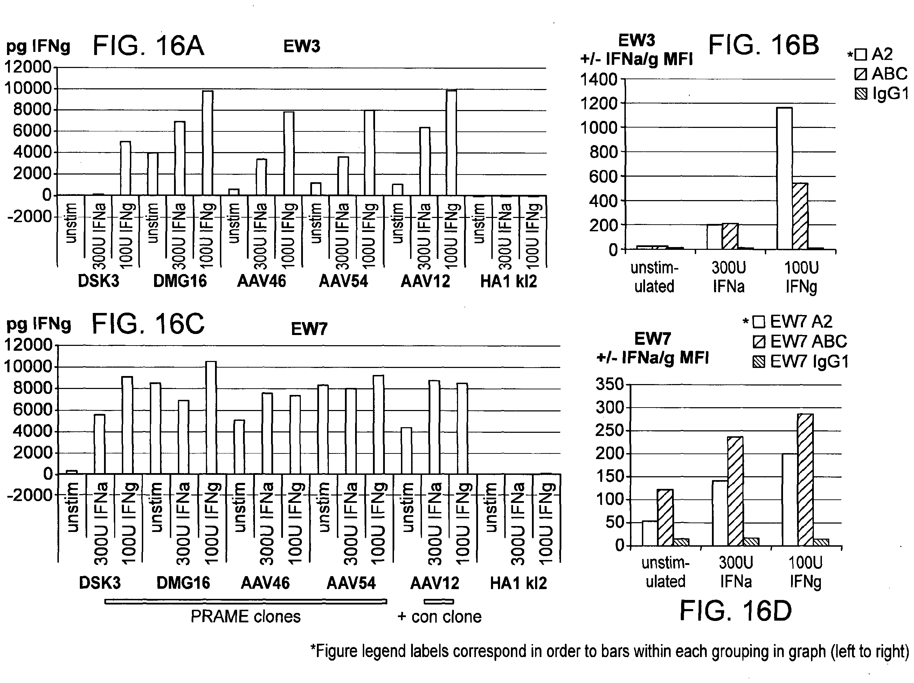

Figure 16 provides are bar graphs showing the recognition of Ewing sarcoma cells by PRAME-specific T cell clones. Ewing sarcoma cell lines were treated with or without IFN-y/IFN-a for 48h. HLA expression with or without IFN-y/IFN-a of the Ewing sarcoma cell lines is shown on the right part of the Figure.

### Examples of PRAME TCR sequences

Figures 17-19 provide examples of PRAME TCR amino acid sequences. Provided herein are amino acid and nucleotide sequences of PRAME TCR clones.

PRAME clone 54SLL. (TRAV8-4*04, TRBV9*01) in a PRAME/icasp9 construct.
SEQ ID NO: 1 a CDR3 AA
   CAVSGQTGANNLFFGTGTRLTVIP
SEQ ID NO: 2 a CDR3 NT 
TGTGCTGTGAGTGGCCAAACTGGGGCAAACAACCTCTTCTTTGGGACTGGAACGAGACTC
ACCGTTATTCCC
SEQ ID NO: 3 a CDR3 NT co* 
TGTGCCGTGAGCGGCCAGACCGGCGCCAACAACCTGTTCTTCGGCACCGGCACCCGGCT
GACAGTGATCCCT
SEQ ID NO: 4 β CDR3 AA
   CASARWDRGGEQYFGPGTRLTVT
SEQ ID NO: 5 β CDR3 NT 
TGTGCCAGCGCGAGGTGGGACAGGGGAGGCGAGCAGTACTTCGGGCCGGGCACCAGGC
TCACGGTCACA
SEQ ID NO: 6 β CDR3 NT co 
TGCGCCAGCGCCAGATGGGATAGAGGCGGCGAGCAGTACTTCGGCCCTGGCACCAGACT
GACCGTGACC
SEQ ID NO: 7 a VJ AA 
MLLLLVPVLEVIFTLGGTRAQSVTQLGSHVSVSERALVLLRCNYSSSVPPYLFWYVQYPNQGL
QLLLKYTSAATLVKGINGFEAEFKKSETSFHLTKPSAHMSDAAEYFCAVSGQTGANNLFFGTG
TRLTVIP
SEQ ID NO: 8 a VJ NT 
ATGCTCCTGCTGCTCGTCCCAGTGCTCGAGGTGATTTTTACCCTGGGAGGAACCAGAGCC
CAGTCGGTGACCCAGCTTGGCAGCCACGTCTCTGTCTCTGAACGAGCCCTGGTTCTGCTG
AGGTGCAACTACTCATCGTCTGTTCCACCATATCTCTTCTGGTATGTGCAATACCCCAACC
AAGGACTCCAGCTTCTCCTGAAGTACACATCAGCGGCCACCCTGGTTAAAGGCATCAACG
GTTTTGAGGCTGAATTTAAGAAGAGTGAAACCTCCTTCCACCTGACGAAACCCTCAGCCCA
TATGAGCGACGCGGCTGAGTACTTCTGTGCTGTGAGTGGCCAAACTGGGGCAAACAACCT
CTTCTTTGGGACTGGAACGAGACTCACCGTTATTCCC
SEQ ID NO: 9 a VJ NT co 
ATGCTGCTGCTGCTGGTGCCCGTGCTGGAAGTGATCTTCACCCTGGGCGGCACCAGAGC
CCAGAGCGTGACACAGCTGGGCAGCCACGTGTCCGTGTCTGAGAGGGCCCTGGTGCTGC
TGAGATGCAACTACTCTTCTAGCGTGCCCCCCTACCTGTTTTGGTACGTGCAGTACCCCAA
CCAGGGGCTGCAGCTGCTCCTGAAGTACACCAGCGCCGCCACACTGGTGAAGGGCATCA
ACGGCTTCGAGGCCGAGTTCAAGAAGTCCGAGACAAGCTTCCACCTGACCAAGCCCAGC
GCCCACATGTCTGACGCCGCCGAGTACTTCTGTGCCGTGAGCGGCCAGACCGGCGCCAA
CAACCTGTTCTTCGGCACCGGCACCCGGCTGACAGTGATCCCT
SEQ ID NO: 10 β VDJ AA 
MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASARWDRGGEQYF
SEQ ID NO: 11 β VDJ NT 
ATGGGCTTCAGGCTCCTCTGCTGTGTGGCCTTTTGTCTCCTGGGAGCAGGCCCAGTGGAT
TCTGGAGTCACACAAACCCCAAAGCACCTGATCACAGCAACTGGACAGCGAGTGACGCTG
AGATGCTCCCCTAGGTCTGGAGACCTCTCTGTGTACTGGTACCAACAGAGCCTGGACCAG
GGCCTCCAGTTCCTCATTCAGTATTATAATGGAGAAGAGAGAGCAAAAGGAAACATTCTTG
AACGATTCTCCGCACAACAGTTCCCTGACTTGCACTCTGAACTAAACCTGAGCTCTCTGGA
 
GCTGGGGGACTCAGCTTTGTATTTCTGTGCCAGCGCGAGGTGGGACAGGGGAGGCGAGC
AGTACTTCGGGCCGGGCACCAGGCTCACGGTCACA
SEQ ID NO: 12 β VDJ NT co 
ATGGGCTTCCGGCTGCTGTGCTGCGTGGCCTTTTGTCTGCTGGGAGCCGGCCCTGTGGA
TAGCGGCGTGACCCAGACCCCCAAGCACCTGATCACCGCCACCGGCCAGAGAGTGACCC
TGCGCTGCAGCCCTAGAAGCGGCGACCTGAGCGTGTACTGGTATCAGCAGAGCCTCGAC
CAGGGCCTGCAGTTCCTGATCCAGTACTACAACGGCGAGGAACGGGCCAAGGGCAACAT
CCTGGAACGGTTCAGCGCCCAGCAGTTCCCCGATCTGCACAGCGAGCTGAACCTGAGCA
GCCTGGAACTGGGCGACAGCGCCCTGTACTTCTGCGCCAGCGCCAGATGGGATAGAGGC
GGCGAGCAGTACTTCGGCCCTGGCACCAGACTGACCGTGACC
SEQ ID NO: 13 α VJ and constant AA 
MLLLLVPVLEVIFTLGGTRAQSVTQLGSHVSVSERALVLLRCNYSSSVPPYLFWYVQYPNQGL
QLLLKYTSAATLVKGINGFEAEFKKSETSFHLTKPSAHMSDAAEYFCAVSGQTGANNLFFGTG
TRLTVIPYIQNPDPAVYQLRDSKSSDKSVCLFTDFDSQTNVSQSKDSDVYITDKCVLDMRSMDF
KSNSAVAWSNKSDFACANAFNNSIIPEDTFFPSPESSCDVKLVEKSFETDTNLNFQNLSVIGFRI
LLLKVAGFNLLMTLRLWSS
SEQ ID NO: 14 α VJ and constant (murine) AA 
MLLLLVPVLEVIFTLGGTRAQSVTQLGSHVSVSERALVLLRCNYSSSVPPYLFWYVQYPNQGL
QLLLKYTSAATLVKGINGFEAEFKKSETSFHLTKPSAHMSDAAEYFCAVSGQTGANNLFFGTG
TRLTVIPDIQNPEPAVYQLKDPRSQDSTLCLFTDFDSQINVPKTMESGTFITDKCVLDMKAMDS
KSNGAIAWSNQTSFTCQDIFKETNATYPSSDVPCDATLTEKSFETDMNLNFQNLSVMGLRILLL
KVAGFNLLMTLRLWSS
SEQ ID NO: 15 α VJ and constant NT 
ATGCTCCTGCTGCTCGTCCCAGTGCTCGAGGTGATTTTTACCCTGGGAGGAACCAGAGCC
CAGTCGGTGACCCAGCTTGGCAGCCACGTCTCTGTCTCTGAACGAGCCCTGGTTCTGCTG
AGGTGCAACTACTCATCGTCTGTTCCACCATATCTCTTCTGGTATGTGCAATACCCCAACC
AAGGACTCCAGCTTCTCCTGAAGTACACATCAGCGGCCACCCTGGTTAAAGGCATCAACG
GTTTTGAGGCTGAATTTAAGAAGAGTGAAACCTCCTTCCACCTGACGAAACCCTCAGCCCA
TATGAGCGACGCGGCTGAGTACTTCTGTGCTGTGAGTGGCCAAACTGGGGCAAACAACCT
 
CTTCTTTGGGACTGGAACGAGACTCACCGTTATTCCCTATATCCAGAACCCTGACCCTGCC
GTGTACCAGCTGAGAGACTCTAAATCCAGTGACAAGTCTGTCTGCCTATTCACCGATTTTG
ATTCTCAAACAAATGTGTCACAAAGTAAGGATTCTGATGTGTATATCACAGACAAATGCGTG
CTAGACATGAGGTCTATGGACTTCAAGAGCAACAGTGCTGTGGCCTGGAGCAACAAATCT
GACTTTGCATGTGCAAACGCCTTCAACAACAGCATTATTCCAGAAGACACCTTCTTCCCCA
GCCCAGAAAGTTCCTGTGATGTCAAGCTGGTCGAGAAAAGCTTTGAAACAGATACGAACCT
AAACTTTCAAAACCTGTCAGTGATTGGGTTCCGAATCCTCCTCCTGAAAGTGGCCGGGTTT
AATCTGCTCATGACGCTGCGGTTGTGGTCCAGCTGA
SEQ ID NO: 16 a VJ and constant NT co 
ATGCTGCTGCTGCTGGTGCCCGTGCTGGAAGTGATCTTCACCCTGGGCGGCACCAGAGC
CCAGAGCGTGACACAGCTGGGCAGCCACGTGTCCGTGTCTGAGAGGGCCCTGGTGCTGC
TGAGATGCAACTACTCTTCTAGCGTGCCCCCCTACCTGTTTTGGTACGTGCAGTACCCCAA
CCAGGGGCTGCAGCTGCTCCTGAAGTACACCAGCGCCGCCACACTGGTGAAGGGCATCA
ACGGCTTCGAGGCCGAGTTCAAGAAGTCCGAGACAAGCTTCCACCTGACCAAGCCCAGC
GCCCACATGTCTGACGCCGCCGAGTACTTCTGTGCCGTGAGCGGCCAGACCGGCGCCAA
CAACCTGTTCTTCGGCACCGGCACCCGGCTGACAGTGATCCCTTACATCCAGAACCCCGA
CCCCGCCGTGTACCAGCTGCGGGACAGCAAGAGCAGCGACAAGAGCGTGTGCCTGTTCA
CCGACTTCGACAGCCAGACCAACGTGTCCCAGAGCAAGGACAGCGACGTGTACATCACC
GATAAGTGCGTGCTGGACATGCGGAGCATGGACTTCAAGAGCAACAGCGCCGTGGCCTG
GTCCAACAAGAGCGACTTCGCCTGCGCCAACGCCTTCAACAACAGCATCATCCCCGAGGA
CACATTCTTCCCAAGCCCCGAGAGCAGCTGCGACGTGAAGCTGGTGGAGAAGTCCTTCGA
GACAGACACCAACCTGAACTTCCAGAACCTGTCCGTGATCGGCTTCAGAATCCTGCTGCT
GAAAGTGGCCGGCTTCAACCTGCTGATGACCCTGCGGCTGTGGAGCAGCTGA
SEQ ID NO: 17 Reserved.
SEQ ID NO: 18 α VJ and constant (murine) NT co 
ATGCTGCTGCTGCTGGTGCCCGTGCTGGAAGTGATCTTCACCCTGGGCGGCACCAGAGC
CCAGAGCGTGACACAGCTGGGCAGCCACGTGTCCGTGTCTGAGAGGGCCCTGGTGCTGC
TGAGATGCAACTACTCTTCTAGCGTGCCCCCCTACCTGTTTTGGTACGTGCAGTACCCCAA
CCAGGGGCTGCAGCTGCTCCTGAAGTACACCAGCGCCGCCACACTGGTGAAGGGCATCA
ACGGCTTCGAGGCCGAGTTCAAGAAGTCCGAGACAAGCTTCCACCTGACCAAGCCCAGC
GCCCACATGTCTGACGCCGCCGAGTACTTCTGTGCCGTGAGCGGCCAGACCGGCGCCAA
CAACCTGTTCTTCGGCACCGGCACCCGGCTGACAGTGATCCCTGACATTCAGAACCCGGA
 
ACCGGCTGTATACCAGCTGAAGGACCCCCGATCTCAGGATAGTACTCTGTGCCTGTTCAC
CGACTTTGATAGTCAGATCAATGTGCCTAAAACCATGGAATCCGGAACTTTTATTACCGAC
AAGTGCGTGCTGGATATGAAAGCCATGGACAGTAAGTCAAACGGCGCCATCGCTTGGAGC
AATCAGACATCCTTCACTTGCCAGGATATCTTCAAGGAGACCAACGCAACATACCCATCCT
CTGACGTGCCCTGTGATGCCACCCTGACAGAGAAGTCTTTCGAAACAGACATGAACCTGA
ATTTTCAGAATCTGAGCGTGATGGGCCTGAGAATCCTGCTGCTGAAGGTCGCTGGGTTTA
ATCTGCTGATGACACTGCGGCTGTGGTCCTCATGA
SEQ ID NO: 19 β VJ and constant AA 
MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASARWDRGGEQYFGP
GTRLTVTEDLKNVFPPEVAVFEPSEAEISHTQKATLVCLATGFYPDHVELSWWVNGKEVHSGV
CTDPQPLKEQPALNDSRYCLSSRLRVSATFWQNPRNHFRCQVQFYGLSENDEWTQDRAKPV
TQIVSAEAWGRADCGFTSESYQQGVLSATILYEILLGKATLYAVLVSALVLMAMVKRKDSRG
SEQ ID NO: 20 β VJ and constant (murine) AA 
MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASARWDRGGEQYFGP
GTRLTVTEDLRNVTPPKVSLFEPSKAEIANKQKATLVCLARGFFPDHVELSWWVNGKEVHSGV
CTDPQAYKESNYSYCLSSRLRVSATFWHNPRNHFRCQVQFHGLSEEDKWPEGSPKPVTQNI
SAEAWGRADCGITSASYHQGVLSATILYEILLGKATLYAVLVSGLVLMAMVKKKNS
SEQ ID NO: 21 β VJ and constant NT 
ATGGGCTTCAGGCTCCTCTGCTGTGTGGCCTTTTGTCTCCTGGGAGCAGGCCCAGTGGAT
TCTGGAGTCACACAAACCCCAAAGCACCTGATCACAGCAACTGGACAGCGAGTGACGCTG
AGATGCTCCCCTAGGTCTGGAGACCTCTCTGTGTACTGGTACCAACAGAGCCTGGACCAG
GGCCTCCAGTTCCTCATTCAGTATTATAATGGAGAAGAGAGAGCAAAAGGAAACATTCTTG
AACGATTCTCCGCACAACAGTTCCCTGACTTGCACTCTGAACTAAACCTGAGCTCTCTGGA
GCTGGGGGACTCAGCTTTGTATTTCTGTGCCAGCGCGAGGTGGGACAGGGGAGGCGAGC
AGTACTTCGGGCCGGGCACCAGGCTCACGGTCACAGAGGACCTGAAAAACGTGTTCCCA
CCCGAGGTCGCTGTGTTTGAGCCATCAGAAGCAGAGATCTCCCACACCCAAAAGGCCACA
CTGGTGTGCCTGGCCACAGGCTTCTACCCCGACCACGTGGAGCTGAGCTGGTGGGTGAA
TGGGAAGGAGGTGCACAGTGGGGTCTGCACAGACCCGCAGCCCCTCAAGGAGCAGCCC
GCCCTCAATGACTCCAGATACTGCCTGAGCAGCCGCCTGAGGGTCTCGGCCACCTTCTGG
 
CAGAACCCCCGCAACCACTTCCGCTGTCAAGTCCAGTTCTACGGGCTCTCGGAGAATGAC
GAGTGGACCCAGGATAGGGCCAAACCTGTCACCCAGATCGTCAGCGCCGAGGCCTGGGG
TAGAGCAGACTGTGGCTTCACCTCCGAGTCTTACCAGCAAGGGGTCCTGTCTGCCACCAT
CCTCTATGAGATCTTGCTAGGGAAGGCCACCTTGTATGCCGTGCTGGTCAGTGCCCTCGT
GCTGATGGCCATGGTCAAGAGAAAGGATTCCAGAGGC
SEQ ID NO: 22 β VJ and constant NT co 
ATGGGCTTCCGGCTGCTGTGCTGCGTGGCCTTTTGTCTGCTGGGAGCCGGCCCTGTGGA
TAGCGGCGTGACCCAGACCCCCAAGCACCTGATCACCGCCACCGGCCAGAGAGTGACCC
TGCGCTGCAGCCCTAGAAGCGGCGACCTGAGCGTGTACTGGTATCAGCAGAGCCTCGAC
CAGGGCCTGCAGTTCCTGATCCAGTACTACAACGGCGAGGAACGGGCCAAGGGCAACAT
CCTGGAACGGTTCAGCGCCCAGCAGTTCCCCGATCTGCACAGCGAGCTGAACCTGAGCA
GCCTGGAACTGGGCGACAGCGCCCTGTACTTCTGCGCCAGCGCCAGATGGGATAGAGGC
GGCGAGCAGTACTTCGGCCCTGGCACCAGACTGACCGTGACCGAGGACCTGAAGAACGT
GTTCCCCCCAGAGGTGGCCGTGTTTGAGCCCAGCGAGGCCGAGATCAGCCACACCCAGA
AAGCCACCCTGGTGTGCCTGGCCACCGGCTTCTACCCCGACCACGTGGAGCTGTCTTGG
TGGGTGAACGGCAAAGAGGTGCACAGCGGCGTCTGCACCGACCCCCAGCCCCTGAAAGA
GCAGCCCGCCCTGAACGACAGCCGGTACTGCCTGAGCAGCAGACTGCGGGTGTCCGCCA
CCTTCTGGCAGAACCCCCGGAACCACTTCCGGTGCCAGGTGCAGTTCTACGGCCTGAGC
GAGAACGACGAGTGGACCCAGGACAGAGCCAAGCCTGTGACCCAGATCGTGTCTGCCGA
AGCCTGGGGCAGAGCCGACTGCGGCTTCACCAGCGAGAGCTACCAGCAGGGCGTGCTG
AGCGCCACCATCCTGTACGAGATCCTGCTGGGCAAGGCCACACTGTACGCCGTGCTGGT
GTCCGCTCTGGTGCTGATGGCCATGGTGAAGCGGAAGGACAGCAGAGGC
SEQ ID NO: 23 Reserved.
SEQ ID NO: 24 β VJ and constant (murine) NT co

ATGGGCTTCCGGCTGCTGTGCTGCGTGGCCTTTTGTCTGCTGGGAGCCGGCCCTGTGGA
TAGCGGCGTGACCCAGACCCCCAAGCACCTGATCACCGCCACCGGCCAGAGAGTGACCC
TGCGCTGCAGCCCTAGAAGCGGCGACCTGAGCGTGTACTGGTATCAGCAGAGCCTCGAC
CAGGGCCTGCAGTTCCTGATCCAGTACTACAACGGCGAGGAACGGGCCAAGGGCAACAT
CCTGGAACGGTTCAGCGCCCAGCAGTTCCCCGATCTGCACAGCGAGCTGAACCTGAGCA
GCCTGGAACTGGGCGACAGCGCCCTGTACTTCTGCGCCAGCGCCAGATGGGATAGAGGC
GGCGAGCAGTACTTCGGCCCTGGCACCAGACTGACCGTGACCGAAGATCTACGTAACGT
GACACCACCCAAAGTCTCACTGTTTGAGCCTAGCAAGGCAGAAATTGCCAACAAGCAGAA

GGCCACCCTGGTGTGCCTGGCAAGAGGGTTCTTTCCAGATCACGTGGAGCTGTCCTGGT
GGGTCAACGGCAAAGAAGTGCATTCTGGGGTCTGCACCGACCCCCAGGCTTACAAGGAG
AGTAATTACTCATATTGTCTGTCAAGCCGGCTGAGAGTGTCCGCCACATTCTGGCACAACC
CTAGGAATCATTTCCGCTGCCAGGTCCAGTTTCACGGCCTGAGTGAGGAAGATAAATGGC
CAGAGGGGTCACCTAAGCCAGTGACACAGAACATCAGCGCAGAAGCCTGGGGACGAGCA
GACTGTGGCATTACTAGCGCCTCCTATCATCAGGGCGTGCTGAGCGCCACTATCCTGTAC
GAGATTCTGCTGGGAAAGGCCACCCTGTATGCTGTGCTGGTCTCCGGCCTGGTGCTGATG
GCCATGGTCAAGAAAAAGAACTCT

PRAME clone 46SLL (TRAV35*02, TRBV28*01).
SEQ ID NO: 25 a CDR3 AA
   CAGIPRDNYGQNFVFGPGTRLSVLP
SEQ ID NO: 26 a CDR3 NT 
TGTGCTGGGATACCCCGGGATAACTATGGTCAGAATTTTGTCTTTGGTCCCGGAACCAGAT
TGTCCGTGCTGCCC
SEQ ID NO: 27 a CDR3 NT co* 
TGCGCCGGCATCCCTCGGGACAACTACGGCCAGAACTTCGTGTTCGGCCCTGGCACCAG
ACTGAGCGTGCTGCCC
SEQ ID NO: 28 β CDR3 AA
   CASTPWLAGGNEQFFGPGTRLTVL
SEQ ID NO: 29 β CDR3 NT 
TGTGCCAGCACCCCGTGGCTAGCGGGAGGCAATGAGCAGTTCTTCGGGCCAGGGACACG
GCTCACCGTGCTA
SEQ ID NO: 30 β CDR3 NT co 
TGTGCCAGCACCCCTTGGCTGGCTGGCGGCAACGAGCAGTTTTTGGCCCTGGCACCCG
GCTGACCGTGCTG
SEQ ID NO: 31 a VJ AA 
MLLEHLLIILWMQLTWVSGQQLNQSPQSMFIQEGEDVSMNCTSSSIFNTWLWYKQDPGEGPV
LLIALYKAGELTSNGRLTAQFGITRKDSFLNISASIPSDVGIYFCAGIPRDNYGQNFVFGPGTRLS
VLP
SEQ ID NO: 32 α VJ NT 
ATGCTCCTTGAACATTTATTAATAATCTTGTGGATGCAGCTGACATGGGTCAGTGGTCAAC
AGCTGAATCAGAGTCCTCAATCTATGTTTATCCAGGAAGGAGAAGATGTCTCCATGAACTG
CACTTCTTCAAGCATATTTAACACCTGGCTATGGTACAAGCAGGACCCTGGGGAAGGTCCT
GTCCTCTTGATAGCCTTATATAAGGCTGGTGAATTGACCTCAAATGGAAGACTGACTGCTC
AGTTTGGTATAACCAGAAAGGACAGCTTCCTGAATATCTCAGCATCCATACCTAGTGATGT
AGGCATCTACTTCTGTGCTGGGATACCCCGGGATAACTATGGTCAGAATTTTGTCTTTGGT
CCCGGAACCAGATTGTCCGTGCTGCCC
SEQ ID NO: 33 α VJ NT co 
ATGCTGCTGGAACATCTGCTGATCATCCTGTGGATGCAGCTGACCTGGGTGTCCGGCCAG
CAGCTGAATCAGAGCCCCCAGAGCATGTTCATCCAGGAAGGCGAGGACGTGTCCATGAAC
TGCACCAGCAGCAGCATCTTCAACACCTGGCTGTGGTACAAGCAGGACCCCGGCGAAGG
ACCCGTGCTGCTGATCGCCCTGTATAAGGCCGGCGAGCTGACCAGCAACGGCAGACTGA
CAGCCCAGTTCGGCATTACCCGGAAGGACAGCTTCCTGAACATCAGCGCCAGCATCCCCA
GCGACGTGGGCATCTACTTTTGCGCCGGCATCCCTCGGGACAACTACGGCCAGAACTTCG
TGTTCGGCCCTGGCACCAGACTGAGCGTGCTGCCC
SEQ ID NO: 34 β VDJ AA 
MGIRLLCRVAFCFLAVGLVDVKVTQSSRYLVKRTGEKVFLECVQDMDHENMFWYRQDPGLGL
RLIYFSYDVKMKEKGDIPEGYSVSREKKERFSLILESASTNQTSMYLCASTPWLAGGNEQFFG
PGTRLTVL
SEQ ID NO: 35 β VDJ NT 
ATGGGAATCAGGCTCCTCTGTCGTGTGGCCTTTTGTTTCCTGGCTGTAGGCCTCGTAGAT
GTGAAAGTAACCCAGAGCTCGAGATATCTAGTCAAAAGGACGGGAGAGAAAGTTTTTCTG
 
GAATGTGTCCAGGATATGGACCATGAAAATATGTTCTGGTATCGACAAGACCCAGGTCTGG
GGCTACGGCTGATCTATTTCTCATATGATGTTAAAATGAAAGAAAAAGGAGATATTCCTGAG
GGGTACAGTGTCTCTAGAGAGAAGAAGGAGCGCTTCTCCCTGATTCTGGAGTCCGCCAGC
ACCAACCAGACATCTATGTACCTCTGTGCCAGCACCCCGTGGCTAGCGGGAGGCAATGAG
CAGTTCTTCGGGCCAGGGACACGGCTCACCGTGCTA
SEQ ID NO: 36 β VDJ NT co 
ATGGGCATCCGGCTGCTGTGCAGAGTGGCCTTCTGCTTTCTGGCCGTGGGCCTGGTGGA
CGTGAAAGTGACCCAGAGCAGCAGATACCTCGTGAAGCGGACCGGCGAGAAGGTGTTCC
TGGAATGCGTGCAGGACATGGACCACGAGAATATGTTCTGGTACAGACAGGACCCCGGC
CTGGGCCTGCGGCTGATCTACTTCAGCTACGACGTGAAGATGAAGGAAAAGGGCGACATC
CCCGAGGGCTACAGCGTGTCCAGAGAGAAGAAAGAGCGGTTCAGCCTGATCCTGGAAAG
CGCCAGCACCAACCAGACCAGCATGTACCTGTGTGCCAGCACCCCTTGGCTGGCTGGCG
GCAACGAGCAGTTTTTTGGCCCTGGCACCCGGCTGACCGTGCTG
37 α VJ and constant AA 
MLLEHLLIILWMQLTWVSGQQLNQSPQSMFIQEGEDVSMNCTSSSIFNTWLWYKQDPGEGPV
LLIALYKAGELTSNGRLTAQFGITRKDSFLNISASIPSDVGIYFCAGIPRDNYGQNFVFGPGTRLS
VLPYIQNPDPAVYQLRDSKSSDKSVCLFTDFDSQTNVSQSKDSDVYITDKTVLDMRSMDFKSN
SAVAWSNKSDFACANAFNNSIIPEDTFFPSPESSCDVKLVEKSFETDTNLNFQNLSVIGFRILLL
KVAGFNLLMTLRLWSS
38 α VJ and constant (murine) AA 
MLLEHLLIILWMQLTWVSGQQLNQSPQSMFIQEGEDVSMNCTSSSIFNTWLWYKQDPGEGPV
LLIALYKAGELTSNGRLTAQFGITRKDSFLNISASIPSDVGIYFCAGIPRDNYGQNFVFGPGTRLS
VLPDIQNPEPAVYQLKDPRSQDSTLCLFTDFDSQINVPKTMESGTFITDKCVLDMKAMDSKSN
GAIAWSNQTSFTCQDIFKETNATYPSSDVPCDATLTEKSFETDMNLNFQNLSVMGLRILLLKVA
GFNLLMTLRLWSS
SEQ ID NO: 39 α VJ and constant NT 
ATGCTCCTTGAACATTTATTAATAATCTTGTGGATGCAGCTGACATGGGTCAGTGGTCAAC
AGCTGAATCAGAGTCCTCAATCTATGTTTATCCAGGAAGGAGAAGATGTCTCCATGAACTG
CACTTCTTCAAGCATATTTAACACCTGGCTATGGTACAAGCAGGACCCTGGGGAAGGTCCT
 
GTCCTCTTGATAGCCTTATATAAGGCTGGTGAATTGACCTCAAATGGAAGACTGACTGCTC
AGTTTGGTATAACCAGAAAGGACAGCTTCCTGAATATCTCAGCATCCATACCTAGTGATGT
AGGCATCTACTTCTGTGCTGGGATACCCCGGGATAACTATGGTCAGAATTTTGTCTTTGGT
CCCGGAACCAGATTGTCCGTGCTGCCCTATATCCAGAACCCTGACCCTGCCGTGTACCAG
CTGAGAGACTCTAAATCCAGTGACAAGTCTGTCTGCCTATTCACCGATTTTGATTCTCAAAC
AAATGTGTCACAAAGTAAGGATTCTGATGTGTATATCACAGACAAAACTGTGCTAGACATG
AGGTCTATGGACTTCAAGAGCAACAGTGCTGTGGCCTGGAGCAACAAATCTGACTTTGCAT
GTGCAAACGCCTTCAACAACAGCATTATTCCAGAAGACACCTTCTTCCCCAGCCCAGAAAG
TTCCTGTGATGTCAAGCTGGTCGAGAAAAGCTTTGAAACAGATACGAACCTAAACTTTCAA
AACCTGTCAGTGATTGGGTTCCGAATCCTCCTCCTGAAAGTGGCCGGGTTTAATCTGCTCA
TGACGCTGCGGTTGTGGTCCAGCTGA
SEQ ID NO: 40 α VJ and constant NT co 
ATGCTGCTGGAACATCTGCTGATCATCCTGTGGATGCAGCTGACCTGGGTGTCCGGCCAG
CAGCTGAATCAGAGCCCCCAGAGCATGTTCATCCAGGAAGGCGAGGACGTGTCCATGAAC
TGCACCAGCAGCAGCATCTTCAACACCTGGCTGTGGTACAAGCAGGACCCCGGCGAAGG
ACCCGTGCTGCTGATCGCCCTGTATAAGGCCGGCGAGCTGACCAGCAACGGCAGACTGA
CAGCCCAGTTCGGCATTACCCGGAAGGACAGCTTCCTGAACATCAGCGCCAGCATCCCCA
GCGACGTGGGCATCTACTTTTGCGCCGGCATCCCTCGGGACAACTACGGCCAGAACTTCG
TGTTCGGCCCTGGCACCAGACTGAGCGTGCTGCCCTACATCCAGAACCCCGACCCTGCC
GTGTACCAGCTGAGAGACAGCAAGAGCAGCGACAAGAGCGTGTGCCTGTTCACCGACTTC
GACAGCCAGACCAACGTGTCCCAGAGCAAGGACTCCGACGTGTACATCACCGACAAGAC
CGTGCTGGACATGCGGAGCATGGACTTCAAGAGCAACAGCGCCGTGGCCTGGTCCAACA
AGAGCGATTTCGCCTGCGCCAACGCCTTCAACAACAGCATTATCCCCGAGGACACATTCTT
CCCAAGCCCCGAGAGCAGCTGCGACGTGAAGCTGGTGGAAAAGAGCTTCGAGACAGACA
CCAACCTGAACTTCCAGAACCTGAGCGTGATCGGCTTCCGGATCCTGCTGCTGAAGGTGG
CCGGCTTCAACCTGCTGATGACCCTGAGACTGTGGTCCAGCTGA
SEQ ID NO: 41 Reserved.
SEQ ID NO: 42 α VJ and constant (murine) NT co 
ATGCTGCTGGAACATCTGCTGATCATCCTGTGGATGCAGCTGACCTGGGTGTCCGGCCAG
CAGCTGAATCAGAGCCCCCAGAGCATGTTCATCCAGGAAGGCGAGGACGTGTCCATGAAC
TGCACCAGCAGCAGCATCTTCAACACCTGGCTGTGGTACAAGCAGGACCCCGGCGAAGG
ACCCGTGCTGCTGATCGCCCTGTATAAGGCCGGCGAGCTGACCAGCAACGGCAGACTGA
 
CAGCCCAGTTCGGCATTACCCGGAAGGACAGCTTCCTGAACATCAGCGCCAGCATCCCCA
GCGACGTGGGCATCTACTTTTGCGCCGGCATCCCTCGGGACAACTACGGCCAGAACTTCG
TGTTCGGCCCTGGCACCAGACTGAGCGTGCTGCCCGACATTCAGAACCCGGAACCGGCT
GTATACCAGCTGAAGGACCCCCGATCTCAGGATAGTACTCTGTGCCTGTTCACCGACTTTG
ATAGTCAGATCAATGTGCCTAAAACCATGGAATCCGGAACTTTTATTACCGACAAGTGCGT
GCTGGATATGAAAGCCATGGACAGTAAGTCAAACGGCGCCATCGCTTGGAGCAATCAGAC
ATCCTTCACTTGCCAGGATATCTTCAAGGAGACCAACGCAACATACCCATCCTCTGACGTG
CCCTGTGATGCCACCCTGACAGAGAAGTCTTTCGAAACAGACATGAACCTGAATTTTCAGA
ATCTGAGCGTGATGGGCCTGAGAATCCTGCTGCTGAAGGTCGCTGGGTTTAATCTGCTGA
TGACACTGCGGCTGTGGTCCTCATGA
SEQ ID NO: 43 β VJ and constant AA 
MGIRLLCRVAFCFLAVGLVDVKVTQSSRYLVKRTGEKVFLECVQDMDHENMFWYRQDPGLGL
RLIYFSYDVKMKEKGDIPEGYSVSREKKERFSLILESASTNQTSMYLCASTPWLAGGNEQFFG
PGTRLTVLEDLKNVFPPEVAVFEPSEAEISHTQKATLVCLATGFYPDHVELSWWVNGKEVHSG
VSTDPQPLKEQPALNDSRYCLSSRLRVSATFWQNPRNHFRCQVQFYGLSENDEWTQDRAKP
VTQIVSAEAWGRADCGFTSESYQQGVLSATILYEILLGKATLYAVLVSALVLMAMVKRKDSRG
SEQ ID NO: 44 β VJ and constant (murine) AA 
MGIRLLCRVAFCFLAVGLVDVKVTQSSRYLVKRTGEKVFLECVQDMDHENMFWYRQDPGLGL
RLIYFSYDVKMKEKGDIPEGYSVSREKKERFSLILESASTNQTSMYLCASTPWLAGGNEQFFG
PGTRLTVLEDLRNVTPPKVSLFEPSKAEIANKQKATLVCLARGFFPDHVELSWWVNGKEVHSG
VCTDPQAYKESNYSYCLSSRLRVSATFWHNPRNHFRCQVQFHGLSEEDKWPEGSPKPVTQN
ISAEAWGRADCGITSASYHQGVLSATILYEILLGKATLYAVLVSGLVLMAMVKKKNS
SEQ ID NO: 45 β VJ and constant NT 
ATGGGAATCAGGCTCCTCTGTCGTGTGGCCTTTTGTTTCCTGGCTGTAGGCCTCGTAGAT
GTGAAAGTAACCCAGAGCTCGAGATATCTAGTCAAAAGGACGGGAGAGAAAGTTTTTCTG
GAATGTGTCCAGGATATGGACCATGAAAATATGTTCTGGTATCGACAAGACCCAGGTCTGG
GGCTACGGCTGATCTATTTCTCATATGATGTTAAAATGAAAGAAAAAGGAGATATTCCTGAG
GGGTACAGTGTCTCTAGAGAGAAGAAGGAGCGCTTCTCCCTGATTCTGGAGTCCGCCAGC
ACCAACCAGACATCTATGTACCTCTGTGCCAGCACCCCGTGGCTAGCGGGAGGCAATGAG
CAGTTCTTCGGGCCAGGGACACGGCTCACCGTGCTAGAGGACCTGAAAAACGTGTTCCCA
CCCGAGGTCGCTGTGTTTGAGCCATCAGAAGCAGAGATCTCCCACACCCAAAAGGCCACA
 
CTGGTATGCCTGGCCACAGGCTTCTACCCCGACCACGTGGAGCTGAGCTGGTGGGTGAA
TGGGAAGGAGGTGCACAGTGGGGTCAGCACAGACCCGCAGCCCCTCAAGGAGCAGCCC
GCCCTCAATGACTCCAGATACTGCCTGAGCAGCCGCCTGAGGGTCTCGGCCACCTTCTGG
CAGAACCCCCGCAACCACTTCCGCTGTCAAGTCCAGTTCTACGGGCTCTCGGAGAATGAC
GAGTGGACCCAGGATAGGGCCAAACCCGTCACCCAGATCGTCAGCGCCGAGGCCTGGG
GTAGAGCAGACTGTGGCTTCACCTCCGAGTCTTACCAGCAAGGGGTCCTGTCTGCCACCA
TCCTCTATGAGATCTTGCTAGGGAAGGCCACCTTGTATGCCGTGCTGGTCAGTGCCCTCG
TGCTGATGGCCATGGTCAAGAGAAAGGATTCCAGAGGCTAG
SEQ ID NO: 46 β VJ and constant NT co 
ATGGGCATCCGGCTGCTGTGCAGAGTGGCCTTCTGCTTTCTGGCCGTGGGCCTGGTGGA
CGTGAAAGTGACCCAGAGCAGCAGATACCTCGTGAAGCGGACCGGCGAGAAGGTGTTCC
TGGAATGCGTGCAGGACATGGACCACGAGAATATGTTCTGGTACAGACAGGACCCCGGC
CTGGGCCTGCGGCTGATCTACTTCAGCTACGACGTGAAGATGAAGGAAAAGGGCGACATC
CCCGAGGGCTACAGCGTGTCCAGAGAGAAGAAAGAGCGGTTCAGCCTGATCCTGGAAAG
CGCCAGCACCAACCAGACCAGCATGTACCTGTGTGCCAGCACCCCTTGGCTGGCTGGCG
GCAACGAGCAGTTTTTTGGCCCTGGCACCCGGCTGACCGTGCTGGAAGATCTGAAGAACG
TGTTCCCCCCAGAGGTGGCCGTGTTCGAGCCTTCTGAGGCCGAGATCAGCCACACCCAG
AAAGCCACCCTCGTGTGTCTGGCCACCGGCTTCTACCCCGACCACGTGGAACTGTCTTGG
TGGGTCAACGGCAAAGAGGTGCACAGCGGCGTGTCCACCGATCCCCAGCCTCTGAAAGA
ACAGCCCGCCCTGAACGACAGCCGGTACTGCCTGTCCAGCAGACTGAGAGTGTCCGCCA
CCTTCTGGCAGAACCCCCGGAACCACTTCAGATGCCAGGTGCAGTTCTACGGCCTGAGCG
AGAACGACGAGTGGACCCAGGACAGAGCCAAGCCCGTGACACAGATCGTGTCTGCCGAA
GCCTGGGGCAGAGCCGATTGCGGCTTTACCAGCGAGAGCTACCAGCAGGGCGTGCTGAG
CGCCACCATCCTGTACGAGATCCTGCTGGGCAAGGCCACCCTGTACGCCGTGCTGGTGT
CTGCCCTGGTGCTGATGGCCATGGTCAAGCGGAAGGACAGCCGGGGCTAA
SEQ ID NO: 47 Reserved.
SEQ ID NO: 48 β VJ and constant (murine) NT co

ATGGGCATCCGGCTGCTGTGCAGAGTGGCCTTCTGCTTTCTGGCCGTGGGCCTGGTGGA
CGTGAAAGTGACCCAGAGCAGCAGATACCTCGTGAAGCGGACCGGCGAGAAGGTGTTCC
TGGAATGCGTGCAGGACATGGACCACGAGAATATGTTCTGGTACAGACAGGACCCCGGC
CTGGGCCTGCGGCTGATCTACTTCAGCTACGACGTGAAGATGAAGGAAAAGGGCGACATC
CCCGAGGGCTACAGCGTGTCCAGAGAGAAGAAAGAGCGGTTCAGCCTGATCCTGGAAAG

CGCCAGCACCAACCAGACCAGCATGTACCTGTGTGCCAGCACCCCTTGGCTGGCTGGCG
GCAACGAGCAGTTTTTGGCCCTGGCACCCGGCTGACCGTGCTGGAAGATCTACGTAACG
TGACACCACCCAAAGTCTCACTGTTTGAGCCTAGCAAGGCAGAAATTGCCAACAAGCAGA
AGGCCACCCTGGTGTGCCTGGCAAGAGGGTTCTTTCCAGATCACGTGGAGCTGTCCTGGT
GGGTCAACGGCAAAGAAGTGCATTCTGGGGTCTGCACCGACCCCCAGGCTTACAAGGAG
AGTAATTACTCATATTGTCTGTCAAGCCGGCTGAGAGTGTCCGCCACATTCTGGCACAACC
CTAGGAATCATTTCCGCTGCCAGGTCCAGTTTCACGGCCTGAGTGAGGAAGATAAATGGC
CAGAGGGGTCACCTAAGCCAGTGACACAGAACATCAGCGCAGAAGCCTGGGGACGAGCA
GACTGTGGCATTACTAGCGCCTCCTATCATCAGGGCGTGCTGAGCGCCACTATCCTGTAC
GAGATTCTGCTGGGAAAGGCCACCCTGTATGCTGTGCTGGTCTCCGGCCTGGTGCTGATG
GCCATGGTCAAGAAAAAGAACTCT

PRAME clone DSK3 QLL (TRAV12-2*01, TRBV9*01).
SEQ ID NO: 49 α CDR3 AA
   CAVKDNAGNMLTFGGGTRLMVKP
SEQ ID NO: 50 α CDR3 NT 
TGTGCCGTGAAGGATAATGCAGGCAACATGCTCACCTTTGGAGGGGGAACAAGGTTAATG
GTCAAACCC
SEQ ID NO: 51 α CDR3 NT co* 
TGCGCCGTGAAGGACAACGCCGGCAACATGCTGACCTTCGGCGGAGGCACCCGGCTGAT
GGTCAAGCCC
SEQ ID NO: 52 β CDR3 AA
   CASSDGGGVYEQYFGPGTRLTVT
SEQ ID NO: 53 β CDR3 NT 
TGTGCCAGCAGCGACGGAGGGGGCGTCTACGAGCAGTACTTCGGGCCGGGCACCAGGC
TCACGGTCACA
SEQ ID NO: 54 β CDR3 NT co 
TGTGCCAGCTCTGATGGCGGCGGAGTGTACGAGCAGTACTTCGGCCCTGGCACCAGACT
GACCGTGACC
SEQ ID NO: 55 a VJ AA 
MMKSLRVLLVILWLQLSWVWSQQKEVEQNSGPLSVPEGAIASLNCTYSDRGSQSFFWYRQY
SGKSPELIMFIYSNGDKEDGRFTAQLNKASQYVSLLIRDSQPSDSATYLCAVKDNAGNMLTFG
GGTRLMVKP
SEQ ID NO: 56 α VJ NT 
ATGATGAAATCCTTGAGAGTTTTACTAGTGATCCTGTGGCTTCAGTTGAGCTGGGTTTGGA
GCCAACAGAAGGAGGTGGAGCAGAATTCTGGACCCCTCAGTGTTCCAGAGGGAGCCATT
GCCTCTCTCAACTGCACTTACAGTGACCGAGGTTCCCAGTCCTTCTTCTGGTACAGACAAT
ATTCTGGGAAAAGCCCTGAGTTGATAATGTTCATATACTCCAATGGTGACAAAGAAGATGG
AAGGTTTACAGCACAGCTCAATAAAGCCAGCCAGTATGTTTCTCTGCTCATCAGAGACTCC
CAGCCCAGTGATTCAGCCACCTACCTCTGTGCCGTGAAGGATAATGCAGGCAACATGCTC
ACCTTTGGAGGGGGAACAAGGTTAATGGTCAAACCC
SEQ ID NO: 57 α VJ NT co 
ATGATGAAGTCCCTGCGGGTGCTGCTCGTGATCCTGTGGCTGCAGCTGAGCTGGGTGTG
GTCCCAGCAGAAAGAGGTGGAACAGAACAGCGGCCCTCTGAGCGTGCCAGAAGGCGCTA
TCGCCAGCCTGAACTGCACCTACAGCGACAGAGGCAGCCAGAGCTTCTTCTGGTACAGAC
AGTACAGCGGCAAGAGCCCCGAGCTGATCATGTTCATCTACAGCAACGGCGACAAAGAGG
ACGGCCGGTTCACCGCCCAGCTGAACAAGGCCAGCCAGTACGTGTCCCTGCTGATCAGA
GACAGCCAGCCCAGCGACAGCGCCACCTATCTGTGCGCCGTGAAGGACAACGCCGGCAA
CATGCTGACCTTCGGCGGAGGCACCCGGCTGATGGTCAAGCCC
SEQ ID NO: 58 β VDJ AA 
MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASSDGGGVYEQYFGP
GTRLTVT
SEQ ID NO: 59 β VDJ NT 
ATGGGCTTCAGGCTCCTCTGCTGTGTGGCCTTTTGTCTCCTGGGAGCAGGCCCAGTGGAT
TCTGGAGTCACACAAACCCCAAAGCACCTGATCACAGCAACTGGACAGCGAGTGACGCTG
AGATGCTCCCCTAGGTCTGGAGACCTCTCTGTGTACTGGTACCAACAGAGCCTGGACCAG
GGCCTCCAGTTCCTCATTCAGTATTATAATGGAGAAGAGAGAGCAAAAGGAAACATTCTTG
AACGATTCTCCGCACAACAGTTCCCTGACTTGCACTCTGAACTAAACCTGAGCTCTCTGGA
GCTGGGGGACTCAGCTTTGTATTTCTGTGCCAGCAGCGACGGAGGGGGCGTCTACGAGC
AGTACTTCGGGCCGGGCACCAGGCTCACGGTCACA
SEQ ID NO: 60 β VDJ NT co 
ATGGGCTTCAGACTGCTGTGCTGCGTGGCCTTCTGTCTGCTGGGAGCCGGCCCTGTGGA
TAGCGGCGTGACACAGACACCCAAGCACCTGATCACCGCCACCGGCCAGCGCGTGACAC
TGAGATGTAGCCCTAGAAGCGGCGACCTGAGCGTGTACTGGTATCAGCAGAGCCTGGAC
CAGGGCCTGCAGTTCCTGATCCAGTACTACAACGGCGAGGAACGGGCCAAGGGCAACAT
CCTGGAACGGTTCAGCGCCCAGCAGTTCCCCGATCTGCACAGCGAGCTGAACCTGAGCA
GCCTGGAACTGGGCGACAGCGCCCTGTACTTCTGTGCCAGCTCTGATGGCGGCGGAGTG
TACGAGCAGTACTTCGGCCCTGGCACCAGACTGACCGTGACC
SEQ ID NO: 61 α VJ and constant AA 
MMKSLRVLLVILWLQLSWVWSQQKEVEQNSGPLSVPEGAIASLNCTYSDRGSQSFFWYRQY
SGKSPELIMFIYSNGDKEDGRFTAQLNKASQYVSLLIRDSQPSDSATYLCAVKDNAGNMLTFG
GGTRLMVKPHIQNPDPAVYQLRDSKSSDKSVCLFTDFDSQTNVSQSKDSDVYITDKTVLDMRS
MDFKSNSAVAWSNKSDFACANAFNNSIIPEDTFFPSPESSCDVKLVEKSFETDTNLNFQNLSVI
GFRILLLKVAGFNLLMTLRLWSS
SEQ ID NO: 62 α VJ and constant (murine) AA 
MMKSLRVLLVILWLQLSWVWSQQKEVEQNSGPLSVPEGAIASLNCTYSDRGSQSFFWYRQY
SGKSPELIMFIYSNGDKEDGRFTAQLNKASQYVSLLIRDSQPSDSATYLCAVKDNAGNMLTFG
GGTRLMVKPDIQNPEPAVYQLKDPRSQDSTLCLFTDFDSQINVPKTMESGTFITDKCVLDMKA
MDSKSNGAIAWSNQTSFTCQDIFKETNATYPSSDVPCDATLTEKSFETDMNLNFQNLSVMGLR
ILLLKVAGFNLLMTLRLWSS
SEQ ID NO: 63 α VJ and constant NT 
ATGATGAAATCCTTGAGAGTTTTACTAGTGATCCTGTGGCTTCAGTTGAGCTGGGTTTGGA
GCCAACAGAAGGAGGTGGAGCAGAATTCTGGACCCCTCAGTGTTCCAGAGGGAGCCATT
GCCTCTCTCAACTGCACTTACAGTGACCGAGGTTCCCAGTCCTTCTTCTGGTACAGACAAT
ATTCTGGGAAAAGCCCTGAGTTGATAATGTTCATATACTCCAATGGTGACAAAGAAGATGG
AAGGTTTACAGCACAGCTCAATAAAGCCAGCCAGTATGTTTCTCTGCTCATCAGAGACTCC
CAGCCCAGTGATTCAGCCACCTACCTCTGTGCCGTGAAGGATAATGCAGGCAACATGCTC
ACCTTTGGAGGGGGAACAAGGTTAATGGTCAAACCCCATATCCAGAACCCTGACCCTGCC
GTGTACCAGCTGAGAGACTCTAAATCCAGTGACAAGTCTGTCTGCCTATTCACCGATTTTG
ATTCTCAAACAAATGTGTCACAAAGTAAGGATTCTGATGTGTATATCACAGACAAAACTGTG
CTAGACATGAGGTCTATGGACTTCAAGAGCAACAGTGCTGTGGCCTGGAGCAACAAATCT
GACTTTGCATGTGCAAACGCCTTCAACAACAGCATTATTCCAGAAGACACCTTCTTCCCCA
GCCCAGAAAGTTCCTGTGATGTCAAGCTGGTCGAGAAAAGCTTTGAAACAGATACGAACCT
AAACTTTCAAAACCTGTCAGTGATTGGGTTCCGAATCCTCCTCCTGAAAGTGGCCGGGTTT
AATCTGCTCATGACGCTGCGGTTGTGGTCCAGCTGA
SEQ ID NO: 64 α VJ and constant NT co 
ATGATGAAGTCCCTGCGGGTGCTGCTCGTGATCCTGTGGCTGCAGCTGAGCTGGGTGTG
GTCCCAGCAGAAAGAGGTGGAACAGAACAGCGGCCCTCTGAGCGTGCCAGAAGGCGCTA
TCGCCAGCCTGAACTGCACCTACAGCGACAGAGGCAGCCAGAGCTTCTTCTGGTACAGAC
AGTACAGCGGCAAGAGCCCCGAGCTGATCATGTTCATCTACAGCAACGGCGACAAAGAGG
ACGGCCGGTTCACCGCCCAGCTGAACAAGGCCAGCCAGTACGTGTCCCTGCTGATCAGA
GACAGCCAGCCCAGCGACAGCGCCACCTATCTGTGCGCCGTGAAGGACAACGCCGGCAA
CATGCTGACCTTCGGCGGAGGCACCCGGCTGATGGTCAAGCCCCACATCCAGAACCCCG
ACCCCGCCGTGTACCAGCTGAGAGACAGCAAGAGCAGCGATAAGAGCGTGTGCCTGTTC
ACCGACTTCGACAGCCAGACCAACGTGTCCCAGAGCAAGGACAGCGACGTGTACATCACC
GACAAGACCGTGCTGGACATGCGGAGCATGGACTTCAAGAGCAACAGCGCCGTGGCCTG
GTCCAACAAGAGCGATTTCGCCTGCGCCAACGCCTTCAACAACAGCATTATCCCCGAGGA
CACATTCTTCCCAAGCCCCGAGAGCAGCTGCGACGTGAAGCTGGTGGAAAAGAGCTTCGA
GACAGACACCAACCTGAACTTCCAGAACCTGAGCGTGATCGGCTTCCGGATCCTGCTGCT
GAAGGTGGCCGGCTTCAACCTGCTGATGACCCTGAGACTGTGGTCCAGCTGA
SEQ ID NO: 65 Reserved.
SEQ ID NO: 66 α VJ and constant (murine) NT co 
ATGATGAAGTCCCTGCGGGTGCTGCTCGTGATCCTGTGGCTGCAGCTGAGCTGGGTGTG
GTCCCAGCAGAAAGAGGTGGAACAGAACAGCGGCCCTCTGAGCGTGCCAGAAGGCGCTA
TCGCCAGCCTGAACTGCACCTACAGCGACAGAGGCAGCCAGAGCTTCTTCTGGTACAGAC
AGTACAGCGGCAAGAGCCCCGAGCTGATCATGTTCATCTACAGCAACGGCGACAAAGAGG
ACGGCCGGTTCACCGCCCAGCTGAACAAGGCCAGCCAGTACGTGTCCCTGCTGATCAGA
GACAGCCAGCCCAGCGACAGCGCCACCTATCTGTGCGCCGTGAAGGACAACGCCGGCAA
CATGCTGACCTTCGGCGGAGGCACCCGGCTGATGGTCAAGCCCGACATTCAGAACCCGG
AACCGGCTGTATACCAGCTGAAGGACCCCCGATCTCAGGATAGTACTCTGTGCCTGTTCA
CCGACTTTGATAGTCAGATCAATGTGCCTAAAACCATGGAATCCGGAACTTTTATTACCGA
CAAGTGCGTGCTGGATATGAAAGCCATGGACAGTAAGTCAAACGGCGCCATCGCTTGGAG
CAATCAGACATCCTTCACTTGCCAGGATATCTTCAAGGAGACCAACGCAACATACCCATCC
TCTGACGTGCCCTGTGATGCCACCCTGACAGAGAAGTCTTTCGAAACAGACATGAACCTG
AATTTTCAGAATCTGAGCGTGATGGGCCTGAGAATCCTGCTGCTGAAGGTCGCTGGGTTT
AATCTGCTGATGACACTGCGGCTGTGGTCCTCATGA
SEQ ID NO: 67 β VJ and constant AA 
MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASSDGGGVYEQYFGP
GTRLTVTEDLKNVFPPEVAVFEPSEAEISHTQKATLVCLATGFYPDHVELSWWVNGKEVHSGV
STDPQPLKEQPALNDSRYCLSSRLRVSATFWQNPRNHFRCQVQFYGLSENDEWTQDRAKPV
TQIVSAEAWGRADCGFTSESYQQGVLSATILYEILLGKATLYAVLVSALVLMAMVKRKDSRG
SEQ ID NO: 68 β VJ and constant (murine) AA 
MGFRLLCCVAFCLLGAGPVDSGVTQTPKHLITATGQRVTLRCSPRSGDLSVYWYQQSLDQGL
QFLIQYYNGEERAKGNILERFSAQQFPDLHSELNLSSLELGDSALYFCASSDGGGVYEQYFGP
GTRLTVTEDLRNVTPPKVSLFEPSKAEIANKQKATLVCLARGFFPDHVELSWWVNGKEVHSGV
CTDPQAYKESNYSYCLSSRLRVSATFWHNPRNHFRCQVQFHGLSEEDKWPEGSPKPVTQNI
SAEAWGRADCGITSASYHQGVLSATILYEILLGKATLYAVLVSGLVLMAMVKKKNS
SEQ ID NO: 69 β VJ and constant NT 
ATGGGCTTCAGGCTCCTCTGCTGTGTGGCCTTTTGTCTCCTGGGAGCAGGCCCAGTGGAT
TCTGGAGTCACACAAACCCCAAAGCACCTGATCACAGCAACTGGACAGCGAGTGACGCTG
AGATGCTCCCCTAGGTCTGGAGACCTCTCTGTGTACTGGTACCAACAGAGCCTGGACCAG
GGCCTCCAGTTCCTCATTCAGTATTATAATGGAGAAGAGAGAGCAAAAGGAAACATTCTTG
 
AACGATTCTCCGCACAACAGTTCCCTGACTTGCACTCTGAACTAAACCTGAGCTCTCTGGA
GCTGGGGGACTCAGCTTTGTATTTCTGTGCCAGCAGCGACGGAGGGGGCGTCTACGAGC
AGTACTTCGGGCCGGGCACCAGGCTCACGGTCACAGAGGACCTGAAAAACGTGTTCCCA
CCCGAGGTCGCTGTGTTTGAGCCATCAGAAGCAGAGATCTCCCACACCCAAAAGGCCACA
CTGGTATGCCTGGCCACAGGCTTCTACCCCGACCACGTGGAGCTGAGCTGGTGGGTGAA
TGGGAAGGAGGTGCACAGTGGGGTCAGCACAGACCCGCAGCCCCTCAAGGAGCAGCCC
GCCCTCAATGACTCCAGATACTGCCTGAGCAGCCGCCTGAGGGTCTCGGCCACCTTCTGG
CAGAACCCCCGCAACCACTTCCGCTGTCAAGTCCAGTTCTACGGGCTCTCGGAGAATGAC
GAGTGGACCCAGGATAGGGCCAAACCCGTCACCCAGATCGTCAGCGCCGAGGCCTGGG
GTAGAGCAGACTGTGGCTTCACCTCCGAGTCTTACCAGCAAGGGGTCCTGTCTGCCACCA
TCCTCTATGAGATCTTGCTAGGGAAGGCCACCTTGTATGCCGTGCTGGTCAGTGCCCTCG
TGCTGATGGCCATGGTCAAGAGAAAGGATTCCAGAGGCTAG
SEQ ID NO: 70 β VJ and constant NT co 
ATGGGCTTCAGACTGCTGTGCTGCGTGGCCTTCTGTCTGCTGGGAGCCGGCCCTGTGGA
TAGCGGCGTGACACAGACACCCAAGCACCTGATCACCGCCACCGGCCAGCGCGTGACAC
TGAGATGTAGCCCTAGAAGCGGCGACCTGAGCGTGTACTGGTATCAGCAGAGCCTGGAC
CAGGGCCTGCAGTTCCTGATCCAGTACTACAACGGCGAGGAACGGGCCAAGGGCAACAT
CCTGGAACGGTTCAGCGCCCAGCAGTTCCCCGATCTGCACAGCGAGCTGAACCTGAGCA
GCCTGGAACTGGGCGACAGCGCCCTGTACTTCTGTGCCAGCTCTGATGGCGGCGGAGTG
TACGAGCAGTACTTCGGCCCTGGCACCAGACTGACCGTGACCGAGGACCTGAAGAACGT
GTTCCCCCCAGAGGTGGCCGTGTTCGAGCCTTCTGAGGCCGAGATCAGCCACACCCAGA
AAGCCACCCTCGTGTGTCTGGCCACCGGCTTCTACCCCGACCACGTGGAACTGTCTTGGT
GGGTCAACGGCAAAGAGGTGCACAGCGGCGTGTCCACCGATCCCCAGCCTCTGAAAGAA
CAGCCCGCCCTGAACGACAGCCGGTACTGCCTGTCCAGCAGACTGAGAGTGTCCGCCAC
CTTCTGGCAGAACCCCCGGAACCACTTCAGATGCCAGGTGCAGTTCTACGGCCTGAGCGA
GAACGACGAGTGGACCCAGGACAGAGCCAAGCCCGTGACCCAGATCGTGTCTGCCGAAG
CCTGGGGCAGAGCCGATTGCGGCTTTACCAGCGAGAGCTACCAGCAGGGCGTGCTGAGC
GCCACCATCCTGTACGAGATCCTGCTGGGCAAGGCCACCCTGTACGCCGTGCTGGTGTCT
GCCCTGGTGCTGATGGCCATGGTCAAGCGGAAGGACAGCCGGGGCTAA
SEQ ID NO: 71 Reserved.
SEQ ID NO: 72 β VJ and constant (murine) NT co

ATGGGCTTCAGACTGCTGTGCTGCGTGGCCTTCTGTCTGCTGGGAGCCGGCCCTGTGGA
TAGCGGCGTGACACAGACACCCAAGCACCTGATCACCGCCACCGGCCAGCGCGTGACAC
TGAGATGTAGCCCTAGAAGCGGCGACCTGAGCGTGTACTGGTATCAGCAGAGCCTGGAC
CAGGGCCTGCAGTTCCTGATCCAGTACTACAACGGCGAGGAACGGGCCAAGGGCAACAT
CCTGGAACGGTTCAGCGCCCAGCAGTTCCCCGATCTGCACAGCGAGCTGAACCTGAGCA
GCCTGGAACTGGGCGACAGCGCCCTGTACTTCTGTGCCAGCTCTGATGGCGGCGGAGTG
TACGAGCAGTACTTCGGCCCTGGCACCAGACTGACCGTGACCGAAGATCTACGTAACGTG
ACACCACCCAAAGTCTCACTGTTTGAGCCTAGCAAGGCAGAAATTGCCAACAAGCAGAAG
GCCACCCTGGTGTGCCTGGCAAGAGGGTTCTTTCCAGATCACGTGGAGCTGTCCTGGTG
GGTCAACGGCAAAGAAGTGCATTCTGGGGTCTGCACCGACCCCCAGGCTTACAAGGAGA
GTAATTACTCATATTGTCTGTCAAGCCGGCTGAGAGTGTCCGCCACATTCTGGCACAACCC
TAGGAATCATTTCCGCTGCCAGGTCCAGTTTCACGGCCTGAGTGAGGAAGATAAATGGCC
AGAGGGGTCACCTAAGCCAGTGACACAGAACATCAGCGCAGAAGCCTGGGGACGAGCAG
ACTGTGGCATTACTAGCGCCTCCTATCATCAGGGCGTGCTGAGCGCCACTATCCTGTACG
AGATTCTGCTGGGAAAGGCCACCCTGTATGCTGTGCTGGTCTCCGGCCTGGTGCTGATGG
CCATGGTCAAGAAAAAGAACTCT

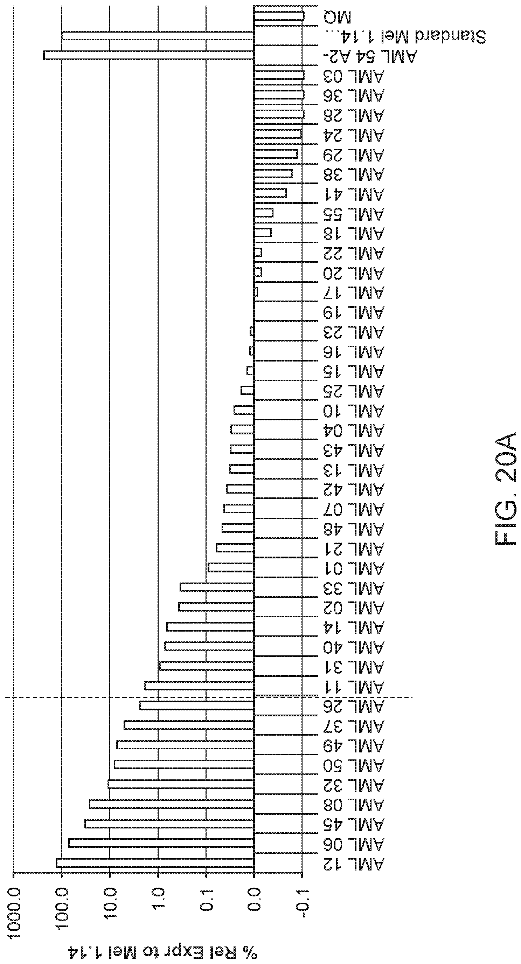

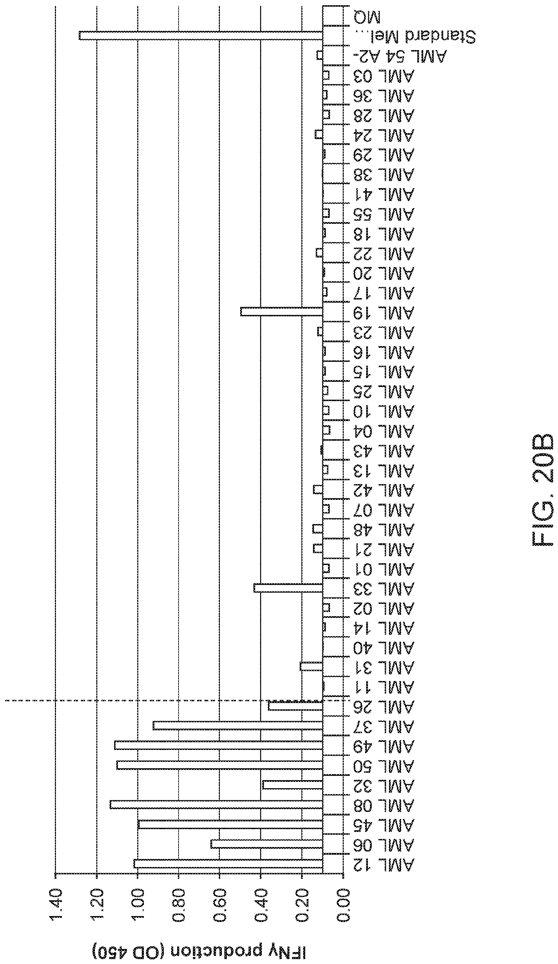

In Figure 20 we demonstrate the reactivity of PRAME specific T cells against different primary AML samples derived from patients suffering from AML at the time of diagnosis. The AML samples that were analyzed were HLA-A*0201 (41 samples), one AML sample was HLA-A2 negative (AML 54). In Figure 20 both the reactivity of the T cell clone (B) and the expression of PRAME measured by qPCR (A) is shown.

Primary AML samples were analyzed for recognition by PRAME specific T cells. Of the 42 AML samples , 41 were HLA-A*02:01 positive, 1 HLA-A*02:01 negative (AML 54). Of the 41 HLA-A*02:01+ AML samples, 9 AML samples were efficiently recognized by PRAME specific T cells. AML samples with PRAME expression >2.5% relative to the level of PRAME expression measured in melanoma cell line Mel1.14 (set at 100%) were efficiently recognized by the PRAME specific T cells.

The results demonstrate that when PRAME expression in the AML samples is >2.5% relative to the level of PRAME expression measured in melanoma cell line Mel1.14.(set at 100%) the PRAME specific T cells are able to efficiently react against the AML samples. These results indicate that approximately 20-25% of the HLA-A*0201 positive AML patients can be treated with PRAME-TCR gene transfer. Furthermore, the results demonstrate that measuring the PRAME expression of the AML sample by qPCR may be used to determine whether it is of potential benefit to the patient to treat with PRAME-TCR modified T cells.

### Example 2: Addition of a suicide gene-- Selective Apoptosis of the Modified Cells

The modified cells that express the PRAME-targeted TCR may be provided with a mechanism to remove some, or all of the cells if the patient experiences negative effects, and there is a need to reduce, or stop treatment. These cells may be used for all TCR-expressing modified T cells, or the cells may be provided with this ability where the TCR is directed against antigens that have previously caused, or are at risk to cause, lethal on-target, off-organ toxicity, where there is a need for an option to rapidly terminate therapy.

An example of a chimeric polypeptide that may be expressed in the modified cells is provided in the present examples. In these examples, a single polypeptide is encoded by the nucleic acid vector. The inducible caspase-9 polypeptide is separated from the CAR polypeptide during translation, due to skipping of a peptide bond. (Donnelly, ML 2001, J. Gen. Virol. 82:1013-25).

### Vector construction and confirmation of expression

A safety switch that can be stably and efficiently expressed in human T cells is presented herein. Expression vectors suitable for use as a therapeutic agent were constructed that included a modified human caspase-9 activity fused to a human FK506 binding protein (FKBP), such as, for example, FKBP12v36. The caspase-9/FK506 hybrid activity can be dimerized using a small molecule pharmaceutical. Full length, truncated, and modified versions of the caspase-9 activity were fused to the ligand binding domain, or multimerization region, and inserted into the retroviral vector MSCV.IRES.GRP, which also allows expression of the fluorescent marker, GFP.

The full-length inducible caspase-9 molecule (F'-F-C-Casp9) includes 2, 3, or more FK506 binding proteins (FKBPs-for example, FKBP12v36 variants) linked with a Gly-Ser-Gly-Gly-Gly-Ser linker to the small and large subunit of the caspase molecule. Full-length inducible caspase-9 (F'F-C-Casp9.I.GFP) has a full-length caspase-9, also includes a caspase recruitment domain (CARD; GenBank NM001 229) linked to 2 12-kDa human FK506 binding proteins (FKBP12; GenBank AH002 818) that contain an F36V mutation. The amino acid sequence of one or more of the FKBPs (F') was codon-wobbled (e.g., the 3rd nucleotide of each amino acid codon was altered by a silent mutation that maintained the originally encoded amino acid) to prevent homologous recombination when expressed in a retrovirus. F'F-C-Casp9C3S includes a cysteine to serine mutation at position 287 that disrupts its activation site. In constructs F'F-Casp9, F-C-Casp9, and F'-Casp9, either the caspase activation domain (CARD), one FKBP, or both, were deleted, respectively. All constructs were cloned into MSCV.IRES.GFP as EcoRI-Xhol fragments.

Coexpression of the inducible caspase-9 constructs of the expected size with the marker gene GFP in transfected 293 T cells was demonstrated by Western blot using a caspase-9 antibody specific for amino acid residues 299-318, present both in the full-length and truncated caspase molecules as well as a GFP-specific antibody.

An initial screen indicated that the full length iCasp9 could not be maintained stably at high levels in T cells, possibly due to transduced cells being eliminated by the basal activity of the transgene. The CARD domain is involved in physiologic dimerization of caspase-9 molecules, by a cytochrome C and adenosine triphosphate (ATP)-driven interaction with apoptotic protease-activating factor 1 (Apaf-1). Because of the use of a CID to induce dimerization and activation of the suicide switch, the function of the CARD domain is superfluous in this context and removal of the CARD domain was investigated as a method of reducing basal activity.

### Using the iCasp9 Suicide Gene to Improve the Safety of Allodepleted T cells after Haploidentical Stem Cell Transplantation

Presented in this example are expression constructs and methods of using the expression constructs to improve the safety of allodepleted T cells after haploidentical stem cell transplantation. Similar methods may be used to express the caspase-9 expression constructs in non allodepleted cells. A retroviral vector encoding iCasp9 and a selectable marker (truncated CD19) was generated as a safety switch for donor T cells. Even after allodepletion (using anti-CD25 immunotoxin), donor T cells could be efficiently transduced, expanded, and subsequently enriched by CD19 immunomagnetic selection to >90% purity. The engineered cells retained anti-viral specificity and functionality, and contained a subset with regulatory phenotype and function. Activating iCasp9 with a small-molecule dimerizer rapidly produced >90% apoptosis. Although transgene expression was downregulated in quiescent T cells, iCasp9 remained an efficient suicide gene, as expression was rapidly upregulated in activated (alloreactive) T cells.

### Materials and Methods

### Generation of allodepleted T cells

Allodepleted cells were generated from healthy volunteers as previously presented. Briefly, peripheral blood mononuclear cells (PBMCs) from healthy donors were co-cultured with irradiated recipient Epstein Barr virus (EBV)-transformed lymphoblastoid cell lines (LCL) at responder-to-stimulator ratio of 40:1 in serum-free medium (AIM V; Invitrogen, Carlsbad, CA). After 72 hours, activated T cells that expressed CD25 were depleted from the co-culture by overnight incubation in RFT5-SMPT-dgA immunotoxin. Allodepletion was considered adequate if the residual CD3⁺CD25⁺ population was <1% and residual proliferation by 3H-thymidine incorporation was <10%.

### Plasmid and retrovirus

SFG.iCasp9.2A.CD19 consists of inducible caspase-9 (iCasp9) linked, via a cleavable 2A-like sequence, to truncated human CD19. iCasp9 consists of a human FK506-binding protein (FKBP12; GenBank AH002 818) with an F36V mutation, connected via a Ser-Gly-Gly-Gly-Ser-Gly linker to human caspase-9 (CASP9; GenBank NM 001229). The F36V mutation increases the binding affinity of FKBP12 to the synthetic homodimerizer, AP20187 or AP1903. The caspase recruitment domain (CARD) has been deleted from the human caspase-9 sequence because its physiological function has been replaced by FKBP12, and its removal increases transgene expression and function. The 2A-like sequence encodes an 20 amino acid peptide from Thosea asigna insect virus, which mediates >99% cleavage between a glycine and terminal proline residue, resulting in 19 extra amino acids in the C terminus of iCasp9, and one extra proline residue in the N terminus of CD19. CD19 consists of full-length CD19 (GenBank NM 001770) truncated at amino acid 333 (TDPTRRF), which shortens the intracytoplasmic domain from 242 to 19 amino acids, and removes all conserved tyrosine residues that are potential sites for phosphorylation.

A stable PG13 clone producing Gibbon ape leukemia virus (Gal-V) pseudotyped retrovirus was made by transiently transfecting Phoenix Eco cell line (ATCC product #SD3444; ATCC, Manassas, VA) with SFG.iCasp9.2A.CD19. This produced Eco-pseudotyped retrovirus. The PG13 packaging cell line (ATCC) was transduced three times with Eco-pseudotyped or retrovirus to generate a producer line that contained multiple SFG.iCasp9.2A.CD19 proviral integrants per cell. Single cell cloning was performed, and the PG13 clone that produced the highest titer was expanded and used for vector production.

### Retroviral transduction

Culture medium for T cell activation and expansion consisted of 45% RPMI 1640 (Hyclone, Logan, UT), 45% Clicks (Irvine Scientific, Santa Ana, CA) and 10% fetal bovine serum (FBS; Hyclone). Allodepleted cells were activated by immobilized anti-CD3 (OKT3; Ortho Biotech, Bridgewater, NJ) for 48 hours before transduction with retroviral vector Selective allodepletion was performed by co-culturing donor PBMC with recipient EBV-LCL to activate alloreactive cells: activated cells expressed CD25 and were subsequently eliminated by anti-CD25 immunotoxin. The allodepleted cells were activated by OKT3 and transduced with the retroviral vector 48 hours later. Immunomagnetic selection was performed on day 4 of transduction; the positive fraction was expanded for a further 4 days and cryopreserved.

In small-scale experiments, non-tissue culture-treated 24-well plates (Becton Dickinson, San Jose, CA) were coated with OKT3 1 g/ml for 2 to 4 hours at 37°C. Allodepleted cells were added at 1×10⁶ cells per well. At 24 hours, 100U/ml of recombinant human interleukin-2 (IL-2) (Proleukin; Chiron, Emeryville, CA) was added. Retroviral transduction was performed 48 hours after activation. Non-tissue culture-treated 24-well plates were coated with 3.5ug/cm² recombinant fibronectin fragment (CH-296; Retronectin; Takara Mirus Bio, Madison, WI) and the wells loaded twice with retroviral vector-containing supernatant at 0.5ml per well for 30 minutes at 37°C, following which OKT3 -activated cells were plated at 5 ×10⁵ cells per well in fresh retroviral vector-containing supernatant and T cell culture medium at a ratio of 3:1, supplemented with 100U/ml IL-2. Cells were harvested after 2 to 3 days and expanded in the presence of 50U/ml IL-2.

### Scaling-up production of gene-modified allodepleted cells

Scale-up of the transduction process for clinical application used non-tissue culture-treated T75 flasks (Nunc, Rochester, NY), which were coated with 10ml of OKT3 1µg/ml or 10ml of fibronectin 7µg/ml at 4°C overnight. Fluorinated ethylene propylene bags corona-treated for increased cell adherence (2PF-0072AC, American Fluoroseal Corporation, Gaithersburg, MD) were also used. Allodepleted cells were seeded in OKT3 -coated flasks at 1×10⁶ cells/ml. 100U/ml IL-2 was added the next day. For retroviral transduction, retronectin-coated flasks or bags were loaded once with 10ml of retrovirus-containing supernatant for 2 to 3 hours. OKT3-activated T cells were seeded at 1×106 cells/ml in fresh retroviral vector-containing medium and T cell culture medium at a ratio of 3:1, supplemented with 100 U/ml IL-2. Cells were harvested the following morning and expanded in tissue-culture treated T75 or T175 flasks in culture medium supplemented with between about 50 to 100U/ml IL-2 at a seeding density of between about 5×10⁵ cells/ ml to 8×10⁵ cells/ ml.

### CD19 immunomagnetic selection

Immunomagnetic selection for CD19 was performed 4 days after transduction. Cells were labeled with paramagnetic microbeads conjugated to monoclonal mouse anti-human CD19 antibodies (Miltenyi Biotech, Auburn, CA) and selected on MS or LS columns in small scale experiments and on a CliniMacs Plus automated selection device in large scale experiments. CD19-selected cells were expanded for a further 4 days and cryopreserved on day 8 post transduction. These cells were referred to as "gene-modified allodepleted cells".

### Immunophenotyping and pentamer analysis

Flow cytometric analysis (FACSCalibur and CellQuest software; Becton Dickinson) was performed using the following antibodies: CD3, CD4, CD8, CD19, CD25, CD27, CD28, CD45RA, CD45RO, CD56 and CD62L. CD19-PE (Clone 4G7; Becton Dickinson) was found to give optimum staining and was used in all subsequent analysis. A non-transduced control was used to set the negative gate for CD19. An HLA-pentamer, HLA-B8-RAKFKQLL (Proimmune, Springfield, VA) was used to detect T cells recognizing an epitope from EBV lytic antigen (BZLF1). HLA-A2-NLVPMVATV pentamer was used to detect T cells recognizing an epitope from CMV-pp65 antigen.

### Induction of apoptosis with chemical inducer of dimerization, AP20187

Suicide gene functionality was assessed by adding a small molecule synthetic homodimerizer, AP20187 (Ariad Pharmaceuticals; Cambridge, MA), at 10nM final concentration the day following CD19 immunomagnetic selection. AP1903 may also be used. Cells were stained with annexin V and 7-amino-actinomycin (7-AAD) (BD Pharmingen) at 24 hours and analyzed by flow cytometry. Cells negative for both annexin V and 7-AAD were considered viable, cells that were annexin V positive were apoptotic, and cells that were both annexin V and 7-AAD positive were necrotic. The percentage killing induced by dimerization was corrected for baseline viability as follows: Percentage killing = 100% - (%Viability in AP20187-treated cells ÷ %Viability in non-treated cells).

### Assessment of transgene expression following extended culture and reactivation

Cells were maintained in T cell medium containing 50U/ml IL-2 until 22 days after transduction. A portion of cells was reactivated on 24-well plates coated with 1 g/ml OKT3 and 1 microgram/ml anti-CD28 (Clone CD28.2, BD Pharmingen, San Jose, CA) for 48 to 72 hours. CD19 expression and suicide gene function in both reactivated and non-reactivated cells were measured on day 24 or 25 post transduction.

In some experiments, cells also were cultured for 3 weeks post transduction and stimulated with 30Gγ̃-irradiated allogeneic PBMC at a responder: stimulator ratio of 1:1. After 4 days of co-culture, a portion of cells was treated with 10nM AP20187. Killing was measured by annexin V/7-AAD staining at 24 hours, and the effect of dimerizer on bystander virus-specific T cells was assessed by pentamer analysis on AP20187-treated and untreated cells.

Optimal culture conditions for maintaining the immunological competence of suicide gene-modified T cells must be determined and defined for each combination of safety switch, selectable marker and cell type, since phenotype, repertoire and functionality can all be affected by the stimulation used for polyclonal T cell activation, the method for selection of transduced cells, and duration of culture.

### Phase I Clinical Trial of Allodepleted T cells Transduced with Inducible caspase-9 Suicide Gene after Haploidentical Stem Cell Transplantation

This example presents results of a phase 1 clinical trial using an alternative suicide gene strategy. Briefly, donor peripheral blood mononuclear cells were co-cultured with recipient irradiated EBV-transformed lymphoblastoid cells (40:1) for 72 hrs, allodepleted with a CD25 immunotoxin and then transduced with a retroviral supernatant carrying the iCasp9 suicide gene and a selection marker (ΔCD19); ΔCD19 allowed enrichment to >90% purity via immunomagnetic selection.

An example of a protocol for generation of a cell therapy product is provided herein.

### Source Material

Up to 240 ml (in 2 collections) of peripheral blood was obtained from the transplant donor according to established protocols. In some cases, dependent on the size of donor and recipient, a leukopheresis was performed to isolate sufficient T cells. 10-30cc of blood also was drawn from the recipient and was used to generate the Epstein Barr virus (EBV)-transformed lymphoblastoid cell line used as stimulator cells. In some cases, dependent on the medical history and/or indication of a low B cell count, the LCLs were generated using appropriate 1st degree relative (e.g., parent, sibling, or offspring) peripheral blood mononuclear cells.

### Generation of Allodepleted Cells

Allodepleted cells were generated from the transplant donors as presented herein. Peripheral blood mononuclear cells (PBMCs) from healthy donors were co-cultured with irradiated recipient Epstein Barr virus (EBV)-transformed lymphoblastoid cell lines (LCL) at responder-to-stimulator ratio of 40:1 in serum-free medium (AIM V; Invitrogen, Carlsbad, CA). After 72 hours, activated T cells that express CD25 were depleted from the co-culture by overnight incubation in RFT5-SMPT-dgA immunotoxin. Allodepletion is considered adequate if the residual CD3⁺CD25⁺ population was <1% and residual proliferation by 3H-thymidine incorporation was <10%.

### Retroviral Production

A retroviral producer line clone was generated for the iCasp9-ΔCD19 construct. A master cell-bank of the producer also was generated. Testing of the master-cell bank was performed to exclude generation of replication competent retrovirus and infection by Mycoplasma, HIV, HBV, HCV and the like. The producer line was grown to confluency, supernatant harvested, filtered, aliquotted and rapidly frozen and stored at -80°C. Additional testing was performed on all batches of retroviral supernatant to exclude Replication Competent Retrovirus (RCR) and issued with a certificate of analysis, as per protocol.

### Transduction of Allodepleted Cells

Allodepleted T-lymphocytes were transduced using Fibronectin. Plates or bags were coated with recombinant Fibronectin fragment CH-296 (RetronectinTM, Takara Shuzo, Otsu, Japan). Virus was attached to retronectin by incubating producer supernatant in coated plates or bags. Cells were then transferred to virus coated plates or bags. After transduction allodepleted T cells were expanded, feeding them with IL-2 twice a week to reach the sufficient number of cells as per protocol.

### CD19 Immunomagnetic Selection

Immunomagnetic selection for CD19 was performed 4 days after transduction. Cells are labeled with paramagnetic microbeads conjugated to monoclonal mouse anti-human CD19 antibodies (Miltenyi Biotech, Auburn, CA) and selected on a CliniMacs Plus automated selection device. Depending upon the number of cells required for clinical infusion cells were either cryopreserved after the CliniMacs selection or further expanded with IL-2 and cryopreserved on day 6 or day 8 post transduction.

### Freezing

Aliquots of cells were removed for testing of transduction efficiency, identity, phenotype and microbiological culture as required for final release testing by the FDA. The cells were cryopreserved prior to administration according to protocol.

### Study Drugs

### RFT5-SMPT-dgA

RFT5-SMPT-dgA is a murine IgG1 anti-CD25 (IL-2 receptor α chain) conjugated via a heterobifunctional crosslinker [N-succinimidyloxycarbonyl-α-methyl-d- (2-pyridylthio) toluene] (SMPT) to chemically deglycosylated ricin A chain (dgA). RFT5-SMPT-dgA is formulated as a sterile solution at 0.5 mg/ml.

### Synthetic homodimerizer, AP1903

Mechanism of Action: AP1903-inducible cell death is achieved by expressing a chimeric protein comprising the intracellular portion of the human (caspase-9 protein) receptor, which signals apoptotic cell death, fused to a drug-binding domain derived from human FK506-binding protein (FKBP). This chimeric protein remains quiescent inside cells until administration of AP1903, which cross-links the FKBP domains, initiating caspase signaling and apoptosis.

Toxicology: AP1903 has been evaluated as an Investigational New Drug (IND) by the FDA and has successfully completed a phase I clinical safety study. No significant adverse effects were noted when API 903 was administered over a 0.01 mg/kg to 1.0mg/kg dose range.

Pharmacology/Pharmacokinetics: Patients received 0.4 mg/kg of AP1903 as a 2 h infusion - based on published Pk data which show plasma concentrations of 10 ng/mL - 1275 ng/mL over the 0.01 mg/kg to 1.0 mg/kg dose range with plasma levels falling to 18% and 7% of maximum at 0.5 and 2 hrs post dose.

Side Effect Profile in Humans: No serious adverse events occurred during the Phase 1 study in volunteers. The incidence of adverse events was very low following each treatment, with all adverse events being mild in severity. Only one adverse event was considered possibly related to API903. This was an episode of vasodilatation, presented as "facial flushing" for 1 volunteer at the 1.0 mg/kg API903 dosage. This event occurred at 3 minutes after the start of infusion and resolved after 32 minutes duration. All other adverse events reported during the study were considered by the investigator to be unrelated or to have improbable relationship to the study drug. These events included chest pain, flu syndrome, halitosis, headache, injection site pain, vasodilatation, increased cough, rhinitis, rash, gum hemorrhage, and ecchymosis.

Patients developing grade 1 GvHD were treated with 0.4mg/kg AP1903 as a 2-hour infusion. Protocols for administration of AP1903 to patients grade 1 GvHD were established as follows. Patients developing GvHD after infusion of allodepleted T cells are biopsied to confirm the diagnosis and receive 0.4 mg/kg of AP1903 as a 2 h infusion. Patients with Grade 1 GvHD received no other therapy initially, however if they showed progression of GvHD conventional GvHD therapy was administered as per institutional guidelines. Patients developing grades 2-4 GvHD were administered standard systemic immunosuppressive therapy per institutional guidelines, in addition to the AP1903 dimerizer drug.

Instructions for preparation and infusion: AP1903 for injection is obtained as a concentrated solution of 2.33 ml in a 3 ml vial, at a concentration of 5 mg/ml, (i.e., 10.66 mg per vial). Prior to administration, the calculated dose was diluted to 100 mL in 0.9% normal saline for infusion. AP1903 for injection (0.4 mg/kg) in a volume of 100 ml was administered via IV infusion over 2 hours, using a non-DEHP, non-ethylene oxide sterilized infusion set and infusion pump.

The iCasp9 suicide gene expression construct (e.g., SFG.iCasp9.2A.ΔCD19) consists of inducible caspase-9 (iCasp9) linked, via a cleavable 2A-like sequence, to truncated human CD19 (ΔCD19). iCasp9 includes a human FK506-binding protein (FKBP12; GenBank AH002 818) with an F36V mutation, connected via a Ser-Gly-Gly-Gly-Ser-Gly linker to human caspase-9 (CASP9; GenBank NM 001229). The F36V mutation may increase the binding affinity of FKBP12 to the synthetic homodimerizer, AP20187 or API903. The caspase recruitment domain (CARD) has been deleted from the human caspase-9 sequence and its physiological function has been replaced by FKBP12. The replacement of CARD with FKBP12 increases transgene expression and function. The 2A-like sequence encodes an 18 amino acid peptide from Thosea Asigna insect virus, which mediates >99% cleavage between a glycine and terminal proline residue, resulting in 17 extra amino acids in the C terminus of iCasp9, and one extra proline residue in the N terminus of CD19. ΔCD19 consists of full length CD19 (GenBank NM 001770) truncated at amino acid 333 (TDPTRRF), which shortens the intracytoplasmic domain from 242 to 19 amino acids, and removes all conserved tyrosine residues that are potential sites for phosphorylation.

### In vivo studies

Three patients received iCasp9⁺ T cells after haplo-CD34⁺stem cell transplantation (SCT), at dose levels between about 1×10⁶ to about 3×10⁶ cells/kg.

Infused T cells were detected in vivo by flow cytometry (CD3⁺ ΔCD19⁺) or qPCR as early as day 7 after infusion, with a maximum fold expansion of 170±5 (day 29±9 after infusion). Two patients developed grade I/II aGvHD and AP1903 administration caused >90% ablation of CD3⁺ ΔCD19⁺ cells, within 30 minutes of infusion, with a further log reduction within 24 hours, and resolution of skin and liver aGvHD within 24hrs, showing that iCasp9 transgene was functional in vivo.

Ex vivo experiments confirmed this data. Furthermore, the residual allodepleted T cells were able to expand and were reactive to viruses (CMV) and fungi (Aspergillus fumigatus) (IFN-γ production). These in vivo studies found that a single dose of dimerizer drug can reduce or eliminate the subpopulation of T cells causing GvHD, but can spare virus specific CTLs, which can then re-expand.

### Immune reconstitution

Depending on availability of patient cells and reagents, immune reconstitution studies (Immunophenotyping, T and B cell function) may be obtained at serial intervals after transplant. Several parameters measuring immune reconstitution resulting from icaspase transduced allodepleted T cells will be analyzed. The analysis includes repeated measurements of total lymphocyte counts, T and CD19 B cell numbers, and FACS analysis of T cell subsets (CD3, CD4, CD8, CD16, CD19, CD27, CD28, CD44, CD62L, CCR7, CD56, CD45RA, CD45RO, alpha/beta and gamma/delta T cell receptors). Depending on the availability of a patients T cells T regulatory cell markers such as CD41CD251FoxP3 also are analyzed. Approximately 10-60 ml of patient blood is taken, when possible, 4 hours after infusion, weekly for 1 month, monthly × 9 months, and then at 1 and 2 years. The amount of blood taken is dependent on the size of the recipient and does not exceed 1-2 cc/kg in total (allowing for blood taken for clinical care and study evaluation) at any one blood draw.

### Administration of non-allodepleted transfected or transformed T cells

The protocols provided herein for generation and administration of T cells that express a PRAME-specific TCR and an inducible caspase polypeptide may also be modified to provide for in vivo T cell allodepletion if necessary after the patient exhibits toxic symptoms. To extend the approach to a larger group of subjects who might benefit from immune reconstitution without acute GvHD, the protocol may be simplified, by providing for an in vivo method of T cell depletion. In the pre-treatment allodepletion method, as discussed herein, EBV-transformed lymphoblastoid cell lines are first prepared from the recipient, which then act as alloantigen presenting cells. This procedure can take up to 8 weeks, and may fail in extensively pretreated subjects with malignancy, particularly if they have received rituximab as a component of their initial therapy. Subsequently, the donor T cells are co-cultured with recipient EBV-LCL, and the alloreactive T cells (which express the activation antigen CD25) are then treated with CD25-ricin conjugated monoclonal antibody. This procedure may take many additional days of laboratory work for each subject.

The process may be simplified by using an in vivo method of allodepletion, building on the observed rapid in vivo depletion of alloreactive T cells by dimerizer drug and the sparing of unstimulated but virus/fungus reactive T cells.

If there is development of Grade I or greater acute GvHD or other toxic event, a single dose of dimerizer drug is administered, for example at a dose of 0.4 mg/kg of AP1903 as a 2 hour intravenous infusion. Up to 3 additional doses of dimerizer drug may be administered at 48 hour intervals if acute GvHD persists. In subjects with Grade II or greater acute GvHD, these additional doses of dimerizer drug may be combined with steroids. For patients with persistent GVHD who cannot receive additional doses of the dimerizer due to a Grade III or IV reaction to the dimerizer, the patient may be treated with steroids alone, after either 0 or 1 doses of the dimerizer.

### Generation of Therapeutic T cells

Up to 240 ml (in 2 collections) of peripheral blood is obtained from the transplant donor according to the procurement consent. If necessary, a leukapheresis is used to obtain sufficient T cells; (either prior to stem cell mobilization or seven days after the last dose of G-CSF). An extra 10-30 mls of blood may also be collected to test for infectious diseases such as hepatitis and HIV.

Peripheral blood mononuclear cells are be activated using anti-human CD3 antibody (e.g. from Orthotech or Miltenyi) on day 0 and expanded in the presence of recombinant human interleukin-2 (rhIL-2) on day 2. CD3 antibody-activated T cells are transduced by the icaspase-9 retroviral vector on flasks or plates coated with recombinant Fibronectin fragment CH-296 (RetronectinTM, Takara Shuzo, Otsu, Japan). Virus is attached to retronectin by incubating producer supernatant in retronectin coated plates or flasks. Cells are then transferred to virus coated tissue culture devices. After transduction T cells are expanded by feeding them with rhlL-2 twice a week to reach the sufficient number of cells as per protocol.

To ensure that the majority of infused T cells carry the suicide gene, a selectable marker, truncated human CD19 (ΔCD19) and a commercial selection device, may be used to select the transduced cells to >90% purity. Immunomagnetic selection for CD19 may be performed 4 days after transduction. Cells are labeled with paramagnetic microbeads conjugated to monoclonal mouse anti-human CD19 antibodies (Miltenyi Biotech, Auburn, CA) and selected on a CliniMacs Plus automated selection device. Depending upon the number of cells required for clinical infusion cells might either be cryopreserved after the CliniMacs selection or further expanded with IL-2 and cryopreserved as soon as sufficient cells have expanded (up to day 14 from product initiation).

Aliquots of cells may be removed for testing of transduction efficiency, identity, phenotype, autonomous growth and microbiological examination as required for final release testing by the FDA. The cells are be cryopreserved prior to administration.

### Alternative generation of therapeutic T cells

Prior to the non-mobilized T cell leukapheresis, the subject's blood count and differential is collected and recorded. Infectious disease monitoring per the established regulatory guidelines is performed. Subject must meet institutional criteria for CBC and platelets prior to initiation of leukapheresis. The leukocyte fraction is collected using standardized continuous flow centrifugation. The subject is monitored during apheresis. A standard apheresis procedure of up to approximately 3-4 blood volumes is processed per institutional standard procedures, including precautions for leukemic patients. The volume processed and the duration of leukapheresis is documented and recorded. If less than 5 × 10⁹ mononuclear cells are collected, the Medical Monitor is consulted.

### Administration of T cells

The transduced T cells are administered to patients from, for example, between 30 and 120 days following stem cell transplantation. The cryopreserved T cells are thawed and infused through a catheter line with normal saline. For children, premedications are dosed by weight. Doses of cells may range from, for example, from about 1 × 10⁴ cells/Kg to 1 × 10⁸ cells/Kg, for example from about 1 × 10⁵ cells/Kg to 1 × 10⁷ cells/Kg, from about 1 × 10⁶ cells/Kg to 5 × 10⁶ cells/Kg, from about 1 × 10⁴ cells/Kg to 5 × 10⁶ cells/Kg, for example, about 1 × 10⁴, about 1 × 10⁵, about 2 × 10⁵, about 3 × 10⁵, about 5 × 10⁵, 6 × 10⁵, about 7 × 10⁵, about 8 × 10⁵, about 9 × 10⁵, about 1 × 10⁶, about 2 × 10⁶, about 3 × 10⁶, about 4 × 10⁶, or about 5 × 10⁶ cells/Kg.

### Treatment of GvHD

Patients who develop grade ≥1 acute GVHD are treated with 0.4mg/kg AP1903 as a 2-hour infusion. AP1903 for injection may be provided, for example, as a concentrated solution of 2.33 ml in a 3 ml vial, at a concentration of 5 mg/ml, (i.e 10.66 mg per vial). Prior to administration, the calculated dose will be diluted to 100 mL in 0.9% normal saline for infusion. AP1903 for Injection (0.4 mg/kg) in a volume of 100 ml may be administered via IV infusion over 2 hours, using a non-DEHP, non-ethylene oxide sterilized infusion set and an infusion pump.

### Sample treatment schedule

| **Time** | **Donor** | **Recipient** |
|---|---|---|
| Pre-transplant | Obtain up to 240 ml of blood or unstimulated leukapheresis from bone marrow transplant donor. Prepare T cells and donor LCLs for later immune reconstitution studies. | |
| Day 0 | Anti-CD3 activation of PBMC | |
| Day 2 | IL-2 feed | |
| Day 3 | Transduction | |
| Day 4 | Expansion | |
| Day 6 | CD19 selection. Cryopreservation (*if required dose is met) | |
| Day 8 | Assess transduction efficiency and iCaspase9 transgene functionality by phenotype. Cryopreservation (*if not yet performed) | |
| Day 10 or Day 12 to Day 14 | Cryopreservation (if not yet performed) | |
| From 30 to 120 days post transplant | | Thaw and infuse T cells 30 to 120 days post stem cell infusion. |

Methods for using chimeric caspase-9 polypeptides to induce apoptosis are discussed in PCT Publication Number WO 2011/146862 by Malcolm K. Brenner et al., titled Methods for Inducing Selective Apoptosis, filed May 20, 2011, and in United States Patent Publication Number US 2011/0286980 by Malcolm K. Brenner et al., titled Methods for Inducing Selective Apoptosis, filed May 20, 2011.

### Literature References Cited or Providing Additional Support to the Present Example

1. Seifert, R. and K. Wenzel-Seifert, Constitutive activity of G-protein-coupled receptors: cause of disease and common property of wild-type receptors. Naunyn Schmiedebergs Arch Pharmacol, 2002. 366(5): p. 381-416.
2. Roose, J.P., et al., T cell receptor-independent basal signaling via Erk and Abl kinases suppresses RAG gene expression. PLoS Biol, 2003. 1(2): p. E53.
3. Tze, L.E., et al., Basal immunoglobulin signaling actively maintains developmental stage in immature B cells. PLoS Biol, 2005. 3(3): p. e82.
4. Schram, B.R., et al., B cell receptor basal signaling regulates antigen-induced Ig light chain rearrangements. J Immunol, 2008. 180(7): p. 4728-41.
5. Randall, K.L., et al., Dock8 mutations cripple B cell immunological synapses, germinal centers and long-lived antibody production. Nat Immunol, 2009. 10(12): p. 1283-91.
6. Kouskoff, V., et al., B cell receptor expression level determines the fate of developing B lymphocytes: receptor editing versus selection. Proc Natl Acad Sci USA, 2000. 97(13): p. 7435-9.
7. Hong, T., et al., A simple theoretical framework for understanding heterogeneous differentiation of CD4+ T cells. BMC Syst Biol, 2012. 6: p. 66.
8. Rudd, M.L., A. Tua-Smith, and D.B. Straus, Lck SH3 domain function is required for T cell receptor signals regulating thymocyte development. Mol Cell Biol, 2006. 26(21): p. 7892-900.
9. Sorkin, A. and M. von Zastrow, Endocytosis and signalling: intertwining molecular networks. Nat Rev Mol Cell Biol, 2009. 10(9): p. 609-22.
10. Luning Prak, E.T., M. Monestier, and R.A. Eisenberg, B cell receptor editing in tolerance and autoimmunity. Ann NY Acad Sci, 2011. 1217: p. 96-121.
11. Boss, W.F., et al., Basal signaling regulates plant growth and development. Plant Physiol, 2010. 154(2): p. 439-43.
12. Tao, Y.X., Constitutive activation of G protein-coupled receptors and diseases: insights into mechanisms of activation and therapeutics. Pharmacol Ther, 2008. 120(2): p. 129-48.
13. Spiegel, A.M., Defects in G protein-coupled signal transduction in human disease. Annu Rev Physiol, 1996. 58: p. 143-70.
14. Shiozaki, E.N., et al., Mechanism of XIAP-mediated inhibition of caspase-9. Mol Cell, 2003. 11(2): p. 519-27.
15. Renatus, M., et al., Dimer formation drives the activation of the cell death protease caspase-9. Proc Natl Acad Sci U S A, 2001. 98(25): p. 14250-5.
16. Shi, Y., Mechanisms of Caspase activation and inhibition during apoptosis. Mol Cell, 2002. 9(3): p. 459-70.
17. Shiozaki, E.N., J. Chai, and Y. Shi, Oligomerization and activation of caspase-9, induced by Apaf-1 CARD. Proc Natl Acad Sci U S A, 2002. 99(7): p. 4197-202.
18. Straathof, K.C., et al., An inducible caspase-9 safety switch for T cell therapy. Blood, 2005. 105(11): p. 4247-54.
19. MacCorkle, R.A., K.W. Freeman, and D.M. Spencer, Synthetic activation of Caspases: artificial death switches. Proc Natl Acad Sci U S A, 1998. 95(7): p. 3655-60.
20. Di Stasi, A., et al., Inducible apoptosis as a safety switch for adoptive cell therapy. N Engl J Med, 2011. 365(18): p. 1673-83.
21. Chang, W.C., et al., Modifying ligand-induced and constitutive signaling of the human 5-HT4 receptor. PLoS One, 2007. 2(12): p. e1317.
22. Bloom, J.D. and F.H. Arnold, In the light of directed evolution: pathways of adaptive protein evolution. Proc Natl Acad Sci U S A, 2009. 106 Suppl 1: p. 9995-10000.
23. Boatright, K.M. and G.S. Salvesen, Mechanisms of Caspase activation. Curr Opin Cell Biol, 2003. 15(6): p. 725-31.
24. Boatright, K.M., et al., A unified model for apical Caspase activation. Mol Cell, 2003. 11(2): p. 529-41.
25. Chao, Y., et al., Engineering a dimeric caspase-9: a re-evaluation of the induced proximity model for Caspase activation. PLoS Biol, 2005. 3(6): p. e183.
26. Stennicke, H.R., et al., caspase-9 can be activated without proteolytic processing. J Biol Chem, 1999. 274(13): p. 8359-62.
27. Brady, S.C., L.A. Allan, and P.R. Clarke, Regulation of caspase-9 through phosphorylation by protein kinase C zeta in response to hyperosmotic stress. Mol Cell Biol, 2005. 25(23): p. 10543-55.
28. Martin, M.C., et al., Protein kinase A regulates caspase-9 activation by Apaf-1 downstream of cytochrome c. J Biol Chem, 2005. 280(15): p. 15449-55.
29. Cardone, M.H., et al., Regulation of cell death protease caspase-9 by phosphorylation. Science, 1998. 282(5392): p. 1318-21.
30. Raina, D., et al., c-Abl tyrosine kinase regulates caspase-9 autocleavage in the apoptotic response to DNA damage. J Biol Chem, 2005. 280(12): p. 11147-51.
31. Papworth, C., Bauer, J. C., Braman, J. and Wright, D. A. , Site-directed mutagenesis in one day with >80% efficiency. Strategies, 1996. 9(3): p. 3-4.
32. Spencer, D.M., et al., Functional analysis of Fas signaling in vivo using synthetic inducers of dimerization. Curr Biol, 1996. 6(7): p. 839-47.
33. Hsiao, E.C., et al., Constitutive Gs activation using a single-construct tetracycline-inducible expression system in embryonic stem cells and mice. Stem Cell Res Ther, 2011. 2(2): p. 11.
34. Waldner, C., et al., Double conditional human embryonic kidney cell line based on FLP and PhiC31 mediated transgene integration. BMC Res Notes, 2011. 4: p. 420.

### Example 3: Additional Sequences

SEQ ID NO: 74 Casp 9 (truncated) nucleotide sequence 
GGATTTGGTGATGTCGGTGCTCTTGAGAGTTTGAGGGGAAATGCAGATTTGGCTTACATCC
TGAGCATGGAGCCCTGTGGCCACTGCCTCATTATCAACAATGTGAACTTCTGCCGTGAGT
CCGGGCTCCGCACCCGCACTGGCTCCAACATCGACTGTGAGAAGTTGCGGCGTCGCTTC
TCCTCGCTGCATTTCATGGTGGAGGTGAAGGGCGACCTGACTGCCAAGAAAATGGTGCTG
GCTTTGCTGGAGCTGGCGCAGCAGGACCACGGTGCTCTGGACTGCTGCGTGGTGGTCAT
TCTCTCTCACGGCTGTCAGGCCAGCCACCTGCAGTTCCCAGGGGCTGTCTACGGCACAGA
TGGATGCCCTGTGTCGGTCGAGAAGATTGTGAACATCTTCAATGGGACCAGCTGCCCCAG
CCTGGGAGGGAAGCCCAAGCTCTTTTTCATCCAGGCCTGTGGTGGGGAGCAGAAAGACC
ATGGGTTTGAGGTGGCCTCCACTTCCCCTGAAGACGAGTCCCCTGGCAGTAACCCCGAGC
CAGATGCCACCCCGTTCCAGGAAGGTTTGAGGACCTTCGACCAGCTGGACGCCATATCTA
GTTTGCCCACACCCAGTGACATCTTTGTGTCCTACTCTACTTTCCCAGGTTTTGTTTCCTGG
AGGGACCCCAAGAGTGGCTCCTGGTACGTTGAGACCCTGGACGACATCTTTGAGCAGTG
GGCTCACTCTGAAGACCTGCAGTCCCTCCTGCTTAGGGTCGCTAATGCTGTTTCGGTGAA
AGGGATTTATAAACAGATGCCTGGTTGCTTTAATTTCCTCCGGAAAAACTTTTCTTTAAAA
CATCA
SEQ ID NO: 75, caspase-9 (truncated) amino acid sequence-CARD domain deleted 
G F G D V G A L E S L R G N A D L A Y I L S M E P C G H C L I I N N V
N F C R E S G L R T R T G S N I D C E K L R R R F S S L H F M V E V K
G D L T A K K M V L A L L E L A Q Q D H G A L D C C V V V I L S H G C
Q A S H L Q F P G A V Y G T D G C P V S V E K I V N I F N G T S C P S
L G G K P K L F F I Q A C G G E Q K D H G F E V A S T S P E D E S P
G S N P E P D A T P F Q E G L R T F D Q L D A I S S L P T P S D I F V
S Y S T F P G F V S W R D P K S G S W Y V E T L D D I F E Q W A H S
E D L Q S L L L R V A N A V S V K G I Y K Q M P G C F N F L R K K L F
F K T S
SEQ ID NO: 76, FKBPv36 (Fv1) nucleotide sequence 
GGCGTTCAAGTAGAAACAATCAGCCCAGGAGACGGAAGGACTTTCCCCAAACGAGGCCAA
ACATGCGTAGTTCATTATACTGGGATGCTCGAAGATGGAAAAAAGTAGATAGTAGTAGAG
ACCGAAACAAACCATTTAAATTTATGTTGGGAAAACAAGAAGTAATAAGGGGCTGGGAAGA
AGGTGTAGCACAAATGTCTGTTGGCCAGCGCGCAAAACTCACAATTTCTCCTGATTATGCT
TACGGAGCTACCGGCCACCCCGGCATCATACCCCCTCATGCCACACTGGTGTTTGACGTC
GAATTGCTCAAACTGGAA
SEQ ID NO: 77, FKBPv36 (Fv1) amino acid sequence 
GVQVETISPGDGRTFPKRGQTCVVHYTGMLEDGKKVDSSRDRNKPFKFMLGKQEVIRGWEE
GVAQMSVGQRAKLTISPDYAYGATGHPGIIPPHATLVFDVELLKLE
SEQ ID NO: 78, FKBPv36 (Fv2) nucleotide sequence 
GGaGTgCAgGTgGAgACgATtAGtCCtGGgGAtGGgAGaACcTTtCCaAAgCGcGGtCAgACcTGtG
TtGTcCAcTAcACcGGtATGCTgGAgGAcGGgAAgAAgGTgGActcTtcacGcGAtCGcAAtAAgCCtT
TcAAgTTcATGcTcGGcAAgCAgGAgGTgATccGGGGgTGGGAgGAgGGcGTgGCtCAgATGTCg
GTcGGgCAaCGaGCgAAgCTtACcATcTCaCCcGAcTAcGCgTAtGGgGCaACgGGgCAtCCgGG
aATtATcCCtCCcCAcGCtACgCTcGTaTTcGAtGTgGAgcTcttgAAgCTtGag
SEQ ID NO: 79, FKBPv36 (Fv2) amino acid sequence 
GVQVETISPGDGRTFPKRGQTCVVHYTGMLEDGKKVDSSRDRNKPFKFMLGKQEVIRGWEE
GVAQMSVGQRAKLTISPDYAYGATGHPGIIPPHATLVFDVELLKLE
SEQ ID NO: 80: T2A.codon optimized nucleotide sequence
   GAAGGCCGAGGGAGCCTGCTGACATGTGGCGATGTGGAGGAAAACCCAGGACCA
SEQ ID NO: 81: T2A.codon optimized amino acid sequence
   EGRGSLLTCGDVEENPGP
SEQ ID NO: 82, Thosea asigna virus-2A from capsid protein precursor nucleotide sequence
   GCCGAGGGCAGGGGAAGTCTTCTAACATGCGGGGACGTGGAGGAAAATCCCGGGCCC
SEQ ID NO: 83, Thosea asigna virus-2A from capsid protein precursor amino acid sequence
   A E G R G S L L T C G D V E E N P G P
SEQ ID NO: 84: FKBP amino acid sequence (with phenylalanine at position 36)

GVQVETISPGDGRTFPKRGQTCVVHYTGMLEDGKKFDSSRDRNKPFKFMLGKQEVIRGWEE
GVAQMSVGQRAKLTISPDYAYGATGHPGIIPPHATLVFDVELLKLE

Citation of the above patents, patent applications, publications and documents is not an admission that any of the foregoing is pertinent prior art, nor does it constitute any admission as to the contents or date of these publications or documents. Their citation is not an indication of a search for relevant disclosures. All statements regarding the date(s) or contents of the documents is based on available information and is not an admission as to their accuracy or correctness.

The technology illustratively described herein suitably may be practiced in the absence of any element(s) not specifically disclosed herein. Thus, for example, in each instance herein any of the terms "comprising," "consisting essentially of," and "consisting of" may be replaced with either of the other two terms. The terms and expressions which have been employed are used as terms of description and not of limitation.

The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%). Thus, it should be understood that although the present technology has been specifically disclosed by representative embodiments and optional features, modification and variation of the concepts herein disclosed may be resorted to by those skilled in the art, and such modifications and variations are considered within the scope of this technology.

Certain embodiments of the technology are set forth in the claim(s) that follow(s).

## Claims

1. A nucleic acid molecule comprising a CDR3-encoding polynucleotide, wherein the CDR3-encoding polynucleotide encodes the CDR3 region of a T cell receptor that specifically binds to the preferentially expressed antigen in melanoma (PRAME), wherein:
a) the CDR3-encoding polynucleotide comprises a first polynucleotide that encodes a first polypeptide comprising the VJ regions of a TCRα polypeptide, wherein the first polypeptide comprises the CDR3 region of the TCRα polypeptide, and wherein the CDR3 region comprises the amino acid sequence of SEQ ID NO: 25; and
b) the CDR3-encoding polynucleotide comprises a second polynucleotide that encodes a second polypeptide comprising the VDJ regions of a TCRβ polypeptide, wherein the second polypeptide comprises the CDR3 region of the TCRβ polypeptide, and wherein the CDR3 region comprises the amino acid sequence of SEQ ID NO: 28;
wherein the CDR3 region of the TCRα polypeptide and the CDR3 region of the TCRβ polypeptide together specifically bind to PRAME.

2. The nucleic acid molecule of claim 1, wherein the first polypeptide comprises the amino acid sequence of SEQ ID NO: 31 and the second polypeptide comprises the amino acid sequence of SEQ ID NO: 34.

3. The nucleic acid molecule of claim 1 or claim 2, wherein the nucleic acid molecule encodes a T cell receptor, and wherein the CDR3 regions of the T cell receptor specifically binds to a PRAME polypeptide comprising the amino acid sequence SLLQHLIGL.

4. The nucleic acid molecule of any one of claims 1 to 3, wherein
a) the first polynucleotide comprises the nucleotide sequence of SEQ ID NO:32 or SEQ ID NO:33; and
b) the second polynucleotide comprises the nucleotide sequence of SEQ ID NO:35 or SEQ ID NO: 36.

5. The nucleic acid molecule of any of claims 1 to 4, wherein the CDR3 region of the T cell receptor specifically binds to a peptide-MHC complex, wherein the MHC molecule is a MHC Class I HLA molecule and the peptide is a PRAME epitope.

6. The nucleic acid molecule of any one of claims 1 to 5, further comprising a promoter operatively linked to the CDR3-encoding polynucleotide.

7. The nucleic acid molecule of any one of claims 1 to 6, wherein the nucleic acid molecule further comprises a polynucleotide encoding a chimeric Caspase-9 polypeptide comprising a multimeric ligand binding region and a Caspase-9 polypeptide.

8. A plasmid vector or viral vector comprising a nucleic acid molecule of any of claims 1 to 7.

9. A modified cell transfected or transduced with a nucleic acid molecule of any of claims 1 to 7.

10. A modified cell transfected or transduced with a nucleic acid molecule encoding a T cell receptor that specifically binds to the preferentially expressed antigen in melanoma (PRAME), wherein the nucleic acid molecule comprises a first polynucleotide encoding a first polypeptide comprising the VJ regions of a TCRα polypeptide, wherein the first polypeptide comprises the amino acid sequence of SEQ ID NO: 25; and a second polynucleotide encodes a second polypeptide comprising the VDJ regions of a TCRβ polypeptide, wherein the second polypeptide comprises the amino acid sequence of SEQ ID NO: 28.

11. The modified cell of claim 10, wherein the first polypeptide comprises the amino acid sequence of SEQ ID NO: 31 and the second polypeptide comprises the amino acid sequence of SEQ ID NO: 34.

12. A pharmaceutical composition comprising a nucleic acid molecule of any one of claims 1 to 7, a vector of claim 8, or a modified cell of claim any one of claims 9 to 11 and a pharmaceutically acceptable carrier.

13. The pharmaceutical composition of claim 12 for use in enhancing an immune response in a subject diagnosed with a hyperproliferative disease or condition, wherein a therapeutically effective amount of the pharmaceutical composition is to be administered to the subject.

14. The pharmaceutical composition of claim 12 for use in stimulating a cell mediated immune response to a target cell population or tissue in a subject, wherein a therapeutically effective amount of the pharmaceutical composition is to be administered to the subject.

15. An in vitro method of expressing a T cell receptor that specifically binds to PRAME in a cell, comprising contacting a nucleic acid molecule of any one of claims 1 to 7 with a cell under conditions in which the nucleic acid is incorporated into the cell, whereby the cell expresses the T cell receptor from the incorporated nucleic acid.

## Patentansprüche

1. Nukleinsäuremolekül, umfassend ein CDR3- kodierendes Polynukleotid, wobei das CDR3- kodierende Polynukleotid für die CDR3-Region eines T-Zell-Rezeptors kodiert, der spezifisch an das bevorzugt exprimierte Antigen in Melanom (PRAME) bindet, wobei:
a) das CDR3- kodierende Polynukleotid ein erstes Polynukleotid umfasst, das für ein erstes Polypeptid kodiert, umfassend die VJ-Regionen eines TCRa-Polypeptids, wobei das erste Polypeptid die CDR3-Region des TCRa-Polypeptids umfasst, und wobei die CDR3-Region die Aminosäuresequenz von SEQ ID NO: 25 umfasst; und
b) das CDR3- kodierende Polynukleotid ein zweites Polynukleotid umfasst, das für ein zweites Polypeptid kodiert, umfassend die VDJ-Regionen eines TCRβ-Polypeptids, wobei das zweite Polypeptid die CDR3-Region des TCRβ-Polypeptids umfasst, und wobei die CDR3-Region die Aminosäuresequenz von SEQ ID NO: 28 umfasst;
wobei die CDR3-Region des TCRa-Polypeptids und die CDR3-Region des TCRβ-Polypeptids zusammen spezifisch an PRAME binden.

2. Nukleinsäuremolekül nach Anspruch 1, wobei das erste Polypeptid die Aminosäuresequenz von SEQ ID NO: 31 umfasst und das zweite Polypeptid die Aminosäuresequenz von SEQ ID NO: 34 umfasst.

3. Nukleinsäuremolekül nach Anspruch 1 oder Anspruch 2, wobei das Nukleinsäuremolekül für einen T-Zell-Rezeptor kodiert, und wobei die CDR3-Regionen des T-Zell-Rezeptors spezifisch an ein PRAME-Polypeptid bindet, umfassend die Aminosäuresequenz SLLQHLIGL.

4. Nukleinsäuremolekül nach einem der Ansprüche 1 bis 3, wobei
a) das erste Polynukleotid die Nukleotidsequenz von SEQ ID NO: 32 oder SEQ ID NO: 33 umfasst; und
b) das zweite Polynukleotid die Nukleotidsequenz von SEQ ID NO: 35 oder SEQ ID NO: 36 umfasst.

5. Nukleinsäuremolekül nach einem der Ansprüche 1 bis 4, wobei die CDR3-Region des T-Zell-Rezeptors spezifisch an einen Peptid-MHC-Komplex bindet, wobei das MHC-Molekül ein I-HLA-Molekül der Klasse MHC ist und das Peptid ein PRAME-Epitop ist.

6. Nukleinsäuremolekül nach einem der Ansprüche 1 bis 5, ferner umfassend einen Promotor, der funktionsfähig mit dem CDR3- kodierenden Polynukleotid verbunden ist.

7. Nukleinsäuremolekül nach einem der Ansprüche 1 bis 6, wobei das Nukleinsäuremolekül ferner ein Polynukleotid umfasst, das für ein chimäres Caspase-9-Polypeptid kodiert, umfassend eine multimere Ligandenbindungsregion und ein Caspase-9-Polypeptid.

8. Plasmidvektor oder viraler Vektor, umfassend ein Nukleinsäuremolekül nach einem der Ansprüche 1 bis 7.

9. Modifizierte Zelle, die mit einem Nukleinsäuremolekül nach einem der Ansprüche 1 bis 7 transfiziert oder transduziert ist.

10. Modifizierte Zelle, die mit einem Nukleinsäuremolekül transfiziert oder transduziert ist, das für einen T-Zell-Rezeptor kodiert, der spezifisch an das bevorzugt exprimierte Antigen in Melanom (PRAME) bindet, wobei das Nukleinsäuremolekül ein erstes Polynukleotid, das für ein erstes Polypeptid kodiert, umfassend die VJ-Regionen eines TCRa-Polypeptids, wobei das erste Polypeptid die Aminosäuresequenz von SEQ ID NO: 25 umfasst; und ein zweites Polynukleotid, das für ein zweites Polypeptid kodiert, umfassend die VDJ-Regionen eines TCRβ-Polypeptids, umfasst, wobei das zweite Polypeptid die Aminosäuresequenz von SEQ ID NO: 28 umfasst.

11. Modifizierte Zelle nach Anspruch 10, wobei das erste Polypeptid die Aminosäuresequenz von SEQ ID NO: 31 umfasst und das zweite Polypeptid die Aminosäuresequenz von SEQ ID NO: 34 umfasst.

12. Pharmazeutische Zusammensetzung, umfassend ein Nukleinsäuremolekül nach einem der Ansprüche 1 bis 7, einen Vektor nach Anspruch 8 oder eine modifizierte Zelle nach einem der Ansprüche 9 bis 11 und einen pharmazeutisch annehmbaren Träger.

13. Pharmazeutische Zusammensetzung nach Anspruch 12 zur Verwendung beim Verstärken einer Immunantwort in einem Subjekt, bei dem eine hyperproliferative Krankheit oder ein hyperproliferativer Zustand diagnostiziert wird, wobei eine therapeutisch wirksame Menge der pharmazeutischen Zusammensetzung an das Subjekt zu verabreichen ist.

14. Pharmazeutische Zusammensetzung nach Anspruch 12 zur Verwendung beim Stimulieren einer zellvermittelten Immunantwort auf eine Zielzellpopulation oder ein Zielgewebe in einem Subjekt, wobei eine therapeutisch wirksame Menge der pharmazeutischen Zusammensetzung an das Subjekt zu verabreichen ist.

15. In vitro-Verfahren zum Exprimieren eines T-Zell-Rezeptors, der spezifisch an PRAME in einer Zelle bindet, umfassend ein Inkontaktbringen eines Nukleinsäuremoleküls nach einem der Ansprüche 1 bis 7 mit einer Zelle unter Bedingungen, unter denen die Nukleinsäure in die Zelle integriert wird, wodurch die Zelle den T-Zell-Rezeptor aus der integrierten Nukleinsäure exprimiert.

## Revendications

1. Molécule d'acide nucléique comprenant un polynucléotide codant CDR3, ledit polynucléotide codant CDR3 codant la région CDR3 d'un récepteur de lymphocytes T qui se lie spécifiquement à l'antigène exprimé préférentiellement dans le mélanome (PRAME) :
a) ledit polynucléotide codant CDR3 comprenant un premier polynucléotide qui code un premier polypeptide comprenant les régions VJ d'un polypeptide TCRα, ledit premier polypeptide comprenant la région CDR3 du polypeptide TCRα, et ladite région CDR3 comprenant la séquence d'acides aminés de SEQ ID n° : 25 ; et
b) ledit polynucléotide codant CDR3 comprenant un second polynucléotide qui code un second polypeptide comprenant les régions VDJ d'un polypeptide TCRβ, ledit second polypeptide comprenant la région CDR3 du polypeptide TCRβ, et ladite région CDR3 comprenant la séquence d'acides aminés de SEQ ID n° : 28 ;
ladite région CDR3 du polypeptide TCRα et ladite région CDR3 du polypeptide TCRβ se liant ensemble spécifiquement à PRAME

2. Molécule d'acide nucléique selon la revendication 1, ledit premier polypeptide comprenant la séquence d'acides aminés de SEQ ID n° : 31 et ledit second polypeptide comprenant la séquence d'acides aminés de SEQ ID n° : 34.

3. Molécule d'acide nucléique selon la revendication 1 ou la revendication 2, ladite molécule d'acide nucléique codant un récepteur de lymphocytes T, et lesdites régions CDR3 du récepteur de lymphocytes T se liant spécifiquement à un polypeptide de PRAME comprenant la séquence d'acides aminés SLLQHLIGL.

4. Molécule d'acide nucléique selon l'une quelconque des revendications 1 à 3,
a) ledit premier polynucléotide comprenant la séquence nucléotidique de SEQ ID n° : 32 ou de SEQ ID n° : 33 ; et
b) ledit second polynucléotide comprenant la séquence nucléotidique de SEQ ID n° : 35 ou de SEQ ID n° : 36.

5. Molécule d'acide nucléique selon l'une quelconque des revendications 1 à 4, ladite région CDR3 du récepteur de lymphocytes T se liant spécifiquement à un complexe peptide-MHC, ladite molécule de MHC étant une molécule HLA de classe I de MHC et ledit peptide étant un épitope de PRAME

6. Molécule d'acide nucléique selon l'une quelconque des revendications 1 à 5, comprenant en outre un promoteur lié de manière fonctionnelle au polynucléotide codant CDR3.

7. Molécule d'acide nucléique selon l'une quelconque des revendications 1 à 6, ladite molécule d'acide nucléique comprenant en outre un polynucléotide codant un polypeptide de caspase-9 chimère comprenant une région de liaison de ligand multimère et un polypeptide de caspase-9.

8. Vecteur plasmidique ou vecteur viral comprenant une molécule d'acide nucléique selon l'une quelconque des revendications 1 à 7.

9. Cellule modifiée transfectée ou transduite avec une molécule d'acide nucléique selon l'une quelconque des revendications 1 à 7.

10. Cellule modifiée transfectée ou transduite avec une molécule d'acide nucléique codant un récepteur de lymphocytes T qui se lie spécifiquement à l'antigène exprimé préférentiellement dans le mélanome (PRAME), ladite molécule d'acide nucléique comprenant un premier polynucléotide codant un premier polypeptide comprenant les régions VJ d'un polypeptide TCRα, ledit premier polypeptide comprenant la séquence d'acides aminés de SEQ ID n°: 25 ; et un second polynucléotide codant un second polypeptide comprenant les régions VDJ d'un polypeptide TCRβ, ledit second polypeptide comprenant la séquence d'acides aminés de SEQ ID n° : 28.

11. Cellule modifiée selon la revendication 10, ledit premier polypeptide comprenant la séquence d'acides aminés de SEQ ID n° : 31 et ledit second polypeptide comprenant la séquence d'acides aminés de SEQ ID n° : 34.

12. Composition pharmaceutique comprenant une molécule d'acide nucléique selon l'une quelconque des revendications 1 à 7, un vecteur selon la revendication 8, ou une cellule modifiée selon l'une quelconque des revendications 9 à 11 et un véhicule pharmaceutiquement acceptable.

13. Composition pharmaceutique selon la revendication 12 pour une utilisation dans l'amélioration d'une réponse immunitaire chez un sujet diagnostiqué avec une maladie ou un état hyperprolifératif, une quantité thérapeutiquement efficace de la composition pharmaceutique devant être administrée au sujet.

14. Composition pharmaceutique selon la revendication 12 pour une utilisation dans la stimulation d'une réponse immunitaire à médiation cellulaire auprès d'une population de cellules ou d'un tissu cible chez un sujet, une quantité thérapeutiquement efficace de la composition pharmaceutique devant être administrée au sujet.

15. Procédé in vitro d'expression d'un récepteur de lymphocytes T qui se lie spécifiquement à PRAME dans une cellule, comprenant la mise en contact d'une molécule d'acide nucléique selon l'une quelconque des revendications 1 à 7 avec une cellule dans des conditions dans lesquelles l'acide nucléique est incorporé dans la cellule, grâce à quoi la cellule exprime le récepteur de lymphocytes T à partir de l'acide nucléique incorporé.
